# EUROPEAN PATENT APPLICATION

(11) **EP 3 858 986 A1**
(43) Date of publication of application: **04.08.2021**
(21) Application number: 20155122.3
(22) Date of filing: 03.02.2020
(51) Int. Cl.: C12N 9/02, C12P 7/22

(54) **P450 BM3 MONOOXYGENASE VARIANTS FOR C19-HYDROXYLATION OF STEROIDS**

(71) Applicant: Bayer Aktiengesellschaft, 51373 Leverkusen (DE); Bayer Pharma Aktiengesellschaft, 13353 Berlin (DE)
(72) Inventor: The designation of the inventor has not yet been filed
(74) Representative: BIP Patents

(57) **Abstract**

The present invention relates to novel recombinant *Bacillus megaterium* cytochrome P450-monooxygenase (P450-BM3) variants for the C19 hydroxylation of steroids and derivatives thereof. In particular, the present invention also relates to methods and processes using P450-BM3 variants for the production of estrone and estradiol. The invention further relates to nucleotide sequences, constructs and vectors for the expression of these P450-BM3 variants

SEQUENCE LISTING

<110>  Bayer Aktiengesellschaft
       Bayer Pharma Aktiengesellschaft

<120>  P450 BM3 MONOOXYGENASE VARIANTS FOR C19-HYDROXYLATION OF STEROIDS

<130>  BHC201002

<160>  116

<170>  PatentIn version 3.5

<210>  1
<211>  1048
<212>  PRT
<213>  Artificial Sequence

<220>
<223>  BM3 WT

<400>  1

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Val Arg Asp
65                  70                  75                  80

Phe Ala Gly Asp Gly Leu Phe Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly

915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  2
<211>  1048
<212>  PRT
<213>  Artificial Sequence

<220>
<223>  BM3-254 (V78Y, A82E, F87A)

<400>  2

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys

                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  3
<211>  1048
<212>  PRT
<213>  Artificial Sequence

<220>

<223>  BM3-263 (V78M, A82E, F87A)

<400>  3

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr

740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  4
<211>  1048
<212>  PRT
<213>  Artificial Sequence

<220>
<223>  BM3-268 (V78I, A82E, F87A)

<400>  4

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr

145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
        660                 665                 670

Gln Gln Pro Gly Ser Ala Arg Ser Thr Arg His Leu Glu Ile Glu Leu
    675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
        740                 745                 750

Val Glu Leu Gln Asp Pro Val Thr Arg Thr Gln Leu Arg Ala Met Ala
    755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
        820                 825                 830

Ser Ser Pro Arg Val Asp Glu Lys Gln Ala Ser Ile Thr Val Ser Val
    835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
        900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  5
<211>  1048
<212>  PRT
<213>  Artificial Sequence

<220>
<223>  BM3 variant (V78L, A82E, F87A)

<400>  5

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr

                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  6
<211>  1048
<212>  PRT

<213>  Artificial Sequence

<220>
<223>  BM3 variant (V78Y, A82P, F87A)

<400>  6

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln

            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 7
<211> 1048
<212> PRT
<213> Artificial Sequence

<220>
<223> BM3 variant (V78M, A82P, F87A)

<400> 7

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys

385                 390                 395                 400

Ile Gly Gln Gln Phe Ala Leu His Glu Ala Thr Leu Val Leu Gly Met
            405                 410                 415

Met Leu Lys His Phe Asp Phe Glu Asp His Thr Asn Tyr Glu Leu Asp
        420                 425                 430

Ile Lys Glu Thr Leu Thr Leu Lys Pro Glu Gly Phe Val Val Lys Ala
    435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
            485                 490                 495

Ala Arg Asp Leu Ala Asp Ile Ala Met Ser Lys Gly Phe Ala Pro Gln
        500                 505                 510

Val Ala Thr Leu Asp Ser His Ala Gly Asn Leu Pro Arg Glu Gly Ala
    515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
            565                 570                 575

Thr Tyr Gln Lys Val Pro Ala Phe Ile Asp Glu Thr Leu Ala Ala Lys
        580                 585                 590

Gly Ala Glu Asn Ile Ala Asp Arg Gly Glu Ala Asp Ala Ser Asp Asp
    595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 8
<211> 1048
<212> PRT
<213> Artificial Sequence

<220>
<223> BM3 variant (V78I, A82P, F87A)

<400> 8

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu

                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210>  9
<211>  1048
<212>  PRT
<213>  Artificial Sequence

<220>
<223>  BM3 variant (V78L, A82P, F87A)

<400>  9

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys

210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  10
<211>  1048
<212>  PRT
<213>  Artificial Sequence

<220>
<223>  BM3 variant (V78Y, A82Q, F87A)

<400>  10

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser

625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  11
<211>  1048
<212>  PRT
<213>  Artificial Sequence

<220>
<223>  BM3 variant (V78M, A82Q, F87A)

<400>  11

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val

35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly

1040                    1045


<210>  12
<211>  1048
<212>  PRT
<213>  Artificial Sequence

<220>
<223>  BM3 variant (V78I, A82Q, F87A)

<400>  12

Ala Ile Lys Glu Met Pro Gln Pro Lys Thr Phe Gly Glu Leu Lys Asn
1               5                   10                  15


Leu Pro Leu Leu Asn Thr Asp Lys Pro Val Gln Ala Leu Met Lys Ile
            20                  25                  30


Ala Asp Glu Leu Gly Glu Ile Phe Lys Phe Glu Ala Pro Gly Arg Val
        35                  40                  45


Thr Arg Tyr Leu Ser Ser Gln Arg Leu Ile Lys Glu Ala Cys Asp Glu
    50                  55                  60


Ser Arg Phe Asp Lys Asn Leu Ser Gln Ala Leu Lys Phe Ile Arg Asp
65                  70                  75                  80


Phe Gln Gly Asp Gly Leu Ala Thr Ser Trp Thr His Glu Lys Asn Trp
                85                  90                  95


Lys Lys Ala His Asn Ile Leu Leu Pro Ser Phe Ser Gln Gln Ala Met
            100                 105                 110


Lys Gly Tyr His Ala Met Met Val Asp Ile Ala Val Gln Leu Val Gln
        115                 120                 125


Lys Trp Glu Arg Leu Asn Ala Asp Glu His Ile Glu Val Pro Glu Asp
    130                 135                 140


Met Thr Arg Leu Thr Leu Asp Thr Ile Gly Leu Cys Gly Phe Asn Tyr
145                 150                 155                 160


Arg Phe Asn Ser Phe Tyr Arg Asp Gln Pro His Pro Phe Ile Thr Ser
                165                 170                 175


Met Val Arg Ala Leu Asp Glu Ala Met Asn Lys Leu Gln Arg Ala Asn
            180                 185                 190


Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu

450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  13
<211>  1048
<212>  PRT
<213>  Artificial Sequence

<220>
<223>  BM3 variant (V78L, A82Q, F87A)

<400>  13

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe

865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  14
<211>  1048
<212>  PRT
<213>  Artificial Sequence

<220>
<223>  BM3 variant (V78F, A82E, F87A, M177Y, A184Y)

<400>  14

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln

        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg Tyr Ala Lys Asp Val Trp Ala Gly
    1040                1045

<210> 15
<211> 1048
<212> PRT
<213> Artificial Sequence

<220>
<223> BM3 variant (V78L, A82E, F87A, M177Y, A184Y)

<400> 15

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro

690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  16
<211>  1048
<212>  PRT
<213>  Artificial Sequence

<220>
<223>  BM3 variant (V78I, A82E, F87A, M177Y, A184Y)

<400>  16

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met

            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  17
<211>  1048
<212>  PRT
<213>  Artificial Sequence

<220>
<223>  BM3 variant (V78M, A82E, F87A, M177Y, A184Y)

<400>  17

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
        20                  25                  30

Ala Asp Glu Leu Gly Glu Ile Phe Lys Phe Glu Ala Pro Gly Arg Val
    35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala

515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 18
<211> 1048
<212> PRT
<213> Artificial Sequence

<220>
<223> BM3 variant (V78Y, A82E, F87A, M177Y, A184Y)

<400> 18

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
        180                 185                 190

Pro Asp Asp Pro Ala Tyr Asp Glu Asn Lys Arg Gln Phe Gln Glu Asp
    195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
        260                 265                 270

Leu Ser Phe Ala Leu Tyr Phe Leu Val Lys Asn Pro His Val Leu Gln
    275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
        340                 345                 350

Leu Met Val Leu Ile Pro Gln Leu His Arg Asp Lys Thr Ile Trp Gly
    355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
        420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu

930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  19
<211>  1048
<212>  PRT
<213>  Artificial Sequence

<220>
<223>  BM3 variant (V78F, A82P, F87A, M177Y, A184Y)

<400>  19

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu

340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  20
<211>  1048
<212>  PRT
<213>  Artificial Sequence

<220>
<223>  BM3 variant (V78L, A82P, F87A, M177Y, A184Y)

<400>  20

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala

        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 21
<211> 1048
<212> PRT
<213> Artificial Sequence

<220>
<223> BM3 variant (V78I, A82P, F87A, M177Y, A184Y)

<400> 21

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser

                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 22
<211> 1048
<212> PRT
<213> Artificial Sequence

<220>
<223> BM3 variant (V78M, A82P, F87A, M177Y, A184Y)

<400> 22

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys

            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  23
<211>  1048
<212>  PRT
<213>  Artificial Sequence

<220>
<223>  BM3 variant (V78Y, A82P, F87A, M177Y, A184Y)

<400>  23

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala

995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  24
<211>  1048
<212>  PRT
<213>  Artificial Sequence

<220>
<223>  BM3 variant (V78F, A82Q, F87A, M177Y, A184Y)

<400>  24

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met

                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
        660                 665                 670

Gln Gln Pro Gly Ser Ala Arg Ser Thr Arg His Leu Glu Ile Glu Leu
    675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  25
<211>  1048
<212>  PRT
<213>  Artificial Sequence

<220>
<223>  BM3 variant (V78L, A82Q, F87A, M177Y, A184Y)

<400>  25

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser

820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  26
<211>  1048
<212>  PRT
<213>  Artificial Sequence

<220>
<223>  BM3 variant (V78I, A82Q, F87A, M177Y, A184Y)

<400>  26

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly

225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
                740                 745                 750

Val Glu Leu Gln Asp Pro Val Thr Arg Thr Gln Leu Arg Ala Met Ala
            755                 760                 765

Ala Lys Thr Val Cys Pro Pro His Lys Val Glu Leu Glu Ala Leu Leu
        770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
                820                 825                 830

Ser Ser Pro Arg Val Asp Glu Lys Gln Ala Ser Ile Thr Val Ser Val
            835                 840                 845

Val Ser Gly Glu Ala Trp Ser Gly Tyr Gly Glu Tyr Lys Gly Ile Ala
        850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
                900                 905                 910

Phe Val Gln Ala Arg Lys Gln Leu Lys Glu Gln Gly Gln Ser Leu Gly
            915                 920                 925

Glu Ala His Leu Tyr Phe Gly Cys Arg Ser Pro His Glu Asp Tyr Leu
        930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
                980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 27
<211> 1048
<212> PRT
<213> Artificial Sequence

<220>
<223> BM3 variant (V78M, A82Q, F87A, M177Y, A184Y)

<400> 27

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala

                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  28
<211>  1048
<212>  PRT
<213>  Artificial Sequence

<220>
<223>  BM3 variant (V78Y, A82Q, F87A, M177Y, A184Y)

<400>  28

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu

50                  55                  60

Ser Arg Phe Asp Lys Asn Leu Ser Gln Ala Leu Lys Phe Tyr Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
                820                 825                 830

Ser Ser Pro Arg Val Asp Glu Lys Gln Ala Ser Ile Thr Val Ser Val
            835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
                900                 905                 910

Phe Val Gln Ala Arg Lys Gln Leu Lys Glu Gln Gly Gln Ser Leu Gly
            915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
                980                 985                 990

Gly Ala His Phe Tyr Ile Cys Gly  Asp Gly Ser Gln Met  Ala Pro Ala
            995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  29

<211>  1048
<212>  PRT
<213>  Artificial Sequence

<220>
<223>  BM3 variant (V78F, A82E, F87A, M177Y)

<400>  29

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr

465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  30
<211>  1048
<212>  PRT
<213>  Artificial Sequence

<220>
<223>  BM3 variant (V78L, A82E, F87A, M177Y)

<400>  30

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr

                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 31
<211> 1048
<212> PRT
<213> Artificial Sequence

<220>
<223> BM3 variant (V78I, A82E, F87A, M177Y)

<400> 31

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser

290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  32
<211>  1048
<212>  PRT
<213>  Artificial Sequence

<220>
<223>  BM3 variant (V78M, A82E, F87A, M177Y)

<400>  32

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu

705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  33
<211>  1048
<212>  PRT
<213>  Artificial Sequence

<220>
<223>  BM3 variant (V78Y, A82E, F87A, M177Y)

<400>  33

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln

        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  34
<211>  1048
<212>  PRT
<213>  Artificial Sequence

<220>
<223>  BM3 variant (V78F, A82P, F87A, M177Y)

<400>  34

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45
Thr Arg Tyr Leu Ser Ser Gln Arg Leu Ile Lys Glu Ala Cys Asp Glu
    50                  55                  60
Ser Arg Phe Asp Lys Asn Leu Ser Gln Ala Leu Lys Phe Phe Arg Asp
65                  70                  75                  80
Phe Pro Gly Asp Gly Leu Ala Thr Ser Trp Thr His Glu Lys Asn Trp
                85                  90                  95
Lys Lys Ala His Asn Ile Leu Leu Pro Ser Phe Ser Gln Gln Ala Met
            100                 105                 110
Lys Gly Tyr His Ala Met Met Val Asp Ile Ala Val Gln Leu Val Gln
        115                 120                 125
Lys Trp Glu Arg Leu Asn Ala Asp Glu His Ile Glu Val Pro Glu Asp
    130                 135                 140
Met Thr Arg Leu Thr Leu Asp Thr Ile Gly Leu Cys Gly Phe Asn Tyr
145                 150                 155                 160
Arg Phe Asn Ser Phe Tyr Arg Asp Gln Pro His Pro Phe Ile Thr Ser
                165                 170                 175
Tyr Val Arg Ala Leu Asp Glu Ala Met Asn Lys Leu Gln Arg Ala Asn
            180                 185                 190
Pro Asp Asp Pro Ala Tyr Asp Glu Asn Lys Arg Gln Phe Gln Glu Asp
        195                 200                 205
Ile Lys Val Met Asn Asp Leu Val Asp Lys Ile Ile Ala Asp Arg Lys
    210                 215                 220
Ala Ser Gly Glu Gln Ser Asp Asp Leu Leu Thr His Met Leu Asn Gly
225                 230                 235                 240
Lys Asp Pro Glu Thr Gly Glu Pro Leu Asp Asp Glu Asn Ile Arg Tyr
                245                 250                 255
Gln Ile Ile Thr Phe Leu Ile Ala Gly His Glu Thr Thr Ser Gly Leu
            260                 265                 270
Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala

530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg Tyr Ala Lys Asp Val Trp Ala Gly
    1040                1045

<210> 35
<211> 1048
<212> PRT
<213> Artificial Sequence

<220>
<223> BM3 variant (V78L, A82P, F87A, M177Y)

<400> 35

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu

945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  36
<211>  1048
<212>  PRT
<213>  Artificial Sequence

<220>
<223>  BM3 variant (V78I, A82P, F87A, M177Y)

<400>  36

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly

        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  37
<211>  1048
<212>  PRT
<213>  Artificial Sequence

<220>
<223>  BM3 variant (V78M, A82P, F87A, M177Y)

<400>  37

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu

770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 38
<211> 1048
<212> PRT
<213> Artificial Sequence

<220>
<223> BM3 variant (V78Y, A82P, F87A, M177Y)

<400> 38

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn

            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  39
<211>  1048
<212>  PRT
<213>  Artificial Sequence

<220>
<223>  BM3 variant (V78F, A82Q, F87A, M177Y)

<400>  39

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp

        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  40
<211>  1048
<212>  PRT
<213>  Artificial Sequence

<220>
<223>  BM3 variant (V78L, A82Q, F87A, M177Y)

<400>  40

Ala Ile Lys Glu Met Pro Gln Pro Lys Thr Phe Gly Glu Leu Lys Asn

1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
        260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val

1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  41
<211>  1048
<212>  PRT
<213>  Artificial Sequence

<220>
<223>  BM3 variant (V78I, A82Q, F87A, M177Y)

<400>  41

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
        180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
        260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
        340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp

            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  42
<211>  1048
<212>  PRT
<213>  Artificial Sequence

<220>
<223>  BM3 variant (V78M, A82Q, F87A, M177Y)

<400>  42

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
        340                 345                 350

Leu Met Val Leu Ile Pro Gln Leu His Arg Asp Lys Thr Ile Trp Gly
    355                 360                 365

Asp Asp Val Glu Glu Phe Arg Pro Glu Arg Phe Glu Asn Pro Ser Ala
370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val

835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  43
<211>  1048
<212>  PRT
<213>  Artificial Sequence

<220>
<223>  BM3 variant (V78Y, A82Q, F87A, M177Y)

<400>  43

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Tyr Val Arg Ala Leu Asp Glu Ala Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr

245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
        500                 505                 510

Val Ala Thr Leu Asp Ser His Ala Gly Asn Leu Pro Arg Glu Gly Ala
    515                 520                 525

Val Leu Ile Val Thr Ala Ser Tyr Asn Gly His Pro Pro Asp Asn Ala
530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 44
<211> 1048
<212> PRT
<213> Artificial Sequence

<220>
<223> BM3 variant (V78F, A82E, F87A, M184Y)

<400> 44

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu

            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  45
<211>  1048
<212>  PRT
<213>  Artificial Sequence

<220>
<223>  BM3 variant (V78L, A82E, F87A, M184Y)

<400>  45

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp

65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
        580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  46
<211>  1048
<212>  PRT
<213>  Artificial Sequence

<220>
<223>  BM3 variant (V78I, A82E, F87A, M184Y)

<400>  46

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr

                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
        740                 745                 750

Val Glu Leu Gln Asp Pro Val Thr Arg Thr Gln Leu Arg Ala Met Ala
    755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
        820                 825                 830

Ser Ser Pro Arg Val Asp Glu Lys Gln Ala Ser Ile Thr Val Ser Val
    835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
        900                 905                 910

Phe Val Gln Ala Arg Lys Gln Leu Lys Glu Gln Gly Gln Ser Leu Gly
    915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
        980                 985                 990

Gly Ala His Phe Tyr Ile Cys Gly  Asp Gly Ser Gln Met  Ala Pro Ala
      995                  1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  47
<211>  1048
<212>  PRT
<213>  Artificial Sequence

<220>
<223>  BM3 variant (V78M, A82E, F87A, M184Y)

<400>  47

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly

900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  48
<211>  1048
<212>  PRT
<213>  Artificial Sequence

<220>
<223>  BM3 variant (V78Y, A82E, F87A, M184Y)

<400>  48

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Glu Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu

305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
                820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
                900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
                980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  49
<211>  1048

<212>  PRT
<213>  Artificial Sequence

<220>
<223>  BM3 variant (V78F, A82P, F87A, M184Y)

<400>  49

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala

725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  50
<211>  1048
<212>  PRT
<213>  Artificial Sequence

<220>
<223>  BM3 variant (V78L, A82P, F87A, M184Y)

<400>  50

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp

130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly Asp Gly Ser Gln Met Ala Pro Ala
        995                 1000                1005

Val Glu Ala Thr Leu Met Lys Ser Tyr Ala Asp Val His Gln Val
    1010                1015                1020

Ser Glu Ala Asp Ala Arg Leu Trp Leu Gln Gln Leu Glu Glu Lys
    1025                1030                1035

Gly Arg Tyr Ala Lys Asp Val Trp Ala Gly
    1040                1045

<210> 51
<211> 1048
<212> PRT
<213> Artificial Sequence

<220>
<223> BM3 variant (V78I, A82P, F87A, M184Y)

<400> 51

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys

545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  52
<211>  1048
<212>  PRT
<213>  Artificial Sequence

<220>
<223>  BM3 variant (V78M, A82P, F87A, M184Y)

<400>  52
   Ala Ile Lys Glu Met Pro Gln Pro Lys Thr Phe Gly Glu Leu Lys Asn
   1               5                   10                  15
   Leu Pro Leu Leu Asn Thr Asp Lys Pro Val Gln Ala Leu Met Lys Ile
               20                  25                  30
   Ala Asp Glu Leu Gly Glu Ile Phe Lys Phe Glu Ala Pro Gly Arg Val
           35                  40                  45
   Thr Arg Tyr Leu Ser Ser Gln Arg Leu Ile Lys Glu Ala Cys Asp Glu
       50                  55                  60
   Ser Arg Phe Asp Lys Asn Leu Ser Gln Ala Leu Lys Phe Met Arg Asp
   65                  70                  75                  80
   Phe Pro Gly Asp Gly Leu Ala Thr Ser Trp Thr His Glu Lys Asn Trp
                   85                  90                  95
   Lys Lys Ala His Asn Ile Leu Leu Pro Ser Phe Ser Gln Gln Ala Met
               100                 105                 110
   Lys Gly Tyr His Ala Met Met Val Asp Ile Ala Val Gln Leu Val Gln
           115                 120                 125
   Lys Trp Glu Arg Leu Asn Ala Asp Glu His Ile Glu Val Pro Glu Asp
       130                 135                 140
   Met Thr Arg Leu Thr Leu Asp Thr Ile Gly Leu Cys Gly Phe Asn Tyr
   145                 150                 155                 160
   Arg Phe Asn Ser Phe Tyr Arg Asp Gln Pro His Pro Phe Ile Thr Ser
                   165                 170                 175
   Met Val Arg Ala Leu Asp Glu Tyr Met Asn Lys Leu Gln Arg Ala Asn
               180                 185                 190
   Pro Asp Asp Pro Ala Tyr Asp Glu Asn Lys Arg Gln Phe Gln Glu Asp
           195                 200                 205
   Ile Lys Val Met Asn Asp Leu Val Asp Lys Ile Ile Ala Asp Arg Lys
       210                 215                 220
   Ala Ser Gly Glu Gln Ser Asp Asp Leu Leu Thr His Met Leu Asn Gly
   225                 230                 235                 240
   Lys Asp Pro Glu Thr Gly Glu Pro Leu Asp Asp Glu Asn Ile Arg Tyr
                   245                 250                 255
   Gln Ile Ile Thr Phe Leu Ile Ala Gly His Glu Thr Thr Ser Gly Leu
               260                 265                 270
   Leu Ser Phe Ala Leu Tyr Phe Leu Val Lys Asn Pro His Val Leu Gln
           275                 280                 285
   Lys Ala Ala Glu Glu Ala Ala Arg Val Leu Val Asp Pro Val Pro Ser
       290                 295                 300
   Tyr Lys Gln Val Lys Gln Leu Lys Tyr Val Gly Met Val Leu Asn Glu
   305                 310                 315                 320
   Ala Leu Arg Leu Trp Pro Thr Ala Pro Ala Phe Ser Leu Tyr Ala Lys
                   325                 330                 335
   Glu Asp Thr Val Leu Gly Gly Glu Tyr Pro Leu Glu Lys Gly Asp Glu
               340                 345                 350
   Leu Met Val Leu Ile Pro Gln Leu His Arg Asp Lys Thr Ile Trp Gly
           355                 360                 365
   Asp Asp Val Glu Glu Phe Arg Pro Glu Arg Phe Glu Asn Pro Ser Ala
       370                 375                 380
   Ile Pro Gln His Ala Phe Lys Pro Phe Gly Asn Gly Gln Arg Ala Cys
   385                 390                 395                 400
   Ile Gly Gln Gln Phe Ala Leu His Glu Ala Thr Leu Val Leu Gly Met
                   405                 410                 415
   Met Leu Lys His Phe Asp Phe Glu Asp His Thr Asn Tyr Glu Leu Asp
               420                 425                 430
   Ile Lys Glu Thr Leu Thr Leu Lys Pro Glu Gly Phe Val Val Lys Ala
           435                 440                 445
   Lys Ser Lys Lys Ile Pro Leu Gly Gly Ile Pro Ser Pro Ser Thr Glu
       450                 455                 460
   Gln Ser Ala Lys Lys Val Arg Lys Lys Ala Glu Asn Ala His Asn Thr
   465                 470                 475                 480
   Pro Leu Leu Val Leu Tyr Gly Ser Asn Met Gly Thr Ala Glu Gly Thr
                   485                 490                 495
   Ala Arg Asp Leu Ala Asp Ile Ala Met Ser Lys Gly Phe Ala Pro Gln
               500                 505                 510
   Val Ala Thr Leu Asp Ser His Ala Gly Asn Leu Pro Arg Glu Gly Ala
           515                 520                 525
   Val Leu Ile Val Thr Ala Ser Tyr Asn Gly His Pro Pro Asp Asn Ala
       530                 535                 540
   Lys Gln Phe Val Asp Trp Leu Asp Gln Ala Ser Ala Asp Glu Val Lys
   545                 550                 555                 560
   Gly Val Arg Tyr Ser Val Phe Gly Cys Gly Asp Lys Asn Trp Ala Thr
                   565                 570                 575
   Thr Tyr Gln Lys Val Pro Ala Phe Ile Asp Glu Thr Leu Ala Ala Lys
               580                 585                 590
   Gly Ala Glu Asn Ile Ala Asp Arg Gly Glu Ala Asp Ala Ser Asp Asp
           595                 600                 605
   Phe Glu Gly Thr Tyr Glu Glu Trp Arg Glu His Met Trp Ser Asp Val
       610                 615                 620
   Ala Ala Tyr Phe Asn Leu Asp Ile Glu Asn Ser Glu Asp Asn Lys Ser
   625                 630                 635                 640
   Thr Leu Ser Leu Gln Phe Val Asp Ser Ala Ala Asp Met Pro Leu Ala
                   645                 650                 655
   Lys Met His Gly Ala Phe Ser Thr Asn Val Val Ala Ser Lys Glu Leu
               660                 665                 670
   Gln Gln Pro Gly Ser Ala Arg Ser Thr Arg His Leu Glu Ile Glu Leu
           675                 680                 685
   Pro Lys Glu Ala Ser Tyr Gln Glu Gly Asp His Leu Gly Val Ile Pro
       690                 695                 700
   Arg Asn Tyr Glu Gly Ile Val Asn Arg Val Thr Ala Arg Phe Gly Leu
   705                 710                 715                 720
   Asp Ala Ser Gln Gln Ile Arg Leu Glu Ala Glu Glu Glu Lys Leu Ala
                   725                 730                 735
   His Leu Pro Leu Ala Lys Thr Val Ser Val Glu Glu Leu Leu Gln Tyr
               740                 745                 750
   Val Glu Leu Gln Asp Pro Val Thr Arg Thr Gln Leu Arg Ala Met Ala
           755                 760                 765
   Ala Lys Thr Val Cys Pro Pro His Lys Val Glu Leu Glu Ala Leu Leu
       770                 775                 780
   Glu Lys Gln Ala Tyr Lys Glu Gln Val Leu Ala Lys Arg Leu Thr Met
   785                 790                 795                 800
   Leu Glu Leu Leu Glu Lys Tyr Pro Ala Cys Glu Met Lys Phe Ser Glu
                   805                 810                 815
   Phe Ile Ala Leu Leu Pro Ser Ile Arg Pro Arg Tyr Tyr Ser Ile Ser
               820                 825                 830
   Ser Ser Pro Arg Val Asp Glu Lys Gln Ala Ser Ile Thr Val Ser Val
           835                 840                 845
   Val Ser Gly Glu Ala Trp Ser Gly Tyr Gly Glu Tyr Lys Gly Ile Ala
       850                 855                 860
   Ser Asn Tyr Leu Ala Glu Leu Gln Glu Gly Asp Thr Ile Thr Cys Phe
   865                 870                 875                 880
   Ile Ser Thr Pro Gln Ser Glu Phe Thr Leu Pro Lys Asp Pro Glu Thr
                   885                 890                 895
   Pro Leu Ile Met Val Gly Pro Gly Thr Gly Val Ala Pro Phe Arg Gly
               900                 905                 910
   Phe Val Gln Ala Arg Lys Gln Leu Lys Glu Gln Gly Gln Ser Leu Gly
           915                 920                 925
   Glu Ala His Leu Tyr Phe Gly Cys Arg Ser Pro His Glu Asp Tyr Leu
       930                 935                 940
   Tyr Gln Glu Glu Leu Glu Asn Ala Gln Ser Glu Gly Ile Ile Thr Leu
   945                 950                 955                 960
   His Thr Ala Phe Ser Arg Met Pro Asn Gln Pro Lys Thr Tyr Val Gln
   965 970 975
   His Val Met Glu Gln Asp Gly Lys Lys Leu Ile Glu Leu Leu Asp Gln
   980 985 990
   Gly Ala His Phe Tyr Ile Cys Gly Asp Gly Ser Gln Met Ala Pro Ala
   995 1000 1005
   Val Glu Ala Thr Leu Met Lys Ser Tyr Ala Asp Val His Gln Val
   1010 1015 1020
   Ser Glu Ala Asp Ala Arg Leu Trp Leu Gln Gln Leu Glu Glu Lys
   1025 1030 1035
   Gly Arg Tyr Ala Lys Asp Val Trp Ala Gly
   1040 1045

<210>  53
<211>  1048
<212>  PRT
<213>  Artificial Sequence

<220>
<223>  BM3 variant (V78Y, A82P, F87A, M184Y)

<400>  53

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Pro Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala

370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  54
<211>  1048
<212>  PRT
<213>  Artificial Sequence

<220>
<223>  BM3 variant (V78F, A82Q, F87A, M184Y)

<400>  54

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Phe Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met

785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg Tyr Ala Lys Asp Val Trp Ala Gly
    1040                1045

<210> 55
<211> 1048
<212> PRT
<213> Artificial Sequence

<220>
<223> BM3 variant (V78L, A82Q, F87A, M184Y)

<400> 55

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Leu Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp

195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  56
<211>  1048
<212>  PRT
<213>  Artificial Sequence

<220>
<223>  BM3 variant (V78I, A82Q, F87A, M184Y)

<400>  56

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Ile Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val

    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                1005

Val Glu  Ala Thr Leu Met Lys  Ser Tyr Ala Asp Val  His Gln Val
    1010                1015                1020

Ser Glu  Ala Asp Ala Arg Leu  Trp Leu Gln Gln Leu  Glu Glu Lys
    1025                1030                1035

Gly Arg  Tyr Ala Lys Asp Val  Trp Ala Gly
    1040                1045

<210>  57
<211>  1048
<212>  PRT
<213>  Artificial Sequence

<220>
<223>  BM3 variant (V78M, A82Q, F87A, M184Y)

<400>  57

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile

20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Met Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala
        435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys

1025                1030                1035

Gly Arg  Tyr Ala Lys Asp Val  Trp Ala Gly
    1040                1045

<210>  58
<211>  1048
<212>  PRT
<213>  Artificial Sequence

<220>
<223>  BM3 variant (V78Y, A82Q, F87A, M184Y)

<400>  58

Ala Ile Lys Glu Met Pro Gln Pro Lys Thr Phe Gly Glu Leu Lys Asn
1               5                   10                  15

Leu Pro Leu Leu Asn Thr Asp Lys Pro Val Gln Ala Leu Met Lys Ile
            20                  25                  30

Ala Asp Glu Leu Gly Glu Ile Phe Lys Phe Glu Ala Pro Gly Arg Val
        35                  40                  45

Thr Arg Tyr Leu Ser Ser Gln Arg Leu Ile Lys Glu Ala Cys Asp Glu
    50                  55                  60

Ser Arg Phe Asp Lys Asn Leu Ser Gln Ala Leu Lys Phe Tyr Arg Asp
65                  70                  75                  80

Phe Gln Gly Asp Gly Leu Ala Thr Ser Trp Thr His Glu Lys Asn Trp
                85                  90                  95

Lys Lys Ala His Asn Ile Leu Leu Pro Ser Phe Ser Gln Gln Ala Met
            100                 105                 110

Lys Gly Tyr His Ala Met Met Val Asp Ile Ala Val Gln Leu Val Gln
        115                 120                 125

Lys Trp Glu Arg Leu Asn Ala Asp Glu His Ile Glu Val Pro Glu Asp
    130                 135                 140

Met Thr Arg Leu Thr Leu Asp Thr Ile Gly Leu Cys Gly Phe Asn Tyr
145                 150                 155                 160

Arg Phe Asn Ser Phe Tyr Arg Asp Gln Pro His Pro Phe Ile Thr Ser
                165                 170                 175

Met Val Arg Ala Leu Asp Glu Tyr Met Asn Lys Leu Gln Arg Ala Asn
            180                 185                 190

Pro Asp Asp Pro Ala Tyr Asp Glu Asn Lys Arg Gln Phe Gln Glu Asp
        195                 200                 205

Ile Lys Val Met Asn Asp Leu Val Asp Lys Ile Ile Ala Asp Arg Lys
    210                 215                 220

Ala Ser Gly Glu Gln Ser Asp Asp Leu Leu Thr His Met Leu Asn Gly
225                 230                 235                 240

Lys Asp Pro Glu Thr Gly Glu Pro Leu Asp Asp Glu Asn Ile Arg Tyr
                245                 250                 255

Gln Ile Ile Thr Phe Leu Ile Ala Gly His Glu Thr Thr Ser Gly Leu
            260                 265                 270

Leu Ser Phe Ala Leu Tyr Phe Leu Val Lys Asn Pro His Val Leu Gln
        275                 280                 285

Lys Ala Ala Glu Glu Ala Ala Arg Val Leu Val Asp Pro Val Pro Ser
    290                 295                 300

Tyr Lys Gln Val Lys Gln Leu Lys Tyr Val Gly Met Val Leu Asn Glu
305                 310                 315                 320

Ala Leu Arg Leu Trp Pro Thr Ala Pro Ala Phe Ser Leu Tyr Ala Lys
                325                 330                 335

Glu Asp Thr Val Leu Gly Gly Glu Tyr Pro Leu Glu Lys Gly Asp Glu
            340                 345                 350

Leu Met Val Leu Ile Pro Gln Leu His Arg Asp Lys Thr Ile Trp Gly
        355                 360                 365

Asp Asp Val Glu Glu Phe Arg Pro Glu Arg Phe Glu Asn Pro Ser Ala
    370                 375                 380

Ile Pro Gln His Ala Phe Lys Pro Phe Gly Asn Gly Gln Arg Ala Cys
385                 390                 395                 400

Ile Gly Gln Gln Phe Ala Leu His Glu Ala Thr Leu Val Leu Gly Met
                405                 410                 415

Met Leu Lys His Phe Asp Phe Glu Asp His Thr Asn Tyr Glu Leu Asp
            420                 425                 430

Ile Lys Glu Thr Leu Thr Leu Lys Pro Glu Gly Phe Val Val Lys Ala

435                 440                 445

Lys Ser Lys Lys Ile Pro Leu Gly Gly Ile Pro Ser Pro Ser Thr Glu
    450                 455                 460

Gln Ser Ala Lys Lys Val Arg Lys Lys Ala Glu Asn Ala His Asn Thr
465                 470                 475                 480

Pro Leu Leu Val Leu Tyr Gly Ser Asn Met Gly Thr Ala Glu Gly Thr
                485                 490                 495

Ala Arg Asp Leu Ala Asp Ile Ala Met Ser Lys Gly Phe Ala Pro Gln
            500                 505                 510

Val Ala Thr Leu Asp Ser His Ala Gly Asn Leu Pro Arg Glu Gly Ala
        515                 520                 525

Val Leu Ile Val Thr Ala Ser Tyr Asn Gly His Pro Pro Asp Asn Ala
    530                 535                 540

Lys Gln Phe Val Asp Trp Leu Asp Gln Ala Ser Ala Asp Glu Val Lys
545                 550                 555                 560

Gly Val Arg Tyr Ser Val Phe Gly Cys Gly Asp Lys Asn Trp Ala Thr
                565                 570                 575

Thr Tyr Gln Lys Val Pro Ala Phe Ile Asp Glu Thr Leu Ala Ala Lys
            580                 585                 590

Gly Ala Glu Asn Ile Ala Asp Arg Gly Glu Ala Asp Ala Ser Asp Asp
        595                 600                 605

Phe Glu Gly Thr Tyr Glu Glu Trp Arg Glu His Met Trp Ser Asp Val
    610                 615                 620

Ala Ala Tyr Phe Asn Leu Asp Ile Glu Asn Ser Glu Asp Asn Lys Ser
625                 630                 635                 640

Thr Leu Ser Leu Gln Phe Val Asp Ser Ala Ala Asp Met Pro Leu Ala
                645                 650                 655

Lys Met His Gly Ala Phe Ser Thr Asn Val Val Ala Ser Lys Glu Leu
            660                 665                 670

Gln Gln Pro Gly Ser Ala Arg Ser Thr Arg His Leu Glu Ile Glu Leu
        675                 680                 685

Pro Lys Glu Ala Ser Tyr Gln Glu Gly Asp His Leu Gly Val Ile Pro
    690                 695                 700

Arg Asn Tyr Glu Gly Ile Val Asn Arg Val Thr Ala Arg Phe Gly Leu
705                 710                 715                 720

Asp Ala Ser Gln Gln Ile Arg Leu Glu Ala Glu Glu Glu Lys Leu Ala
                725                 730                 735

His Leu Pro Leu Ala Lys Thr Val Ser Val Glu Glu Leu Leu Gln Tyr
            740                 745                 750

Val Glu Leu Gln Asp Pro Val Thr Arg Thr Gln Leu Arg Ala Met Ala
        755                 760                 765

Ala Lys Thr Val Cys Pro Pro His Lys Val Glu Leu Glu Ala Leu Leu
    770                 775                 780

Glu Lys Gln Ala Tyr Lys Glu Gln Val Leu Ala Lys Arg Leu Thr Met
785                 790                 795                 800

Leu Glu Leu Leu Glu Lys Tyr Pro Ala Cys Glu Met Lys Phe Ser Glu
                805                 810                 815

Phe Ile Ala Leu Leu Pro Ser Ile Arg Pro Arg Tyr Tyr Ser Ile Ser
            820                 825                 830

Ser Ser Pro Arg Val Asp Glu Lys Gln Ala Ser Ile Thr Val Ser Val
        835                 840                 845

Val Ser Gly Glu Ala Trp Ser Gly Tyr Gly Glu Tyr Lys Gly Ile Ala
    850                 855                 860

Ser Asn Tyr Leu Ala Glu Leu Gln Glu Gly Asp Thr Ile Thr Cys Phe
865                 870                 875                 880

Ile Ser Thr Pro Gln Ser Glu Phe Thr Leu Pro Lys Asp Pro Glu Thr
                885                 890                 895

Pro Leu Ile Met Val Gly Pro Gly Thr Gly Val Ala Pro Phe Arg Gly
            900                 905                 910

Phe Val Gln Ala Arg Lys Gln Leu Lys Glu Gln Gly Gln Ser Leu Gly
        915                 920                 925

Glu Ala His Leu Tyr Phe Gly Cys Arg Ser Pro His Glu Asp Tyr Leu
    930                 935                 940

Tyr Gln Glu Glu Leu Glu Asn Ala Gln Ser Glu Gly Ile Ile Thr Leu
945                 950                 955                 960

His Thr Ala Phe Ser Arg Met Pro Asn Gln Pro Lys Thr Tyr Val Gln
                965                 970                 975

His Val Met Glu Gln Asp Gly Lys Lys Leu Ile Glu Leu Leu Asp Gln
            980                 985                 990

Gly Ala His Phe Tyr Ile Cys Gly  Asp Gly Ser Gln Met  Ala Pro Ala
        995                 1000                 1005

Val Glu  Ala Thr Leu Met Lys  Ser Tyr Ala Asp Val  His Gln Val
    1010                 1015                 1020

Ser Glu  Ala Asp Ala Arg Leu  Trp Leu Gln Gln Leu  Glu Glu Lys
    1025                 1030                 1035

Gly Arg  Tyr Ala Lys Asp Val  Trp Ala Gly
    1040                 1045

<210>  59
<211>  3228
<212>  DNA
<213>  Artificial Sequence

<220>
<223>  BM3 WT

<400>  59
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat ttgtacgtga ttttgcagga gacgggttat ttacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagta tggtccgtgc actggatgaa gcaatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 60
<211> 3228
<212> DNA
<213> Artificial Sequence

<220>
<223> BM3-254 (V78Y, A82E, F87A)

<400> 60
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtt taattaaaga agcatgcgat gaatcacgct ttgataaaaa cttaagtcaa 300

gcgcttaaat tttatcgtga ttttgaggga gacgggttag cgacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagctagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 61
<211> 3228
<212> DNA
<213> Artificial Sequence

<220>
<223> BM3-263 (V78M, A82E, F87A)

<400> 61
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtt taattaaaga agcatgcgat gaatcacgct ttgataaaaa cttaagtcaa 300

gcgcttaaat ttatgcgtga ttttgaggga gacgggttag cgacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagctagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 62
<211> 3228
<212> DNA
<213> Artificial Sequence

<220>
<223> BM3-268 (V78I, A82E, F87A)

<400> 62
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 63
<211> 3228
<212> DNA

<213>  Artificial Sequence

<220>
<223>  BM3 variant (V78L, A82E, F87A)

<400>  63
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat ttctgcgtga ttttgaggga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagta tggtccgtgc actggatgaa gcaatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg    1680

gcgtcttata acggtcatcc gcctgataac gcaaagcaat ttgtcgactg gttagaccaa    1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 64
<211> 3228
<212> DNA
<213> Artificial Sequence

<220>
<223> BM3 variant (V78Y, A82P, F87A)

<400> 64
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 65
<211> 3228
<212> DNA
<213> Artificial Sequence

<220>
<223> BM3 variant (V78M, A82P, F87A)

<400> 65
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 66
<211> 3228
<212> DNA
<213> Artificial Sequence

<220>
<223> BM3 variant (V78I, A82P, F87A)

<400> 66
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 67
<211> 3228
<212> DNA
<213> Artificial Sequence

<220>
<223> BM3 variant (V78L, A82P, F87A)

<400> 67
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 68
<211> 3228
<212> DNA
<213> Artificial Sequence

<220>
<223> BM3 variant (V78Y, A82Q, F87A)

<400> 68
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 69
<211> 3228
<212> DNA
<213> Artificial Sequence

<220>
<223> BM3 variant (V78M, A82Q, F87A)

<400> 69
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 70
<211> 3228
<212> DNA
<213> Artificial Sequence

<220>
<223> BM3 variant (V78I, A82Q, F87A)

<400> 70
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 71
<211> 3228
<212> DNA
<213> Artificial Sequence

<220>
<223>  BM3 variant (V78L, A82Q, F87A)

<400>  71
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat ttctgcgtga ttttcaggga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagta tggtccgtgc actggatgaa gcaatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg    1680

gcgtcttata acggtcatcc gcctgataac gcaaagcaat ttgtcgactg gttagaccaa    1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 72
<211> 3228
<212> DNA
<213> Artificial Sequence

<220>
<223> BM3 variant (V78F, A82E, F87A, M177Y, A184Y)

<400> 72
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 73
<211> 3228
<212> DNA
<213> Artificial Sequence

<220>
<223> BM3 variant (V78L, A82E, F87A, M177Y, A184Y)

<400> 73
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 74
<211> 3228
<212> DNA
<213> Artificial Sequence

<220>
<223> BM3 variant (V78I, A82E, F87A, M177Y, A184Y)

<400> 74
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 75
<211> 3228
<212> DNA
<213> Artificial Sequence

<220>
<223> BM3 variant (V78M, A82E, F87A, M177Y, A184Y)

<400> 75
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 76
<211> 3228
<212> DNA
<213> Artificial Sequence

<220>
<223> BM3 variant (V78Y, A82E, F87A, M177Y, A184Y)

<400> 76
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 77
<211> 3228
<212> DNA
<213> Artificial Sequence

<220>
<223> BM3 variant (V78F, A82P, F87A, M177Y, A184Y)

<400> 77
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 78
<211> 3228
<212> DNA
<213> Artificial Sequence

<220>
<223> BM3 variant (V78L, A82P, F87A, M177Y, A184Y)

<400> 78
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 79
<211> 3228
<212> DNA
<213> Artificial Sequence

<220>
<223>  BM3 variant (V78I, A82P, F87A, M177Y, A184Y)

<400>  79
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat ttattcgtga ttttcctgga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagtt atgtccgtgc actggatgaa tatatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg    1680

gcgtcttata acggtcatcc gcctgataac gcaaagcaat ttgtcgactg gttagaccaa    1740

gcgtctgctg atgaagtaaa aggcgttcgc tactccgtat ttggatgcgg cgataaaaac    1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 80
<211> 3228
<212> DNA
<213> Artificial Sequence

<220>
<223> BM3 variant (V78M, A82P, F87A, M177Y, A184Y)

<400> 80
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 81
<211> 3228
<212> DNA
<213> Artificial Sequence

<220>
<223> BM3 variant (V78Y, A82P, F87A, M177Y, A184Y)

<400> 81
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 82
<211> 3228
<212> DNA
<213> Artificial Sequence

<220>
<223> BM3 variant (V78F, A82Q, F87A, M177Y, A184Y)

<400> 82
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 83
<211> 3228
<212> DNA
<213> Artificial Sequence

<220>
<223> BM3 variant (V78L, A82Q, F87A, M177Y, A184Y)

<400> 83
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 84
<211> 3228
<212> DNA
<213> Artificial Sequence

<220>
<223> BM3 variant (V78I, A82Q, F87A, M177Y, A184Y)

<400> 84
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 85
<211> 3228
<212> DNA
<213> Artificial Sequence

<220>
<223> BM3 variant (V78M, A82Q, F87A, M177Y, A184Y)

<400> 85
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 86
<211> 3228
<212> DNA
<213> Artificial Sequence

<220>
<223> BM3 variant (V78Y, A82Q, F87A, M177Y, A184Y)

<400> 86
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 87
<211> 3228
<212> DNA
<213> Artificial Sequence

<220>

<223>  BM3 variant (V78F, A82E, F87A, M177Y)

<400>  87
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat tttttcgtga ttttgaggga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagtt acgtccgtgc actggatgaa gcaatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg    1680

gcgtcttata acggtcatcc gcctgataac gcaaagcaat ttgtcgactg gttagaccaa    1740

gcgtctgctg atgaagtaaa aggcgttcgc tactccgtat ttggatgcgg cgataaaaac    1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 88
<211> 3228
<212> DNA
<213> Artificial Sequence

<220>
<223> BM3 variant (V78L, A82E, F87A, M177Y)

<400> 88
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt acgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 89
<211> 3228
<212> DNA
<213> Artificial Sequence

<220>
<223> BM3 variant (V78I, A82E, F87A, M177Y)

<400> 89
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt acgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 90
<211> 3228
<212> DNA
<213> Artificial Sequence

<220>
<223> BM3 variant (V78M, A82E, F87A, M177Y)

<400> 90
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt acgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 91
<211> 3228
<212> DNA
<213> Artificial Sequence

<220>
<223> BM3 variant (V78Y, A82E, F87A, M177Y)

<400> 91
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt acgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 92
<211> 3228
<212> DNA
<213> Artificial Sequence

<220>
<223> BM3 variant (V78F, A82P, F87A, M177Y)

<400> 92
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 93
<211> 3228
<212> DNA
<213> Artificial Sequence

<220>
<223> BM3 variant (V78L, A82P, F87A, M177Y)

<400> 93
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 94
<211> 3228
<212> DNA
<213> Artificial Sequence

<220>
<223> BM3 variant (V78I, A82P, F87A, M177Y)

<400> 94
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 95
<211> 3228
<212> DNA
<213> Artificial Sequence

<220>
<223> BM3 variant (V78M, A82P, F87A, M177Y)

<400> 95
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 96
<211> 3228
<212> DNA
<213> Artificial Sequence

<220>
<223> BM3 variant (V78Y, A82P, F87A, M177Y)

<400> 96
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 97
<211> 3228
<212> DNA
<213> Artificial Sequence

<220>
<223> BM3 variant (V78F, A82Q, F87A, M177Y)

<400> 97
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 98
<211> 3228
<212> DNA
<213> Artificial Sequence

<220>
<223> BM3 variant (V78L, A82Q, F87A, M177Y)

<400> 98
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 99
<211> 3228
<212> DNA
<213> Artificial Sequence

<220>
<223> BM3 variant (V78I, A82Q, F87A, M177Y)

<400> 99
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 100
<211> 3228
<212> DNA
<213> Artificial Sequence

<220>
<223> BM3 variant (V78M, A82Q, F87A, M177Y)

<400> 100
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 101
<211> 3228
<212> DNA
<213> Artificial Sequence

<220>
<223> BM3 variant (V78Y, A82Q, F87A, M177Y)

<400> 101
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagtt atgtccgtgc actggatgaa gcaatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210>  102
<211>  3228
<212>  DNA
<213>  Artificial Sequence

<220>
<223>  BM3 variant (V78F, A82E, F87A, M184Y)

<400>  102
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat tttttcgtga ttttgaggga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagta tggtccgtgc actggatgaa tacatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 103
<211> 3228
<212> DNA
<213> Artificial Sequence

<220>
<223> BM3 variant (V78L, A82E, F87A, M184Y)

<400> 103
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tacatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 104
<211> 3228
<212> DNA
<213> Artificial Sequence

<220>
<223> BM3 variant (V78I, A82E, F87A, M184Y)

<400> 104
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tacatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 105
<211> 3228
<212> DNA
<213> Artificial Sequence

<220>
<223> BM3 variant (V78M, A82E, F87A, M184Y)

<400> 105
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tacatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 106
<211> 3228
<212> DNA
<213> Artificial Sequence

<220>
<223> BM3 variant (V78Y, A82E, F87A, M184Y)

<400> 106
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttgaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tacatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 107
<211> 3228
<212> DNA
<213> Artificial Sequence

<220>
<223> BM3 variant (V78F, A82P, F87A, M184Y)

<400> 107
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 108
<211> 3228
<212> DNA
<213> Artificial Sequence

<220>
<223> BM3 variant (V78L, A82P, F87A, M184Y)

<400> 108
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 109
<211> 3228
<212> DNA
<213> Artificial Sequence

<220>
<223> BM3 variant (V78I, A82P, F87A, M184Y)

<400> 109
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 110

<211>  3228
<212>  DNA
<213>  Artificial Sequence

<220>
<223>  BM3 variant (V78M, A82P, F87A, M184Y)

<400>  110
atgaaacatc accatcacca tcaccccatg agcgattacg acatccccac tactgagaat      60

ctttattttc agggcgccat ggcaattaaa gaaatgcctc agccaaaaac gtttggagag     120

cttaaaaatt taccgttatt aaacacagat aaaccggttc aagctttgat gaaaattgcg     180

gatgaattag gagaaatctt taaattcgag gcgcctggtc gtgtaacgcg ctacttatca     240

agtcagcgtc taattaaaga agcatgcgat gaatcacgct ttgataaaaa cttaagtcaa     300

gcgcttaaat ttatgcgtga ttttcctgga gacgggttag caacaagctg gacgcatgaa     360

aaaaattgga aaaaagcgca taatatctta cttccaagct tcagtcagca ggcaatgaaa     420

ggctatcatg cgatgatggt cgatatcgcc gtgcagcttg ttcaaaagtg ggagcgtcta     480

aatgcagatg agcatattga agtaccggaa gacatgacac gtttaacgct tgatacaatt     540

ggtctttgcg gctttaacta tcgctttaac agcttttacc gagatcagcc tcatccattt     600

attacaagta tggtccgtgc actggatgaa tatatgaaca agctgcagcg agcaaatcca     660

gacgacccag cttatgatga aaacaagcgc cagtttcaag aagatatcaa ggtgatgaac     720

gacctagtag ataaaattat tgcagatcgc aaagcaagcg gtgaacaaag cgatgattta     780

ttaacgcata tgctaaacgg aaaagatcca gaaacgggtg agccgcttga tgacgagaac     840

attcgctatc aaattattac attcttaatt gcgggacacg aaacaacaag tggtctttta     900

tcatttgcgc tgtatttctt agtgaaaaat ccacatgtat tacaaaaagc agcagaagaa     960

gcagcacgag ttctagtaga tcctgttcca agctacaaac aagtcaaaca gcttaaatat    1020

gtcggcatgg tcttaaacga agcgctgcgc ttatggccaa ctgctcctgc gttttcccta    1080

tatgcaaaag aagatacggt gcttggagga gaatatcctt tagaaaaagg cgacgaacta    1140

atggttctga ttcctcagct tcaccgtgat aaaacaattt ggggagacga tgtggaagag    1200

ttccgtccag agcgttttga aaatccaagt gcgattccgc agcatgcgtt taaaccgttt    1260

ggaaacggtc agcgtgcgtg tatcggtcag cagttcgctc ttcatgaagc aacgctggta    1320

cttggtatga tgctaaaaca ctttgacttt gaagatcata caaactacga gctggatatt    1380

aaagaaactt taacgttaaa acctgaaggc tttgtggtaa aagcaaaatc gaaaaaaatt    1440

ccgcttggcg gtattccttc acctagcact gaacagtctg ctaaaaaagt acgcaaaaag    1500

gcagaaaacg ctcataatac gccgctgctt gtgctatacg gttcaaatat gggaacagct    1560

gaaggaacgg cgcgtgattt agcagatatt gcaatgagca aaggatttgc accgcaggtc    1620

gcaacgcttg attcacacgc cggaaatctt ccgcgcgaag gagctgtatt aattgtaacg    1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 111
<211> 3228
<212> DNA
<213> Artificial Sequence

<220>
<223> BM3 variant (V78Y, A82P, F87A, M184Y)

<400> 111

atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcctgga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 112
<211> 3228
<212> DNA
<213> Artificial Sequence

<220>
<223> BM3 variant (V78F, A82Q, F87A, M184Y)

<400> 112
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttttcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 113
<211> 3228
<212> DNA
<213> Artificial Sequence

<220>
<223> BM3 variant (V78L, A82Q, F87A, M184Y)

<400> 113
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttctgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 114
<211> 3228
<212> DNA
<213> Artificial Sequence

<220>
<223> BM3 variant (V78I, A82Q, F87A, M184Y)

<400> 114
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttattcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 115
<211> 3228
<212> DNA
<213> Artificial Sequence

<220>
<223> BM3 variant (V78M, A82Q, F87A, M184Y)

<400> 115
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat ttatgcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

<210> 116
<211> 3228
<212> DNA
<213> Artificial Sequence

<220>
<223> BM3 variant (V78Y, A82Q, F87A, M184Y)

<400> 116
atgaaacatc accatcacca tcaccccatg agcgattacg acatccccac tactgagaat 60

ctttattttc agggcgccat ggcaattaaa gaaatgcctc agccaaaaac gtttggagag 120

cttaaaaatt taccgttatt aaacacagat aaaccggttc aagctttgat gaaaattgcg 180

gatgaattag gagaaatctt taaattcgag gcgcctggtc gtgtaacgcg ctacttatca 240

agtcagcgtc taattaaaga agcatgcgat gaatcacgct ttgataaaaa cttaagtcaa 300

gcgcttaaat tttatcgtga ttttcaggga gacgggttag caacaagctg gacgcatgaa 360

aaaaattgga aaaaagcgca taatatctta cttccaagct tcagtcagca ggcaatgaaa 420

ggctatcatg cgatgatggt cgatatcgcc gtgcagcttg ttcaaaagtg ggagcgtcta 480

aatgcagatg agcatattga agtaccggaa gacatgacac gtttaacgct tgatacaatt 540

ggtctttgcg gctttaacta tcgctttaac agcttttacc gagatcagcc tcatccattt 600

attacaagta tggtccgtgc actggatgaa tatatgaaca agctgcagcg agcaaatcca 660

gacgacccag cttatgatga aaacaagcgc cagtttcaag aagatatcaa ggtgatgaac 720

gacctagtag ataaaattat tgcagatcgc aaagcaagcg gtgaacaaag cgatgattta 780

ttaacgcata tgctaaacgg aaaagatcca gaaacgggtg agccgcttga tgacgagaac 840

attcgctatc aaattattac attcttaatt gcgggacacg aaacaacaag tggtctttta 900

tcatttgcgc tgtatttctt agtgaaaaat ccacatgtat tacaaaaagc agcagaagaa 960

gcagcacgag ttctagtaga tcctgttcca agctacaaac aagtcaaaca gcttaaatat 1020

gtcggcatgg tcttaaacga agcgctgcgc ttatggccaa ctgctcctgc gttttcccta 1080

tatgcaaaag aagatacggt gcttggagga gaatatcctt tagaaaaagg cgacgaacta 1140

atggttctga ttcctcagct tcaccgtgat aaaacaattt ggggagacga tgtggaagag 1200

ttccgtccag agcgttttga aaatccaagt gcgattccgc agcatgcgtt taaaccgttt 1260

ggaaacggtc agcgtgcgtg tatcggtcag cagttcgctc ttcatgaagc aacgctggta 1320

cttggtatga tgctaaaaca ctttgacttt gaagatcata caaactacga gctggatatt 1380

aaagaaactt taacgttaaa acctgaaggc tttgtggtaa aagcaaaatc gaaaaaaatt 1440

ccgcttggcg gtattccttc acctagcact gaacagtctg ctaaaaaagt acgcaaaaag 1500

gcagaaaacg ctcataatac gccgctgctt gtgctatacg gttcaaatat gggaacagct 1560

gaaggaacgg cgcgtgattt agcagatatt gcaatgagca aaggatttgc accgcaggtc 1620

gcaacgcttg attcacacgc cggaaatctt ccgcgcgaag gagctgtatt aattgtaacg 1680

gcgtcttata acggtcatcc gcctgataac gcaaagcaat ttgtcgactg gttagaccaa 1740

gcgtctgctg atgaagtaaa aggcgttcgc tactccgtat ttggatgcgg cgataaaaac 1800

tgggctacta cgtatcaaaa agtgcctgct tttatcgatg aaacgcttgc cgctaaaggg 1860

gcagaaaaca tcgctgaccg cggtgaagca gatgcaagcg acgactttga aggcacatat 1920

gaagaatggc gtgaacatat gtggagtgac gtagcagcct actttaacct cgacattgaa 1980

aacagtgaag ataataaatc tactctttca cttcaatttg tcgacagcgc cgcggatatg 2040

ccgcttgcga aaatgcacgg tgcgttttca acgaacgtcg tagcaagcaa agaacttcaa 2100

cagccaggca gtgcacgaag cacgcgacat cttgaaattg aacttccaaa agaagcttct 2160

tatcaagaag gagatcattt aggtgttatt cctcgcaact atgaaggaat agtaaaccgt 2220

gtaacagcaa ggttcggcct agatgcatca cagcaaatcc gtctggaagc agaagaagaa 2280

aaattagctc atttgccact cgctaaaaca gtatccgtag aagagcttct gcaatacgtg 2340

gagcttcaag atcctgttac gcgcacgcag cttcgcgcaa tggctgctaa aacggtctgc 2400

ccgccgcata aagtagagct tgaagccttg cttgaaaagc aagcctacaa agaacaagtg 2460

ctggcaaaac gtttaacaat gcttgaactg cttgaaaaat acccggcgtg tgaaatgaaa 2520

ttcagcgaat ttatcgccct tctgccaagc atacgcccgc gctattactc gatttcttca 2580

tcacctcgtg tcgatgaaaa acaagcaagc atcacggtca gcgttgtctc aggagaagcg 2640

tggagcggat atggagaata taaaggaatt gcgtcgaact atcttgccga gctgcaagaa 2700

ggagatacga ttacgtgctt tatttccaca ccgcagtcag aatttacgct gccaaaagac 2760

cctgaaacgc cgcttatcat ggtcggaccg ggaacaggcg tcgcgccgtt tagaggcttt 2820

gtgcaggcgc gcaaacagct aaaagaacaa ggacagtcac ttggagaagc acatttatac 2880

ttcggctgcc gttcacctca tgaagactat ctgtatcaag aagagcttga aaacgcccaa 2940

agcgaaggca tcattacgct tcataccgct ttttctcgca tgccaaatca gccgaaaaca 3000

tacgttcagc acgtaatgga acaagacggc aagaaattga ttgaacttct tgatcaagga 3060

gcgcacttct atatttgcgg agacggaagc caaatggcac ctgccgttga agcaacgctt 3120

atgaaaagct atgctgacgt tcaccaagtg agtgaagcag acgctcgctt atggctgcag 3180

cagctagaag aaaaaggccg atacgcaaaa gacgtgtggg ctgggtaa 3228

## Description

### FIELD OF THE DISCLOSURE

The present invention relates to novel recombinant *Bacillus megaterium* cytochrome P450-monooxygenase (P450-BM3) variants for the C19 hydroxylation of steroids and derivatives thereof. In particular, the present invention also relates to methods and processes using P450-BM3 variants for the production of estrone and estradiol. The invention further relates to nucleotide sequences, constructs and vectors for the expression of these P450-BM3 variants.

### BACKGROUND

The steroid hormones estrone and estradiol and their derivatives are used as medications, for instance in menopausal hormone therapy, contraception and oncology or are important intermediates for the synthesis of further steroid products. Estrone and estradiol can be generated in several steps starting from phytosterols. The different degradation products of phytosterols contain a methyl group in position C19 of the steroid ring systems which needs to be removed in order to synthesize estrone and estradiol and their derivatives under aromatization of the A ring of the steroid ring system. Inhoffen describes this aromatization by thermolysis for the synthesis of estradiol (Inhoffen 1947) and Hershberg *et al* describe this aromatization by thermolysis for the synthesis of estrone (Hershberg 1950). However, yields are not optimal and various side products are formed. Templeton *et al.* and Numazawa *et al.* describe this aromatization through steroid products which have been oxidized in position C19 (F. Templeton 1997), (Mitsuteru Numazawa 2009). Therefore, processes hydroxylating the methyl group in position C19 and thus allowing for further derivatization and eventually removal of the substituent in position 19 can pave the way for novel and improved syntheses of estrone and estradiol and their derivatives.

Enzymes as catalysts are typically characterized by an excellent regio- and/or stereoselectivity for the natural substrates but may show low thermal and solvent stabilities. Due to a narrow substrate spectrum, enzymes are often very specialized compared to chemical catalyst, which limits their broad applicability. It is therefore a challenging endeavor to find or build an enzyme which catalyzes a specific reaction with commercially exploitable yield and sufficient purity.

Cytochrome P450 monooxgenases (P450s) comprise a large group of heme enzymes that are ubiquitous in the natural world. Cytochrome P450 BM3 (P450-BM3, BM3), obtained from *Bacillus megaterium* has previously been described to catalyze the NADPH-dependent hydroxylation of long-chain fatty acids, alcohols, and amides, as well as the epoxidation of unsaturated fatty acids (see e.g. (Narhi 1986) and (Capdevila 1996)). For P450-BM3 the reductase (65 kDa) and monooxygenase (55 kDa) domains of the enzyme are fused and produced as a catalytically self-sufficient 120 kDa enzyme. In consequence, P450-BM3 enzymes exhibit the highest rate of catalysis amongst P450 monooxygenases due to the efficient electron transfer between the fused reductase and heme domains, see e.g., (Noble MA 1999); and (Munro 1996).

BM3 variants have been described for various biotransformation processes. Wild-type and mutant P450 BM3 were applied as biocatalysts in the production of chemicals, including pharmaceuticals (Hazel M Girvan 2016). For example, (WO200107630A1) discloses processes for the microbiological oxidation of different organic substrates, such as N-heterocyclic aromatic compounds, particularly methods for the preparation of indigo and indian ruby using special cytochrome P450-monooxygenases with altered substrate specificity. (EP1196603A1) claims a P450 BM3 variant with an altered profile in enzymatic hydroxylation of aliphatic carboxylic acids, owing to site-specific mutagenesis of its substrate-binding region, wherein F87 is replaced by Val, Ala or Leu, and L188 is replaced by Asn, Gln, Arg, Lys, Ala, Gly, Ser or Trp, and optionally at least one of amino acid positions 26, 47, 72, 74 and 354 is altered. (WO2016007623A1) discloses cytochrome P450 BM3 variants to obtain improved activity with regard to substrates selected from nifedipine, propranolol, verapamil and diclofenac. (EP1131440A2) discloses a process for oxidising a substrate which is an acyclic or cyclic terpene selected from monoterpenes, sesquiterpenes and diterpenes, or a cycloalkene; or a substituted derivative thereof, which process comprises oxidising said compound with a mutant P450 BM3 enzyme, the mutant comprising the substitution of an amino acid in the active site by an amino acid with a less polar side-chain.

None of the mentioned documents discloses the specific structure of the BM3 variants according to the current invention. Furthermore, none of the above indicated documents relates to the C19 hydroxylation of steroid derivatives, e.g. for the production of estradiol or estrone. Instead it has been described that BM3 wildtype (WT) does not accept testosterone nor steroids in general as a substrate.

(Kille Sabrina 2011), (Kille 2010) and (Acevedo-Rocha 2018) have described a library of P450 BM3 variants and the use of directed evolution of P450 BM3. Acevedo-Rocha *et al.* have reported the directed evolution of P450 BM3 for the selective hydroxylation of testosterone. With the resulting mutants, however, relevant amounts of hydroxylation of testosterone were obtained only at positions 16α/β. These findings discouraged further developments of BM3 variants for C19 hydroxylation, because there was a prejudice to obtain only trace amounts at the position C19 due to preferential hydroxylation at other positions (Acevedo-Rocha 2018).

According to the current invention it was nevertheless surprisingly found that specific novel variants of bacterial cytochrome P450 BM3 monooxygenases (CYPs) can be used to catalyze the efficient synthesis of estrone and estradiol and their derivatives through C19-hydroxylation, also in an efficient and commercially exploitable way. In particular, it was surprisingly found that C19-hydroxylation of substrates testosterone, delta-1-testosterone and androsta-1,4-dien-3,17-dione can be achieved using mutants of bacterial cytochrome P450 BM3 monooxygenases as biocatalysts.

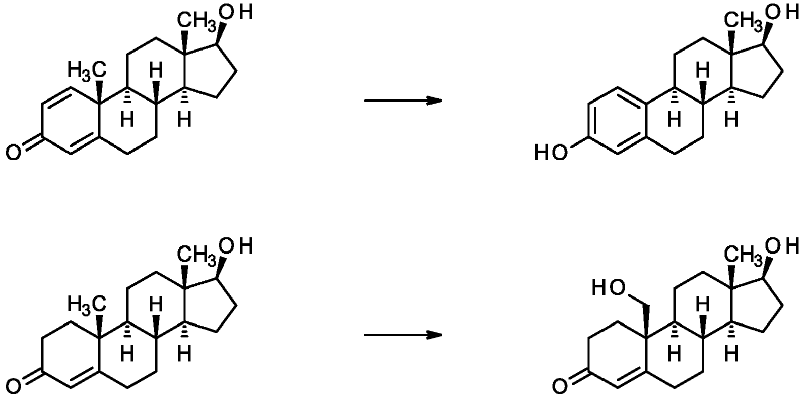

### SUMMARY

According to a first aspect of the invention there is provided a Cytochrome P450 BM3 monooxygenase (BM3) variant for catalyzing the C19 hydroxylation of a steroid or steroid derivative. According to a first embodiment of the first aspect, the BM3 variant comprises the mutation F87A and at least one and preferably two further mutations selected from (i) a mutation at position V78, preferably V78F, V78Y, V78M, V78I or V78L, (ii) a mutation at position A82, preferably A82E, A82Q or A82P; and optionally further mutations. According to a second aspect of the current invention, there is provided a nucleic acid encoding for a Cytochrome P450 BM3 monooxygenase variant as described herein. According to a third aspect of the current invention, there is provided a host cell for the production of a Cytochrome P450 BM3 monooxygenase variant. In a preferred embodiment, the host cell comprises a nucleic acid encoding for a BM3 variant according to any of the aspects dercribed herein. According to a fourth aspect of the current invention, there is provided the use of a Cytochrome P450 BM3 monooxygenase (BM3) variant for the production of a compound according to formula I, wherein R1 and R2 form a six-membered ring as part of a steroid.

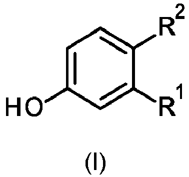

According to a fifth aspect of the current invention, there is provided the use of a BM3 variant for the C19-hydroxylation of a steroid or steroid derivative. According to a sixth aspect of the current invention, there is provided a process for C19-hydroxylation of a steroid or derivative thereof comprising (i) (a) culturing a recombinant Cytochrome P450 BM3 monooxygenase (BM3) variant producing microorganism in a culture medium, in the presence of an exogenous or intermediately formed substrate; or (b) incubating a substrate-containing reaction medium with a Cytochrome P450 BM3 monooxygenase; and (ii) isolating the oxidation product formed, or a secondary product thereof, from the medium; said process being further characterized in that said BM3 variant is a BM3 variant as described herein. According to a seventh aspect there is provided a method for obtaining optimized BM3 variants for the C19 hydroxylation of steroids, said method comprising (i) (a) culturing a recombinant microorganism expressing a BM3 variant (test variant) in a culture medium, in the presence of an exogenous or intermediately formed steroid or steroid derivative; or (b) incubating a steroid or steroid derivative-containing reaction medium with a BM3 variant (test variant); and (ii) comparing the obtained product yield or selectiviy factor for the C19 hydroxylation product formed by the test variant or a secondary product thereof with the respective value obtained for a parent variant of the test variant capable of catalyzing the C19 hydroxylation of a steroid, and (iii) selecting the test variant as optimized for the C19 hydroxylation of steroids, if the test variant has an improved product yield/and or selectivity factor compared to the parent variant.

### BRIEF DESCRIPTION OF THE DRAWINGS

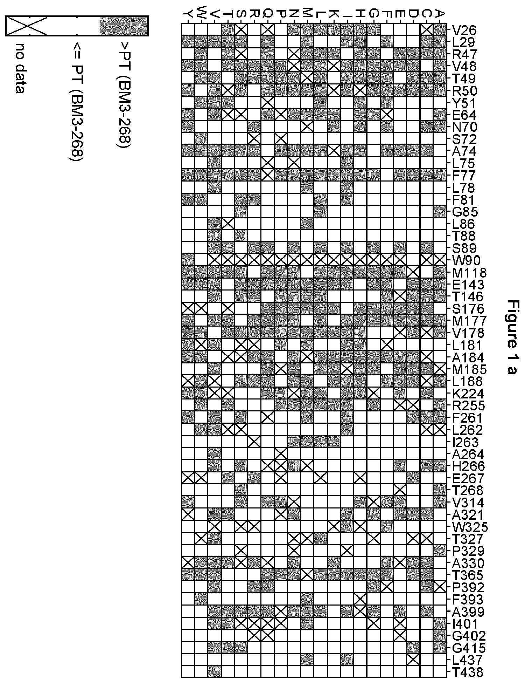

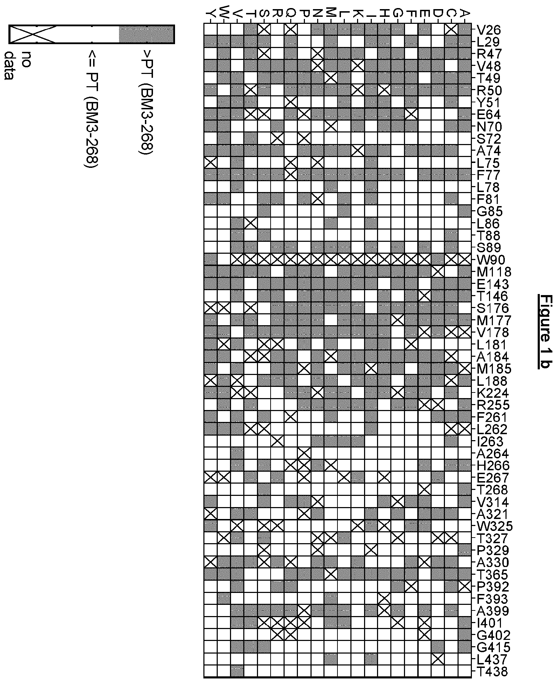

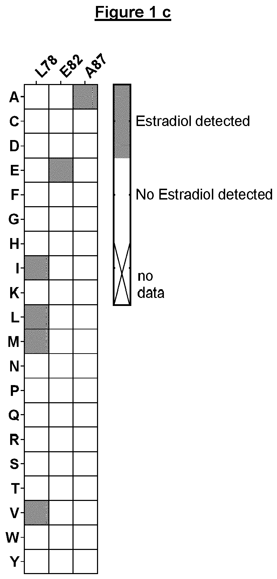

**Figure 1** **a** shows the estradiol yield obtained in biotransformation reactions for BM3-268 daughter variants. The estradiol yield (from 50 mg/L Δ1-testosterone as substrate) is given as ratio to the parent type. In comparison with the parent type, estradiol yields could be further improved by introducing modifications at positions V26, L29, R47, V48, T49, R50, Y51, E64, N70, S72, A74, L75, F77, L78, F81, G85, L86, T88, S89, W90, M118, E143, T146, S176, M177, V178, L181, A184, M185, L188, K224, R255, F261, L262, I263, A264, H266, E267, T268, V314, A321, W325, T327, P329, A330, T365, P392, F393, A399, 1401, G402, G415, L437, T438.
**Figure 1 b** shows the selectivity factor for estradiol obtained in biotransformation reactions for BM3-268 daughter variants. The selectivity factor (from 50 mg/L Δ1-testosterone as substrate) is given as ratio to the parent type. In comparison with the parent type, selectivity factors could be further improved by introducing modifications at positions V26, L29, R47, V48, T49, R50, Y51, E64, N70, S72, A74, L75, F77, L78, F81, G85, L86, T88, S89, W90, M118, E143, T146, S176, M177, V178, L181, A184, M185, L188, K224, R255, F261, L262, I263, A264, H266, E267, T268, V314, A321, W325, T327, P329, A330, T365, P392, F393, A399, 1401, G402, G415, L437, T438.
**Figure 1 c** shows Δ1-testosterone to estradiol conversion obtained in biotransformation reactions (50 mg/L Δ1-testosterone as substrate) with BM3-268 (SEQ ID No. 5) variants, wherein the positions L78, E82 and A87 have been altered. Estradiol was obtained with BM3-268 and variants L78[I, L, M, V] of BM3-268.

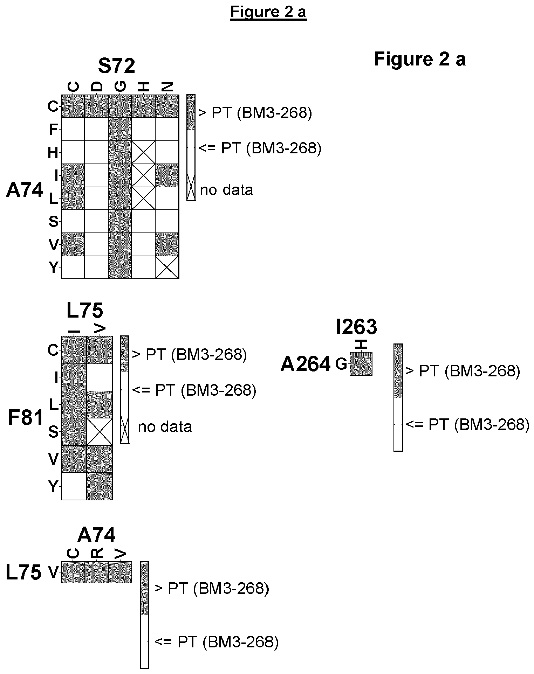

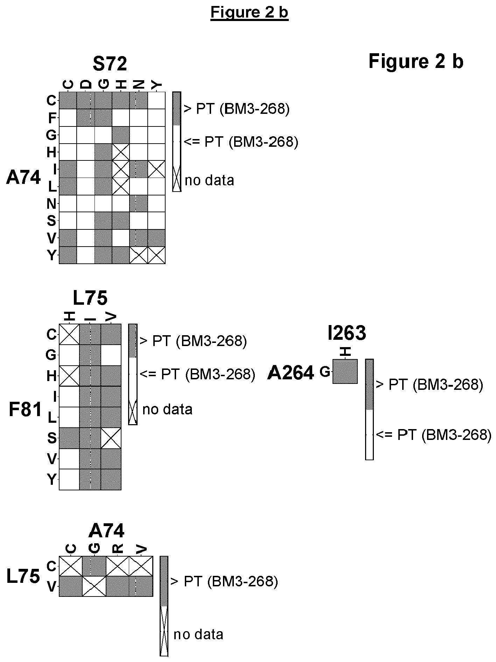

**Figure 2 a, b** show the estradiol product yield (a) and selectivity factor (b), respectively, obtained in biotransformation reactions with BM3-268 (SEQ ID No. 5) variants comprising the depicted double mutations. Both estradiol yield (from 50 mg/L Δ1-testosterone as substrate) and selectivity factor are given as ratio to the parent type. Variants having an improved yield or selectivity factor compared to BM3-268 are depicted in grey. In comparison with the BM3-268 parent variant, product yields could be further improved by introducing the following mutations: (S72C, A74[C, I, L, V]), (S72D, A74C), (S72G, A74[C, F, H, I, L, S, V, Y]), (S72H, A74C), (S72N, A74[C, I, V]), (L75I, F81[C, I, L, S, V]), (L75V, F81[C, L, V, Y]), (I263H, A264G), (A74[C, R, V], L75V). In comparison with the BM3-268 parent variant, selectivity factors could be further improved by introducing the following mutations: (S72C, A74[C, I, L, V, Y]), (S72D, A74[C, F]), (S72G, A74[C, F, H, I, L, S, V, Y]), (S72H, A74[C, G, S, Y]), (S72N, A74[C, I, N, V]), (S72Y, A74V), (L75H, F81S), (L75I, F81[C, G, H, I, L, S, V, Y]), (L75V, F81[C, H, I, L, V, Y]), (I263H, A264G), (A74G, L75C), (A74[C, R, V], L75V).

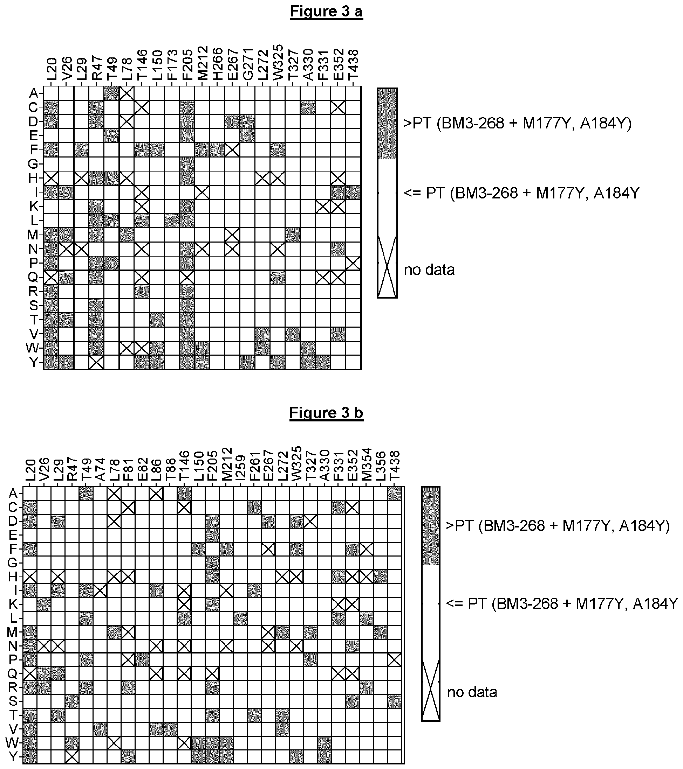

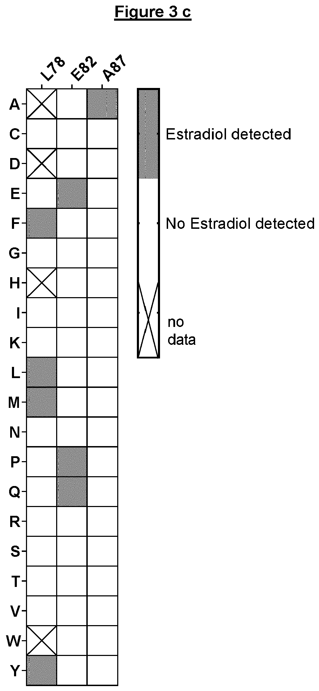

**Figure 3 a, b** show the estradiol product yield (a) and selectivity factor (b), respectively, obtained in biotransformation reactions with variants of BM3-268; M177Y, A184Y (SEQ ID No. 15) and further mutations as depicted. Both, estradiol yield and selectivity factor (from 100 mg/L Δ1-testosterone as substrate) are given as ratio to the parent type. Variants of SEQ ID No. 15 comprising an additional mutation at position L20, V26, L29, R47, T49, L78, T146, L150, F173, F205, M212, H266, E267, G271, L272, W325, T327, A330, F331, E352, and/or T438 were surprisingly found to have a further improved product yield compared with the parent variant according to SEQ ID No. 15. Variants of SEQ ID No. 15 comprising an additional mutation at position L20, V26, L29, R47, T49, A74, L78, F81, E82, L86, T88, T146, L150, F205, M212, I259, F261, E267, L272, W325, T327, A330, F331, E352, M354, L356, and/or T438 were surprisingly found to have an improved selectivity compared with the parent variant according to SEQ ID No. 15.
**Figure 3c** shows Δ1-testosterone to estradiol conversion obtained in biotransformation reactions with 100 mg/L Δ1-testosterone as substrate, using BM3-268; M177Y, A184Y (SEQ ID No. 15) and comprising mutation(s) at position L78, E82 or A87 as specified. Estradiol was obtained with the variant having the SEQ ID No. 15. Furthermore, estradiol was obtained with SEQ ID No. 15 derived variants comprising at least mutation(s) L78[L, F, M, Y] or E82[E, P, Q], or combinations thereof.

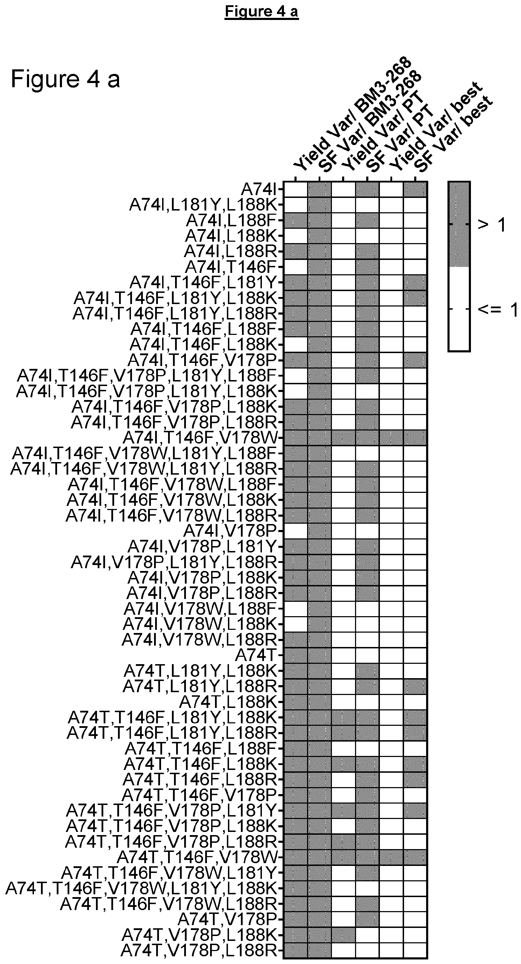

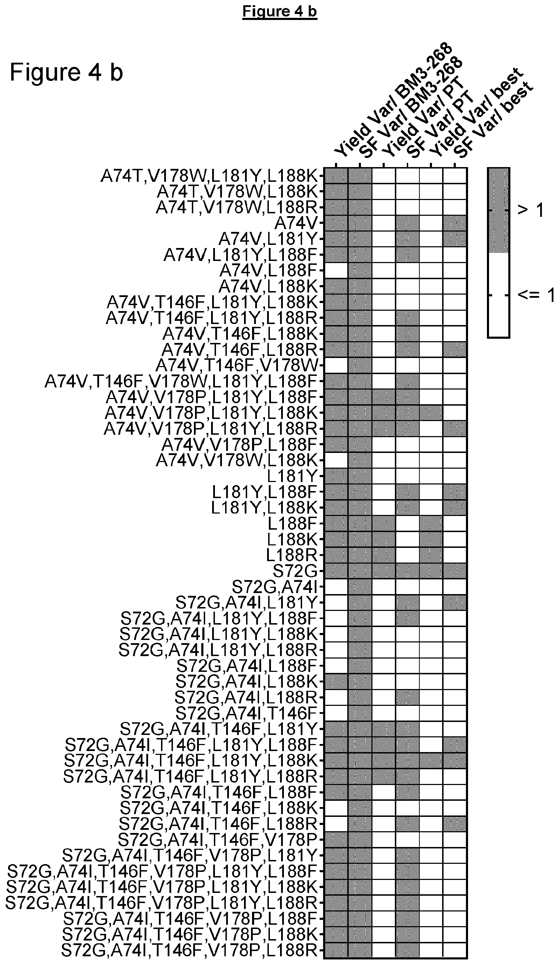

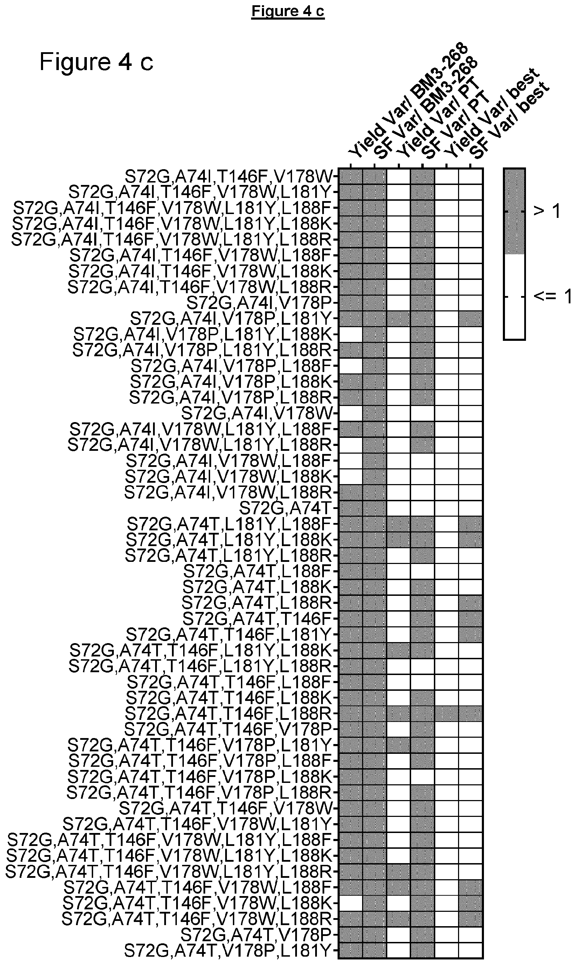

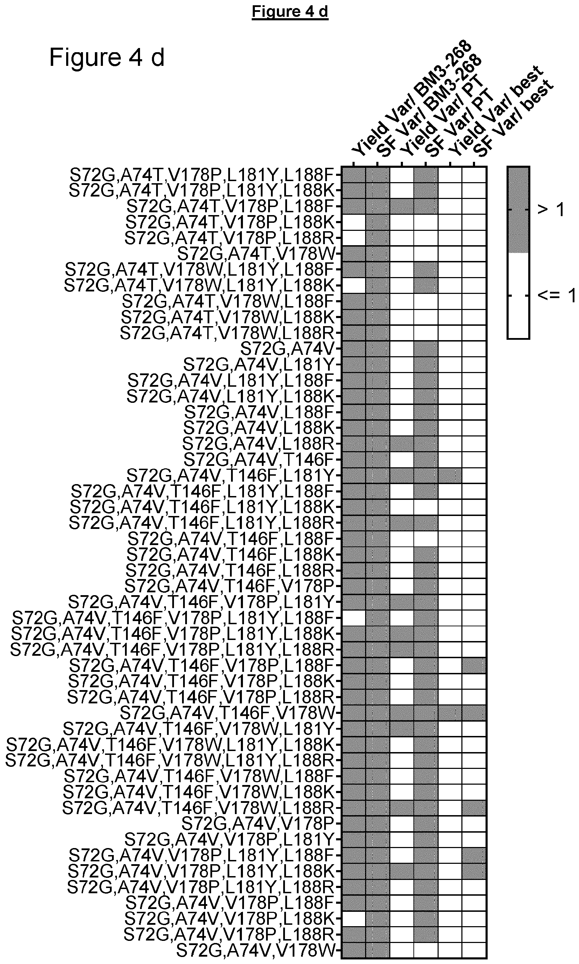

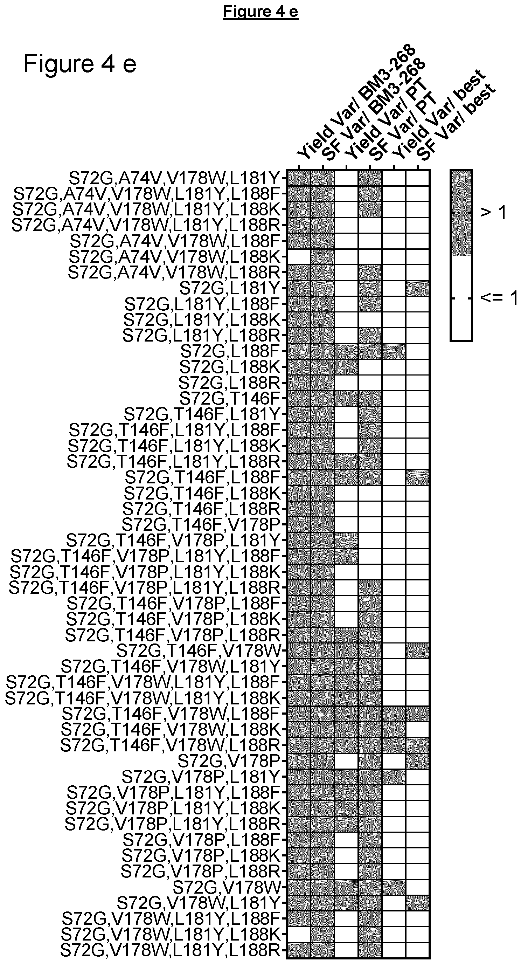

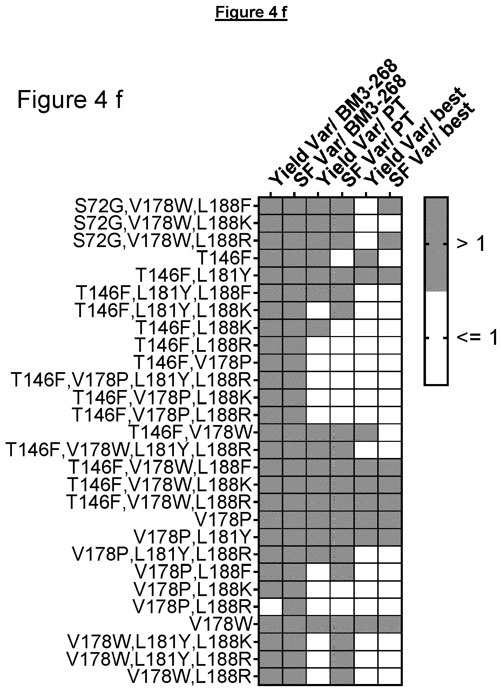

**Figure 4 a, b, c, d, e, f** show product yield and selectivity factor for estradiol obtained in biotransformation reactions with BM3-268; M177Y, A184Y (SEQ ID No. 15) variants (Var) having up to six mutations. 100 mg/L Δ1-testosterone were used as substrate. Each variant was compared to BM3-268 (SEQ ID No. 5) and improved variant BM3-268; M177Y, A184Y (PT) (SEQ ID No. 15), respectively. Furthermore, the last two columns show whether a variant (having the depicted set of mutations) was improved in yield or selectivity (SF) compared to the best analyzed variant (best) from a set of variants comprising the same subset of mutations but at least one mutation less.

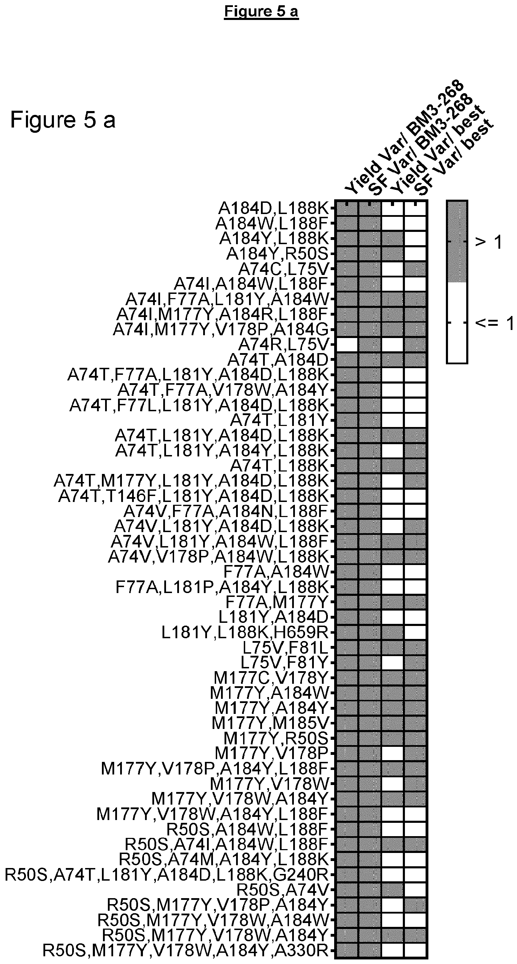

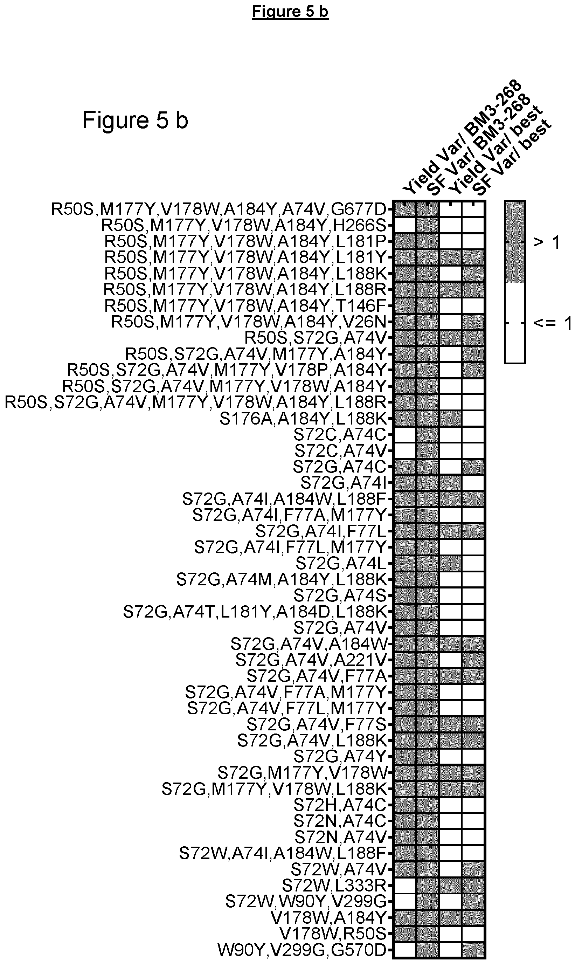

**Figure 5a** **and b** show estradiol product yield and selectivity factor (SF) obtained in biotransformation reactions with BM3-268 variants having up to seven mutations. 50 mg/L Δ1-testosterone were used as substrate. Each variant was compared to BM3-268 (SEQ ID No. 5) with regard to product yield and selectivity factor (columns 1 and 2). Furthermore, columns 3 and 4 show whether a variant (having the depicted set of mutations) was improved in yield or selectivity (SF) compared to the best analyzed variant (best) from a set of variants comprising the same subset of mutations but at least one mutation less.

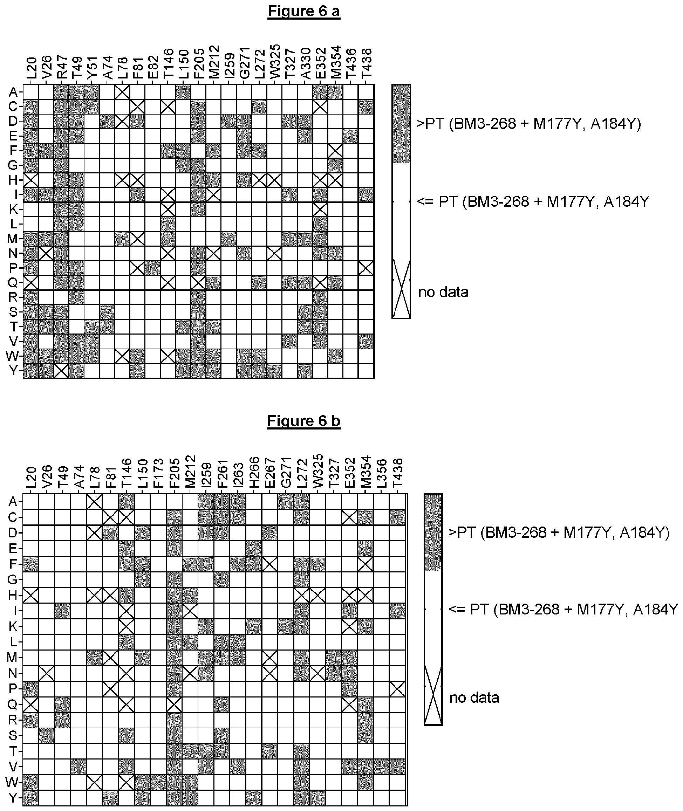

**Figures 6a** **and b** show product yield (a) and selectivity factor (b), respectively, for estrone obtained in biotransformation reactions with BM3-268; M177Y, A184Y (SEQ ID No. 15) variants having the depicted modifications. Estrone yield/selectivity factor from 100 mg/L ADD as substrate is given as ratio to the parent type (SEQ ID No. 15). In comparison with the parent type, estradiol yields could be further improved by introducing modifications at positions L20, V26, R47, T49, Y51, A74, L78, F81, E82, T146, L150, F205, M212, I259, G271, L272, W325, T327, A330, E352, M354, T436, T438. Selectivity factors could be further improved in comparison to the parent type by introducing modifications at positions L20, V26, T49, A74, L78, F81, T146, L150, F173, F205, M212, I259, F261, I263, H266, E267, G271, L272, W325, T327, E352, M354, L356, T438.

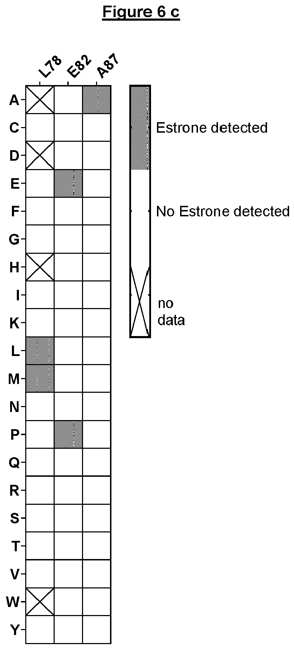

**Figure 6 c** shows ADD to estrone conversion obtained in biotransformation reactions with 100 mg/L ADD as substrate using BM3-268; M177Y, A184Y (SEQ ID No. 15) variants having a mutation at position L78, E82 or A87. Estrone was obtained with the variant having SEQ ID No. 15 and variants L78[M], E82[P] of SEQ ID No. 15.

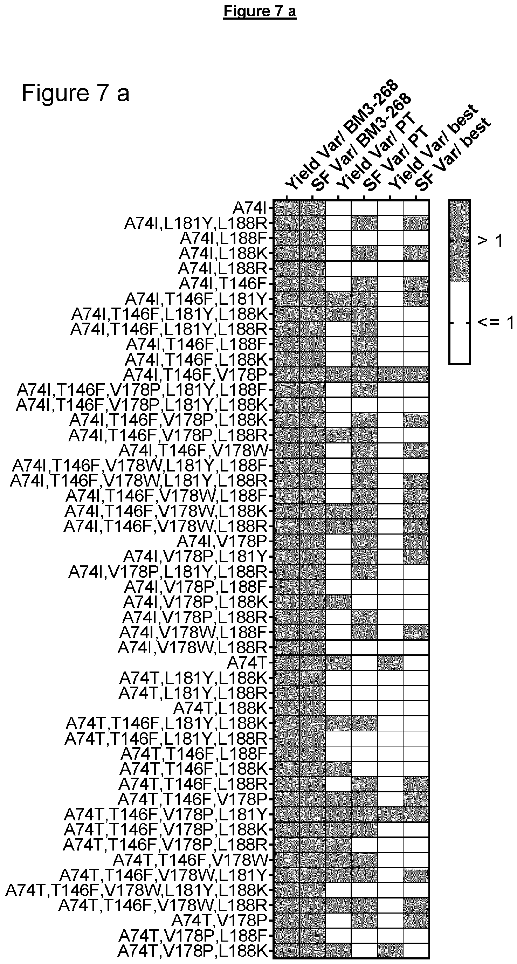

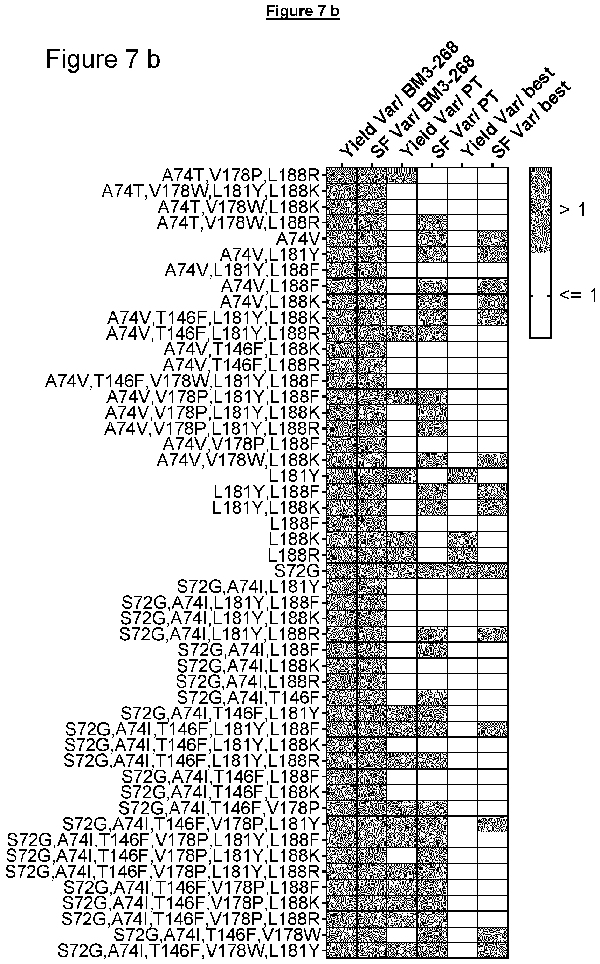

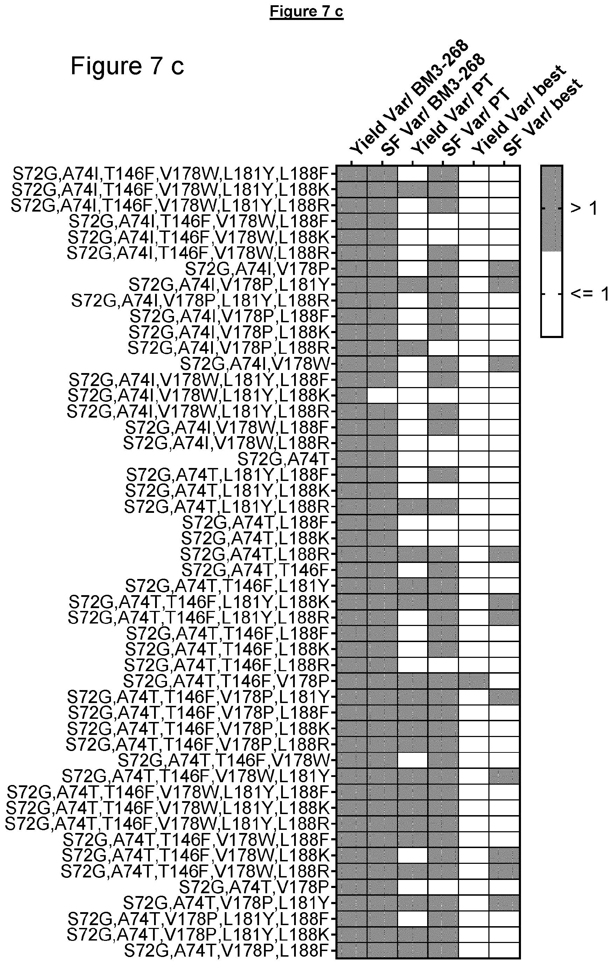

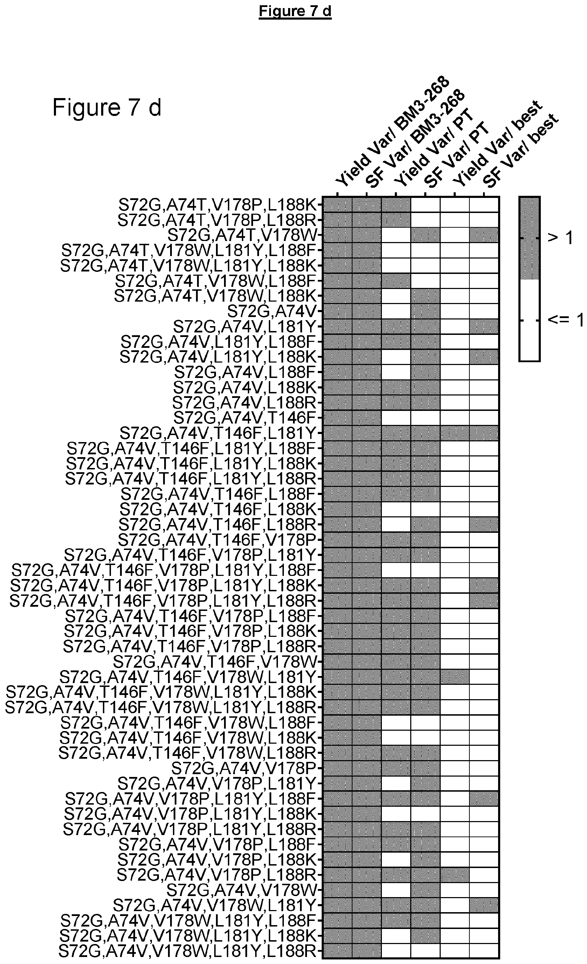

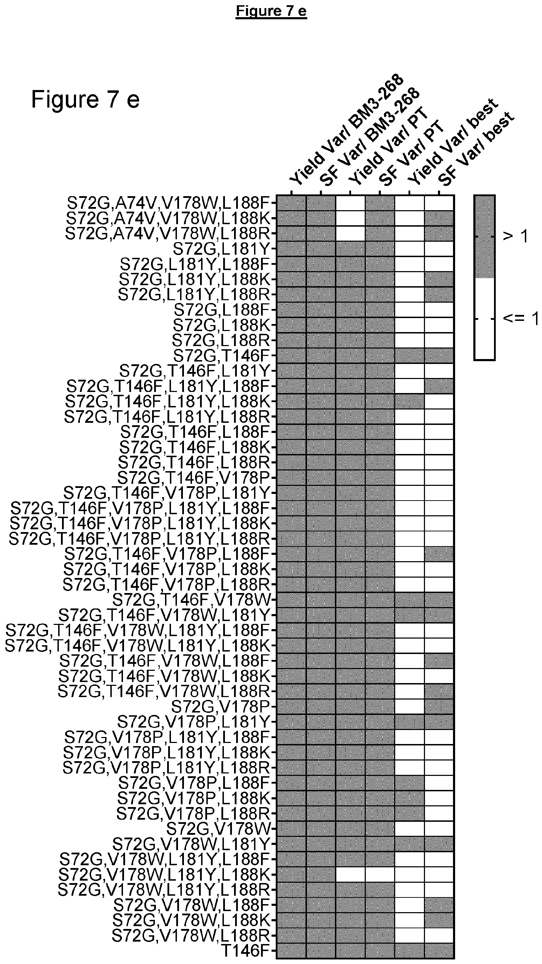

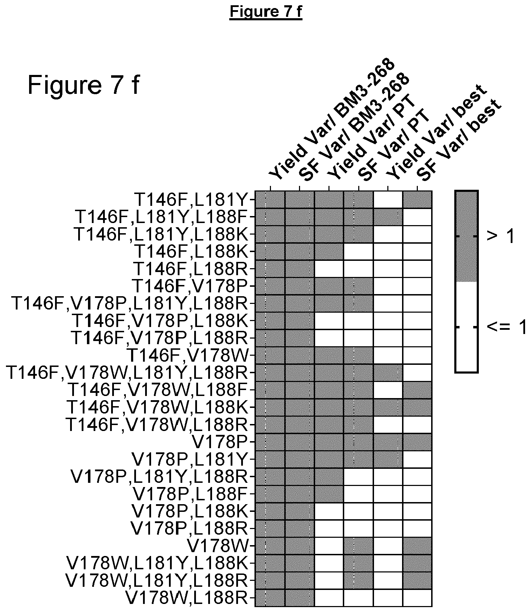

**Figure 7 a, b, c, d, e, f** show product yield and selectivity factor for estrone obtained in biotransformation reactions with BM3-268; M177Y, A184Y (SEQ ID No. 15) variants (Var) having up to six mutations. 100 mg/L ADD were used as substrate. Each variant was compared to BM3-268 (SEQ ID No. 5) and improved variant BM3-268; M177Y, A184Y (PT) (SEQ ID No. 15). Furthermore, the last two columns show whether a variant (having the depicted set of mutations) was improved in yield or selectivity (SF) compared to the best analyzed variant (best) from a set of variants comprising the same subset of mutations but at least one mutation less.

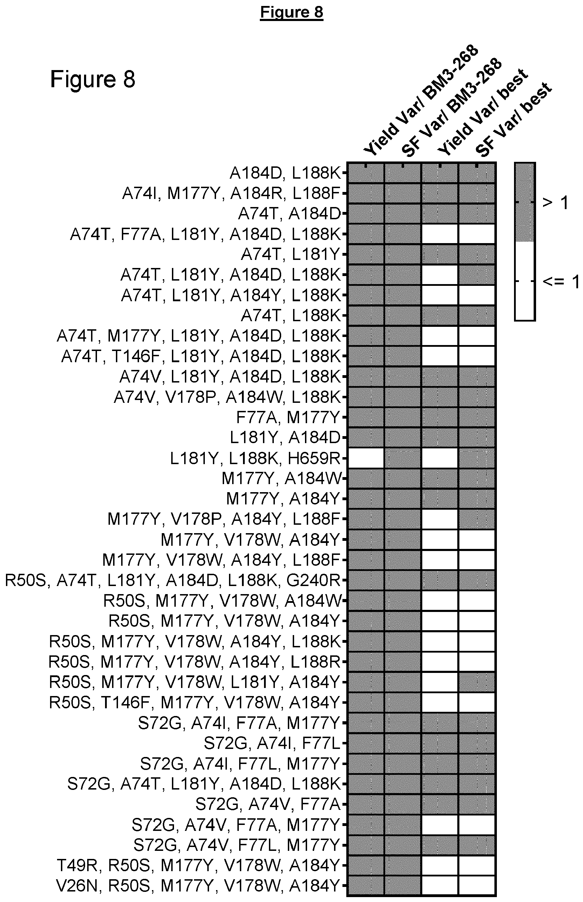

**Figure 8** shows product yield and selectivity (SF) for estrone obtained in biotransformation reactions with BM3-268 variants having multiple mutations. 100 mg/L ADD were used as substrate. Each variant was compared to BM3-268 (SEQ ID No. 5) with regard to product yield and selectivity (columns 1 and 2). Furthermore, columns 3 and 4 show whether a variant (having the depicted set of mutations) was improved in yield or selectivity (SF) compared to the best analyzed variant (best) from a set of variants comprising the same subset of mutations but at least one mutation less.

### BRIEF DESCRIPTION OF THE SEQUENCE IDs

The Sequence Listing associated with this application is filed in electronic format and hereby incorporated by reference into the specification in its entirety.

| SEQ ID NO. | VARIANT | MUTATIONS | TYPE |
|---|---|---|---|
| 1 | WT | | PRT |
| 2 | BM3-254 | V78Y, A82E, F87A | PRT |
| 3 | BM3-261 | V78M, A82E, F87A | PRT |
| 4 | BM3-263 | V78I, A82E, F87A | PRT |
| 5 | BM3-268 | V78L, A82E, F87A | PRT |
| 6 | | V78Y, A82P, F87A | PRT |
| 7 | | V78M, A82P, F87A | PRT |
| 8 | | V78I, A82P, F87A | PRT |
| 9 | | V78L, A82P, F87A | PRT |
| 10 | | V78Y, A82Q, F87A | PRT |
| 11 | | V78M, A82Q, F87A | PRT |
| 12 | | V78I, A82Q, F87A | PRT |
| 13 | | V78L, A82Q, F87A | PRT |
| 14 | | V78F, A82E, F87A, M177Y, A184Y | PRT |
| 15 | | V78L, A82E, F87A, M177Y, A184Y | PRT |
| 16 | | V78I, A82E, F87A, M177Y, A184Y | PRT |
| 17 | | V78M, A82E, F87A, M177Y, A184Y | PRT |
| 18 | | V78Y, A82E, F87A, M177Y, A184Y | PRT |
| 19 | | V78F, A82P, F87A, M177Y, A184Y | PRT |
| 20 | | V78L, A82P, F87A, M177Y, A184Y | PRT |
| 21 | | V78I, A82P, F87A, M177Y, A184Y | PRT |
| 22 | | V78M, A82P, F87A, M177Y, A184Y | PRT |
| 23 | | V78Y, A82P, F87A, M177Y, A184Y | PRT |
| 24 | | V78F, A82Q, F87A, M177Y, A184Y | PRT |
| 25 | | V78L, A82Q, F87A, M177Y, A184Y | PRT |
| 26 | | V78I, A82Q, F87A, M177Y, A184Y | PRT |
| 27 | | V78M, A82Q, F87A, M177Y, A184Y | PRT |
| 28 | | V78Y, A82Q, F87A, M177Y, A184Y | PRT |
| 29 | | V78F, A82E, F87A, M177Y | PRT |
| 30 | | V78L, A82E, F87A, M177Y | PRT |
| 31 | | V78I, A82E, F87A, M177Y | PRT |
| 32 | | V78M, A82E, F87A, M177Y | PRT |
| 33 | | V78Y, A82E, F87A, M177Y | PRT |
| 34 | | V78F, A82P, F87A, M177Y | PRT |
| 35 | | V78L, A82P, F87A, M177Y | PRT |
| 36 | | V78I, A82P, F87A, M177Y | PRT |
| 37 | | V78M, A82P, F87A, M177Y | PRT |
| 38 | | V78Y, A82P, F87A, M177Y | PRT |
| 39 | | V78F, A82Q, F87A, M177Y | PRT |
| 40 | | V78L, A82Q, F87A, M177Y | PRT |
| 41 | | V78I, A82Q, F87A, M177Y | PRT |
| 42 | | V78M, A82Q, F87A, M177Y | PRT |
| 43 | | V78Y, A82Q, F87A, M177Y | PRT |
| 44 | | V78F, A82E, F87A, A184Y | PRT |
| 45 | | V78L, A82E, F87A, A184Y | PRT |
| 46 | | V78I, A82E, F87A, A184Y | PRT |
| 47 | | V78M, A82E, F87A, A184Y | PRT |
| 48 | | V78Y, A82E, F87A, A184Y | PRT |
| 49 | | V78F, A82P, F87A, A184Y | PRT |
| 50 | | V78L, A82P, F87A, A184Y | PRT |
| 51 | | V78I, A82P, F87A, A184Y | PRT |
| 52 | | V78M, A82P, F87A, A184Y | PRT |
| 53 | | V78Y, A82P, F87A, A184Y | PRT |
| 54 | | V78F, A82Q, F87A, A184Y | PRT |
| 55 | | V78L, A82Q, F87A, A184Y | PRT |
| 56 | | V78I, A82Q, F87A, A184Y | PRT |
| 57 | | V78M, A82Q, F87A, A184Y | PRT |
| 58 | | V78Y, A82Q, F87A, A184Y | PRT |
| 59 | WT | | NUC |
| 60 | BM3-254 | V78Y, A82E, F87A | NUC |
| 61 | BM3-261 | V78M, A82E, F87A | NUC |
| 62 | BM3-263 | V78I, A82E, F87A | NUC |
| 63 | BM3-268 | V78L, A82E, F87A | NUC |
| 64 | | V78Y, A82P, F87A | NUC |
| 65 | | V78M, A82P, F87A | NUC |
| 66 | | V78I, A82P, F87A | NUC |
| 67 | | V78L, A82P, F87A | NUC |
| 68 | | V78Y, A82Q, F87A | NUC |
| 69 | | V78M, A82Q, F87A | NUC |
| 70 | | V78I, A82Q, F87A | NUC |
| 71 | | V78L, A82Q, F87A | NUC |
| 72 | | V78F, A82E, F87A, M177Y, A184Y | NUC |
| 73 | | V78L, A82E, F87A, M177Y, A184Y | NUC |
| 74 | | V78I, A82E, F87A, M177Y, A184Y | NUC |
| 75 | | V78M, A82E, F87A, M177Y, A184Y | NUC |
| 76 | | V78Y, A82E, F87A, M177Y, A184Y | NUC |
| 77 | | V78F, A82P, F87A, M177Y, A184Y | NUC |
| 78 | | V78L, A82P, F87A, M177Y, A184Y | NUC |
| 79 | | V78I, A82P, F87A, M177Y, A184Y | NUC |
| 80 | | V78M, A82P, F87A, M177Y, A184Y | NUC |
| 81 | | V78Y, A82P, F87A, M177Y, A184Y | NUC |
| 82 | | V78F, A82Q, F87A, M177Y, A184Y | NUC |
| 83 | | V78L, A82Q, F87A, M177Y, A184Y | NUC |
| 84 | | V78I, A82Q, F87A, M177Y, A184Y | NUC |
| 85 | | V78M, A82Q, F87A, M177Y, A184Y | NUC |
| 86 | | V78Y, A82Q, F87A, M177Y, A184Y | NUC |
| 87 | | V78F, A82E, F87A, M177Y | NUC |
| 88 | | V78L, A82E, F87A, M177Y | NUC |
| 89 | | V78I, A82E, F87A, M177Y | NUC |
| 90 | | V78M, A82E, F87A, M177Y | NUC |
| 91 | | V78Y, A82E, F87A, M177Y | NUC |
| 92 | | V78F, A82P, F87A, M177Y | NUC |
| 93 | | V78L, A82P, F87A, M177Y | NUC |
| 94 | | V78I, A82P, F87A, M177Y | NUC |
| 95 | | V78M, A82P, F87A, M177Y | NUC |
| 96 | | V78Y, A82P, F87A, M177Y | NUC |
| 97 | | V78F, A82Q, F87A, M177Y | NUC |
| 98 | | V78L, A82Q, F87A, M177Y | NUC |
| 99 | | V78I, A82Q, F87A, M177Y | NUC |
| 100 | | V78M, A82Q, F87A, M177Y | NUC |
| 101 | | V78Y, A82Q, F87A, M177Y | NUC |
| 102 | | V78F, A82E, F87A, A184Y | NUC |
| 103 | | V78L, A82E, F87A, A184Y | NUC |
| 104 | | V78I, A82E, F87A, A184Y | NUC |
| 105 | | V78M, A82E, F87A, A184Y | NUC |
| 106 | | V78Y, A82E, F87A, A184Y | NUC |
| 107 | | V78F, A82P, F87A, A184Y | NUC |
| 108 | | V78L, A82P, F87A, A184Y | NUC |
| 109 | | V78I, A82P, F87A, A184Y | NUC |
| 110 | | V78M, A82P, F87A, A184Y | NUC |
| 111 | | V78Y, A82P, F87A, A184Y | NUC |
| 112 | | V78F, A82Q, F87A, A184Y | NUC |
| 113 | | V78L, A82Q, F87A, A184Y | NUC |
| 114 | | V78I, A82Q, F87A, A184Y | NUC |
| 115 | | V78M, A82Q, F87A, A184Y | NUC |
| 116 | | V78Y, A82Q, F87A, A184Y | NUC |

### DETAILED DESCRIPTION

### DEFINITIONS

Unless otherwise defined, all scientific and technical terms used in the description, figures and claims have their ordinary meaning as commonly understood by one of ordinary skill in the art. All publications, patent applications, patents, and other references mentioned herein are incorporated by reference in their entirety. In case of conflict, the present specification, including definitions, will prevail. If two or more documents incorporated by reference include conflicting and/or inconsistent disclosure with respect to each other, then the document having the later effective date shall control. The materials, methods, and examples are illustrative only and not intended to be limiting. Unless stated otherwise, the following terms used in this document, including the description and claims, have the definitions given below.

The terms **"comprising", "including", "containing", "having"** etc. shall be read expansively or open-ended and without limitation. Singular forms such as **"a", "an"** or **"the"** include plural references unless the context clearly indicates otherwise. Unless otherwise indicated, the term **"at least"** preceding a series of elements is to be understood to refer to every element in the series. The terms **"at least one"** and **"at least one of"** include for example, one, two, three, four, five, six, seven, eight, nine, ten or more elements.

It is furthermore understood that slight variations above and below a stated range can be used to achieve substantially the same results as a value within the range. Also, unless indicated otherwise, the disclosure of ranges is intended as a continuous range including every value between the minimum and maximum values.

Where protein or amino acid sequences are provided throughout the application it is also understood by the skilled person that single or multiple amino acids may be exchanged by amino acids with similar properties to achieve substantially the same effect, i.e.an equivalent result. The skilled person furthermore knows that a defined protein or amino acid sequence may be encoded by various nucleic acid sequences. For a given amino acid sequence as defined herein, each of the countable nucleic acid sequences encoding the specific amino acid sequence shall be deemed to be disclosed herein. Where nucleic acid sequences are provided throughout the application it is furthermore understood that silent mutations may be introduced.

The terms **"cytochrome P450 BM3 monooxygenase", "cytochrome P450-BM3", "P450-BM3"** (also **BM3, BM-3**) refer to the cytochrome P450 enzyme obtained from *Bacillus megaterium* that catalyzes the hydroxylation of long-chain fatty acids, alcohols, and amides, as well as the epoxidation of unsaturated fatty acids. Without being bound by theory, P450-BM3 is naturally expressed by *Bacillus megaterium strains ATCC 14581, DSM 32, JCM 2506, NBRC 15308, NCIMB 9376*, *NCTC 10342, VKM B-512.* Alternative names comprise *"Bifunctional cytochrome P450*/*NADPH--P450 reductase".* The P450-BM3 protein is encoded by the gene cyp102A1. Sequence(s) are accessible via UniProt Identifier P14779 (CPXB_BACMB). Different isoforms and variants may exist for the different strains and are all comprised by the term. Where a specific mutation can be exchanged without changing the described catalytic properties of the intitial sequence, it is clear that the sequence having such a functionally silent mutation is equivalent with regard to the initial sequence. In addition, the protein may furthermore be subject to various modifications, e.g, synthetic or naturally occurring modifications.

The gene encoding the cytochrome P450-monooxygenases (P450-BM3) from *Bacillus megaterium* was originally amplified from genomic DNA by PCR and subsequently cloned using restriction endonucleases *NcoI* and *SacI* into the polylinker sequence of plasmid pETM11, resulting in pETM11-BM3 (Kille 2010). This procedure adds a hexa-histidine tag and an 18 amino acid linker sequence to the N-terminus of the resulting protein, which is however not essential for function. Introducing the *NcoI* site at the 5' end of the gene sequence resulted in modification of the second codon of the P450-BM3 gene and to an amino acid exchange in the resulting protein (T1A).

An **amino acid position** is typically given by specifying the number of the respective position behind the amino acid of the wildtype using the one letter code (e.g. V78 where the wildtype has a valine at position 78). A list of comma separated letters in squared brackets specifies the **mutation or amino acid exchange.** For example, where at position 78 of the wildtype variant the valine is replaced by any one of isoleucine, leucine, or methionine, or is not mutated at all, this is abbreviated as V78[I, L, M, V].

Throughout this application **counting of amino acid positions** of the P450-BM3 amino acid sequence starts with the first amino acid that appears in the protein sequence underlying the crystal structure of a P450-BM3 variant (PDB ID: 2UWH) that was used for designing protein libraries. This is also common practice in P450-BM3-related literature, see e.g. (Kille 2010), (Kille Sabrina 2011), (Acevedo-Rocha 2018). The N-terminal methionine is ignored such that counting starts with threonine 1, which was converted to an alanine in the cloning process of pETM11-BM3 and which is codon 28 according to the SEQ IDs listed herein.

The term **"P450-BM3 variant"** refers to a **P450-BM3** comprising at least one mutation relative to the wildtype sequence. In particular, the term refers to proteins having at least 60 %, 70 %, 80 % or 90 % sequence identity with the P450-BM3 wildtype (SEQ ID No. 1). Preferably, P450-BM3 variants are functional, i.e. catalytically active with regard to at least one substrate. Where mutations of a variant are specified, these mutations are restrictive, i.e. a BM3-268; M177Y, A184Y (SEQ ID No. 15) variant having up to six mutations is required to have the mutations M177Y and A184Y.

A **"daughter variant"** of a **"parent variant"** is a variant having the sequence of its parent variant and at least one further mutation. A daughter variant can have multiple parent variants.

According to the IUPAC definition **"steroids"** are naturally occurring compounds and synthetic analogues, which are based on the cyclopenta[a]phenanthrene carbon skeleton, and can be partially or completely hydrogenated. Usually but not necessarily, there are methyl groups at C-10 and C-13, and often but not necessarily there is an alkyl group at C-17. By extension, one or more bond scissions, ring expansions and/or ring contractions of the skeleton may or may not have occurred (IUPAC kein Datum). Throughout this application, steroids are numbered and rings are lettered as in the following formula:

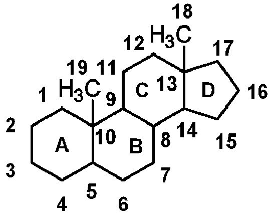

When the rings of a steroid are denoted as projections onto the plane of the paper, the formula is normally to be oriented as in the follwing formula:

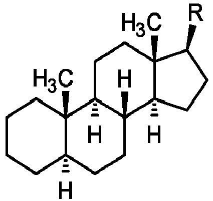

An atom or group attached to a ring depicted as in this orientation is termed **"alpha"** if it lies below the plane of the paper or **"beta"** if it lies above the plane of the paper. Examples for steroids include androgens, estrogens, and progestogens, corticosteroids, glucocorticoids, mineralocorticoids, cholesterol, estradiol, testosterone, dexamethasone, lanosterol, progesterone, medrogestone, β-sitosterol.

The term **steroid derivative** refers to molecules derived from a steroid, i.e. molecules comprising a cyclopenta[a]phenanthrene carbon skeleton.

The term **"C19 hydroxylation"** refers to the introduction of a hydroxyl group (-OH) into a steroid or steroid derivative at the position C19.

The **"selectivity factor"** of a reaction is calculated by dividing the measured product concentration by the consumed substrate concentration (which is the difference between the initial substrate concentration and the measured substrate concentration after the biotransformation process). Product and substrate concentration can be measured as known in the art and as described in the examples. Usual methods comprise HPLC analyses using substrate and product standards as references and for quantification. If not specified otherwise the selectivity factor for the C19 hydroxylation of at least one steroid or derivative thereof is calculated based on the steroid or derivative thereof (for the consumed substrate concentration) and the C19 hydroxylated steroid or derivative thereof (for the product concentration).

The **"product yield"** is calculated by dividing the measured product concentration by the initial substrate concentration in the experiment. The product yield for a given P450-BM3 variant is therefore specific for a given substrate and a given product. For example, the substrate may be a steroid and the product may be the C19 hydroxylated steroid. Where the C19 hydroxylated steroid is further converted into a secondary product, product yield may also be calculated with regard to the commercially relevant product.

The term **"expression system"** refers to any protein production system known in the art and suitable for production of P450-BM3 variants according to the current invention. Suitable expression systems are well known in the art and example systems comprise *E. coli* strains such as DH5alpha, BL21 (DE3), or Rosetta (DE3), but also various other bacterial and non bacterial systems. Suitable expression systems include gram positive bacteria, such as *Bacillus* or *Rhodococcus,* such as *Bacillus megaterium* or *Bacillus subtilis.*

The term **"vector",** as used herein, refers to a nucleic acid molecule capable of propagating a nucleic acid molecule to which it is linked. The term further comprises plasmids (non-viral) and viral vectors.

### EMBODIMENTS

According to a **first aspect** of the invention there is provided a Cytochrome P450 BM3 monooxygenase (BM3) variant for catalyzing the C19 hydroxylation of a steroid or steroid derivative.

According to a **first embodiment** of the first aspect, the BM3 variant comprises the mutation F87A and at least one and preferably two further mutations selected from
(i) a mutation at position V78, preferably V78F, V78Y, V78M, V78I or V78L,
(ii) a mutation at position A82, preferably A82E, A82Q or A82P,
and optionally further mutations.

For example the mutation according to (i) can be V78F and the mutation according to (ii) can be A82E, A82Q or A82P. For example the mutation according to (i) can be V78Y and the mutation according to (ii) can be A82E, A82Q or A82P. For example the mutation according to (i) can be V78M and the mutation according to (ii) can be A82E, A82Q or A82P. For example the mutation according to (i) can be V78I and the mutation according to (ii) can be A82E, A82Q or A82P. For example the mutation according to (i) can be V78L and the mutation according to (ii) can be A82E, A82Q or A82P. For example the mutation according to (ii) can be A82E and the mutation according to (i) can be V78F, V78Y, V78M, V78I or V78L. For example the mutation according to (ii) can be A82Q and the mutation according to (i) can be V78F, V78Y, V78M, V78I or V78L. For example the mutation according to (ii) can be A82P and the mutation according to (i) can be V78F, V78Y, V78M, V78I or V78L.

The optional further mutations may comprise a mutation at positon M177 and/or A184. For example the mutations at positon M177 and/or A184 can be M177Y and/or A184Y.

Generation of enzyme variants according to the current invention may be performed as known in the art. For example, mutations can be introduced into the nucleic acid sequences which encode the BM3 wildtype or BM3 variants by any means appropriate for replacing nucleotides in nucleic acid sequences. A useful method for preparing a mutated nucleic acid sequence according to the invention and the corresponding protein comprises carrying out site-directed mutagenesis on codons encoding one or more amino acids which are selected in advance, thereby changing the selected codons in a way that they encode for different amino acids. The methods for obtaining these site-directed mutations are well known to the skilled person and widely described in the literature (in particular: (McPherson 1991)). Various kits are commercially available, for example the QUIKCHANGE™ lightening mutagenesis kit from Qiagen or Stratagene.

According to a second **embodiment** of the first aspect, there are provided variants which may or may not be variants according to the first embodiment. The P450-BM3 variants according to the second embodiment comprise
(i) at least the mutations
   a) V78Y, A82E and F87A (BM3-254, SEQ ID No. 2),
   b) V78M, A82E and F87A (BM3-261, SEQ ID No. 3),
   c) V78I, A82E and F87A (BM3-263, SEQ ID No. 4),
   d) V78L, A82E and F87A (BM3-268, SEQ ID No. 5).
   e) V78Y, A82P and F87A (SEQ ID No. 6),
   f) V78M, A82P and F87A (SEQ ID No. 7),
   g) V78I, A82P and F87A (SEQ ID No. 8),
   h) V78L, A82P and F87A (SEQ ID No. 9),
   i) V78Y, A82Q and F87A (SEQ ID No. 10),
   j) V78M, A82Q and F87A (SEQ ID No. 11),
   k) V78I, A82Q and F87A (SEQ ID No. 12), or
   l) V78L, A82Q and F87A (SEQ ID No. 13),
   and at least one, two, three, four, five, six, seven, eight, nine or ten further mutation(s), or
(ii) at least the mutations
   m) V78F, A82E, F87A, M177Y and A184Y (SEQ ID No. 14),
   n) V78L, A82E, F87A, M177Y and A184Y (SEQ ID No. 15),
   o) V78I, A82E, F87A, M177Y and A184Y (SEQ ID No. 16),
   p) V78M, A82E, F87A, M177Y and A184Y (SEQ ID No. 17),
   q) V78Y, A82E, F87A, M177Y and A184Y (SEQ ID No. 18),
   r) V78F, A82P, F87A, M177Y and A184Y (SEQ ID No. 19),
   s) V78L, A82P, F87A, M177Y and A184Y (SEQ ID No. 20),
   t) V78I, A82P, F87A, M177Y and A184Y (SEQ ID No. 21),
   u) V78M, A82P, F87A, M177Y and A184Y (SEQ ID No. 22),
   v) V78Y, A82P, F87A, M177Y and A184Y (SEQ ID No. 23),
   w) V78F, A82Q, F87A, M177Y and A184Y (SEQ ID No. 24),
   x) V78L, A82Q, F87A, M177Y and A184Y (SEQ ID No. 25),
   y) V78I, A82Q, F87A, M177Y and A184Y (SEQ ID No. 26),
   z) V78M, A82Q, F87A, M177Y and A184Y (SEQ ID No. 27), or
   aa) V78Y, A82Q, F87A, M177Y and A184Y (SEQ ID No. 28),
   and optionally further mutation(s).

**Some of the variants according to the second embodiment of the first aspect** comprise at least one further mutation, preferably at least one, two, three, four, five, six, seven, eigth, nine, ten, eleven, twelve, thirteen, fourteen, fifteen or more further mutations.

While the P450-BM3 wild type as well as hundreds of tested variants from literature or generated *de novo* were not able to catalyze the C19 hydroxylation of steroids or steroid derivatives, the variants according to the first aspect were surprisingly found to be suitable catalysts for the efficient C19 hydroxylation of steroids or derivatives thereof.

Without being bound by theory, the described variants according to the aspect at hand, in particular the variants according to SEQ ID No. 2 to 28 are technically related, such that they induce a tertiary protein structure which enables not only the binding of steroids but also their efficient C19 hydroxylation, without supporting side reactions, such as other oxidation reactions, e.g. as mentioned herein.

In brief, the P450-BM3 variants according to the first aspect recognize steroids as substrates, in particular steroids comprising a 1,4-dien-3-one-A-ring, or steroids comprising a 4-en-3-one-A-ring. Furthermore, the P450-BM3 variants according to the first aspect can catalyze the C19 hydroxylation of these steroids.

Without being bound by theory, for steroids with a **1,4-dien-3-one-A-ring** such as (17beta)-17-hydroxyandrosta-1,4-dien-3-one (synonyms: 1-dehydrotestosterone, Δ1-testosterone, boldenone) or androsta-1,4-dien-3,17-dione (synonym: ADD) a C19-hydroxylation is believed to lead to an instable intermediate undergoing immediate aromatization and formation of estradiol or estrone and their derivatives.

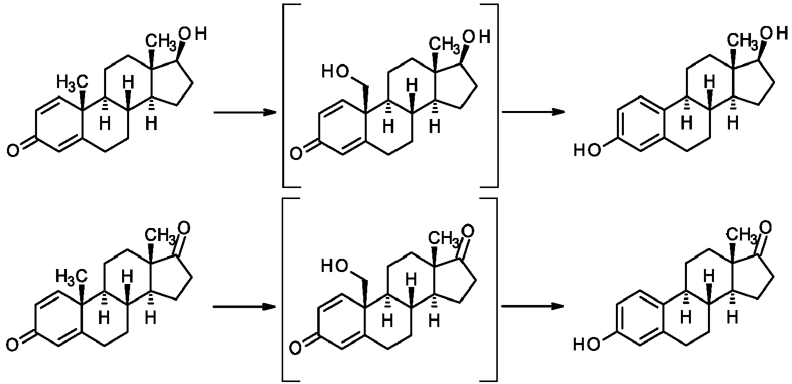

Example 8, Table E2 shows the obtained screening yields of estradiol based on delta-1-testosterone as a substrate for P450-BM3 variants BM3-254 (SEQ ID No. 2), BM3-261 (SEQ ID No. 3), BM3-263 (SEQ ID No. 4) and BM3-268 (SEQ ID No. 5).

Without being bound by theory, for steroids with a **4-en-3-one-A-ring** like (17beta)-17-hydroxyandrost-4-en-3-one (synonym: testosterone) the product of C19-hydroxylation is typically stable and can be isolated. These kind of products can then by further functionalization under dehydrogenation form the same instable intermediate and subsequently under aromatization estradiol and estradiol derivatives (as shown for similar estrone derivatives by (F. Templeton 1997).

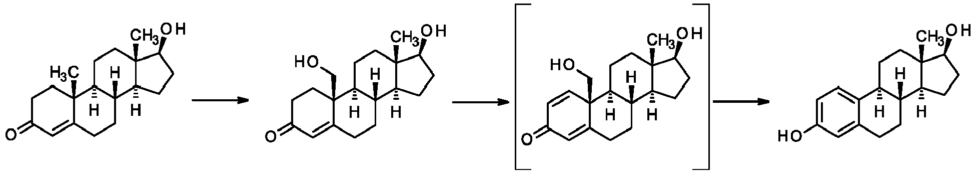

Example 9, Table E5 shows the obtained screening yields for C19 hydroxylated products based on testosterone as a substrate for P450-BM3 variants BM3-254 (SEQ ID No. 2), BM3-261 (SEQ ID No. 3), BM3-263 (SEQ ID No. 4) and BM3-268 (SEQ ID No. 5).

In consequence, the described P450-BM3 variants are suitable catalysts for the C19-hydroxylation of steroids, thereby enabling production of compounds of formula (I) 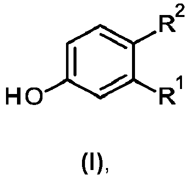 wherein R¹ and R² form a six-membered ring as part of a steroid. In particular, the described P450-BM3 variants according to the first aspect are suitable catalysts for the C19-hydroxylation of steroids, thereby enabling production of compounds of formula (II) 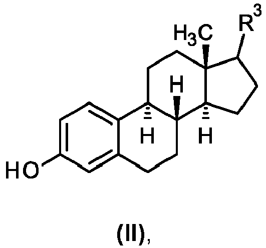 wherein R³ is a beta-hydroxy group (-OH) or an oxo group (=O).

According to some **highly preferred embodiments** the P450-BM3 variants according to the first aspect catalyze the C19-hydroxylation of steroids for the production of compounds of formula (I) wherein the formula (I) is preferably formula (II).

Without being bound by theory, by using the P450-BM3 variants as catalysts, the compounds of formula (I) and (II) can thus be synthesized in the following ways:

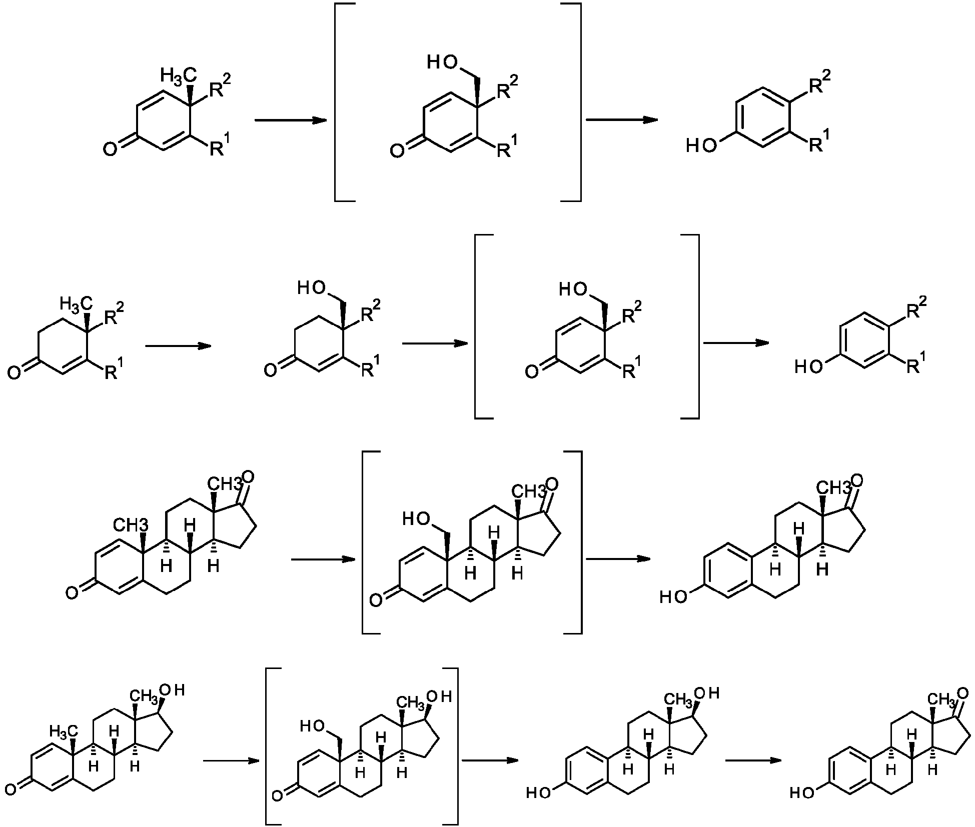

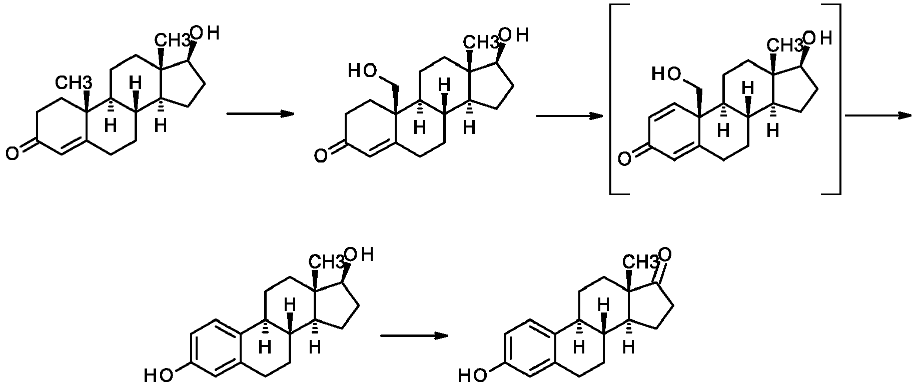

Estradiol and estradiol derivatives can be oxidized to estrone and estrone derivatives as described by (Kawahara R 2012).

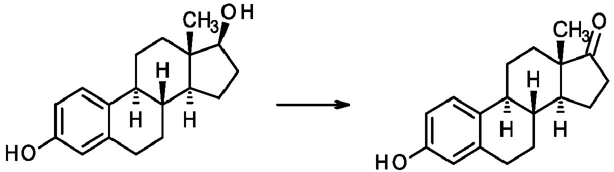

According to a **third embodiment of the first aspect**, there are provided variants which are characterized by a product yield for at least one C19 hydroxylated steroid or derivative thereof or a secondary product thereof which is higher than 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 92, 92, 93, 94 or 95 %.

For example, said product yield for the at least one C19 hydroxylated steroid or derivative thereof, or a secondary product thereof can be > 5 %, preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %. For example, said product yield can be the product yield for the product estradiole or estrone, or derivatives thereof. For example, said steroid or steroid derivative can be a a steroid or steroid derivative comprising a 1,4-dien-3-one-A-ring or a 4-en-3-one-A-ring, such as d1-testosterone or ADD.

The third embodiment according to the first aspect can be and is suggested to be combined with each embodiment or example according to the first aspect and in particular the first, second, fourth, fifth or sixth embodiment of the first aspect.

For example there is provided a BM3 variant comprising the mutation F87A and at least one and preferably two further mutations selected from
(i) a mutation at position V78, preferably V78F, V78Y, V78M, V78I or V78L,
(ii) a mutation at position A82, preferably A82E, A82Q or A82P,
and optionally comprising further mutations, said BM3 variant having a product yield for the at least one C19 hydroxylated steroid or derivative thereof or a secondary product thereof > 5 %, preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %.

For example, the BM3 variant may comprise the mutations M177Y and/or A184Y.

For example there is provided a BM3 variant comprising
(i) at least the mutations
   a) V78Y, A82E and F87A (BM3-254, SEQ ID No. 2),
   b) V78M, A82E and F87A (BM3-261, SEQ ID No. 3),
   c) V78I, A82E and F87A (BM3-263, SEQ ID No. 4),
   d) V78L, A82E and F87A (BM3-268, SEQ ID No. 5).
   e) V78Y, A82P and F87A (SEQ ID No. 6),
   f) V78M, A82P and F87A (SEQ ID No. 7),
   g) V78I, A82P and F87A (SEQ ID No. 8),
   h) V78L, A82P and F87A (SEQ ID No. 9),
   i) V78Y, A82Q and F87A (SEQ ID No. 10),
   j) V78M, A82Q and F87A (SEQ ID No. 11),
   k) V78I, A82Q and F87A (SEQ ID No. 12), or
   l) V78L, A82Q and F87A (SEQ ID No. 13),
   and at least one, two, three, four, five, six, seven, eight, nine or ten further mutation(s), or
(ii) at least the mutations
   m) V78F, A82E, F87A, M177Y and A184Y (SEQ ID No. 14),
   n) V78L, A82E, F87A, M177Y and A184Y (SEQ ID No. 15),
   o) V78I, A82E, F87A, M177Y and A184Y (SEQ ID No. 16),
   p) V78M, A82E, F87A, M177Y and A184Y (SEQ ID No. 17),
   q) V78Y, A82E, F87A, M177Y and A184Y (SEQ ID No. 18),
   r) V78F, A82P, F87A, M177Y and A184Y (SEQ ID No. 19),
   s) V78L, A82P, F87A, M177Y and A184Y (SEQ ID No. 20),
   t) V78I, A82P, F87A, M177Y and A184Y (SEQ ID No. 21),
   u) V78M, A82P, F87A, M177Y and A184Y (SEQ ID No. 22),
   v) V78Y, A82P, F87A, M177Y and A184Y (SEQ ID No. 23),
   w) V78F, A82Q, F87A, M177Y and A184Y (SEQ ID No. 24),
   x) V78L, A82Q, F87A, M177Y and A184Y (SEQ ID No. 25),
   y) V78I, A82Q, F87A, M177Y and A184Y (SEQ ID No. 26),
   z) V78M, A82Q, F87A, M177Y and A184Y (SEQ ID No. 27), or
   aa) V78Y, A82Q, F87A, M177Y and A184Y (SEQ ID No. 28),
and optionally further mutation(s),
said BM3 variant having a product yield for at least one C19 hydroxylated steroid or derivative thereof or a secondary product thereof > 5 %, preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %.

**Table A1 shows estradiol product yields for d1-testosterone to estradiol conversion from 50 mg/L substrate for variants derived from the parent type V78L, A82E, F87A (SEQ ID No. 5) having further mutations as depicted.**

| **Mutation** | **Yield Estradiol [%]** | **Mutation** | **Yield Estradiol [%]** | **Mutation** | **Yield Estradiol [%]** | **Mutation** | **Yield Estradiol [%]** |
|---|---|---|---|---|---|---|---|
| A74I | 52.8 | V178M | 10.9 | L181I | 7.7 | M177G | 6.0 |
| A74V | 42.2 | A184Q | 10.8 | A399Q | 7.7 | M118K | 6.0 |
| S72G, A74V | 40.2 | A321G | 10.7 | R50D | 7.7 | L29Y | 6.0 |
| F77A | 38.3 | P392Q | 10.5 | M177A | 7.6 | A399L | 6.0 |
| A184G | 37.4 | A399E | 10.5 | S72C, A74C | 7.6 | M177I | 6.0 |
| M177Y | 36.5 | M177F | 10.4 | S89V | 7.6 | T49H | 6.0 |
| V178P | 36.3 | T365I | 10.3 | R50S | 7.5 | R47C | 6.0 |
| A184Y | 34.2 | L75V, F81L | 10.3 | V371A | 7.5 | I401V | 5.9 |
| A74T | 33.3 | F77P | 10.2 | E143I | 7.5 | T146A | 5.9 |
| A184R | 31.5 | V314P | 10.2 | T49Q | 7.5 | S176L | 5.9 |
| R50S, A74V | 30.7 | S72G, A74Y | 10.2 | T365G | 7.5 | S72G, A74F | 5.9 |
| S72G, A74I | 30.2 | S89E | 10.1 | F77M | 7.5 | K224W | 5.9 |
| A74C | 29.2 | M118E | 10.0 | T146D | 7.5 | E267T | 5.9 |
| M185V | 29.1 | T146R | 10.0 | R47H | 7.4 | M185D | 5.8 |
| L188K | 27.0 | V26I | 9.9 | R255K | 7.4 | Y51L | 5.8 |
| A330R | 26.3 | E64Q | 9.9 | E143V | 7.4 | M118S | 5.8 |
| K224P | 26.0 | W90Y | 9.7 | V314F | 7.3 | M118T | 5.8 |
| V48H | 25.9 | L29T | 9.6 | A330F | 7.3 | A399G | 5.8 |
| V26N | 25.9 | T327V | 9.6 | Y51W | 7.3 | A330C | 5.8 |
| A184E | 24.9 | F81W | 9.6 | M118A | 7.3 | K224Y | 5.8 |
| F77V | 24.8 | H266N | 9.6 | T49D | 7.3 | S89G | 5.7 |
| A184W | 24.7 | A184F | 9.6 | E267D | 7.2 | S72N, A74I | 5.7 |
| A74M | 24.2 | V48S | 9.5 | F261Y | 7.2 | S72C, A74L | 5.7 |
| F77S | 24.1 | M177T | 9.5 | T365L | 7.2 | E143Q | 5.7 |
| A74L | 23.8 | G85A | 9.5 | W90D | 7.2 | R47D | 5.7 |
| V26H | 23.5 | M118Y | 9.5 | L29M | 7.2 | A264V | 5.6 |
| F77L | 23.0 | L437I | 9.5 | L75I | 7.2 | R47M | 5.6 |
| A184D | 22.3 | R50Y | 9.4 | V26A | 7.2 | N70R | 5.6 |
| M177V | 21.9 | M118W | 9.4 | T49W | 7.2 | I401M | 5.6 |
| F77T | 20.5 | A74R, L75V | 9.3 | R50P | 7.2 | F393W | 5.6 |
| T146F | 20.3 | E64V | 9.3 | L29V | 7.2 | A74D, F81V | 5.6 |
| V178W | 20.0 | A321N | 9.2 | R47T | 7.1 | G415D | 5.6 |
| L188R | 19.7 | M118L | 9.2 | S72N, A74C | 7.1 | L75I, F81I | 5.6 |
| A74F | 19.7 | L188A | 9.2 | V48Y | 7.1 | T49G | 5.6 |
| F77K | 19.7 | T365W | 9.2 | T146C | 7.1 | S89N | 5.5 |
| L188N | 19.5 | T49C | 9.2 | E143M | 7.1 | E64A | 5.5 |
| T49R | 19.4 | I263L | 9.1 | V48F | 7.0 | E143G | 5.5 |
| S89T | 19.4 | T146M | 9.1 | R255P | 7.0 | A330N | 5.5 |
| L188W | 19.1 | T49A | 9.1 | R50I | 7.0 | H266E | 5.5 |
| A74W | 19.0 | V48T | 9.0 | H266A | 7.0 | R50F | 5.5 |
| V178Y | 19.0 | M118P | 8.9 | M118V | 7.0 | F77G | 5.4 |
| A74Y | 18.9 | V314E | 8.9 | L29A | 7.0 | R47E | 5.4 |
| M177C | 18.8 | M177L | 8.8 | E143K | 7.0 | R255A | 5.4 |
| A184K | 18.4 | F77Y | 8.8 | E64Y | 6.9 | A74V, L75V | 5.4 |
| A184N | 18.2 | R50G | 8.8 | L29I | 6.9 | L78M | 5.4 |
| H266S | 18.1 | R47L | 8.8 | F77D | 6.9 | T146N | 5.3 |
| S89C | 18.1 | R50N | 8.7 | T49F | 6.8 | F261V | 5.3 |
| L188F | 17.9 | F77H | 8.7 | S89I | 6.8 | K224L | 5.3 |
| V178Q | 17.6 | E143F | 8.7 | F81Y | 6.8 | I401A | 5.3 |
| S176P | 17.5 | F261I | 8.7 | V48R | 6.8 | R47W | 5.3 |
| F77E | 17.2 | V178R | 8.7 | R255N | 6.8 | T365D | 5.3 |
| L188I | 17.0 | A184L | 8.7 | V178N | 6.8 | N70F | 5.2 |
| S72W | 16.9 | A74R | 8.6 | S72N, A74V | 6.8 | N70C | 5.2 |
| S72G, A74C | 16.9 | T49L | 8.6 | E267R | 6.7 | S176N | 5.2 |
| A399V | 16.8 | P392G | 8.6 | E143P | 6.7 | L86V | 5.2 |
| L181Y | 16.4 | F77N | 8.6 | L75V | 6.7 | E143N | 5.2 |
| S176G | 15.8 | F261M | 8.6 | R50C | 6.6 | V48M | 5.2 |
| L181P | 15.8 | S89Q | 8.6 | T438V | 6.6 | K224M | 5.1 |
| S176M | 15.4 | T365Q | 8.6 | V48L | 6.6 | A321T | 5.1 |
| G415A | 15.4 | E143C | 8.6 | R47A | 6.6 | A321E | 5.1 |
| F77W | 15.2 | F77I | 8.5 | E143A | 6.6 | E64L | 5.1 |
| T49V | 15.2 | M177H | 8.5 | A330T | 6.5 | G85S | 5.1 |
| M118G | 15.2 | V26M | 8.5 | V48I | 6.5 | T146V | 5.1 |
| A330K | 14.9 | V178S | 8.5 | L75V, F81Y | 6.5 | L75I, F81L | 5.1 |
| K224H | 14.9 | V314A | 8.5 | L86M | 6.5 | M177E | 5.1 |
| S72G, A74L | 14.8 | I263K | 8.5 | S176K | 6.5 | M118N | 5.1 |
| A184H | 14.7 | V178L | 8.5 | S72H, A74C | 6.5 | I401T | 5.0 |
| M177Q | 14.6 | T49N | 8.4 | A321I | 6.5 | | |
| A330W | 14.5 | A330V | 8.4 | L29Q | 6.4 | | |
| L188H | 14.2 | E143D | 8.3 | T365K | 6.4 | | |
| A399I | 14.0 | F77R | 8.3 | S176H | 6.4 | | |
| V178D | 14.0 | S176F | 8.3 | M177D | 6.4 | | |
| T327L | 13.7 | A399N | 8.2 | S89L | 6.4 | | |
| A74H | 13.6 | G415T | 8.2 | E344K | 6.3 | | |
| G415S | 13.5 | R47Q | 8.2 | K224C | 6.3 | | |
| T365V | 13.4 | E64G | 8.1 | F81S | 6.3 | | |
| S176C | 13.1 | V26K | 8.1 | L29H | 6.3 | | |
| V314H | 13.0 | R50M | 8.1 | F261C | 6.3 | | |
| T365Y | 12.8 | A74P | 8.1 | W325F | 6.3 | | |
| S72G, A74S | 12.8 | S176D | 8.1 | F393M | 6.3 | | |
| T365P | 12.7 | L29C | 8.1 | E143Y | 6.3 | | |
| A74S | 12.6 | A321V | 8.1 | A321D | 6.3 | | |
| F77C | 12.6 | R50V | 8.0 | K224I | 6.2 | | |
| L188M | 12.5 | E143R | 8.0 | S72G, A74H | 6.2 | | |
| R50Q | 12.5 | V314Q | 8.0 | T365F | 6.2 | | |
| R255I | 12.4 | E64H | 8.0 | V178T | 6.2 | | |
| V178I | 12.4 | P392R | 8.0 | M118H | 6.2 | | |
| V314S | 12.2 | L188S | 8.0 | V26G | 6.2 | | |
| A399M | 12.0 | R47K | 8.0 | F261A | 6.2 | | |
| A74C, L75V | 12.0 | I263N | 7.9 | E344G | 6.2 | | |
| L188D | 11.8 | V178K | 7.9 | S176Q | 6.2 | | |
| T49P | 11.6 | V26L | 7.9 | K224E | 6.1 | | |
| P392V | 11.6 | F81L | 7.8 | A74N | 6.1 | | |
| L188E | 11.5 | S72C, A74V | 7.8 | M177N | 6.1 | | |
| T49I | 11.4 | R50L | 7.8 | V178A | 6.1 | | |
| A399C | 11.4 | V371P | 7.8 | L75I, F81V | 6.1 | | |
| M118F | 11.3 | V48W | 7.8 | R255L | 6.1 | | |
| E143W | 11.1 | V48D | 7.8 | S176E | 6.1 | | |
| L29F | 10.9 | L262I | 7.7 | A330D | 6.1 | | |

**Table A2 shows estradiol product yields for Δ1-testosterone to estradiol conversion from 100 mg/L substrate for variants derived from the parent type V78L, A82E, F87A, M177Y, A184Y (SEQ ID No. 15) having further mutations as depicted.**

| **Mutation** | **Yield Estradiol [%]** | **Mutation** | **Yield Estradiol [%]** | **Mutation** | **Yield Estradiol [%]** | **Mutation** | **Yield Estradiol [%]** |
|---|---|---|---|---|---|---|---|
| L150W | 65.2 | L20R | 39.4 | F205R | 44.0 | L20C | 33.3 |
| L78M | 64.0 | T149P | 39.3 | A330W | 43.8 | T146V, A191T | 33.2 |
| L20W | 59.5 | F205K | 38.8 | F205D | 43.4 | L20Y | 32.3 |
| M212F | 52.2 | W325Y | 38.6 | F205D. D208N | 43.2 | L20M | 31.4 |
| L20F | 52.2 | P9S | 38.3 | L150Y | 43.1 | T49A | 31.3 |
| A330Y | 50.1 | V26Q | 37.0 | T327M | 42.3 | T146A. L272W | 30.8 |
| M212Y | 48.9 | R47S | 36.4 | L20N | 41.9 | T49L | 30.7 |
| E267D | 48.3 | L272V | 36.1 | K210E | 41.7 | L150F | 30.2 |
| L20P | 47.5 | S89T | 35.5 | T146A, L150A | 41.7 | T146L | 28.5 |
| M212W | 47.1 | F205H | 35.1 | R47W | 41.5 | L20D | 28.2 |
| F205Y | 46.8 | W325F | 34.4 | F205E | 41.4 | L20S | 28.1 |
| F205G | 46.4 | E93G | 34.2 | F77V | 41.2 | L20I | 27.7 |
| F205W | 45.0 | T49P | 34.0 | L20T | 40.7 | L20V | 26.6 |
| T146A, F205G | 44.2 | E352N | 33.5 | F205T | 39.7 | T49I | 25.9 |

**Table A3 shows product yield for estradiol obtained in biotransformation reactions with BM3-268; M177Y, A184Y (SEQ ID No. 15) variants having up to six mutations. 100 mg/L Δ1-testosterone were used as substrate.**

| **Mutations** | **Yield EstradioI [%]** | **Mutations** | **Yield Estradio I [%]** |
|---|---|---|---|
| S72G,A74V,T146F,V178W,L181Y | 75.9 | T146F,V178W,L188F | 49.1 |
| S72G,A74T,T146F,V178W,L181Y,L18 8R | 72.8 | A74T,T146F,V178P,L181Y | 47.9 |
| S72G,A74V,T146F,V178W,L188R | 72.3 | S72G,V178W | 47.7 |
| S72G,A74T,T146F,V178W,L188R | 72.2 | V178P | 47.0 |
| S72G,A74V,T146F,L181Y,L188R | 72.1 | T146F,V178W,L188K | 46.1 |
| S72G,T146F,V178W,L188F | 65.9 | S72G,V178W,L181Y | 45.8 |
| S72G,A74V,T146F,L181Y | 65.8 | S72G,A74T,L181Y,L188F | 45.1 |
| S72G,V178P,L181Y | 64.8 | S72G,V178W,L188F | 45.0 |
| A74T,T146F,L181Y,L188R | 63.9 | S72G,L188F | 44.9 |
| S72G,A74I,V178P,L181Y | 62.6 | S72G,A74T,T146F,V178W,L188F | 44.7 |
| S72G,A74V,T146F,V178W | 61.2 | S72G,A74V,T146F,V178P,L181Y,L188R | 44.5 |
| A74I,T146F,V178W | 61.0 | T146F,V178W | 43.7 |
| S72G,T146F,V178W,L188R | 61.0 | S72G | 43.6 |
| S72G,A74I,T146F,L181Y,L188K | 60.7 | S72G,A74T,T146F,L188R | 43.1 |
| S72G,T146F,V178W,L181Y,L188F | 59.0 | S72G,T146F,V178W | 42.9 |
| T146F,V178W,L188R | 58.9 | S72G,A74V,T146F,V178P,L181Y,L188K | 41.9 |
| A74V,V178P,L181Y,L188K | 58.4 | S72G,V178W,L188R | 39.9 |
| T146F,L181Y,L188F | 58.1 | S72G,T146F,L188F | 38.8 |
| S72G,V178P,L181Y,L188F | 57.6 | S72G,A74I,T146F,L181Y,L188F | 35.0 |
| V178P,L181Y | 56.9 | S72G,T146F | 34.7 |
| S72G,V178P,L181Y,L188K | 56.0 | A74V,V178P,L181Y,L188F | 34.4 |
| S72G,A74V,T146F,V178P,L181Y | 56.0 | S72G,A74T,L181Y,L188K | 33.7 |
| S72G,V178P,L181Y,L188R | 55.8 | S72G,A741,T146F,L181Y,L188R | 29.9 |
| S72G,T146F,V178W,L181Y | 55.4 | A74T,T146F,L188K | 29.8 |
| S72G,T146F,V178W,L188K | 55.4 | S72G,A74V,L188R | 29.5 |
| T146F,L181Y | 55.3 | S72G,A74T,T146F,V178P,L181Y | 29.2 |
| S72G,A74T,T146F,L181Y,L188K | 54.8 | S72G,V178W,L188K | 29.1 |
| S72G,A74V,V178P,L181Y,L188K | 54.1 | S72G,A74I,T146F,L181Y | 28.8 |
| S72G,T146F,V178W,L181Y,L188K | 53.9 | S72G,T146F,V178P,L188R | 28.3 |
| A74T,T146F,L181Y,L188K | 53.9 | S72G,A74T,V178P,L188F | 28.2 |
| T146F,V178W,L181Y,L188R | 52.6 | V178W | 28.2 |
| A74V,V178P,L181Y,L188R | 52.1 | S72G,T146F,L181Y,L188R | 26.6 |
| V178P,L181Y,L188R | 52.0 | A74T,T146F,V178P,L188R | 26.2 |
| A74T,T146F,V178W | 51.3 | | |

**Table A4 shows estrone product yields for ADD to estrone conversion from 100 mg/L substrate for variants derived from the parent type BM3-268; M177Y, A184Y (SEQ ID No. 15) having further mutations as depicted.**

| **Mutation(s)** | **Yield Estrone** | **Mutation(s)** | **Yield Estrone** | **Mutation(s)** | **Yield Estrone** |
|---|---|---|---|---|---|
| F205Y | 24.5 | F205K | 8.4 | L272W | 6.3 |
| M212Y | 18.9 | L150G | 8.1 | L272F | 6.3 |
| L150Y | 18.4 | F205P | 8.0 | T146A, L272H | 6.1 |
| M212F | 17.2 | E352I | 7.9 | E352N | 5.8 |
| F205H | 15.8 | L20F | 7.9 | T438V | 5.8 |
| F205R | 15.6 | M212T | 7.6 | E93G | 5.7 |
| L20W | 15.4 | E352V | 7.5 | T49Q | 5.7 |
| F205G | 15.2 | F81D | 7.4 | F205S | 5.6 |
| L78M | 14.0 | L20P | 7.4 | D363G, T438C | 5.5 |
| F205W | 13.2 | F173Y, I174F | 6.8 | W325Y | 5.4 |
| M212W | 13.0 | T49I | 6.7 | M212H | 5.4 |
| K210E | 11.6 | T49R | 6.7 | T438C | 5.3 |
| L20Y | 11.3 | L272Y | 6.7 | L150W | 5.3 |
| T146F | 10.7 | L20R | 6.7 | F205I | 5.1 |
| L150F | 10.5 | F205N | 6.6 | F205V | 5.1 |
| S89T | 10.0 | E352M | 6.6 | F81Y | 5.1 |
| T146A, F205G | 9.7 | R203C | 6.5 | | |
| F205D, D208N | 9.5 | F205D | 6.4 | | |

**Table A5 shows the product yield for estrone obtained in biotransformation reactions with SEQ ID No. 15 derived variants having up to six mutations. 100 mg/L ADD were used as substrate.**

| **Mutation(s)** | **Yield Estrone [%]** | **Mutation(s)** | **Yield Estrone [%]** | **Mutation(s)** | **Yield Estrone [%]** |
|---|---|---|---|---|---|
| S72G,T146F,V178W,L181Y | 66.6 | S72G,V178P,L181Y,L188R | 14.8 | S72G,V178W,L188 R | 7.9 |
| S72G,T146F,V178W,L181Y,L188F | 58.4 | S72G,V178W,L181Y | 14.7 | S72G,T146F,V178P, L188F | 7.6 |
| S72G,T146F,V178W | 55.2 | S72G,A74T,T146F,V178P | 14.6 | S72G,A74I,T146F,L 181Y,L188R | 7.6 |
| S72G,T146F,V178W,L188F | 53.1 | S72G,A74V,V178W,L181Y | 14.6 | S72G,A74V,T146F, V178P,L188R | 7.5 |
| S72G,V178P,L181Y | 38.1 | S72G,A74V,V178P,L188R | 14.1 | S72G,A74I,T146F,V 178P,L188K | 7.5 |
| S72G,T146F,L181Y,L188R | 37.4 | S72G,A74I,T146F,V178P,L181Y | 14.0 | S72G,T146F,V178P, L181Y,L188F | 7.2 |
| S72G,T146F,L181Y,L188F | 37.3 | S72G,T146F | 13.6 | A74T,T146F,V178P, L188K | 7.0 |
| S72G,A74V,T146F,V178W,L181Y | 36.3 | S72G,T146F,V178W,L188K | 13.5 | S72G,A74I,T146F,V 178P,L181Y,L188F | 6.9 |
| S72G,T146F,V178W,L188R | 33.5 | S72G,V178P,L188R | 13.2 | S72G,A74V,T146F,L 181Y,L188K | 6.9 |
| S72G,A74T,T146F,V178W,L181Y,L188R | 30.0 | S72G,A74V,L181Y | 13.1 | S72G,A74I,T146F,V 178P,L188F | 6.9 |
| S72G,A74V,T146F,L181Y | 29.4 | S72G,A74V,T146F,V178P,L181Y,L188R | 13.0 | S72G,L188K | 6.8 |
| S72G,T146F,V178P,L181Y | 29.0 | T146F,L181Y,L188F | 12.9 | S72G,A74V,V178P, L181Y,L188R | 6.6 |
| S72G,A74T,V178P,L181Y | 27.6 | S72G | 12.7 | S72G,T146F,L188K | 6.6 |
| S72G,A74T,T146F,V178W,L181Y | 26.5 | S72G,A74T,T146F,V178P,L181Y | 12.3 | S72G,A74I,T146F,L 181Y | 6.5 |
| S72G,V178P,L181Y,L188F | 25.2 | S72G,L188F | 11.9 | S72G,A74V,T146F, V178W,L188R | 6.3 |
| T146F,L181Y | 25.2 | S72G,A74I,V178P,L181Y | 11.8 | T146F,V178W,L188 R | 6.2 |
| S72G,A74V,T146F,L181Y,L188F | 24.1 | S72G,A74T,V178P,L188F | 11.7 | A741,T146F,L181Y | 6.2 |
| S72G,A74V,V178P,L181Y,L188F | 23.1 | A74T,T146F,V178P,L181Y | 11.5 | A74T,T146F,V178W | 6.2 |
| S72G,A74T,T146F,V178W,L188F | 23.0 | S72G,A74V,T146F,V178W | 11.3 | S72G,A74V,V178P | 6.1 |
| S72G,A74T,T146F,V178W,L181Y,L188K | 22.7 | S72G,T146F,V178P | 11.3 | T146F,V178W,L188 F | 6.0 |
| S72G,T146F,L181Y,L188K | 21.4 | S72G,V178P | 11.1 | V178P | 6.0 |
| S72G,A74V,T146F,V178P,L181Y | 20.8 | A74I,T146F,V178P | 11.0 | S72G,A74V,T146F,L 188F | 5.9 |
| S72G,A74I,T146F,V178W,L181Y | 20.7 | S72G,V178W,L188F | 10.8 | S72G,A74I,T146F,V 178P,L188R | 5.9 |
| S72G,A74V,T146F,L181Y,L188R | 20.6 | T146F | 10.7 | S72G,L188R | 5.9 |
| S72G,A74T,V178P,L181Y,L188K | 20.3 | S72G,A74V,T146F,V178P,L188F | 10.3 | S72G,V178W,L188K | 5.8 |
| T146F,V178W,L181Y,L188R | 19.6 | S72G,V178P,L181Y,L188K | 10.0 | S72G,A74I,T146F,V 178P | 5.8 |
| S72G,L181Y,L188R | 18.8 | S72G,A74V,T146F,V178W,L181Y,L188R | 10.0 | S72G,A74T,T146F, V178P,L188R | 5.7 |
| S72G,T146F ,V178W,L181Y,L188K | 18.8 | V178P,L181Y | 9.9 | S72G,T146F,L188R | 5.6 |
| A74T,T146F,V178W,L181Y | 18.3 | A74T,T146F,L181Y,L188K | 9.3 | S72G,T146F,L181Y | 5.6 |
| S72G,A74T,T146F,L181Y,L188K | 18.0 | S72G,A74V,V178P,L188F | 9.3 | T146F,L181Y,L188K | 5.4 |
| S72G,L181Y,L188K | 18.0 | S72G,V178W,L181Y,L188R | 9.0 | A74I,T146F,V178P, L188R | 5.3 |
| S72G,T146F,V178P,L 181Y,L188R | 17.4 | S72G,T146F,V178P,L188R | 8.9 | S72G,L181Y | 5.3 |
| S72G,A74V,T146F,V178P,L181Y,L188K | 16.8 | A74T,T146F,V178P | 8.8 | S72G,A74I,T146F,L 181Y,L188F | 5.3 |
| S72G,V178P,L188F | 16.5 | S72G,A74T,T146F,V178P,L188F | 8.7 | S72G,A74T,T146F, V178W,L181Y,L188 F | 5.2 |
| A74I,T146F,V178W,L188R | 15.9 | S72G,T146F,L188F | 8.6 | S72G,A74I,T146F,V 178P,L181Y,L188R | 5.2 |
| S72G,V178P,L188K | 15.3 | T146F,V178P,L181Y,L188R | 8.5 | A74V,T146F,L181Y, L188R | 5.1 |
| T146F,V178W,L188K | 15.3 | T146F,V178W | 8.4 | T146F,V178P | 5.1 |
| S72G,A74T,T146F,V178W,L188R | 14.9 | S72G,T146F,V178P,L181Y,L188K | 8.2 | | |

According a fourth embodiment of the first aspect, there are provided variants which are characterized by a selectivity factor for the C19 hydroxylation of at least one steroid or derivative thereof, which is higher than 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98 or 0.99.

For example, said selectivity factor for the C19 hydroxylation of a steroid or steroid derivative can be > 0.1, preferably > 0.2 or > 0.3, even more preferably > 0.4 or > 0.5, most preferably > 0.5, 0.6, 0.7, 0.8 or 0.9. For example, said steroid or steroid derivative can be a a steroid or steroid derivative comprising a 1,4-dien-3-one-A-ring or a 4-en-3-one-A-ring, such as d1-testosterone or ADD.

The fourth embodiment according to the first aspect can be and is suggested to be combined with each embodiment according to the first aspect and in particular with the first, second, third, fifth, or sixth embodiment of the first aspect.

For example there is provided a BM3 variant comprising the mutation F87A and at least one and preferably two further mutations selected from
(iii) a mutation at position V78, preferably V78F, V78Y, V78M, V78I or V78L,
(iv) a mutation at position A82, preferably A82E, A82Q or A82P,
and optionally comprising further mutations, said BM3 variant having a selectivity factor for the C19 hydroxylation of at least one steroid or steroid derivative > 0.1, preferably > 0.2 or > 0.3, even more preferably > 0.4 or > 0.5, most preferably > 0.5, 0.6, 0.7, 0.8 or 0.9. Preferably, the BM3 variant is furthermore characterized by a product yield for the C19 hydroxylation of a steroid or steroid derivative > 5 %, more preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %.

Preferably, the BM3 variant comprises the mutations M177Y and/or A184Y.

For example there is provided a BM3 variant comprising
(i) at least the mutations
   a) V78Y, A82E and F87A (BM3-254, SEQ ID No. 2),
   b) V78M, A82E and F87A (BM3-261, SEQ ID No. 3),
   c) V78I, A82E and F87A (BM3-263, SEQ ID No. 4),
   d) V78L, A82E and F87A (BM3-268, SEQ ID No. 5).
   e) V78Y, A82P and F87A (SEQ ID No. 6),
   f) V78M, A82P and F87A (SEQ ID No. 7),
   g) V78I, A82P and F87A (SEQ ID No. 8),
   h) V78L, A82P and F87A (SEQ ID No. 9),
   i) V78Y, A82Q and F87A (SEQ ID No. 10),
   j) V78M, A82Q and F87A (SEQ ID No. 11),
   k) V78I, A82Q and F87A (SEQ ID No. 12), or
   l) V78L, A82Q and F87A (SEQ ID No. 13),
   and at least one, two, three, four, five, six, seven, eight, nine or ten further mutation(s), or
(ii) at least the mutations
   m) V78F, A82E, F87A, M177Y and A184Y (SEQ ID No. 14),
   n) V78L, A82E, F87A, M177Y and A184Y (SEQ ID No. 15),
   o) V78I, A82E, F87A, M177Y and A184Y (SEQ ID No. 16),
   p) V78M, A82E, F87A, M177Y and A184Y (SEQ ID No. 17),
   q) V78Y, A82E, F87A, M177Y and A184Y (SEQ ID No. 18),
   r) V78F, A82P, F87A, M177Y and A184Y (SEQ ID No. 19),
   s) V78L, A82P, F87A, M177Y and A184Y (SEQ ID No. 20),
   t) V78I, A82P, F87A, M177Y and A184Y (SEQ ID No. 21),
   u) V78M, A82P, F87A, M177Y and A184Y (SEQ ID No. 22),
   v) V78Y, A82P, F87A, M177Y and A184Y (SEQ ID No. 23),
   w) V78F, A82Q, F87A, M177Y and A184Y (SEQ ID No. 24),
   x) V78L, A82Q, F87A, M177Y and A184Y (SEQ ID No. 25),
   y) V78I, A82Q, F87A, M177Y and A184Y (SEQ ID No. 26),
   z) V78M, A82Q, F87A, M177Y and A184Y (SEQ ID No. 27), or
   aa) V78Y, A82Q, F87A, M177Y and A184Y (SEQ ID No. 28),
   and optionally further mutations,
said BM3 variant having a selectivity factor for the C19 hydroxylation of a steroid or steroid derivative > 0.1, preferably > 0.2 or > 0.3, even more preferably > 0.4 or > 0.5, most preferably > 0.5, 0.6, 0.7, 0.8 or 0.9. Preferably, the BM3 variant is furthermore characterized by a product yield for the C19 hydroxylation of a steroid or steroid derivative > 5 %, more preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %.

The P450-BM3 parent variants, in particular the variants according to SEQ ID No. 2 to 5 and variants derived thereof catalyze not only C19 hydroxylation but also further oxidative reactions. Significant generation of products with C19-hydroxylation and an additional oxidation in other positions of the steroid molecule was observed. With testosterone as substrate (2beta)-2,19-dihydroxytestosterone and (15beta)-15,19-dihydroxytestosterone were isolated, with delta-1-testosterone as substrate (1beta,2beta)-1,2-epoxy-19-hydroxytestosterone was isolated.

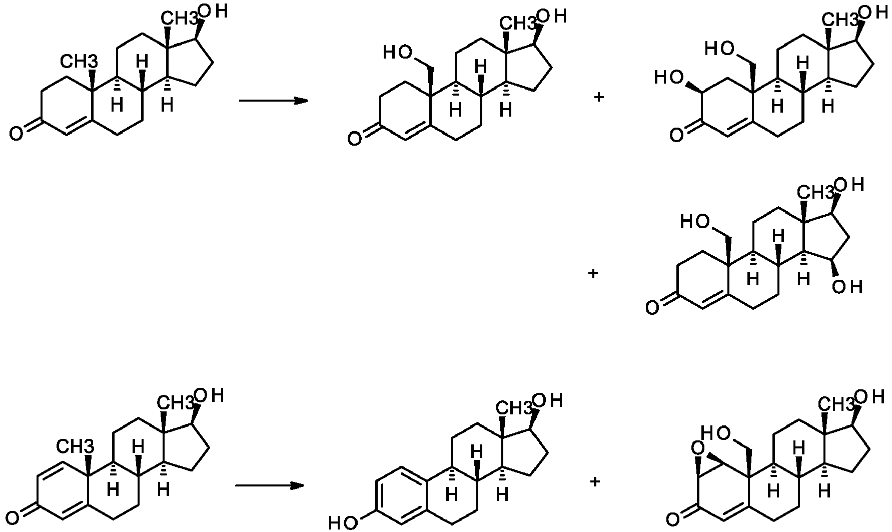

Thus, for example the P450-BM3 variants may catalyze formation of (6beta)-OH, (15beta)-OH or (1beta,2beta)-1,2-epoxy derivatives either instead of or in combination with C19 hydroxylation (see Examples 8, 9, Tables E3, E5). The formation of these side products leads to an impaired selectivity factor. However, for the listed BM3 parent variants it was surprisingly found that even where side reactions occur, the resulting C19 hydroxylation (overoxidation) products may still be relevant for the commercial steroid synthesis. Compared with their respective parent variant the variants according to the fourth embodiment of the first aspect were superior with regard to selectivity.

**Table B1 shows estradiol selectivity factors for d1-testosterone to estradiol conversion from 50 mg/L substrate for variants derived from the parent type BM3-268 (SEQ ID No. 5) having further mutations as depicted.**

| **Mutation** | **SF (Estradiol)** | **Mutation** | **SF (Estradiol)** | **Mutation** | **SF (Estradiol)** | **Mutation** | **SF (Estradiol)** |
|---|---|---|---|---|---|---|---|
| V26E | 0.58 | L188H | 0.14 | M177N | 0.09 | V26G | 0.07 |
| A74I | 0.53 | I263L | 0.14 | H266V | 0.09 | L86M | 0.07 |
| G85L | 0.48 | V178D | 0.14 | E64V | 0.09 | T146C | 0.07 |
| A74V | 0.42 | T146M | 0.14 | M118N | 0.09 | E143M | 0.07 |
| I263C | 0.42 | S72C, A74I | 0.14 | A321N | 0.09 | V48F | 0.07 |
| V178P | 0.42 | T327L | 0.14 | M118L | 0.09 | R50I | 0.07 |
| S72G, A74V | 0.40 | L29Q | 0.14 | E143R | 0.09 | A321D | 0.07 |
| F77A | 0.38 | S72N, A74I | 0.14 | L75I, F81G | 0.09 | R255L | 0.07 |
| A184G | 0.37 | G415S | 0.13 | L188A | 0.09 | M118V | 0.07 |
| M177Y | 0.37 | S89Q | 0.13 | T365W | 0.09 | L29A | 0.07 |
| A330V | 0.36 | T365V | 0.13 | T49C | 0.09 | T365K | 0.07 |
| A184Y | 0.34 | L75V, F81L | 0.13 | E143D | 0.09 | E64Y | 0.07 |
| A74T | 0.33 | S176C | 0.13 | V178K | 0.09 | L29I | 0.07 |
| H266S | 0.32 | L75I, F81L | 0.13 | V48T | 0.09 | L181H | 0.07 |
| T146F | 0.31 | G402A | 0.13 | V178R | 0.09 | T49F | 0.07 |
| A184R | 0.31 | V314H | 0.13 | M118P | 0.09 | V48R | 0.07 |
| A184F | 0.31 | T365Y | 0.13 | R50E | 0.09 | V178N | 0.07 |
| R50S, A74V | 0.31 | M177T | 0.13 | G415T | 0.09 | S72D, A74C | 0.07 |
| S72G, A74I | 0.30 | T365P | 0.13 | M177L | 0.09 | E267R | 0.07 |
| A74C | 0.29 | L188D | 0.13 | S72N, A74V | 0.09 | K224I | 0.07 |
| M185V | 0.29 | L262Y | 0.13 | R50G | 0.09 | S176Q | 0.07 |
| A330R | 0.28 | P392V | 0.13 | V48W | 0.09 | L75V | 0.07 |
| M177K | 0.28 | A74S | 0.13 | R47L | 0.09 | L75I, F81V | 0.07 |
| L188K | 0.28 | F77C | 0.13 | R50N | 0.09 | V48L | 0.07 |
| I263N | 0.27 | L188M | 0.13 | F81Y | 0.09 | R47A | 0.07 |
| A184W | 0.27 | I263H, A264G | 0.13 | F77H | 0.09 | E143A | 0.07 |
| A74M | 0.27 | R50Q | 0.12 | E143F | 0.09 | L78M | 0.07 |
| S89L | 0.27 | R255I | 0.12 | S176F | 0.09 | A330T | 0.07 |
| K224P | 0.26 | S72G, A74Y | 0.12 | L75I, F81Y | 0.09 | V48I | 0.07 |
| V48H | 0.26 | V178I | 0.12 | F261I | 0.09 | S176K | 0.07 |
| V26N | 0.26 | W90D | 0.12 | F77D | 0.09 | | |
| A399I | 0.26 | L75I, F81S | 0.12 | A184L | 0.09 | | |
| A74L | 0.26 | P392G | 0.12 | F77N | 0.09 | | |
| A184E | 0.25 | V314S | 0.12 | F261M | 0.09 | | |
| F77V | 0.25 | V178M | 0.12 | E143C | 0.09 | | |
| F77S | 0.24 | A74R | 0.12 | F77I | 0.09 | | |
| V178W | 0.24 | M177F | 0.12 | V26M | 0.08 | | |
| V26H | 0.24 | A74G, L75C | 0.12 | V178S | 0.08 | | |
| L181P | 0.23 | L29S | 0.12 | V314A | 0.08 | | |
| F77L | 0.23 | L75I, F81H | 0.12 | R47K | 0.08 | | |
| F77W | 0.23 | I263K | 0.12 | T49N | 0.08 | | |
| A184D | 0.22 | A399M | 0.12 | R50P | 0.08 | | |
| S72W | 0.22 | V178L | 0.12 | L75I, F81C | 0.08 | | |
| M177V | 0.22 | F261V | 0.12 | F77R | 0.08 | | |
| V178Y | 0.22 | S72Y, A74V | 0.12 | L29H | 0.08 | | |
| A74H | 0.21 | A399C | 0.12 | T49W | 0.08 | | |
| L181V | 0.21 | S72C, A74L | 0.12 | V48D | 0.08 | | |
| A74C, L75V | 0.21 | L188E | 0.11 | A399N | 0.08 | | |
| S176P | 0.21 | G415D | 0.11 | R47Q | 0.08 | | |
| F77T | 0.21 | T49I | 0.11 | E64G | 0.08 | | |
| S89E | 0.20 | M118F | 0.11 | V26K | 0.08 | | |
| M177R | 0.20 | L75V, F81C | 0.11 | R50M | 0.08 | | |
| T49R | 0.20 | T88C | 0.11 | A321V | 0.08 | | |
| A74Y | 0.20 | E143W | 0.11 | R50V | 0.08 | | |
| A74W | 0.20 | V314P | 0.11 | V314Q | 0.08 | | |
| M177C | 0.20 | F81W | 0.11 | E64H | 0.08 | | |
| S72C, A74V | 0.20 | L181I | 0.11 | P392R | 0.08 | | |
| L188R | 0.20 | S89V | 0.11 | M185L | 0.08 | | |
| A74F | 0.20 | T438V | 0.11 | L188S | 0.08 | | |
| F77K | 0.20 | S176D | 0.11 | V26L | 0.08 | | |
| A74R, L75V | 0.19 | L86V | 0.11 | F81L | 0.08 | | |
| F261D | 0.19 | L29F | 0.11 | L29Y | 0.08 | | |
| L188N | 0.19 | I401T | 0.11 | R50L | 0.08 | | |
| S89T | 0.19 | A184Q | 0.11 | V371P | 0.08 | | |
| L188W | 0.19 | A321G | 0.11 | E267D | 0.08 | | |
| M177H | 0.19 | M118Y | 0.11 | L262W | 0.08 | | |
| T49P | 0.19 | W90Y | 0.11 | A399Q | 0.08 | | |
| F81S | 0.19 | L262I | 0.11 | M177A | 0.08 | | |
| A184K | 0.18 | T146R | 0.11 | E143G | 0.08 | | |
| A184N | 0.18 | L29C | 0.11 | V371A | 0.08 | | |
| M177Q | 0.18 | P392Q | 0.10 | M177P | 0.08 | | |
| L75H, F81S | 0.18 | A399E | 0.10 | S72N, A74C | 0.08 | | |
| S89C | 0.18 | H266E | 0.10 | R50S | 0.08 | | |
| S89M | 0.18 | S72C, A74Y | 0.10 | S176E | 0.08 | | |
| L188F | 0.18 | T49A | 0.10 | E143I | 0.08 | | |
| L181Y | 0.18 | S72C, A74C | 0.10 | T49Q | 0.08 | | |
| V178Q | 0.18 | L75V, F81Y | 0.10 | T365G | 0.07 | | |
| F77E | 0.18 | T365I | 0.10 | F77M | 0.07 | | |
| F261C | 0.17 | F77P | 0.10 | R50C | 0.07 | | |
| L188I | 0.17 | R50Y | 0.10 | T146D | 0.07 | | |
| F261S | 0.17 | S72G, A74F | 0.10 | R47H | 0.07 | | |
| S72G, A74C | 0.17 | M118E | 0.10 | R255K | 0.07 | | |
| A399V | 0.17 | S72H, A74C | 0.10 | E143V | 0.07 | | |
| A330K | 0.17 | A74P | 0.10 | R255P | 0.07 | | |
| S72G, A74H | 0.17 | V26I | 0.10 | V48Y | 0.07 | | |
| F261A | 0.16 | E64Q | 0.10 | V314F | 0.07 | | |
| S176M | 0.16 | L75V, F81I | 0.10 | A330F | 0.07 | | |
| A74N | 0.16 | V314E | 0.10 | Y51W | 0.07 | | |
| S176G | 0.16 | T146P | 0.10 | M118A | 0.07 | | |
| T365Q | 0.16 | T49L | 0.10 | E143P | 0.07 | | |
| S89N | 0.16 | M177E | 0.10 | T49D | 0.07 | | |
| G415A | 0.15 | L29T | 0.10 | E143K | 0.07 | | |
| A264V | 0.15 | T327V | 0.10 | R255N | 0.07 | | |
| S89I | 0.15 | H266N | 0.10 | H266A | 0.07 | | |
| T49V | 0.15 | V48S | 0.10 | F261Y | 0.07 | | |
| M118G | 0.15 | R50D | 0.10 | T365L | 0.07 | | |
| K224H | 0.15 | G85A | 0.09 | L29M | 0.07 | | |
| S72G, A74L | 0.15 | L437I | 0.09 | L75I | 0.07 | | |
| A184H | 0.15 | F77Y | 0.09 | V26A | 0.07 | | |
| A330W | 0.15 | T146S | 0.09 | L29V | 0.07 | | |
| S72G, A74S | 0.14 | M118W | 0.09 | R47T | 0.07 | | |

**Table B2 shows estradiol selectivity factors for Δ1-testosterone to estradiol conversion from 100 mg/L substrate for variants derived from the parent type BM3-268; M177Y, A184Y (SEQ ID No. 15) having further mutations as depicted.**

| **Mutation** | **SF Estradiol** | **Mutation** | **SF Estradiol** | **Mutation** | **SF Estradiol** |
|---|---|---|---|---|---|
| L150W | 0.65 | T49I | 0.51 | F205Y | 0.47 |
| L78M | 0.64 | F205G | 0.51 | K210E | 0.47 |
| W325Y | 0.61 | A330W | 0.51 | L20I | 0.47 |
| L20W | 0.60 | L20N | 0.50 | F205R | 0.46 |
| E93G | 0.59 | F205H | 0.50 | T49A | 0.46 |
| T149P | 0.59 | F205D, D208N | 0.50 | L20D | 0.46 |
| V26Q | 0.57 | L150Y | 0.50 | L20V | 0.46 |
| W325F | 0.56 | E267D | 0.50 | L20S | 0.46 |
| M212F | 0.55 | L20C | 0.49 | L20M | 0.45 |
| S89T | 0.55 | T146L | 0.49 | T146A, F205G | 0.45 |
| M212W | 0.54 | M212Y | 0.49 | R47W | 0.45 |
| L20T | 0.54 | F205T | 0.49 | T146A, L272W | 0.45 |
| L150F | 0.53 | L20R | 0.49 | F205W | 0.45 |
| A330Y | 0.53 | F77V | 0.48 | R47S | 0.45 |
| L20F | 0.53 | F205E | 0.48 | T327M | 0.43 |
| T49P | 0.52 | T49L | 0.48 | F205K | 0.42 |
| L20P | 0.52 | T146V, A191T | 0.47 | T146A, L150A | 0.42 |
| L272V | 0.52 | F205D | 0.47 | E352N | 0.38 |
| L20Y | 0.51 | P9S | 0.47 | | |

**Table B3 shows selectivity factors for estradiol obtained in biotransformation reactions from 100 mg/L Δ1-testosterone as substrate with BM3-268; M177Y, A184Y (SEQ ID No. 15) variants having up to six mutations.**

| **Mutations** | **SF Estradiol** | **Mutations** | **SF Estradiol** |
|---|---|---|---|
| S72G,A74I,V178P,L181Y | 0.83 | S72G,V178P,L181Y,L188F | 0.61 |
| S72G,A74V,V178P,L181Y,L188K | 0.83 | S72G,V178W,L188R | 0.61 |
| A74V,V178P,L181Y,L188R | 0.83 | T146F,L181Y | 0.6 |
| S72G,A74V,T146F,V178P,L181Y,L188R | 0.82 | T146F,V178W,L188R | 0.59 |
| A74V,V178P,L181Y,L188K | 0.8 | S72G,T146F,V178W | 0.59 |
| S72G,A74V,T146F,V178P,L181Y,L188K | 0.8 | T146F,L181Y,L188F | 0.58 |
| S72G,A74V,T146F,V178P,L181Y | 0.79 | A74T,T146F,L181Y,L188K | 0.58 |
| S72G,A74I,T146F,L181Y,L188F | 0.77 | A74T,T146F,V178W | 0.58 |
| S72G,A74V,T146F,V178W,L181Y | 0.76 | V178P | 0.58 |
| S72G,A74T,T146F,V178W,L188R | 0.76 | S72G,A74T,T146F,L188R | 0.58 |
| S72G,A74V,T146F,L181Y,L188R | 0.75 | S72G,V178P,L181Y,L188K | 0.57 |
| S72G,A74V,T146F,L181Y | 0.75 | S72G,V178W,L188F | 0.57 |
| A74V,V178P,L181Y,L188F | 0.74 | S72G,V178P,L181Y,L188R | 0.56 |
| S72G,A74T,T146F,V178W,L181Y,L188R | 0.73 | S72G,T146F,V178W,L181Y,L188K | 0.56 |
| S72G,A74V,T146F,V178W,L188R | 0.72 | S72G,T146F,V178W,L188K | 0.55 |
| A74T,T146F,V178P,L181Y | 0.72 | T146F,V178W,L181Y,L188R | 0.55 |
| A741,T146F,V178W | 0.68 | V178P,L181Y,L188R | 0.55 |
| S72G,A74T,T146F,L181Y,L188K | 0.68 | T146F,V178W,L188F | 0.55 |
| S72G,A74T,L181Y,L188F | 0.68 | S72G,A74V,L188R | 0.55 |
| S72G,A74T,T146F,V178P,L181Y | 0.68 | S72G,A74T,V178P,L188F | 0.55 |
| S72G,A74T,T146F,V178W,L188F | 0.67 | S72G,T146F,V178P,L188R | 0.54 |
| S72G,T146F,V178W,L188F | 0.66 | S72G,V178W,L188K | 0.53 |
| S72G,A74I,T146F,L181Y,L188K | 0.66 | V178W | 0.53 |
| S72G,A74I,T146F,L181Y,L188R | 0.66 | S72G,V178W | 0.52 |
| S72G,V178P,L181Y | 0.65 | S72G,T146F,L188F | 0.52 |
| A74T,T146F,L181Y,L188R | 0.65 | S72G,T146F,L181Y,L188R | 0.52 |
| V178P,L181Y | 0.65 | A74T,T146F,V178P,L188R | 0.51 |
| S72G,V178W,L181Y | 0.65 | T146F,V178W | 0.49 |
| S72G,T146F,V178W,L181Y,L188F | 0.63 | S72G | 0.47 |
| S72G,T146F,V178W,L181Y | 0.63 | A74T,T146F,L188K | 0.47 |
| S72G,A74T,L181Y,L188K | 0.63 | T146F,V178W,L188K | 0.46 |
| S72G,A74I,T146F,L181Y | 0.63 | S72G,L188F | 0.46 |
| S72G,A74V,T146F,V178W | 0.62 | S72G,T146F | 0.46 |

**Table B4 shows estrone selectivity factors for ADD to estrone conversion from 100 mg/L substrate for variants derived from the parent type BM3-268; M177Y, A184Y (SEQ ID No. 15) having further mutations as depicted.**

| **Mutation(s)** | **Estrone** | **Mutation(s)** | **Estrone** | **Mutation(s)** | **Estrone** |
|---|---|---|---|---|---|
| I259M | 0.75 | T146A, F205G | 0.29 | L20R | 0.23 |
| L150W | 0.72 | F205E | 0.29 | F205W | 0.23 |
| L272C | 0.65 | F205S | 0.29 | V26S | 0.23 |
| F205D | 0.45 | M354Q | 0.29 | E352A | 0.23 |
| F205N | 0.44 | W325Y | 0.29 | E3521 | 0.23 |
| R203C | 0.44 | F2051 | 0.28 | M212F | 0.22 |
| F205V | 0.42 | F205G | 0.28 | M212T | 0.22 |
| E93G | 0.41 | M212H | 0.28 | L272W | 0.21 |
| F173Y, I174F | 0.40 | T438V | 0.28 | E352M | 0.20 |
| F205T | 0.39 | T438C | 0.27 | L150F | 0.20 |
| M212Y | 0.37 | M354W | 0.27 | T49Q | 0.20 |
| F205P | 0.36 | T49R | 0.27 | L272Y | 0.20 |
| L78M | 0.36 | T327M | 0.27 | L20F | 0.20 |
| F205Y | 0.34 | F81Y | 0.27 | T146F | 0.20 |
| F205D, D208N | 0.34 | F205R | 0.26 | L150G | 0.19 |
| M212W | 0.34 | S89T | 0.26 | K210E | 0.19 |
| T146L | 0.32 | I259D | 0.26 | T49I | 0.18 |
| D363G, T438C | 0.31 | L150Y | 0.25 | F81D | 0.18 |
| F205K | 0.31 | L20Y | 0.24 | L20P | 0.17 |
| L20W | 0.30 | T438I | 0.24 | L272F | 0.16 |
| F205C | 0.30 | F205H | 0.24 | E352N | 0.15 |
| T146A, L272H | 0.30 | E352V | 0.24 | | |

**Table B5 shows the selectivity factor for estrone obtained in biotransformation reactions from 100 mg/L ADD with BM3-268; M177Y, A184Y (SEQ ID No. 15) derived variants having up to six mutations.**

| **Mutation(s)** | **SF Estr one** | **Mutation(s)** | **SF Estr one** | **Mutation(s)** | **SF Estr one** |
|---|---|---|---|---|---|
| S72G,A74V,V178P,L181Y,L188F | 0.94 | S72G,A74T,L188R | 0.54 | S72G,A74I,T146F,V178P,L188F | 0.37 |
| S72G,A74V,L181Y,L188F | 0.88 | A74V,V178P,L181Y,L188F | 0.54 | T146F,V178W,L188F | 0.37 |
| S72G,A74V,T146F,V178P,L181Y,L188K | 0.85 | S72G,L181Y,L188R | 0.53 | S72G,L181Y,L188F | 0.37 |
| S72G,A74T,V178P,L181Y | 0.84 | S72G,A74T,T146F,V178P,L181Y | 0.53 | A74I,T146F,V178P | 0.36 |
| S72G,A74V,T146F,V178P,L181Y,L188R | 0.83 | S72G,T146F,L181Y,L18 8R | 0.52 | S72G,A74I,T146F,L181Y | 0.36 |
| S72G,A74V,T146F,L181Y,L188K | 0.83 | S72G,A74V,T146F,L181Y,L188R | 0.52 | S72G,L181Y | 0.36 |
| S72G,A74V,T146F,V178W,L181Y,L188K | 0.82 | A74T,T146F,V178P,L181Y | 0.51 | S72G,A74T,T146F,V178W,L181Y,L188F | 0.36 |
| S72G,A74V,V178W,L181Y | 0.81 | S72G,V178P,L188F | 0.50 | S72G,A74V,T146F,V178 P | 0.36 |
| S72G,A74V,V178W,L181Y,L188F | 0.79 | A74T,T146F,V178W,L188R | 0.50 | S72G,A74T,T146F,V178P,L188F | 0.35 |
| S72G,A74T,T146F,V178W,L181Y | 0.78 | S72G,L181Y,L188K | 0.49 | S72G,A74V,V178P,L188R | 0.34 |
| S72G,A741,T146F,V178W,L181Y | 0.78 | S72G,A741,V178P,L181Y | 0.49 | S72G,T146F,V178P,L181Y,L188F | 0.34 |
| S72G,A74V,T146F,V178W,L181Y,L188R | 0.78 | S72G,A74V,V178P,L188F | 0.49 | V178P,L181Y | 0.33 |
| S72G,A74I,T146F,V178P,L181Y,L188R | 0.77 | S72G,T146F,L188F | 0.49 | S72G,V178W,L181Y,L188F | 0.33 |
| S72G,A74V,T146F,L181Y,L188F | 0.76 | S72G,V178P,L181Y,L188F | 0.48 | S72G,A741,T146F,V178P | 0.32 |
| S72G,V178W,L181Y | 0.75 | S72G,A74T,T146F,V178P | 0.48 | S72G,T146F,L188R | 0.32 |
| S72G,V178W,L181Y,L188R | 0.73 | S72G,V178W,L188R | 0.48 | T146F,V178P | 0.32 |
| S72G,A74V,T146F,V178P,L181Y | 0.71 | S72G,A74T,T146F,V178P,L188R | 0.48 | S72G,A74T,T146F,L181Y | 0.32 |
| S72G,A741,T146F,V178W,L181Y,L188K | 0.71 | S72G,T146F,V178W,L 181Y,L188K | 0.47 | T146F,V178W,L 181Y,L18 8R | 0.31 |
| S72G,T146F,V178W,L181Y | 0.69 | S72G,A741,T146F,V178P,L181Y | 0.47 | S72G,V178P,L188K | 0.31 |
| S72G,A74T,T146F,V178W,L181Y,L188K | 0.69 | S72G,T146F,V178P,L181Y,L188K | 0.47 | S72G,L188F | 0.31 |
| A741,T146F,V178W,L188R | 0.69 | S72G,T146F,V178P,L181Y | 0.45 | S72G,V178P,L181Y,L188 K | 0.30 |
| S72G,A74T,V178P,L181Y,L188K | 0.68 | S72G,T146F,L188K | 0.44 | S72G,A74T,V178P,L188F | 0.29 |
| S72G,T146F,V178W,L181Y,L188F | 0.67 | S72G,A74T,T146F,L181 Y.L188K | 0.43 | S72G,T146F,V178P,L188 R | 0.29 |
| S72G,A74V,T146F,V178P,L188F | 0.66 | S72G,A74V,T146F,V178 W,L188R | 0.43 | S72G,A74V,V178P | 0.29 |
| S72G,A74V,T146F,L188F | 0.66 | S72G,V178P,L188R | 0.42 | V178P | 0.29 |
| S72G,A74T,T146F,V178W,L188R | 0.65 | T146F,L181Y,L188F | 0.42 | S72G,A74I,T146F,V178P, L188R | 0.29 |
| S72G,A74V,L181Y | 0.65 | T146F,L181Y | 0.41 | S72G,V178W | 0.29 |
| S72G,T146F,V178W,L188F | 0.64 | S72G,T146F,V178W,L188K | 0.41 | A74I,T146F,L181Y,L188K | 0.29 |
| S72G,A74V,T146F,V178W,L181Y | 0.64 | A74T,T146F,V178P | 0.41 | A74T,T146F,L181Y,L188 K | 0.28 |
| S72G,A74T,L181Y,L188R | 0.64 | S72G,A74V,T146F,V178P,L188R | 0.41 | S72G,A74V,V178P,L181 Y,L188R | 0.28 |
| S72G,T146F,V178W,L188R | 0.63 | T146F,L181Y,L188K | 0.41 | S72G,L188R | 0.27 |
| A74T,T146F,V178W,L181Y | 0.63 | S72G,T146F | 0.40 | S72G,A74V,T146F,V178 P,L188K | 0.27 |
| S72G,A741,T146F,L181Y,L188F | 0.63 | S72G | 0.40 | S72G,A74V,L188R | 0.26 |
| S72G,A74V,T146F,L181Y | 0.62 | S72G,A741,T146F,V178P,L188K | 0.40 | S72G,T146F,V178P,L188 K | 0.26 |
| S72G,A74T,T146F,V178W,L181Y,L188R | 0.61 | S72G,A741,T146F,V178P,L181Y,L188F | 0.40 | A74I,T146F,V178W,L188 K | 0.26 |
| S72G,T146F,L181Y,L188K | 0.61 | S72G,T146F,L181Y | 0.40 | T146F,V178P,L181Y,L18 8R | 0.25 |
| A74I,T146F,L181Y | 0.61 | A741,T146F,V178P,L188R | 0.40 | T146F,V178W | 0.25 |
| S72G,T146F,V178W | 0.59 | A74V,T146F,L181Y,L188R | 0.40 | A74T,T146F,V178W | 0.25 |
| S72G,T146F,L181Y,L188F | 0.59 | S72G,A74V,T146F,V178W | 0.39 | S72G,L188K | 0.24 |
| S72G,T146F,V178P,L188F | 0.56 | S72G,V178P | 0.39 | S72G,A74V,L188K | 0.24 |
| S72G,V178W,L188K | 0.56 | S72G,A74I,T146F,L181Y,L188R | 0.39 | T146F,V178W,L188R | 0.23 |
| S72G,T146F,V178P | 0.55 | S72G,V178P,L181Y | 0.38 | T146F,V178W,L188K | 0.21 |
| S72G,V178W,L188F | 0.55 | S72G,V178P,L181Y,L188R | 0.38 | T146F | 0.20 |
| S72G,A74T,T146F,V178P,L188K | 0.55 | A74T,T146F,V178P,L188K | 0.38 | | |
| S72G,A74T,T146F,V178W,L188F | 0.54 | S72G,T146F,V178P,L181Y,L188R | 0.37 | | |

Provided are variants according to a **fifth embodiment** according to the first aspect, wherein said P450-BM3 variant comprises at least one, two, three, four, five, six, seven, eight, nine, ten or more further mutations. In particular the variants according to the fifth embodiment can be variants according to the first or second embodiment of the first aspect, such as variants derived from variants according to SEQ ID No. 2, 3, 4, 5, 6, 7, 8, 9, 10, 11, 12, 13, 14, 15, 16, 17, 18, 19, 20, 21, 22, 23, 24, 25, 26, 27 or 28.

Preferrably, said further mutation(s) comprise at least one, two, three, four, five, six, seven, eight, nine, ten or more position(s) selected from (A184) or (A191) or (A221) or (A264) or (A321) or (A328) or (A33) or (A330) or (A399) or (A74) or (D168) or (D208) or (D222) or (D363) or (E13) or (E143) or (E267) or (E352) or (E64) or (E82) or (E93) or (F173) or (F205) or (F261) or (F331) or (F393) or (F77) or (F81) or (G240) or (G271) or (G402) or (G415) or (G570) or (G677) or (G85) or (H266) or (H659) or (1153) or (1174) or (I258) or (I259) or (I263) or (1401) or (K210) or (K224) or (L150) or (L181) or (L188) or (L20) or (L262) or (L272) or (L29) or (L324) or (L333) or (L356) or (L437) or (L75) or (L78) or (L86) or (M118) or (M177) or (M185) or (M212) or (M354) or (N70) or (P243) or (P326) or (P329) or (P392) or (P9) or (R147) or (R161) or (R190) or (R203) or (R255) or (R323) or (R47) or (R50) or (S164) or (S176) or (S54) or (S72) or (S89) or (T146) or (T149) or (T260) or (T268) or (T269) or (T327) or (T365) or (T436) or (T438) or (T49) or (T88) or (V178) or (V26) or (V299) or (V314) or (V371) or (V48) or (W325) or (W367) or (W90) or (Y51).

Preferably said BM3 variant has an improved selectivity and/or product yield for C19 hydroxylation compared with its corresponding parent BM3 variant. Preferably, the BM3 variant comprises the mutations M177Y and/or A184Y.

It was surprisingly found that targeting the(se) position(s) and introducing a "suitable" amino acid exchange could further improve either product yield (cf. Tables A1, A2, A3, A4 and A5) or selectivity factor (cf. Tables B1, B2, B3, B4 and B5) of the C19 hydroxylation, thereby further improving the efficiency of the C19 hydroxylation process. Evaluation which amino acid is "suitable" for a position to obtain the improvement is easily feasible where the targeting position is disclosed herein. To evaluate which of the 20 amino acids is "suitable" for the given position, one simply has to mutate the given position by introducing the amino acid as known in the art (see also Example 1) and evaluate the performance of the obtained variant for C19 hydroxylation, e.g in comparison with the parent variant and/or in comparison with the remaining 18 amino acids.

There is provided a first preferred subset comprising variants according to the fifth embodiment, wherein the P450-BM3 variants have an improved product yield for C19 hydroxylation in comparison with the parent variant. Comprised within this subset are variants wherein said further mutation(s) comprise at least one, two, three, four, five, six, seven, eight, nine, ten or more position(s) selected from (A184) or (A191) or (A221) or (A264) or (A321) or (A33) or (A330) or (A399) or (A74) or (D208) or (D222) or (D363) or (E13) or (E143) or (E267) or (E352) or (E64) or (E82) or (E93) or (F173) or (F205) or (F261) or (F331) or (F393) or (F77) or (F81) or (G240) or (G271) or (G402) or (G415) or (G677) or (G85) or (H266) or (H659) or (I153) or (1174) or (I258) or (I259) or (I263) or (1401) or (K210) or (K224) or (L150) or (L181) or (L188) or (L20) or (L262) or (L272) or (L29) or (L324) or (L356) or (L437) or (L75) or (L78) or (L86) or (M118) or (M177) or (M185) or (M212) or (M354) or (N70) or (P243) or (P329) or (P392) or (P9) or (R147) or (R161) or (R190) or (R203) or (R255) or (R47) or (R50) or (S176) or (S54) or (S72) or (S89) or (T146) or (T149) or (T260) or (T268) or (T269) or (T327) or (T365) or (T436) or (T438) or (T49) or (T88) or (V178) or (V26) or (V314) or (V371) or (V48) or (W325) or (W367) or (W90) or (Y51).

There is provided a second preferred subset comprising variants according to the **fifth embodiment,** wherein the P450-BM3 variants have an improved **selectivity factor** for C19 hydroxylation in comparison with the parent variant (see figure 1b). Comprised within this subset are variants wherein said further mutation(s) comprise at least one, two, three, four, five, six, seven, eight, nine, ten or more position(s) selected from (A184) or (A191) or (A221) or (A264) or (A321) or (A328) or (A33) or (A330) or (A399) or (A74) or (D168) or (D208) or (D222) or (D363) or (E143) or (E267) or (E352) or (E64) or (E82) or (E93) or (F173) or (F205) or (F261) or (F331) or (F393) or (F77) or (F81) or (G240) or (G271) or (G402) or (G415) or (G570) or (G677) or (G85) or (H266) or (H659) or (1153) or (1174) or (I258) or (I259) or (I263) or (1401) or (K210) or (K224) or (L150) or (L181) or (L188) or (L20) or (L262) or (L272) or (L29) or (L324) or (L333) or (L356) or (L437) or (L75) or (L78) or (L86) or (M118) or (M177) or (M185) or (M212) or (M354) or (N70) or (P243) or (P326) or (P329) or (P392) or (P9) or (R147) or (R161) or (R190) or (R203) or (R255) or (R323) or (R47) or (R50) or (S164) or (S176) or (S54) or (S72) or (S89) or (T146) or (T149) or (T260) or (T268) or (T269) or (T327) or (T365) or (T436) or (T438) or (T49) or (T88) or (V178) or (V26) or (V299) or (V314) or (V48) or (W325) or (W367) or (W90) or (Y51).

There is provided a third preferred subset comprising variants according to the **fifth embodiment,** wherein the P450-BM3 variants have an improved **selectivity factor and an improved product yield** for C19 hydroxylation in comparison with the parent variant. Comprised within this subset are variants wherein said further mutation(s) comprise at least one, two, three, four, five, six, seven, eight, nine, ten or more position(s) selected from (A184) or (A191) or (A221) or (A264) or (A321) or (A33) or (A330) or (A399) or (A74) or (D208) or (D222) or (D363) or (E13) or (E143) or (E267) or (E352) or (E64) or (E82) or (E93) or (F173) or (F205) or (F261) or (F331) or (F393) or (F77) or (F81) or (G240) or (G271) or (G402) or (G677) or (G85) or (H266) or (H659) or (1153) or (1174) or (I258) or (I259) or (I263) or (1401) or (K210) or (K224) or (L150) or (L181) or (L188) or (L20) or (L262) or (L272) or (L29) or (L324) or (L356) or (L437) or (L75) or (L78) or (L86) or (M118) or (M177) or (M185) or (M212) or (M354) or (N70) or (P243) or (P329) or (P392) or (P9) or (R147) or (R161) or (R190) or (R203) or (R255) or (R47) or (R50) or (S176) or (S54) or (S72) or (S89) or (T146) or (T149) or (T268) or (T269) or (T327) or (T365) or (T436) or (T438) or (T49) or (T88) or (V178) or (V26) or (V314) or (V48) or (W325) or (W367) or (W90) or (Y51).

The fifth embodiment and each of the subsets provided can be and is suggested to be combined with all previous embodiments, as understood by the skilled person, and may or may not be an embodiment according to the **second embodiment** of the first aspect.

Provided are variants according to a **sixth embodiment** according to the first aspect, wherein said P450-BM3 variant comprise at least one, two, three, four, five, six, seven, eight, nine, ten or more further mutations. In particular the variants according to the sixth embodiment can be variants according to the first or second embodiment of the first aspect, such as variants derived from variants according to SEQ ID No. 2, 3, 4, 5, 6, 7, 8, 9, 10, 11, 12, 13, 14, 15, 16, 17, 18, 19, 20, 21, 22, 23, 24, 25, 26, 27 or 28. The sixth embodiment can be and is suggested to be combined with all previous embodiments, and may or may not be an embodiment according to the fifth embodiment.

Preferrably, said further mutation(s) according to the sixth embodiment of the first aspect are (A184D) or (A184D, L188K) or (A184E) or (A184F) or (A184G) or (A184H) or (A184I) or (A184K) or (A184L) or (A184N) or (A184Q) or (A184R) or (A184W) or (A184W, L188F) or (A184Y) or (A184Y, L188K) or (A184Y, R50S) or (A221S) or (A264V) or (A321C) or (A321D) or (A321E) or (A321G) or (A321I) or (A321N) or (A321T) or (A321V) or (A328P) or (A330C) or (A330D) or (A330E) or (A330F) or (A330G) or (A330H) or (A330I) or (A330K) or (A330L) or (A330M) or (A330N) or (A330Q) or (A330R) or (A330S) or (A330T) or (A330T, E352A) or (A330T, F331V) or (A330V) or (A330W) or (A330Y) or (A330Y, W367C) or (A33V) or (A399C) or (A399E) or (A399G) or (A399I) or (A399L) or (A399M) or (A399N) or (A399Q) or (A399R) or (A399S) or (A399T) or (A399V) or (A74C) or (A74C, L75V) or (A74D) or (A74D, F81V) or (A74E) or (A74F) or (A74G) or (A74G, L75C) or (A74H) or (A74I) or (A74I, A184W, L188F) or (A74I, F77A, L181Y, A184W) or (A74I, L181Y, L188K) or (A74I, L181Y, L188R) or (A74I, L188F) or (A74I, L188K) or (A74I, L188R) or (A74I, M177Y, A184R, L188F) or (A74I, M177Y, V178P, A184G) or (A74I, T146F) or (A74I, T146F, L181Y) or (A74I, T146F, L181Y, L188K) or (A74I, T146F, L181Y, L188R) or (A74I, T146F, L188F) or (A74I, T146F, L188K) or (A74I, T146F, V178P) or (A74I, T146F, V178P, L181Y, L188F) or (A74I, T146F, V178P, L181Y, L188K) or (A74I, T146F, V178P, L188K) or (A74I, T146F, V178P, L188R) or (A74I, T146F, V178W) or (A74I, T146F, V178W, L181Y, L188F) or (A74I, T146F, V178W, L181Y, L188R) or (A74I, T146F, V178W, L188F) or (A74I, T146F, V178W, L188K) or (A74I, T146F, V178W, L188R) or (A74I, V178P) or (A74I, V178P, L181Y) or (A74I, V178P, L181Y, L188R) or (A74I, V178P, L188F) or (A74I, V178P, L188K) or (A74I, V178P, L188R) or (A74I, V178W, L188F) or (A74I, V178W, L188K) or (A74I, V178W, L188R) or (A74K) or (A74L) or (A74M) or (A74N) or (A74P) or (A74Q) or (A74R) or (A74R, L75V) or (A74S) or (A74T) or (A74T, A184D) or (A74T, F77A, L181Y, A184D, L188K) or (A74T, F77A, V178W, A184Y) or (A74T, F77L, L181Y, A184D, L188K) or (A74T, L181Y) or (A74T, L181Y, A184D, L188K) or (A74T, L181Y, A184Y, L188K) or (A74T, L181Y, L188K) or (A74T, L181Y, L188R) or (A74T, L188K) or (A74T, M177Y, L181Y, A184D, L188K) or (A74T, T146F, L181Y, A184D, L188K) or (A74T, T146F, L181Y, L188K) or (A74T, T146F, L181Y, L188R) or (A74T, T146F, L188F) or (A74T, T146F, L188K) or (A74T, T146F, L188R) or (A74T, T146F, V178P) or (A74T, T146F, V178P, L181Y) or (A74T, T146F, V178P, L188K) or (A74T, T146F, V178P, L188R) or (A74T, T146F, V178W) or (A74T, T146F, V178W, L181Y) or (A74T, T146F, V178W, L181Y, L188K) or (A74T, T146F, V178W, L188R) or (A74T, V178P) or (A74T, V178P, L188F) or (A74T, V178P, L188K) or (A74T, V178P, L188R) or (A74T, V178W, L181Y, L188K) or (A74T, V178W, L188K) or (A74T, V178W, L188R) or (A74V) or (A74V, F77A, A184N, L188F) or (A74V, L181Y) or (A74V, L181Y, A184D, L188K) or (A74V, L181Y, A184W, L188F) or (A74V, L181Y, L188F) or (A74V, L188F) or (A74V, L188K) or (A74V, L75V) or (A74V, T146F, L181Y, L188K) or (A74V, T146F, L181Y, L188R) or (A74V, T146F, L188K) or (A74V, T146F, L188R) or (A74V, T146F, V178W) or (A74V, T146F, V178W, L181Y, L188F) or (A74V, V178P, A184W, L188K) or (A74V, V178P, L181Y, L188F) or (A74V, V178P, L181Y, L188K) or (A74V, V178P, L181Y, L188R) or (A74V, V178P, L188F) or (A74V, V178W, L188K) or (A74W) or (A74Y) or (D168Y, F173D) or (D363G, T438C) or (E13D, R47L) or (E143A) or (E143C) or (E143D) or (E143F) or (E143G) or (E143I) or (E143K) or (E143L) or (E143M) or (E143N) or (E143P) or (E143Q) or (E143R) or (E143S) or (E143V) or (E143W) or (E143Y) or (E267A) or (E267C) or (E267D) or (E267G) or (E267K) or (E267P) or (E267R) or (E267S) or (E267T) or (E267Y) or (E352A) or (E352D) or (E352F) or (E352G) or (E352I) or (E352L) or (E352M) or (E352N) or (E352P) or (E352R) or (E352S) or (E352T) or (E352V) or (E352W) or (E352Y) or (E64A) or (E64G) or (E64H) or (E64I) or (E64K) or (E64L) or (E64M) or (E64N) or (E64Q) or (E64V) or (E64W) or (E64Y) or (E82P) or (E93G) or (F173C) or (F173C, F205G) or (F173D) or (F173I) or (F173K) or (F173L) or (F173M) or (F173N) or (F173P) or (F173Q) or (F173R) or (F173S) or (F173S, F205P) or (F173V) or (F173W) or (F173Y) or (F173Y, I174F) or (F205A) or (F205C) or (F205D) or (F205D, D208N) or (F205E) or (F205G) or (F205H) or (F205I) or (F205K) or (F205L) or (F205M) or (F205N) or (F205P) or (F205R) or (F205S) or (F205T) or (F205V) or (F205W) or (F205Y) or (F261A) or (F261C) or (F261D) or (F261G) or (F261I) or (F261L) or (F261M) or (F261N) or (F261Q) or (F261S) or (F261T) or (F261V) or (F261W) or (F261Y) or (F331C) or (F331H) or (F331I) or (F331L) or (F331M) or (F331N) or (F331P) or (F331T) or (F331V) or (F331W) or (F331Y) or (F393M) or (F393W) or (F77A) or (F77A, A184W) or (F77A, L181P, A184Y, L188K) or (F77A, M177Y) or (F77C) or (F77D) or (F77E) or (F77G) or (F77H) or (F77I) or (F77K) or (F77L) or (F77M) or (F77N) or (F77P) or (F77R) or (F77S) or (F77T) or (F77V) or (F77V, L86M) or (F77W) or (F77Y) or (F81A) or (F81C) or (F81D) or (F81I) or (F81L) or (F81P) or (F81R) or (F81S) or (F81T) or (F81V) or (F81W) or (F81Y) or (G271A) or (G271C) or (G271D) or (G271E) or (G271F) or (G271H) or (G271K) or (G271L) or (G271M) or (G271N) or (G271P) or (G271Q) or (G271R) or (G271S) or (G271T) or (G271V) or (G271W) or (G271Y) or (G402A) or (G415A) or (G415D) or (G415S) or (G415T) or (G415V) or (G85A) or (G85L) or (G85S) or (H266A) or (H266C) or (H266D) or (H266E) or (H266F) or (H266G) or (H266I) or (H266K) or (H266M) or (H266N) or (H266P) or (H266Q) or (H266R) or (H266S) or (H266T) or (H266V) or (H266W) or (H266Y) or (I153F, G271L) or (I153L) or (I153L, F173Y) or (I259A) or (I259C) or (I259D) or (I259F) or (I259G) or (I259H) or (I259K) or (I259L) or (I259M) or (I259N) or (I259Q) or (I259S) or (I259T) or (I259V) or (I259W) or (I259Y) or (I263A) or (I263C) or (I263E) or (I263F) or (I263G) or (I263H, A264G) or (I263K) or (I263L) or (I263M) or (I263N) or (I263Q) or (I263S) or (I263T) or (I263V) or (I263Y) or (I401A) or (I401L) or (I401M) or (I401T) or (I401V) or (K210E) or (K210T, G271V) or (K224C) or (K224E) or (K224F) or (K224H) or (K224I) or (K224L) or (K224M) or (K224P) or (K224Q) or (K224W) or (K224Y) or (L150A) or (L150C) or (L150D) or (L150E) or (L150F) or (L150G) or (L150H) or (L150I) or (L150K) or (L150M) or (L150N) or (L150Q) or (L150R) or (L150R, F205R) or (L150S) or (L150S, F173L) or (L150T) or (L150V) or (L150W) or (L150Y) or (L181H) or (L181I) or (L181M) or (L181P) or (L181V) or (L181Y) or (L181Y, A184D) or (L181Y, L188F) or (L181Y, L188K) or (L181Y, L188K, H659R) or (L188A) or (L188D) or (L188E) or (L188F) or (L188H) or (L188I) or (L188K) or (L188M) or (L188N) or (L188Q) or (L188R) or (L188S) or (L188W) or (L20C) or (L20D) or (L20E) or (L20F) or (L20G) or (L20G, R47L) or (L20I) or (L20M) or (L20N) or (L20P) or (L20R) or (L20S) or (L20T) or (L20V) or (L20W) or (L20Y) or (L262I) or (L262V) or (L262W) or (L262Y) or (L272A) or (L272C) or (L272E) or (L272F) or (L272G) or (L272I) or (L272K) or (L272M) or (L272N) or (L272Q) or (L272R) or (L272S) or (L272T) or (L272V) or (L272W) or (L272Y) or (L29A) or (L29C) or (L29D) or (L29F) or (L29H) or (L29I) or (L29M) or (L29M, R47G) or (L29P) or (L29Q) or (L29S) or (L29T) or (L29V) or (L29W) or (L29Y) or (L324F) or (L356C) or (L356F) or (L356H) or (L356I) or (L356M) or (L356N) or (L356Q) or (L356S) or (L356T) or (L356V) or (L356W) or (L437I) or (L437M) or (L75H, F81S) or (L75I) or (L75I, F81C) or (L75I, F81G) or (L75I, F81H) or (L75I, F81I) or (L75I, F81L) or (L75I, F81S) or (L75I, F81V) or (L75I, F81Y) or (L75V) or (L75V, F81C) or (L75V, F81H) or (L75V, F81I) or (L75V, F81L) or (L75V, F81V) or (L75V, F81Y) or (L78F) or (L78I) or (L78M) or (L78V) or (L78Y) or (L86A, S89T) or (L86I) or (L86I, S89T) or (L86M) or (L86M, S89T) or (L86N, S89T) or (L86V) or (L86V, S89T) or (M118A) or (M118E) or (M118F) or (M118G) or (M118H) or (M118I) or (M118K) or (M118L) or (M118N) or (M118P) or (M118Q) or (M118S) or (M118T) or (M118V) or (M118W) or (M118Y) or (M177A) or (M177C) or (M177C, V178Y) or (M177D) or (M177E) or (M177F) or (M177G) or (M177H) or (M177I) or (M177K) or (M177L) or (M177N) or (M177P) or (M177Q) or (M177R) or (M177T) or (M177V) or (M177Y) or (M177Y, A184W) or (M177Y, A184Y) or (M177Y, M185V) or (M177Y, R50S) or (M177Y, V178P) or (M177Y, V178P, A184Y, L188F) or (M177Y, V178W) or (M177Y, V178W, A184Y) or (M177Y, V178W, A184Y, L188F) or (M185C) or (M185D) or (M185E) or (M185G) or (M185H) or (M185K) or (M185L) or (M185N) or (M185Q) or (M185R) or (M185V) or (M185Y) or (M212A) or (M212C) or (M212C, I259L) or (M212D) or (M212E) or (M212F) or (M212G) or (M212H) or (M212K) or (M212L) or (M212L, I259F) or (M212P) or (M212Q) or (M212R) or (M212S) or (M212T) or (M212V) or (M212W) or (M212Y) or (M354A) or (M354C) or (M354D) or (M354E) or (M354G) or (M354I) or (M354K) or (M354K, D363Y) or (M354L) or (M354N) or (M354Q) or (M354R) or (M354S) or (M354T) or (M354V) or (M354W) or (M354Y) or (N70A) or (N70C) or (N70F) or (N70G) or (N70H) or (N70K) or (N70R) or (N70W) or (N70Y) or (P243T, H266L) or (P326C) or (P326D) or (P326G) or (P326N) or (P326S) or (P326T) or (P329A) or (P329C) or (P329G) or (P329K) or (P329S) or (P329T) or (P392C) or (P392G) or (P392Q) or (P392R) or (P392V) or (P9S) or (R147C, H266G) or (R161C, G271D) or (R190L, F261V) or (R203C) or (R255A) or (R255F) or (R255G) or (R255I) or (R255K) or (R255L) or (R255M) or (R255N) or (R255P) or (R255T) or (R255W) or (R323C) or (R47A) or (R47C) or (R47D) or (R47E) or (R47F) or (R47G) or (R47H) or (R47H, A74W) or (R47I) or (R47K) or (R47L) or (R47M) or (R47N) or (R47P) or (R47Q) or (R47S) or (R47T) or (R47V) or (R47W) or (R50A) or (R50C) or (R50D) or (R50E) or (R50F) or (R50G) or (R50I) or (R50L) or (R50M) or (R50N) or (R50P) or (R50Q) or (R50S) or (R50S, A184W, L188F) or (R50S, A74I, A184W, L188F) or (R50S, A74M, A184Y, L188K) or (R50S, A74T, L181Y, A184D, L188K, G240R) or (R50S, A74V) or (R50S, M177Y, V178P, A184Y) or (R50S, M177Y, V178W, A184W) or (R50S, M177Y, V178W, A184Y) or (R50S, M177Y, V178W, A184Y, A330R) or (R50S, M177Y, V178W, A184Y, A74V, G677D) or (R50S, M177Y, V178W, A184Y, H266S) or (R50S, M177Y, V178W, A184Y, L181P) or (R50S, M177Y, V178W, A184Y, L181Y) or (R50S, M177Y, V178W, A184Y, L188K) or (R50S, M177Y, V178W, A184Y, L188R) or (R50S, M177Y, V178W, A184Y, T146F) or (R50S, M177Y, V178W, A184Y, V26N) or (R50S, M177Y, V178W, L181Y, A184Y) or (R50S, S72G, A74V) or (R50S, S72G, A74V, M177Y, A184Y) or (R50S, S72G, A74V, M177Y, V178P, A184Y) or (R50S, S72G, A74V, M177Y, V178W, A184Y) or (R50S, S72G, A74V, M177Y, V178W, A184Y, L188R) or (R50S, T146F, M177Y, V178W, A184Y) or (R50V) or (R50Y) or (S164N, F261L) or (S176A, A184Y, L188K) or (S176C) or (S176D) or (S176E) or (S176F) or (S176G) or (S176H) or (S176K) or (S176L) or (S176M) or (S176N) or (S176P) or (S176Q) or (S176R) or (S72C, A74C) or (S72C, A74I) or (S72C, A74L) or (S72C, A74V) or (S72C, A74Y) or (S72D, A74C) or (S72D, A74F) or (S72G) or (S72G, A74C) or (S72G, A74F) or (S72G, A74H) or (S72G, A74I) or (S72G, A74I, A184W, L188F) or (S72G, A74I, F77A, M177Y) or (S72G, A74I, F77L) or (S72G, A74I, F77L, M177Y) or (S72G, A74I, L181Y) or (S72G, A74I, L181Y, L188F) or (S72G, A74I, L181Y, L188K) or (S72G, A74I, L181Y, L188R) or (S72G, A74I, L188F) or (S72G, A74I, L188K) or (S72G, A74I, L188R) or (S72G, A74I, T146F) or (S72G, A74I, T146F, L181Y) or (S72G, A74I, T146F, L181Y, L188F) or (S72G, A74I, T146F, L181Y, L188K) or (S72G, A74I, T146F, L181Y, L188R) or (S72G, A74I, T146F, L188F) or (S72G, A74I, T146F, L188K) or (S72G, A74I, T146F, L188R) or (S72G, A74I, T146F, V178P) or (S72G, A74I, T146F, V178P, L181Y) or (S72G, A74I, T146F, V178P, L181Y, L188F) or (S72G, A74I, T146F, V178P, L181Y, L188K) or (S72G, A74I, T146F, V178P, L181Y, L188R) or (S72G, A74I, T146F, V178P, L188F) or (S72G, A74I, T146F, V178P, L188K) or (S72G, A74I, T146F, V178P, L188R) or (S72G, A74I, T146F, V178W) or (S72G, A74I, T146F, V178W, L181Y) or (S72G, A74I, T146F, V178W, L181Y, L188F) or (S72G, A74I, T146F, V178W, L181Y, L188K) or (S72G, A74I, T146F, V178W, L181Y, L188R) or (S72G, A74I, T146F, V178W, L188F) or (S72G, A74I, T146F, V178W, L188K) or (S72G, A74I, T146F, V178W, L188R) or (S72G, A74I, V178P) or (S72G, A74I, V178P, L181Y) or (S72G, A74I, V178P, L181Y, L188K) or (S72G, A74I, V178P, L181Y, L188R) or (S72G, A74I, V178P, L188F) or (S72G, A74I, V178P, L188K) or (S72G, A74I, V178P, L188R) or (S72G, A74I, V178W) or (S72G, A74I, V178W, L181Y, L188F) or (S72G, A74I, V178W, L181Y, L188K) or (S72G, A74I, V178W, L181Y, L188R) or (S72G, A74I, V178W, L188F) or (S72G, A74I, V178W, L188K) or (S72G, A74I, V178W, L188R) or (S72G, A74L) or (S72G, A74M, A184Y, L188K) or (S72G, A74S) or (S72G, A74T) or (S72G, A74T, L181Y, A184D, L188K) or (S72G, A74T, L181Y, L188F) or (S72G, A74T, L181Y, L188K) or (S72G, A74T, L181Y, L188R) or (S72G, A74T, L188F) or (S72G, A74T, L188K) or (S72G, A74T, L188R) or (S72G, A74T, T146F) or (S72G, A74T, T146F, L181Y) or (S72G, A74T, T146F, L181Y, L188K) or (S72G, A74T, T146F, L181Y, L188R) or (S72G, A74T, T146F, L188F) or (S72G, A74T, T146F, L188K) or (S72G, A74T, T146F, L188R) or (S72G, A74T, T146F, V178P) or (S72G, A74T, T146F, V178P, L181Y) or (S72G, A74T, T146F, V178P, L188F) or (S72G, A74T, T146F, V178P, L188K) or (S72G, A74T, T146F, V178P, L188R) or (S72G, A74T, T146F, V178W) or (S72G, A74T, T146F, V178W, L181Y) or (S72G, A74T, T146F, V178W, L181Y, L188F) or (S72G, A74T, T146F, V178W, L181Y, L188K) or (S72G, A74T, T146F, V178W, L181Y, L188R) or (S72G, A74T, T146F, V178W, L188F) or (S72G, A74T, T146F, V178W, L188K) or (S72G, A74T, T146F, V178W, L188R) or (S72G, A74T, V178P) or (S72G, A74T, V178P, L181Y) or (S72G, A74T, V178P, L181Y, L188F) or (S72G, A74T, V178P, L181Y, L188K) or (S72G, A74T, V178P, L188F) or (S72G, A74T, V178P, L188K) or (S72G, A74T, V178P, L188R) or (S72G, A74T, V178W) or (S72G, A74T, V178W, L181Y, L188F) or (S72G, A74T, V178W, L181Y, L188K) or (S72G, A74T, V178W, L188F) or (S72G, A74T, V178W, L188K) or (S72G, A74T, V178W, L188R) or (S72G, A74V) or (S72G, A74V, A184W) or (S72G, A74V, A221V) or (S72G, A74V, F77A) or (S72G, A74V, F77A, M177Y) or (S72G, A74V, F77L, M177Y) or (S72G, A74V, F77S) or (S72G, A74V, L181Y) or (S72G, A74V, L181Y, L188F) or (S72G, A74V, L181Y, L188K) or (S72G, A74V, L188F) or (S72G, A74V, L188K) or (S72G, A74V, L188R) or (S72G, A74V, T146F) or (S72G, A74V, T146F, L181Y) or (S72G, A74V, T146F, L181Y, L188F) or (S72G, A74V, T146F, L181Y, L188K) or (S72G, A74V, T146F, L181Y, L188R) or (S72G, A74V, T146F, L188F) or (S72G, A74V, T146F, L188K) or (S72G, A74V, T146F, L188R) or (S72G, A74V, T146F, V178P) or (S72G, A74V, T146F, V178P, L181Y) or (S72G, A74V, T146F, V178P, L181Y, L188F) or (S72G, A74V, T146F, V178P, L181Y, L188K) or (S72G, A74V, T146F, V178P, L181Y, L188R) or (S72G, A74V, T146F, V178P, L188F) or (S72G, A74V, T146F, V178P, L188K) or (S72G, A74V, T146F, V178P, L188R) or (S72G, A74V, T146F, V178W) or (S72G, A74V, T146F, V178W, L181Y) or (S72G, A74V, T146F, V178W, L181Y, L188K) or (S72G, A74V, T146F, V178W, L181Y, L188R) or (S72G, A74V, T146F, V178W, L188F) or (S72G, A74V, T146F, V178W, L188K) or (S72G, A74V, T146F, V178W, L188R) or (S72G, A74V, V178P) or (S72G, A74V, V178P, L181Y) or (S72G, A74V, V178P, L181Y, L188F) or (S72G, A74V, V178P, L181Y, L188K) or (S72G, A74V, V178P, L181Y, L188R) or (S72G, A74V, V178P, L188F) or (S72G, A74V, V178P, L188K) or (S72G, A74V, V178P, L188R) or (S72G, A74V, V178W) or (S72G, A74V, V178W, L181Y) or (S72G, A74V, V178W, L181Y, L188F) or (S72G, A74V, V178W, L181Y, L188K) or (S72G, A74V, V178W, L181Y, L188R) or (S72G, A74V, V178W, L188F) or (S72G, A74V, V178W, L188K) or (S72G, A74V, V178W, L188R) or (S72G, A74Y) or (S72G, L181Y) or (S72G, L181Y, L188F) or (S72G, L181Y, L188K) or (S72G, L181Y, L188R) or (S72G, L188F) or (S72G, L188K) or (S72G, L188R) or (S72G, M177Y, V178W) or (S72G, M177Y, V178W, L188K) or (S72G, T146F) or (S72G, T146F, L181Y) or (S72G, T146F, L181Y, L188F) or (S72G, T146F, L181Y, L188K) or (S72G, T146F, L181Y, L188R) or (S72G, T146F, L188F) or (S72G, T146F, L188K) or (S72G, T146F, L188R) or (S72G, T146F, V178P) or (S72G, T146F, V178P, L181Y) or (S72G, T146F, V178P, L181Y, L188F) or (S72G, T146F, V178P, L181Y, L188K) or (S72G, T146F, V178P, L181Y, L188R) or (S72G, T146F, V178P, L188F) or (S72G, T146F, V178P, L188K) or (S72G, T146F, V178P, L188R) or (S72G, T146F, V178W) or (S72G, T146F, V178W, L181Y) or (S72G, T146F, V178W, L181Y, L188F) or (S72G, T146F, V178W, L181Y, L188K) or (S72G, T146F, V178W, L188F) or (S72G, T146F, V178W, L188K) or (S72G, T146F, V178W, L188R) or (S72G, V178P) or (S72G, V178P, L181Y) or (S72G, V178P, L181Y, L188F) or (S72G, V178P, L181Y, L188K) or (S72G, V178P, L181Y, L188R) or (S72G, V178P, L188F) or (S72G, V178P, L188K) or (S72G, V178P, L188R) or (S72G, V178W) or (S72G, V178W, L181Y) or (S72G, V178W, L181Y, L188F) or (S72G, V178W, L181Y, L188K) or (S72G, V178W, L181Y, L188R) or (S72G, V178W, L188F) or (S72G, V178W, L188K) or (S72G, V178W, L188R) or (S72H, A74C) or (S72H, A74G) or (S72H, A74S) or (S72H, A74Y) or (S72N, A74C) or (S72N, A74I) or (S72N, A74N) or (S72N, A74V) or (S72T) or (S72W) or (S72W, A74I, A184W, L188F) or (S72W, A74V) or (S72W, L333R) or (S72W, W90Y, V299G) or (S72Y, A74V) or (S89C) or (S89E) or (S89G) or (S89I) or (S89L) or (S89M) or (S89N) or (S89Q) or (S89R) or (S89T) or (S89V) or (T146A) or (T146A, D222Y, I263M) or (T146A, F173D) or (T146A, F173V) or (T146A, F205G) or (T146A, F261C) or (T146A, F261S) or (T146A, F261V) or (T146A, G271R) or (T146A, G271S) or (T146A, G271T) or (T146A, H266T) or (T146A, H266V) or (T146A, I258T, I259S) or (T146A, I259H) or (T146A, I263F) or (T146A, I263L) or (T146A, I263N) or (T146A, I263V) or (T146A, L150A) or (T146A, L150G) or (T146A, L150K) or (T146A, L150R, F173I) or (T146A, L272H) or (T146A, L272R) or (T146A, L272S) or (T146A, L272W) or (T146A, M212A) or (T146C) or (T146D) or (T146E) or (T146F) or (T146F, L181Y) or (T146F, L181Y, L188F) or (T146F, L181Y, L188K) or (T146F, L188K) or (T146F, L188R) or (T146F, V178P) or (T146F, V178P, L181Y, L188R) or (T146F, V178P, L188K) or (T146F, V178P, L188R) or (T146F, V178W) or (T146F, V178W, L181Y, L188R) or (T146F, V178W, L188F) or (T146F, V178W, L188K) or (T146F, V178W, L188R) or (T146G) or (T146H) or (T146L) or (T146M) or (T146N) or (T146P) or (T146R) or (T146S) or (T146V) or (T146V, A191T) or (T146Y) or (T149P) or (T260A) or (T260S) or (T260W) or (T268A) or (T268S) or (T269K) or (T327A) or (T327A, A330W) or (T327C) or (T327D) or (T327E) or (T327G) or (T327I) or (T327L) or (T327M) or (T327N) or (T327P) or (T327Q) or (T327S) or (T327V) or (T365A) or (T365C) or (T365D) or (T365F) or (T365G) or (T365H) or (T365I) or (T365K) or (T365L) or (T365N) or (T365P) or (T365Q) or (T365V) or (T365W) or (T365Y) or (T436A) or (T436C) or (T436D) or (T436E) or (T436F) or (T436G) or (T436H) or (T436I) or (T436K) or (T436L) or (T436M) or (T436N) or (T436P) or (T436Q) or (T436R) or (T436S) or (T436V) or (T436W) or (T436Y) or (T438A) or (T438C) or (T438G) or (T438I) or (T438L) or (T438S) or (T438V) or (T49A) or (T49C) or (T49D) or (T49E) or (T49F) or (T49G) or (T49H) or (T49I) or (T49K) or (T49L) or (T49L, S54N) or (T49M) or (T49N) or (T49P) or (T49Q) or (T49R) or (T49R, R50C) or (T49R, R50S, M177Y, V178W, A184Y) or (T49S) or (T49V) or (T49W) or (T49Y) or (T88A) or (T88C) or (T88S) or (T88V) or (V178A) or (V178D) or (V178F) or (V178H) or (V178I) or (V178K) or (V178L) or (V178M) or (V178N) or (V178P) or (V178P, L181Y) or (V178P, L181Y, L188R) or (V178P, L188F) or (V178P, L188K) or (V178P, L188R) or (V178Q) or (V178R) or (V178S) or (V178T) or (V178W) or (V178W, A184Y) or (V178W, L181Y, L188K) or (V178W, L181Y, L188R) or (V178W, L188R) or (V178W, R50S) or (V178Y) or (V26A) or (V26C) or (V26E) or (V26F) or (V26G) or (V26G, R47S) or (V26H) or (V26I) or (V26K) or (V26L) or (V26M) or (V26N) or (V26N, R50S, M177Y, V178W, A184Y) or (V26Q) or (V26R) or (V26S) or (V26T) or (V26W) or (V26Y) or (V314A) or (V314E) or (V314F) or (V314H) or (V314P) or (V314Q) or (V314S) or (V314Y) or (V371 P) or (V48A) or (V48D) or (V48E) or (V48F) or (V48G) or (V48H) or (V48I) or (V48L) or (V48M) or (V48P) or (V48Q) or (V48R) or (V48S) or (V48T) or (V48W) or (V48Y) or (W325A) or (W325C) or (W325D) or (W325E) or (W325F) or (W325G) or (W325I) or (W325K) or (W325L) or (W325M) or (W325Q) or (W325R) or (W325S) or (W325T) or (W325V) or (W325Y) or (W90D) or (W90Y) or (W90Y, V299G, G570D) or (Y51A) or (Y51C) or (Y51E) or (Y51F) or (Y51G) or (Y51H) or (Y51I) or (Y51L) or (Y51M) or (Y51N) or (Y51P) or (Y51Q) or (Y51S) or (Y51S, F77V) or (Y51T) or (Y51V) or (Y51W).

The variants according to the **sixth embodiment** were surprisingly found to show an improved product yield or selectivity of the C19 hydroxylation, thereby further improving eficiency of the C19 hydroxylation process in comparison with the parent variant.

There is provided a first preferred subset comprising variants according to the **sixth embodiment,** wherein the P450-BM3 variants have an improved **product yield** for C19 hydroxylation in comparison with the parent variant. Comprised within this subset are variants comprising at least the mutation(s) (A184D) or (A184D, L188K) or (A184E) or (A184F) or (A184G) or (A184H) or (A184I) or (A184K) or (A184L) or (A184N) or (A184Q) or (A184R) or (A184W) or (A184W, L188F) or (A184Y) or (A184Y, L188K) or (A184Y, R50S) or (A221S) or (A264V) or (A321C) or (A321D) or (A321E) or (A321G) or (A321I) or (A321N) or (A321T) or (A321V) or (A330C) or (A330D) or (A330E) or (A330F) or (A330G) or (A330H) or (A330I) or (A330K) or (A330L) or (A330M) or (A330N) or (A330Q) or (A330R) or (A330S) or (A330T) or (A330T, E352A) or (A330V) or (A330W) or (A330Y) or (A330Y, W367C) or (A33V) or (A399C) or (A399E) or (A399G) or (A399I) or (A399L) or (A399M) or (A399N) or (A399Q) or (A399R) or (A399S) or (A399T) or (A399V) or (A74C) or (A74C, L75V) or (A74D) or (A74D, F81V) or (A74E) or (A74F) or (A74H) or (A74I) or (A74I, A184W, L188F) or (A74I, F77A, L181Y, A184W) or (A74I, L181Y, L188R) or (A74I, L188F) or (A74I, L188K) or (A74I, L188R) or (A74I, M177Y, A184R, L188F) or (A74I, M177Y, V178P, A184G) or (A74I, T146F) or (A74I, T146F, L181Y) or (A74I, T146F, L181Y, L188K) or (A74I, T146F, L181Y, L188R) or (A74I, T146F, L188F) or (A74I, T146F, L188K) or (A74I, T146F, V178P) or (A74I, T146F, V178P, L181Y, L188F) or (A74I, T146F, V178P, L181Y, L188K) or (A74I, T146F, V178P, L188K) or (A74I, T146F, V178P, L188R) or (A74I, T146F, V178W) or (A74I, T146F, V178W, L181Y, L188F) or (A74I, T146F, V178W, L181Y, L188R) or (A74I, T146F, V178W, L188F) or (A74I, T146F, V178W, L188K) or (A74I, T146F, V178W, L188R) or (A74I, V178P) or (A74I, V178P, L181Y) or (A74I, V178P, L181Y, L188R) or (A74I, V178P, L188F) or (A74I, V178P, L188K) or (A74I, V178P, L188R) or (A74I, V178W, L188F) or (A74I, V178W, L188R) or (A74K) or (A74L) or (A74M) or (A74N) or (A74P) or (A74Q) or (A74R) or (A74R, L75V) or (A74S) or (A74T) or (A74T, A184D) or (A74T, F77A, L181Y, A184D, L188K) or (A74T, F77A, V178W, A184Y) or (A74T, F77L, L181Y, A184D, L188K) or (A74T, L181Y) or (A74T, L181Y, A184D, L188K) or (A74T, L181Y, A184Y, L188K) or (A74T, L181Y, L188K) or (A74T, L181Y, L188R) or (A74T, L188K) or (A74T, M177Y, L181Y, A184D, L188K) or (A74T, T146F, L181Y, A184D, L188K) or (A74T, T146F, L181Y, L188K) or (A74T, T146F, L181Y, L188R) or (A74T, T146F, L188F) or (A74T, T146F, L188K) or (A74T, T146F, L188R) or (A74T, T146F, V178P) or (A74T, T146F, V178P, L181Y) or (A74T, T146F, V178P, L188K) or (A74T, T146F, V178P, L188R) or (A74T, T146F, V178W) or (A74T, T146F, V178W, L181Y) or (A74T, T146F, V178W, L181Y, L188K) or (A74T, T146F, V178W, L188R) or (A74T, V178P) or (A74T, V178P, L188F) or (A74T, V178P, L188K) or (A74T, V178P, L188R) or (A74T, V178W, L181Y, L188K) or (A74T, V178W, L188K) or (A74T, V178W, L188R) or (A74V) or (A74V, F77A, A184N, L188F) or (A74V, L181Y) or (A74V, L181Y, A184D, L188K) or (A74V, L181Y, A184W, L188F) or (A74V, L181Y, L188F) or (A74V, L188F) or (A74V, L188K) or (A74V, L75V) or (A74V, T146F, L181Y, L188K) or (A74V, T146F, L181Y, L188R) or (A74V, T146F, L188K) or (A74V, T146F, L188R) or (A74V, T146F, V178W, L181Y, L188F) or (A74V, V178P, A184W, L188K) or (A74V, V178P, L181Y, L188F) or (A74V, V178P, L181Y, L188K) or (A74V, V178P, L181Y, L188R) or (A74V, V178P, L188F) or (A74V, V178W, L188K) or (A74W) or (A74Y) or (D363G, T438C) or (E13D, R47L) or (E143A) or (E143C) or (E143D) or (E143F) or (E143G) or (E143I) or (E143K) or (E143L) or (E143M) or (E143N) or (E143P) or (E143Q) or (E143R) or (E143S) or (E143V) or (E143W) or (E143Y) or (E267D) or (E267R) or (E267T) or (E352A) or (E352D) or (E352F) or (E352G) or (E352I) or (E352L) or (E352M) or (E352N) or (E352P) or (E352R) or (E352S) or (E352T) or (E352V) or (E352W) or (E352Y) or (E64A) or (E64G) or (E64H) or (E64K) or (E64L) or (E64M) or (E64N) or (E64Q) or (E64V) or (E64Y) or (E82P) or (E93G) or (F173C) or (F173C, F205G) or (F173D) or (F173I) or (F173K) or (F173L) or (F173M) or (F173N) or (F173P) or (F173Q) or (F173R) or (F173S) or (F173S, F205P) or (F173V) or (F173W) or (F173Y) or (F173Y, I174F) or (F205A) or (F205C) or (F205D) or (F205D, D208N) or (F205E) or (F205G) or (F205H) or (F205I) or (F205K) or (F205L) or (F205M) or (F205N) or (F205P) or (F205R) or (F205S) or (F205T) or (F205V) or (F205W) or (F205Y) or (F261A) or (F261C) or (F261D) or (F261G) or (F261I) or (F261L) or (F261M) or (F261Q) or (F261S) or (F261T) or (F261V) or (F261Y) or (F331H) or (F331I) or (F331L) or (F331M) or (F331V) or (F331W) or (F331Y) or (F393M) or (F393W) or (F77A) or (F77A, A184W) or (F77A, L181P, A184Y, L188K) or (F77A, M177Y) or (F77C) or (F77D) or (F77E) or (F77G) or (F77H) or (F77I) or (F77K) or (F77L) or (F77M) or (F77N) or (F77P) or (F77R) or (F77S) or (F77T) or (F77V) or (F77V, L86M) or (F77W) or (F77Y) or (F81D) or (F81I) or (F81L) or (F81R) or (F81S) or (F81V) or (F81W) or (F81Y) or (G271A) or (G271C) or (G271D) or (G271E) or (G271F) or (G271H) or (G271K) or (G271L) or (G271M) or (G271N) or (G271P) or (G271Q) or (G271S) or (G271T) or (G271V) or (G271W) or (G271Y) or (G402A) or (G415A) or (G415D) or (G415S) or (G415T) or (G415V) or (G85A) or (G85L) or (G85S) or (H266A) or (H266C) or (H266E) or (H266F) or (H266I) or (H266K) or (H266M) or (H266N) or (H266Q) or (H266S) or (H266V) or (H266W) or (H266Y) or (I153F, G271L) or (I153L) or (I153L, F173Y) or (I259A) or (I259C) or (I259D) or (I259F) or (I259H) or (I259K) or (I259L) or (I259M) or (I259N) or (I259Q) or (I259S) or (I259T) or (I259V) or (I263A) or (I263C) or (I263E) or (I263F) or (I263H, A264G) or (I263K) or (I263L) or (I263M) or (I263N) or (I263Q) or (I263S) or (I263V) or (I263Y) or (I401A) or (I401M) or (I401T) or (I401V) or (K210E) or (K210T, G271V) or (K224C) or (K224E) or (K224H) or (K224I) or (K224L) or (K224M) or (K224P) or (K224Q) or (K224W) or (K224Y) or (L150A) or (L150C) or (L150D) or (L150E) or (L150F) or (L150G) or (L150H) or (L150I) or (L150K) or (L150M) or (L150N) or (L150Q) or (L150R) or (L150R, F205R) or (L150S) or (L150S, F173L) or (L150T) or (L150V) or (L150W) or (L150Y) or (L181H) or (L181I) or (L181M) or (L181P) or (L181V) or (L181Y) or (L181Y, A184D) or (L181Y, L188F) or (L181Y, L188K) or (L181Y, L188K, H659R) or (L188A) or (L188D) or (L188E) or (L188F) or (L188H) or (L188I) or (L188K) or (L188M) or (L188N) or (L188Q) or (L188R) or (L188S) or (L188W) or (L20C) or (L20D) or (L20E) or (L20F) or (L20G) or (L20G, R47L) or (L20I) or (L20M) or (L20N) or (L20P) or (L20R) or (L20S) or (L20T) or (L20V) or (L20W) or (L20Y) or (L262I) or (L262V) or (L262W) or (L272A) or (L272C) or (L272E) or (L272F) or (L272G) or (L272I) or (L272K) or (L272M) or (L272N) or (L272Q) or (L272S) or (L272T) or (L272V) or (L272W) or (L272Y) or (L29A) or (L29C) or (L29D) or (L29F) or (L29H) or (L29I) or (L29M) or (L29M, R47G) or (L29Q) or (L29S) or (L29T) or (L29V) or (L29W) or (L29Y) or (L324F) or (L356C) or (L356F) or (L356I) or (L356M) or (L356V) or (L437I) or (L437M) or (L75I) or (L75I, F81C) or (L75I, F81I) or (L75I, F81L) or (L75I, F81S) or (L75I, F81V) or (L75V) or (L75V, F81C) or (L75V, F81L) or (L75V, F81V) or (L75V, F81Y) or (L78F) or (L78I) or (L78M) or (L78V) or (L86I) or (L86I, S89T) or (L86M) or (L86M, S89T) or (L86V) or (M118A) or (M118E) or (M118F) or (M118G) or (M118H) or (M118I) or (M118K) or (M118L) or (M118N) or (M118P) or (M118Q) or (M118S) or (M118T) or (M118V) or (M118W) or (M118Y) or (M177A) or (M177C) or (M177C, V178Y) or (M177D) or (M177E) or (M177F) or (M177G) or (M177H) or (M177I) or (M177L) or (M177N) or (M177P) or (M177Q) or (M177R) or (M177T) or (M177V) or (M177Y) or (M177Y, A184W) or (M177Y, A184Y) or (M177Y, M185V) or (M177Y, R50S) or (M177Y, V178P) or (M177Y, V178P, A184Y, L188F) or (M177Y, V178W) or (M177Y, V178W, A184Y) or (M177Y, V178W, A184Y, L188F) or (M185C) or (M185D) or (M185E) or (M185G) or (M185H) or (M185K) or (M185L) or (M185N) or (M185Q) or (M185V) or (M212A) or (M212C) or (M212D) or (M212E) or (M212F) or (M212G) or (M212H) or (M212K) or (M212L) or (M212L, I259F) or (M212Q) or (M212R) or (M212S) or (M212T) or (M212V) or (M212W) or (M212Y) or (M354A) or (M354C) or (M354D) or (M354E) or (M354G) or (M354I) or (M354K) or (M354K, D363Y) or (M354L) or (M354N) or (M354Q) or (M354R) or (M354S) or (M354T) or (M354V) or (M354W) or (M354Y) or (N70A) or (N70C) or (N70F) or (N70G) or (N70H) or (N70K) or (N70R) or (N70Y) or (P243T, H266L) or (P329A) or (P392C) or (P392G) or (P392Q) or (P392R) or (P392V) or (P9S) or (R147C, H266G) or (R161C, G271D) or (R190L, F261V) or (R203C) or (R255A) or (R255G) or (R255I) or (R255K) or (R255L) or (R255M) or (R255N) or (R255P) or (R255T) or (R255W) or (R47A) or (R47C) or (R47D) or (R47E) or (R47F) or (R47G) or (R47H) or (R47H, A74W) or (R47I) or (R47K) or (R47L) or (R47M) or (R47N) or (R47P) or (R47Q) or (R47S) or (R47T) or (R47V) or (R47W) or (R50A) or (R50C) or (R50D) or (R50E) or (R50F) or (R50G) or (R50I) or (R50L) or (R50M) or (R50N) or (R50P) or (R50Q) or (R50S) or (R50S, A184W, L188F) or (R50S, A74I, A184W, L188F) or (R50S, A74M, A184Y, L188K) or (R50S, A74T, L181Y, A184D, L188K, G240R) or (R50S, A74V) or (R50S, M177Y, V178P, A184Y) or (R50S, M177Y, V178W, A184W) or (R50S, M177Y, V178W, A184Y) or (R50S, M177Y, V178W, A184Y, A330R) or (R50S, M177Y, V178W, A184Y, A74V, G677D) or (R50S, M177Y, V178W, A184Y, L181P) or (R50S, M177Y, V178W, A184Y, L181Y) or (R50S, M177Y, V178W, A184Y, L188K) or (R50S, M177Y, V178W, A184Y, L188R) or (R50S, M177Y, V178W, A184Y, T146F) or (R50S, M177Y, V178W, A184Y, V26N) or (R50S, M177Y, V178W, L181Y, A184Y) or (R50S, S72G, A74V) or (R50S, S72G, A74V, M177Y, A184Y) or (R50S, S72G, A74V, M177Y, V178P, A184Y) or (R50S, S72G, A74V, M177Y, V178W, A184Y) or (R50S, S72G, A74V, M177Y, V178W, A184Y, L188R) or (R50S, T146F, M177Y, V178W, A184Y) or (R50V) or (R50Y) or (S176A, A184Y, L188K) or (S176C) or (S176D) or (S176E) or (S176F) or (S176G) or (S176H) or (S176K) or (S176L) or (S176M) or (S176N) or (S176P) or (S176Q) or (S72C, A74C) or (S72C, A74I) or (S72C, A74L) or (S72C, A74V) or (S72D, A74C) or (S72G) or (S72G, A74C) or (S72G, A74F) or (S72G, A74H) or (S72G, A74I) or (S72G, A74I, A184W, L188F) or (S72G, A74I, F77A, M177Y) or (S72G, A74I, F77L) or (S72G, A74I, F77L, M177Y) or (S72G, A74I, L181Y) or (S72G, A74I, L181Y, L188F) or (S72G, A74I, L181Y, L188K) or (S72G, A74I, L181Y, L188R) or (S72G, A74I, L188F) or (S72G, A74I, L188K) or (S72G, A74I, L188R) or (S72G, A74I, T146F) or (S72G, A74I, T146F, L181Y) or (S72G, A74I, T146F, L181Y, L188F) or (S72G, A74I, T146F, L181Y, L188K) or (S72G, A74I, T146F, L181Y, L188R) or (S72G, A74I, T146F, L188F) or (S72G, A74I, T146F, L188K) or (S72G, A74I, T146F, V178P) or (S72G, A74I, T146F, V178P, L181Y) or (S72G, A74I, T146F, V178P, L181Y, L188F) or (S72G, A74I, T146F, V178P, L181Y, L188K) or (S72G, A74I, T146F, V178P, L181Y, L188R) or (S72G, A74I, T146F, V178P, L188F) or (S72G, A74I, T146F, V178P, L188K) or (S72G, A74I, T146F, V178P, L188R) or (S72G, A74I, T146F, V178W) or (S72G, A74I, T146F, V178W, L181Y) or (S72G, A74I, T146F, V178W, L181Y, L188F) or (S72G, A74I, T146F, V178W, L181Y, L188K) or (S72G, A74I, T146F, V178W, L181Y, L188R) or (S72G, A74I, T146F, V178W, L188F) or (S72G, A74I, T146F, V178W, L188K) or (S72G, A74I, T146F, V178W, L188R) or (S72G, A74I, V178P) or (S72G, A74I, V178P, L181Y) or (S72G, A74I, V178P, L181Y, L188R) or (S72G, A74I, V178P, L188F) or (S72G, A74I, V178P, L188K) or (S72G, A74I, V178P, L188R) or (S72G, A74I, V178W) or (S72G, A74I, V178W, L181Y, L188F) or (S72G, A74I, V178W, L181Y, L188K) or (S72G, A74I, V178W, L181Y, L188R) or (S72G, A74I, V178W, L188F) or (S72G, A74I, V178W, L188R) or (S72G, A74L) or (S72G, A74M, A184Y, L188K) or (S72G, A74S) or (S72G, A74T) or (S72G, A74T, L181Y, A184D, L188K) or (S72G, A74T, L181Y, L188F) or (S72G, A74T, L181Y, L188K) or (S72G, A74T, L181Y, L188R) or (S72G, A74T, L188F) or (S72G, A74T, L188K) or (S72G, A74T, L188R) or (S72G, A74T, T146F) or (S72G, A74T, T146F, L181Y) or (S72G, A74T, T146F, L181Y, L188K) or (S72G, A74T, T146F, L181Y, L188R) or (S72G, A74T, T146F, L188F) or (S72G, A74T, T146F, L188K) or (S72G, A74T, T146F, L188R) or (S72G, A74T, T146F, V178P) or (S72G, A74T, T146F, V178P, L181Y) or (S72G, A74T, T146F, V178P, L188F) or (S72G, A74T, T146F, V178P, L188K) or (S72G, A74T, T146F, V178P, L188R) or (S72G, A74T, T146F, V178W) or (S72G, A74T, T146F, V178W, L181Y) or (S72G, A74T, T146F, V178W, L181Y, L188F) or (S72G, A74T, T146F, V178W, L181Y, L188K) or (S72G, A74T, T146F, V178W, L181Y, L188R) or (S72G, A74T, T146F, V178W, L188F) or (S72G, A74T, T146F, V178W, L188K) or (S72G, A74T, T146F, V178W, L188R) or (S72G, A74T, V178P) or (S72G, A74T, V178P, L181Y) or (S72G, A74T, V178P, L181Y, L188F) or (S72G, A74T, V178P, L181Y, L188K) or (S72G, A74T, V178P, L188F) or (S72G, A74T, V178P, L188K) or (S72G, A74T, V178P, L188R) or (S72G, A74T, V178W) or (S72G, A74T, V178W, L181Y, L188F) or (S72G, A74T, V178W, L181Y, L188K) or (S72G, A74T, V178W, L188F) or (S72G, A74T, V178W, L188K) or (S72G, A74T, V178W, L188R) or (S72G, A74V) or (S72G, A74V, A184W) or (S72G, A74V, A221V) or (S72G, A74V, F77A) or (S72G, A74V, F77A, M177Y) or (S72G, A74V, F77L, M177Y) or (S72G, A74V, F77S) or (S72G, A74V, L181Y) or (S72G, A74V, L181Y, L188F) or (S72G, A74V, L181Y, L188K) or (S72G, A74V, L188F) or (S72G, A74V, L188K) or (S72G, A74V, L188R) or (S72G, A74V, T146F) or (S72G, A74V, T146F, L181Y) or (S72G, A74V, T146F, L181Y, L188F) or (S72G, A74V, T146F, L181Y, L188K) or (S72G, A74V, T146F, L181Y, L188R) or (S72G, A74V, T146F, L188F) or (S72G, A74V, T146F, L188K) or (S72G, A74V, T146F, L188R) or (S72G, A74V, T146F, V178P) or (S72G, A74V, T146F, V178P, L181Y) or (S72G, A74V, T146F, V178P, L181Y, L188F) or (S72G, A74V, T146F, V178P, L181Y, L188K) or (S72G, A74V, T146F, V178P, L181Y, L188R) or (S72G, A74V, T146F, V178P, L188F) or (S72G, A74V, T146F, V178P, L188K) or (S72G, A74V, T146F, V178P, L188R) or (S72G, A74V, T146F, V178W) or (S72G, A74V, T146F, V178W, L181Y) or (S72G, A74V, T146F, V178W, L181Y, L188K) or (S72G, A74V, T146F, V178W, L181Y, L188R) or (S72G, A74V, T146F, V178W, L188F) or (S72G, A74V, T146F, V178W, L188K) or (S72G, A74V, T146F, V178W, L188R) or (S72G, A74V, V178P) or (S72G, A74V, V178P, L181Y) or (S72G, A74V, V178P, L181Y, L188F) or (S72G, A74V, V178P, L181Y, L188K) or (S72G, A74V, V178P, L181Y, L188R) or (S72G, A74V, V178P, L188F) or (S72G, A74V, V178P, L188K) or (S72G, A74V, V178P, L188R) or (S72G, A74V, V178W) or (S72G, A74V, V178W, L181Y) or (S72G, A74V, V178W, L181Y, L188F) or (S72G, A74V, V178W, L181Y, L188K) or (S72G, A74V, V178W, L181Y, L188R) or (S72G, A74V, V178W, L188F) or (S72G, A74V, V178W, L188K) or (S72G, A74V, V178W, L188R) or (S72G, A74Y) or (S72G, L181Y) or (S72G, L181Y, L188F) or (S72G, L181Y, L188K) or (S72G, L181Y, L188R) or (S72G, L188F) or (S72G, L188K) or (S72G, L188R) or (S72G, M177Y, V178W) or (S72G, M177Y, V178W, L188K) or (S72G, T146F) or (S72G, T146F, L181Y) or (S72G, T146F, L181Y, L188F) or (S72G, T146F, L181Y, L188K) or (S72G, T146F, L181Y, L188R) or (S72G, T146F, L188F) or (S72G, T146F, L188K) or (S72G, T146F, L188R) or (S72G, T146F, V178P) or (S72G, T146F, V178P, L181Y) or (S72G, T146F, V178P, L181Y, L188F) or (S72G, T146F, V178P, L181Y, L188K) or (S72G, T146F, V178P, L181Y, L188R) or (S72G, T146F, V178P, L188F) or (S72G, T146F, V178P, L188K) or (S72G, T146F, V178P, L188R) or (S72G, T146F, V178W) or (S72G, T146F, V178W, L181Y) or (S72G, T146F, V178W, L181Y, L188F) or (S72G, T146F, V178W, L181Y, L188K) or (S72G, T146F, V178W, L188F) or (S72G, T146F, V178W, L188K) or (S72G, T146F, V178W, L188R) or (S72G, V178P) or (S72G, V178P, L181Y) or (S72G, V178P, L181Y, L188F) or (S72G, V178P, L181Y, L188K) or (S72G, V178P, L181Y, L188R) or (S72G, V178P, L188F) or (S72G, V178P, L188K) or (S72G, V178P, L188R) or (S72G, V178W) or (S72G, V178W, L181Y) or (S72G, V178W, L181Y, L188F) or (S72G, V178W, L181Y, L188K) or (S72G, V178W, L181Y, L188R) or (S72G, V178W, L188F) or (S72G, V178W, L188K) or (S72G, V178W, L188R) or (S72H, A74C) or (S72N, A74C) or (S72N, A74I) or (S72N, A74V) or (S72W) or (S72W, A74V) or (S89C) or (S89E) or (S89G) or (S89I) or (S89L) or (S89N) or (S89Q) or (S89R) or (S89T) or (S89V) or (T146A) or (T146A, D222Y, I263M) or (T146A, F173D) or (T146A, F173V) or (T146A, F205G) or (T146A, F261C) or (T146A, F261S) or (T146A, F261V) or (T146A, G271S) or (T146A, G271T) or (T146A, H266V) or (T146A, I258T, I259S) or (T146A, I259H) or (T146A, I263F) or (T146A, I263L) or (T146A, I263N) or (T146A, I263V) or (T146A, L150A) or (T146A, L150G) or (T146A, L150K) or (T146A, L150R, F173I) or (T146A, L272H) or (T146A, L272S) or (T146A, L272W) or (T146A, M212A) or (T146C) or (T146D) or (T146E) or (T146F) or (T146F, L181Y) or (T146F, L181Y, L188F) or (T146F, L181Y, L188K) or (T146F, L188K) or (T146F, L188R) or (T146F, V178P) or (T146F, V178P, L181Y, L188R) or (T146F, V178P, L188K) or (T146F, V178P, L188R) or (T146F, V178W) or (T146F, V178W, L181Y, L188R) or (T146F, V178W, L188F) or (T146F, V178W, L188K) or (T146F, V178W, L188R) or (T146G) or (T146H) or (T146L) or (T146M) or (T146N) or (T146P) or (T146R) or (T146S) or (T146V) or (T146V, A191T) or (T146Y) or (T149P) or (T260W) or (T268A) or (T268S) or (T269K) or (T327A) or (T327A, A330W) or (T327C) or (T327D) or (T327E) or (T327I) or (T327L) or (T327M) or (T327N) or (T327P) or (T327Q) or (T327S) or (T327V) or (T365C) or (T365D) or (T365F) or (T365G) or (T365H) or (T365I) or (T365K) or (T365L) or (T365N) or (T365P) or (T365Q) or (T365V) or (T365W) or (T365Y) or (T436A) or (T436C) or (T436D) or (T436E) or (T436F) or (T436G) or (T436H) or (T436I) or (T436K) or (T436L) or (T436M) or (T436N) or (T436P) or (T436Q) or (T436R) or (T436S) or (T436V) or (T436W) or (T436Y) or (T438A) or (T438C) or (T438I) or (T438S) or (T438V) or (T49A) or (T49C) or (T49D) or (T49E) or (T49F) or (T49G) or (T49H) or (T49I) or (T49K) or (T49L) or (T49L, S54N) or (T49M) or (T49N) or (T49P) or (T49Q) or (T49R) or (T49R, R50C) or (T49R, R50S, M177Y, V178W, A184Y) or (T49S) or (T49V) or (T49W) or (T49Y) or (T88C) or (T88S) or (T88V) or (V178A) or (V178D) or (V178H) or (V178I) or (V178K) or (V178L) or (V178M) or (V178N) or (V178P) or (V178P, L181Y) or (V178P, L181Y, L188R) or (V178P, L188F) or (V178P, L188K) or (V178P, L188R) or (V178Q) or (V178R) or (V178S) or (V178T) or (V178W) or (V178W, A184Y) or (V178W, L181Y, L188K) or (V178W, L181Y, L188R) or (V178W, L188R) or (V178W, R50S) or (V178Y) or (V26A) or (V26C) or (V26F) or (V26G) or (V26H) or (V26I) or (V26K) or (V26L) or (V26M) or (V26N) or (V26N, R50S, M177Y, V178W, A184Y) or (V26Q) or (V26S) or (V26T) or (V26W) or (V26Y) or (V314A) or (V314E) or (V314F) or (V314H) or (V314P) or (V314Q) or (V314S) or (V314Y) or (V371P) or (V48A) or (V48D) or (V48E) or (V48F) or (V48G) or (V48H) or (V48I) or (V48L) or (V48M) or (V48P) or (V48Q) or (V48R) or (V48S) or (V48T) or (V48W) or (V48Y) or (W325A) or (W325C) or (W325D) or (W325E) or (W325F) or (W325G) or (W325I) or (W325K) or (W325L) or (W325M) or (W325Q) or (W325S) or (W325T) or (W325V) or (W325Y) or (W90Y) or (Y51A) or (Y51C) or (Y51F) or (Y51H) or (Y51I) or (Y51L) or (Y51M) or (Y51P) or (Y51S, F77V) or (Y51T) or (Y51V) or (Y51W).

There is provided a second preferred subset comprising variants according to the **sixth embodiment,** wherein the P450-BM3 variants have an improved **selectivity factor** for C19 hydroxylation in comparison with the parent variant (see figure 1b). Comprised within this subset are variants comprising at least the mutation(s) (A184D) or (A184D, L188K) or (A184E) or (A184F) or (A184G) or (A184H) or (A184I) or (A184K) or (A184L) or (A184N) or (A184Q) or (A184R) or (A184W) or (A184W, L188F) or (A184Y) or (A184Y, L188K) or (A184Y, R50S) or (A221S) or (A264V) or (A321C) or (A321D) or (A321E) or (A321G) or (A321I) or (A321N) or (A321T) or (A321V) or (A328P) or (A330C) or (A330D) or (A330E) or (A330F) or (A330G) or (A330H) or (A330I) or (A330K) or (A330L) or (A330M) or (A330N) or (A330Q) or (A330R) or (A330S) or (A330T) or (A330T, E352A) or (A330T, F331V) or (A330V) or (A330W) or (A330Y) or (A330Y, W367C) or (A33V) or (A399C) or (A399E) or (A399G) or (A399I) or (A399L) or (A399M) or (A399N) or (A399Q) or (A399R) or (A399S) or (A399T) or (A399V) or (A74C) or (A74C, L75V) or (A74D) or (A74D, F81V) or (A74E) or (A74F) or (A74G) or (A74G, L75C) or (A74H) or (A74I) or (A74I, A184W, L188F) or (A74I, F77A, L181Y, A184W) or (A74I, L181Y, L188K) or (A74I, L181Y, L188R) or (A74I, L188F) or (A74I, L188K) or (A74I, L188R) or (A74I, M177Y, A184R, L188F) or (A74I, M177Y, V178P, A184G) or (A74I, T146F) or (A74I, T146F, L181Y) or (A74I, T146F, L181Y, L188K) or (A74I, T146F, L181Y, L188R) or (A74I, T146F, L188F) or (A74I, T146F, L188K) or (A74I, T146F, V178P) or (A74I, T146F, V178P, L181Y, L188F) or (A74I, T146F, V178P, L181Y, L188K) or (A74I, T146F, V178P, L188K) or (A74I, T146F, V178P, L188R) or (A74I, T146F, V178W) or (A74I, T146F, V178W, L181Y, L188F) or (A74I, T146F, V178W, L181Y, L188R) or (A74I, T146F, V178W, L188F) or (A74I, T146F, V178W, L188K) or (A74I, T146F, V178W, L188R) or (A74I, V178P) or (A74I, V178P, L181Y) or (A74I, V178P, L181Y, L188R) or (A74I, V178P, L188F) or (A74I, V178P, L188K) or (A74I, V178P, L188R) or (A74I, V178W, L188F) or (A74I, V178W, L188K) or (A74I, V178W, L188R) or (A74K) or (A74L) or (A74M) or (A74N) or (A74P) or (A74Q) or (A74R) or (A74R, L75V) or (A74S) or (A74T) or (A74T, A184D) or (A74T, F77A, L181Y, A184D, L188K) or (A74T, F77A, V178W, A184Y) or (A74T, F77L, L181Y, A184D, L188K) or (A74T, L181Y) or (A74T, L181Y, A184D, L188K) or (A74T, L181Y, A184Y, L188K) or (A74T, L181Y, L188K) or (A74T, L181Y, L188R) or (A74T, L188K) or (A74T, M177Y, L181Y, A184D, L188K) or (A74T, T146F, L181Y, A184D, L188K) or (A74T, T146F, L181Y, L188K) or (A74T, T146F, L181Y, L188R) or (A74T, T146F, L188F) or (A74T, T146F, L188K) or (A74T, T146F, L188R) or (A74T, T146F, V178P) or (A74T, T146F, V178P, L181Y) or (A74T, T146F, V178P, L188K) or (A74T, T146F, V178P, L188R) or (A74T, T146F, V178W) or (A74T, T146F, V178W, L181Y) or (A74T, T146F, V178W, L181Y, L188K) or (A74T, T146F, V178W, L188R) or (A74T, V178P) or (A74T, V178P, L188F) or (A74T, V178P, L188K) or (A74T, V178P, L188R) or (A74T, V178W, L181Y, L188K) or (A74T, V178W, L188K) or (A74T, V178W, L188R) or (A74V) or (A74V, F77A, A184N, L188F) or (A74V, L181Y) or (A74V, L181Y, A184D, L188K) or (A74V, L181Y, A184W, L188F) or (A74V, L181Y, L188F) or (A74V, L188F) or (A74V, L188K) or (A74V, L75V) or (A74V, T146F, L181Y, L188K) or (A74V, T146F, L181Y, L188R) or (A74V, T146F, L188K) or (A74V, T146F, L188R) or (A74V, T146F, V178W) or (A74V, T146F, V178W, L181Y, L188F) or (A74V, V178P, A184W, L188K) or (A74V, V178P, L181Y, L188F) or (A74V, V178P, L181Y, L188K) or (A74V, V178P, L181Y, L188R) or (A74V, V178P, L188F) or (A74V, V178W, L188K) or (A74W) or (A74Y) or (D168Y, F173D) or (D363G, T438C) or (E13D, R47L) or (E143A) or (E143C) or (E143D) or (E143F) or (E143G) or (E143I) or (E143K) or (E143L) or (E143M) or (E143N) or (E143P) or (E143Q) or (E143R) or (E143S) or (E143V) or (E143W) or (E143Y) or (E267A) or (E267C) or (E267D) or (E267G) or (E267K) or (E267P) or (E267R) or (E267S) or (E267T) or (E267Y) or (E352A) or (E352D) or (E352F) or (E352G) or (E352I) or (E352L) or (E352M) or (E352N) or (E352P) or (E352R) or (E352S) or (E352T) or (E352V) or (E352W) or (E352Y) or (E64A) or (E64G) or (E64H) or (E64I) or (E64K) or (E64L) or (E64M) or (E64N) or (E64Q) or (E64V) or (E64W) or (E64Y) or (E82P) or (E93G) or (F173C) or (F173C, F205G) or (F173D) or (F173I) or (F173K) or (F173L) or (F173M) or (F173N) or (F173P) or (F173Q) or (F173R) or (F173S) or (F173S, F205P) or (F173V) or (F173W) or (F173Y) or (F173Y, I174F) or (F205A) or (F205C) or (F205D) or (F205D, D208N) or (F205E) or (F205G) or (F205H) or (F205I) or (F205K) or (F205L) or (F205M) or (F205N) or (F205P) or (F205R) or (F205S) or (F205T) or (F205V) or (F205W) or (F205Y) or (F261A) or (F261C) or (F261D) or (F261G) or (F261I) or (F261L) or (F261M) or (F261N) or (F261Q) or (F261S) or (F261T) or (F261V) or (F261W) or (F261Y) or (F331C) or (F331H) or (F331I) or (F331L) or (F331M) or (F331N) or (F331P) or (F331T) or (F331V) or (F331W) or (F331Y) or (F393M) or (F393W) or (F77A) or (F77A, A184W) or (F77A, L181P, A184Y, L188K) or (F77A, M177Y) or (F77C) or (F77D) or (F77E) or (F77G) or (F77H) or (F77I) or (F77K) or (F77L) or (F77M) or (F77N) or (F77P) or (F77R) or (F77S) or (F77T) or (F77V) or (F77V, L86M) or (F77W) or (F77Y) or (F81A) or (F81C) or (F81D) or (F81I) or (F81L) or (F81P) or (F81R) or (F81S) or (F81T) or (F81V) or (F81W) or (F81Y) or (G271A) or (G271C) or (G271 D) or (G271E) or (G271F) or (G271H) or (G271K) or (G271L) or (G271M) or (G271N) or (G271P) or (G271Q) or (G271R) or (G271S) or (G271T) or (G271V) or (G271W) or (G271Y) or (G402A) or (G415A) or (G415D) or (G415S) or (G415T) or (G415V) or (G85A) or (G85L) or (G85S) or (H266A) or (H266C) or (H266D) or (H266E) or (H266F) or (H266G) or (H266I) or (H266K) or (H266M) or (H266N) or (H266P) or (H266Q) or (H266R) or (H266S) or (H266T) or (H266V) or (H266W) or (H266Y) or (I153F, G271L) or (I153L) or (I153L, F173Y) or (I259A) or (I259C) or (I259D) or (I259F) or (I259G) or (I259H) or (I259K) or (I259L) or (I259M) or (I259N) or (I259Q) or (I259S) or (I259T) or (I259V) or (I259W) or (I259Y) or (I263A) or (I263C) or (I263E) or (I263F) or (I263G) or (I263H, A264G) or (I263K) or (I263L) or (I263M) or (I263N) or (I263Q) or (I263S) or (I263T) or (I263V) or (I263Y) or (I401A) or (I401L) or (I401M) or (I401T) or (I401V) or (K210E) or (K210T, G271V) or (K224C) or (K224E) or (K224F) or (K224H) or (K224I) or (K224L) or (K224M) or (K224P) or (K224Q) or (K224W) or (K224Y) or (L150A) or (L150C) or (L150D) or (L150E) or (L150F) or (L150G) or (L150H) or (L150I) or (L150K) or (L150M) or (L150N) or (L150Q) or (L150R) or (L150R, F205R) or (L150S) or (L150S, F173L) or (L150T) or (L150V) or (L150W) or (L150Y) or (L181H) or (L181I) or (L181M) or (L181P) or (L181V) or (L181Y) or (L181Y, A184D) or (L181Y, L188F) or (L181Y, L188K) or (L181Y, L188K, H659R) or (L188A) or (L188D) or (L188E) or (L188F) or (L188H) or (L188I) or (L188K) or (L188M) or (L188N) or (L188Q) or (L188R) or (L188S) or (L188W) or (L20C) or (L20D) or (L20E) or (L20F) or (L20G) or (L20G, R47L) or (L20I) or (L20M) or (L20N) or (L20P) or (L20R) or (L20S) or (L20T) or (L20V) or (L20W) or (L20Y) or (L262I) or (L262V) or (L262W) or (L262Y) or (L272A) or (L272C) or (L272E) or (L272F) or (L272G) or (L272I) or (L272K) or (L272M) or (L272N) or (L272Q) or (L272R) or (L272S) or (L272T) or (L272V) or (L272W) or (L272Y) or (L29A) or (L29C) or (L29D) or (L29F) or (L29H) or (L29I) or (L29M) or (L29M, R47G) or (L29P) or (L29Q) or (L29S) or (L29T) or (L29V) or (L29W) or (L29Y) or (L324F) or (L356C) or (L356F) or (L356H) or (L356I) or (L356M) or (L356N) or (L356Q) or (L356S) or (L356T) or (L356V) or (L356W) or (L437I) or (L437M) or (L75H, F81S) or (L75I) or (L75I, F81C) or (L75I, F81G) or (L75I, F81H) or (L75I, F81I) or (L75I, F81L) or (L75I, F81S) or (L75I, F81V) or (L75I, F81Y) or (L75V) or (L75V, F81C) or (L75V, F81H) or (L75V, F81I) or (L75V, F81L) or (L75V, F81V) or (L75V, F81Y) or (L78F) or (L78I) or (L78M) or (L78V) or (L78Y) or (L86A, S89T) or (L86I) or (L86I, S89T) or (L86M) or (L86M, S89T) or (L86N, S89T) or (L86V) or (L86V, S89T) or (M118A) or (M118E) or (M118F) or (M118G) or (M118H) or (M1181) or (M118K) or (M118L) or (M118N) or (M118P) or (M118Q) or (M118S) or (M118T) or (M118V) or (M118W) or (M118Y) or (M177A) or (M177C) or (M177C, V178Y) or (M177D) or (M177E) or (M177F) or (M177H) or (M177I) or (M177K) or (M177L) or (M177N) or (M177P) or (M177Q) or (M177R) or (M177T) or (M177V) or (M177Y) or (M177Y, A184W) or (M177Y, A184Y) or (M177Y, M185V) or (M177Y, R50S) or (M177Y, V178P) or (M177Y, V178P, A184Y, L188F) or (M177Y, V178W) or (M177Y, V178W, A184Y) or (M177Y, V178W, A184Y, L188F) or (M185C) or (M185D) or (M185E) or (M185G) or (M185H) or (M185K) or (M185L) or (M185N) or (M185Q) or (M185R) or (M185V) or (M185Y) or (M212A) or (M212C) or (M212C, I259L) or (M212D) or (M212E) or (M212F) or (M212G) or (M212H) or (M212K) or (M212L) or (M212L, I259F) or (M212P) or (M212Q) or (M212R) or (M212S) or (M212T) or (M212V) or (M212W) or (M212Y) or (M354A) or (M354C) or (M354D) or (M354E) or (M354G) or (M354I) or (M354K) or (M354K, D363Y) or (M354L) or (M354N) or (M354Q) or (M354R) or (M354S) or (M354T) or (M354V) or (M354W) or (M354Y) or (N70A) or (N70C) or (N70F) or (N70G) or (N70H) or (N70K) or (N70R) or (N70W) or (N70Y) or (P243T, H266L) or (P326C) or (P326D) or (P326G) or (P326N) or (P326S) or (P326T) or (P329A) or (P329C) or (P329G) or (P329K) or (P329S) or (P329T) or (P392C) or (P392G) or (P392Q) or (P392R) or (P392V) or (P9S) or (R147C, H266G) or (R161C, G271D) or (R190L, F261V) or (R203C) or (R255A) or (R255F) or (R255G) or (R255I) or (R255K) or (R255L) or (R255M) or (R255N) or (R255P) or (R255T) or (R255W) or (R323C) or (R47A) or (R47C) or (R47D) or (R47E) or (R47F) or (R47G) or (R47H) or (R47H, A74W) or (R47I) or (R47K) or (R47L) or (R47M) or (R47N) or (R47P) or (R47Q) or (R47S) or (R47T) or (R47V) or (R47W) or (R50A) or (R50C) or (R50D) or (R50E) or (R50F) or (R50G) or (R50I) or (R50L) or (R50M) or (R50N) or (R50P) or (R50Q) or (R50S) or (R50S, A184W, L188F) or (R50S, A74I, A184W, L188F) or (R50S, A74M, A184Y, L188K) or (R50S, A74T, L181Y, A184D, L188K, G240R) or (R50S, A74V) or (R50S, M177Y, V178P, A184Y) or (R50S, M177Y, V178W, A184W) or (R50S, M177Y, V178W, A184Y) or (R50S, M177Y, V178W, A184Y, A330R) or (R50S, M177Y, V178W, A184Y, A74V, G677D) or (R50S, M177Y, V178W, A184Y, H266S) or (R50S, M177Y, V178W, A184Y, L181P) or (R50S, M177Y, V178W, A184Y, L181Y) or (R50S, M177Y, V178W, A184Y, L188K) or (R50S, M177Y, V178W, A184Y, L188R) or (R50S, M177Y, V178W, A184Y, T146F) or (R50S, M177Y, V178W, A184Y, V26N) or (R50S, M177Y, V178W, L181Y, A184Y) or (R50S, S72G, A74V) or (R50S, S72G, A74V, M177Y, A184Y) or (R50S, S72G, A74V, M177Y, V178P, A184Y) or (R50S, S72G, A74V, M177Y, V178W, A184Y) or (R50S, S72G, A74V, M177Y, V178W, A184Y, L188R) or (R50S, T146F, M177Y, V178W, A184Y) or (R50V) or (R50Y) or (S164N, F261L) or (S176A, A184Y, L188K) or (S176C) or (S176D) or (S176E) or (S176F) or (S176G) or (S176H) or (S176K) or (S176L) or (S176M) or (S176N) or (S176P) or (S176Q) or (S176R) or (S72C, A74C) or (S72C, A74I) or (S72C, A74L) or (S72C, A74V) or (S72C, A74Y) or (S72D, A74C) or (S72D, A74F) or (S72G) or (S72G, A74C) or (S72G, A74F) or (S72G, A74H) or (S72G, A74I) or (S72G, A74I, A184W, L188F) or (S72G, A74I, F77A, M177Y) or (S72G, A74I, F77L) or (S72G, A74I, F77L, M177Y) or (S72G, A74I, L181Y) or (S72G, A74I, L181Y, L188F) or (S72G, A74I, L181Y, L188K) or (S72G, A74I, L181Y, L188R) or (S72G, A74I, L188F) or (S72G, A74I, L188K) or (S72G, A74I, L188R) or (S72G, A74I, T146F) or (S72G, A74I, T146F, L181Y) or (S72G, A74I, T146F, L181Y, L188F) or (S72G, A74I, T146F, L181Y, L188K) or (S72G, A74I, T146F, L181Y, L188R) or (S72G, A74I, T146F, L188F) or (S72G, A74I, T146F, L188K) or (S72G, A74I, T146F, L188R) or (S72G, A74I, T146F, V178P) or (S72G, A74I, T146F, V178P, L181Y) or (S72G, A74I, T146F, V178P, L181Y, L188F) or (S72G, A74I, T146F, V178P, L181Y, L188K) or (S72G, A74I, T146F, V178P, L181Y, L188R) or (S72G, A74I, T146F, V178P, L188F) or (S72G, A74I, T146F, V178P, L188K) or (S72G, A74I, T146F, V178P, L188R) or (S72G, A74I, T146F, V178W) or (S72G, A74I, T146F, V178W, L181Y) or (S72G, A74I, T146F, V178W, L181Y, L188F) or (S72G, A74I, T146F, V178W, L181Y, L188K) or (S72G, A74I, T146F, V178W, L181Y, L188R) or (S72G, A74I, T146F, V178W, L188F) or (S72G, A74I, T146F, V178W, L188K) or (S72G, A74I, T146F, V178W, L188R) or (S72G, A74I, V178P) or (S72G, A74I, V178P, L181Y) or (S72G, A74I, V178P, L181Y, L188K) or (S72G, A74I, V178P, L181Y, L188R) or (S72G, A74I, V178P, L188F) or (S72G, A74I, V178P, L188K) or (S72G, A74I, V178P, L188R) or (S72G, A74I, V178W) or (S72G, A74I, V178W, L181Y, L188F) or (S72G, A74I, V178W, L181Y, L188R) or (S72G, A74I, V178W, L188F) or (S72G, A74I, V178W, L188K) or (S72G, A74I, V178W, L188R) or (S72G, A74L) or (S72G, A74M, A184Y, L188K) or (S72G, A74S) or (S72G, A74T) or (S72G, A74T, L181Y, A184D, L188K) or (S72G, A74T, L181Y, L188F) or (S72G, A74T, L181Y, L188K) or (S72G, A74T, L181Y, L188R) or (S72G, A74T, L188F) or (S72G, A74T, L188K) or (S72G, A74T, L188R) or (S72G, A74T, T146F) or (S72G, A74T, T146F, L181Y) or (S72G, A74T, T146F, L181Y, L188K) or (S72G, A74T, T146F, L181Y, L188R) or (S72G, A74T, T146F, L188F) or (S72G, A74T, T146F, L188K) or (S72G, A74T, T146F, L188R) or (S72G, A74T, T146F, V178P) or (S72G, A74T, T146F, V178P, L181Y) or (S72G, A74T, T146F, V178P, L188F) or (S72G, A74T, T146F, V178P, L188K) or (S72G, A74T, T146F, V178P, L188R) or (S72G, A74T, T146F, V178W) or (S72G, A74T, T146F, V178W, L181Y) or (S72G, A74T, T146F, V178W, L181Y, L188F) or (S72G, A74T, T146F, V178W, L181Y, L188K) or (S72G, A74T, T146F, V178W, L181Y, L188R) or (S72G, A74T, T146F, V178W, L188F) or (S72G, A74T, T146F, V178W, L188K) or (S72G, A74T, T146F, V178W, L188R) or (S72G, A74T, V178P) or (S72G, A74T, V178P, L181Y) or (S72G, A74T, V178P, L181Y, L188F) or (S72G, A74T, V178P, L181Y, L188K) or (S72G, A74T, V178P, L188F) or (S72G, A74T, V178P, L188K) or (S72G, A74T, V178P, L188R) or (S72G, A74T, V178W) or (S72G, A74T, V178W, L181Y, L188F) or (S72G, A74T, V178W, L181Y, L188K) or (S72G, A74T, V178W, L188F) or (S72G, A74T, V178W, L188K) or (S72G, A74T, V178W, L188R) or (S72G, A74V) or (S72G, A74V, A184W) or (S72G, A74V, A221V) or (S72G, A74V, F77A) or (S72G, A74V, F77A, M177Y) or (S72G, A74V, F77L, M177Y) or (S72G, A74V, F77S) or (S72G, A74V, L181Y) or (S72G, A74V, L181Y, L188F) or (S72G, A74V, L181Y, L188K) or (S72G, A74V, L188F) or (S72G, A74V, L188K) or (S72G, A74V, L188R) or (S72G, A74V, T146F) or (S72G, A74V, T146F, L181Y) or (S72G, A74V, T146F, L181Y, L188F) or (S72G, A74V, T146F, L181Y, L188K) or (S72G, A74V, T146F, L181Y, L188R) or (S72G, A74V, T146F, L188F) or (S72G, A74V, T146F, L188K) or (S72G, A74V, T146F, L188R) or (S72G, A74V, T146F, V178P) or (S72G, A74V, T146F, V178P, L181Y) or (S72G, A74V, T146F, V178P, L181Y, L188F) or (S72G, A74V, T146F, V178P, L181Y, L188K) or (S72G, A74V, T146F, V178P, L181Y, L188R) or (S72G, A74V, T146F, V178P, L188F) or (S72G, A74V, T146F, V178P, L188K) or (S72G, A74V, T146F, V178P, L188R) or (S72G, A74V, T146F, V178W) or (S72G, A74V, T146F, V178W, L181Y) or (S72G, A74V, T146F, V178W, L181Y, L188K) or (S72G, A74V, T146F, V178W, L181Y, L188R) or (S72G, A74V, T146F, V178W, L188F) or (S72G, A74V, T146F, V178W, L188K) or (S72G, A74V, T146F, V178W, L188R) or (S72G, A74V, V178P) or (S72G, A74V, V178P, L181Y) or (S72G, A74V, V178P, L181Y, L188F) or (S72G, A74V, V178P, L181Y, L188K) or (S72G, A74V, V178P, L181Y, L188R) or (S72G, A74V, V178P, L188F) or (S72G, A74V, V178P, L188K) or (S72G, A74V, V178P, L188R) or (S72G, A74V, V178W) or (S72G, A74V, V178W, L181Y) or (S72G, A74V, V178W, L181Y, L188F) or (S72G, A74V, V178W, L181Y, L188K) or (S72G, A74V, V178W, L181Y, L188R) or (S72G, A74V, V178W, L188F) or (S72G, A74V, V178W, L188K) or (S72G, A74V, V178W, L188R) or (S72G, A74Y) or (S72G, L181Y) or (S72G, L181Y, L188F) or (S72G, L181Y, L188K) or (S72G, L181Y, L188R) or (S72G, L188F) or (S72G, L188K) or (S72G, L188R) or (S72G, M177Y, V178W) or (S72G, M177Y, V178W, L188K) or (S72G, T146F) or (S72G, T146F, L181Y) or (S72G, T146F, L181Y, L188F) or (S72G, T146F, L181Y, L188K) or (S72G, T146F, L181Y, L188R) or (S72G, T146F, L188F) or (S72G, T146F, L188K) or (S72G, T146F, L188R) or (S72G, T146F, V178P) or (S72G, T146F, V178P, L181Y) or (S72G, T146F, V178P, L181Y, L188F) or (S72G, T146F, V178P, L181Y, L188K) or (S72G, T146F, V178P, L181Y, L188R) or (S72G, T146F, V178P, L188F) or (S72G, T146F, V178P, L188K) or (S72G, T146F, V178P, L188R) or (S72G, T146F, V178W) or (S72G, T146F, V178W, L181Y) or (S72G, T146F, V178W, L181Y, L188F) or (S72G, T146F, V178W, L181Y, L188K) or (S72G, T146F, V178W, L188F) or (S72G, T146F, V178W, L188K) or (S72G, T146F, V178W, L188R) or (S72G, V178P) or (S72G, V178P, L181Y) or (S72G, V178P, L181Y, L188F) or (S72G, V178P, L181Y, L188K) or (S72G, V178P, L181Y, L188R) or (S72G, V178P, L188F) or (S72G, V178P, L188K) or (S72G, V178P, L188R) or (S72G, V178W) or (S72G, V178W, L181Y) or (S72G, V178W, L181Y, L188F) or (S72G, V178W, L181Y, L188K) or (S72G, V178W, L181Y, L188R) or (S72G, V178W, L188F) or (S72G, V178W, L188K) or (S72G, V178W, L188R) or (S72H, A74C) or (S72H, A74G) or (S72H, A74S) or (S72H, A74Y) or (S72N, A74C) or (S72N, A74I) or (S72N, A74N) or (S72N, A74V) or (S72T) or (S72W) or (S72W, A74I, A184W, L188F) or (S72W, A74V) or (S72W, L333R) or (S72W, W90Y, V299G) or (S72Y, A74V) or (S89C) or (S89E) or (S89G) or (S89I) or (S89L) or (S89M) or (S89N) or (S89Q) or (S89R) or (S89T) or (S89V) or (T146A) or (T146A, D222Y, I263M) or (T146A, F173D) or (T146A, F173V) or (T146A, F205G) or (T146A, F261C) or (T146A, F261S) or (T146A, F261V) or (T146A, G271R) or (T146A, G271S) or (T146A, G271T) or (T146A, H266T) or (T146A, H266V) or (T146A, I258T, I259S) or (T146A, I259H) or (T146A, I263F) or (T146A, I263L) or (T146A, I263N) or (T146A, I263V) or (T146A, L150A) or (T146A, L150G) or (T146A, L150K) or (T146A, L150R, F173I) or (T146A, L272H) or (T146A, L272R) or (T146A, L272S) or (T146A, L272W) or (T146A, M212A) or (T146C) or (T146D) or (T146E) or (T146F) or (T146F, L181Y) or (T146F, L181Y, L188F) or (T146F, L181Y, L188K) or (T146F, L188K) or (T146F, L188R) or (T146F, V178P) or (T146F, V178P, L181Y, L188R) or (T146F, V178P, L188K) or (T146F, V178P, L188R) or (T146F, V178W) or (T146F, V178W, L181Y, L188R) or (T146F, V178W, L188F) or (T146F, V178W, L188K) or (T146F, V178W, L188R) or (T146G) or (T146H) or (T146L) or (T146M) or (T146N) or (T146P) or (T146R) or (T146S) or (T146V) or (T146V, A191T) or (T146Y) or (T149P) or (T260A) or (T260S) or (T268A) or (T268S) or (T269K) or (T327A) or (T327A, A330W) or (T327C) or (T327D) or (T327E) or (T327G) or (T327I) or (T327L) or (T327M) or (T327N) or (T327P) or (T327Q) or (T327S) or (T327V) or (T365A) or (T365C) or (T365D) or (T365F) or (T365G) or (T365H) or (T365I) or (T365K) or (T365L) or (T365N) or (T365P) or (T365Q) or (T365V) or (T365W) or (T365Y) or (T436A) or (T436C) or (T436D) or (T436E) or (T436F) or (T436G) or (T436H) or (T436I) or (T436K) or (T436L) or (T436M) or (T436N) or (T436P) or (T436Q) or (T436R) or (T436S) or (T436V) or (T436W) or (T436Y) or (T438A) or (T438C) or (T438G) or (T438I) or (T438L) or (T438S) or (T438V) or (T49A) or (T49C) or (T49D) or (T49E) or (T49F) or (T49G) or (T49H) or (T49I) or (T49K) or (T49L) or (T49L, S54N) or (T49M) or (T49N) or (T49P) or (T49Q) or (T49R) or (T49R, R50C) or (T49R, R50S, M177Y, V178W, A184Y) or (T49S) or (T49V) or (T49W) or (T49Y) or (T88A) or (T88C) or (T88S) or (T88V) or (V178D) or (V178F) or (V178H) or (V178I) or (V178K) or (V178L) or (V178M) or (V178N) or (V178P) or (V178P, L181Y) or (V178P, L181Y, L188R) or (V178P, L188F) or (V178P, L188K) or (V178P, L188R) or (V178Q) or (V178R) or (V178S) or (V178T) or (V178W) or (V178W, A184Y) or (V178W, L181Y, L188K) or (V178W, L181Y, L188R) or (V178W, L188R) or (V178W, R50S) or (V178Y) or (V26A) or (V26C) or (V26E) or (V26F) or (V26G) or (V26G, R47S) or (V26H) or (V26I) or (V26K) or (V26L) or (V26M) or (V26N) or (V26N, R50S, M177Y, V178W, A184Y) or (V26Q) or (V26R) or (V26S) or (V26T) or (V26W) or (V26Y) or (V314A) or (V314E) or (V314F) or (V314H) or (V314P) or (V314Q) or (V314S) or (V314Y) or (V48A) or (V48D) or (V48E) or (V48F) or (V48G) or (V48H) or (V48I) or (V48L) or (V48M) or (V48P) or (V48Q) or (V48R) or (V48S) or (V48T) or (V48W) or (V48Y) or (W325A) or (W325C) or (W325D) or (W325E) or (W325F) or (W325G) or (W325I) or (W325K) or (W325L) or (W325M) or (W325Q) or (W325R) or (W325S) or (W325T) or (W325V) or (W325Y) or (W90D) or (W90Y) or (W90Y, V299G, G570D) or (Y51A) or (Y51C) or (Y51E) or (Y51F) or (Y51G) or (Y51H) or (Y51I) or (Y51L) or (Y51M) or (Y51N) or (Y51P) or (Y51Q) or (Y51S) or (Y51S, F77V) or (Y51T) or (Y51V) or (Y51W).

There is provided a **third preferred subset** comprising variants according to the **sixth embodiment,** wherein the P450-BM3 variants have an improved **product yield and selectivity factor** for C19 hydroxylation in comparison with the parent variant. Comprised within this subset are variants comprising at least the mutation(s) (A184D) or (A184D, L188K) or (A184E) or (A184F) or (A184G) or (A184H) or (A184I) or (A184K) or (A184L) or (A184N) or (A184Q) or (A184R) or (A184W) or (A184W, L188F) or (A184Y) or (A184Y, L188K) or (A184Y, R50S) or (A221S) or (A264V) or (A321C) or (A321D) or (A321E) or (A321G) or (A321I) or (A321N) or (A321T) or (A321V) or (A330C) or (A330D) or (A330E) or (A330F) or (A330G) or (A330H) or (A330I) or (A330K) or (A330L) or (A330M) or (A330N) or (A330Q) or (A330R) or (A330S) or (A330T) or (A330T, E352A) or (A330V) or (A330W) or (A330Y) or (A330Y, W367C) or (A33V) or (A399C) or (A399E) or (A399G) or (A399I) or (A399L) or (A399M) or (A399N) or (A399Q) or (A399R) or (A399S) or (A399T) or (A399V) or (A74C) or (A74C, L75V) or (A74D) or (A74D, F81V) or (A74E) or (A74F) or (A74H) or (A74I) or (A74I, A184W, L188F) or (A74I, F77A, L181Y, A184W) or (A74I, L181Y, L188R) or (A74I, L188F) or (A74I, L188K) or (A74I, L188R) or (A74I, M177Y, A184R, L188F) or (A74I, M177Y, V178P, A184G) or (A74I, T146F) or (A74I, T146F, L181Y) or (A74I, T146F, L181Y, L188K) or (A74I, T146F, L181Y, L188R) or (A74I, T146F, L188F) or (A74I, T146F, L188K) or (A74I, T146F, V178P) or (A74I, T146F, V178P, L181Y, L188F) or (A74I, T146F, V178P, L181Y, L188K) or (A74I, T146F, V178P, L188K) or (A74I, T146F, V178P, L188R) or (A74I, T146F, V178W) or (A74I, T146F, V178W, L181Y, L188F) or (A74I, T146F, V178W, L181Y, L188R) or (A74I, T146F, V178W, L188F) or (A74I, T146F, V178W, L188K) or (A74I, T146F, V178W, L188R) or (A74I, V178P) or (A74I, V178P, L181Y) or (A74I, V178P, L181Y, L188R) or (A74I, V178P, L188F) or (A74I, V178P, L188K) or (A74I, V178P, L188R) or (A74I, V178W, L188F) or (A74I, V178W, L188R) or (A74K) or (A74L) or (A74M) or (A74N) or (A74P) or (A74Q) or (A74R) or (A74R, L75V) or (A74S) or (A74T) or (A74T, A184D) or (A74T, F77A, L181Y, A184D, L188K) or (A74T, F77A, V178W, A184Y) or (A74T, F77L, L181Y, A184D, L188K) or (A74T, L181Y) or (A74T, L181Y, A184D, L188K) or (A74T, L181Y, A184Y, L188K) or (A74T, L181Y, L188K) or (A74T, L181Y, L188R) or (A74T, L188K) or (A74T, M177Y, L181Y, A184D, L188K) or (A74T, T146F, L181Y, A184D, L188K) or (A74T, T146F, L181Y, L188K) or (A74T, T146F, L181Y, L188R) or (A74T, T146F, L188F) or (A74T, T146F, L188K) or (A74T, T146F, L188R) or (A74T, T146F, V178P) or (A74T, T146F, V178P, L181Y) or (A74T, T146F, V178P, L188K) or (A74T, T146F, V178P, L188R) or (A74T, T146F, V178W) or (A74T, T146F, V178W, L181Y) or (A74T, T146F, V178W, L181Y, L188K) or (A74T, T146F, V178W, L188R) or (A74T, V178P) or (A74T, V178P, L188F) or (A74T, V178P, L188K) or (A74T, V178P, L188R) or (A74T, V178W, L181Y, L188K) or (A74T, V178W, L188K) or (A74T, V178W, L188R) or (A74V) or (A74V, F77A, A184N, L188F) or (A74V, L181Y) or (A74V, L181Y, A184D, L188K) or (A74V, L181Y, A184W, L188F) or (A74V, L181Y, L188F) or (A74V, L188F) or (A74V, L188K) or (A74V, L75V) or (A74V, T146F, L181Y, L188K) or (A74V, T146F, L181Y, L188R) or (A74V, T146F, L188K) or (A74V, T146F, L188R) or (A74V, T146F, V178W, L181Y, L188F) or (A74V, V178P, A184W, L188K) or (A74V, V178P, L181Y, L188F) or (A74V, V178P, L181Y, L188K) or (A74V, V178P, L181Y, L188R) or (A74V, V178P, L188F) or (A74V, V178W, L188K) or (A74W) or (A74Y) or (D363G, T438C) or (E13D, R47L) or (E143A) or (E143C) or (E143D) or (E143F) or (E143G) or (E143I) or (E143K) or (E143L) or (E143M) or (E143N) or (E143P) or (E143Q) or (E143R) or (E143S) or (E143V) or (E143W) or (E143Y) or (E267D) or (E267R) or (E267T) or (E352A) or (E352D) or (E352F) or (E352G) or (E352I) or (E352L) or (E352M) or (E352N) or (E352P) or (E352R) or (E352S) or (E352T) or (E352V) or (E352W) or (E352Y) or (E64A) or (E64G) or (E64H) or (E64K) or (E64L) or (E64M) or (E64N) or (E64Q) or (E64V) or (E64Y) or (E82P) or (E93G) or (F173C) or (F173C, F205G) or (F173D) or (F173I) or (F173K) or (F173L) or (F173M) or (F173N) or (F173P) or (F173Q) or (F173R) or (F173S) or (F173S, F205P) or (F173V) or (F173W) or (F173Y) or (F173Y, I174F) or (F205A) or (F205C) or (F205D) or (F205D, D208N) or (F205E) or (F205G) or (F205H) or (F205I) or (F205K) or (F205L) or (F205M) or (F205N) or (F205P) or (F205R) or (F205S) or (F205T) or (F205V) or (F205W) or (F205Y) or (F261A) or (F261C) or (F261D) or (F261G) or (F261I) or (F261L) or (F261M) or (F261Q) or (F261S) or (F261T) or (F261V) or (F261Y) or (F331H) or (F331I) or (F331L) or (F331M) or (F331V) or (F331W) or (F331Y) or (F393M) or (F393W) or (F77A) or (F77A, A184W) or (F77A, L181P, A184Y, L188K) or (F77A, M177Y) or (F77C) or (F77D) or (F77E) or (F77G) or (F77H) or (F77I) or (F77K) or (F77L) or (F77M) or (F77N) or (F77P) or (F77R) or (F77S) or (F77T) or (F77V) or (F77V, L86M) or (F77W) or (F77Y) or (F81D) or (F81I) or (F81L) or (F81R) or (F81S) or (F81V) or (F81W) or (F81Y) or (G271A) or (G271C) or (G271D) or (G271E) or (G271F) or (G271H) or (G271K) or (G271L) or (G271M) or (G271N) or (G271P) or (G271Q) or (G271S) or (G271T) or (G271V) or (G271W) or (G271Y) or (G402A) or (G415A) or (G415D) or (G415S) or (G415T) or (G415V) or (G85A) or (G85L) or (G85S) or (H266A) or (H266C) or (H266E) or (H266F) or (H266I) or (H266K) or (H266M) or (H266N) or (H266Q) or (H266S) or (H266V) or (H266W) or (H266Y) or (I153F, G271L) or (I153L) or (I153L, F173Y) or (I259A) or (I259C) or (I259D) or (I259F) or (I259H) or (I259K) or (I259L) or (I259M) or (I259N) or (I259Q) or (I259S) or (I259T) or (I259V) or (I263A) or (I263C) or (I263E) or (I263F) or (I263H, A264G) or (I263K) or (I263L) or (I263M) or (I263N) or (I263Q) or (I263S) or (I263V) or (I263Y) or (I401A) or (I401M) or (I401T) or (I401V) or (K210E) or (K210T, G271V) or (K224C) or (K224E) or (K224H) or (K224I) or (K224L) or (K224M) or (K224P) or (K224Q) or (K224W) or (K224Y) or (L150A) or (L150C) or (L150D) or (L150E) or (L150F) or (L150G) or (L150H) or (L150I) or (L150K) or (L150M) or (L150N) or (L150Q) or (L150R) or (L150R, F205R) or (L150S) or (L150S, F173L) or (L150T) or (L150V) or (L150W) or (L150Y) or (L181H) or (L181I) or (L181M) or (L181P) or (L181V) or (L181Y) or (L181Y, A184D) or (L181Y, L188F) or (L181Y, L188K) or (L181Y, L188K, H659R) or (L188A) or (L188D) or (L188E) or (L188F) or (L188H) or (L188I) or (L188K) or (L188M) or (L188N) or (L188Q) or (L188R) or (L188S) or (L188W) or (L20C) or (L20D) or (L20E) or (L20F) or (L20G) or (L20G, R47L) or (L20I) or (L20M) or (L20N) or (L20P) or (L20R) or (L20S) or (L20T) or (L20V) or (L20W) or (L20Y) or (L262I) or (L262V) or (L262W) or (L272A) or (L272C) or (L272E) or (L272F) or (L272G) or (L272I) or (L272K) or (L272M) or (L272N) or (L272Q) or (L272S) or (L272T) or (L272V) or (L272W) or (L272Y) or (L29A) or (L29C) or (L29D) or (L29F) or (L29H) or (L29I) or (L29M) or (L29M, R47G) or (L29Q) or (L29S) or (L29T) or (L29V) or (L29W) or (L29Y) or (L324F) or (L356C) or (L356F) or (L356I) or (L356M) or (L356V) or (L437I) or (L437M) or (L75I) or (L75I, F81C) or (L75I, F81I) or (L75I, F81L) or (L75I, F81S) or (L75I, F81V) or (L75V) or (L75V, F81C) or (L75V, F81L) or (L75V, F81V) or (L75V, F81Y) or (L78F) or (L78I) or (L78M) or (L78V) or (L86I) or (L86I, S89T) or (L86M) or (L86M, S89T) or (L86V) or (M118A) or (M118E) or (M118F) or (M118G) or (M118H) or (M118I) or (M118K) or (M118L) or (M118N) or (M118P) or (M118Q) or (M118S) or (M118T) or (M118V) or (M118W) or (M118Y) or (M177A) or (M177C) or (M177C, V178Y) or (M177D) or (M177E) or (M177F) or (M177H) or (M177I) or (M177L) or (M177N) or (M177P) or (M177Q) or (M177R) or (M177T) or (M177V) or (M177Y) or (M177Y, A184W) or (M177Y, A184Y) or (M177Y, M185V) or (M177Y, R50S) or (M177Y, V178P) or (M177Y, V178P, A184Y, L188F) or (M177Y, V178W) or (M177Y, V178W, A184Y) or (M177Y, V178W, A184Y, L188F) or (M185C) or (M185D) or (M185E) or (M185G) or (M185H) or (M185K) or (M185L) or (M185N) or (M185Q) or (M185V) or (M212A) or (M212C) or (M212D) or (M212E) or (M212F) or (M212G) or (M212H) or (M212K) or (M212L) or (M212L, I259F) or (M212Q) or (M212R) or (M212S) or (M212T) or (M212V) or (M212W) or (M212Y) or (M354A) or (M354C) or (M354D) or (M354E) or (M354G) or (M354I) or (M354K) or (M354K, D363Y) or (M354L) or (M354N) or (M354Q) or (M354R) or (M354S) or (M354T) or (M354V) or (M354W) or (M354Y) or (N70A) or (N70C) or (N70F) or (N70G) or (N70H) or (N70K) or (N70R) or (N70Y) or (P243T, H266L) or (P329A) or (P392C) or (P392G) or (P392Q) or (P392R) or (P392V) or (P9S) or (R147C, H266G) or (R161C, G271D) or (R190L, F261V) or (R203C) or (R255A) or (R255G) or (R255I) or (R255K) or (R255L) or (R255M) or (R255N) or (R255P) or (R255T) or (R255W) or (R47A) or (R47C) or (R47D) or (R47E) or (R47F) or (R47G) or (R47H) or (R47H, A74W) or (R47I) or (R47K) or (R47L) or (R47M) or (R47N) or (R47P) or (R47Q) or (R47S) or (R47T) or (R47V) or (R47W) or (R50A) or (R50C) or (R50D) or (R50E) or (R50F) or (R50G) or (R50I) or (R50L) or (R50M) or (R50N) or (R50P) or (R50Q) or (R50S) or (R50S, A184W, L188F) or (R50S, A74I, A184W, L188F) or (R50S, A74M, A184Y, L188K) or (R50S, A74T, L181Y, A184D, L188K, G240R) or (R50S, A74V) or (R50S, M177Y, V178P, A184Y) or (R50S, M177Y, V178W, A184W) or (R50S, M177Y, V178W, A184Y) or (R50S, M177Y, V178W, A184Y, A330R) or (R50S, M177Y, V178W, A184Y, A74V, G677D) or (R50S, M177Y, V178W, A184Y, L181P) or (R50S, M177Y, V178W, A184Y, L181Y) or (R50S, M177Y, V178W, A184Y, L188K) or (R50S, M177Y, V178W, A184Y, L188R) or (R50S, M177Y, V178W, A184Y, T146F) or (R50S, M177Y, V178W, A184Y, V26N) or (R50S, M177Y, V178W, L181Y, A184Y) or (R50S, S72G, A74V) or (R50S, S72G, A74V, M177Y, A184Y) or (R50S, S72G, A74V, M177Y, V178P, A184Y) or (R50S, S72G, A74V, M177Y, V178W, A184Y) or (R50S, S72G, A74V, M177Y, V178W, A184Y, L188R) or (R50S, T146F, M177Y, V178W, A184Y) or (R50V) or (R50Y) or (S176A, A184Y, L188K) or (S176C) or (S176D) or (S176E) or (S176F) or (S176G) or (S176H) or (S176K) or (S176L) or (S176M) or (S176N) or (S176P) or (S176Q) or (S72C, A74C) or (S72C, A74I) or (S72C, A74L) or (S72C, A74V) or (S72D, A74C) or (S72G) or (S72G, A74C) or (S72G, A74F) or (S72G, A74H) or (S72G, A74I) or (S72G, A74I, A184W, L188F) or (S72G, A74I, F77A, M177Y) or (S72G, A74I, F77L) or (S72G, A74I, F77L, M177Y) or (S72G, A74I, L181Y) or (S72G, A74I, L181Y, L188F) or (S72G, A74I, L181Y, L188K) or (S72G, A74I, L181Y, L188R) or (S72G, A74I, L188F) or (S72G, A74I, L188K) or (S72G, A74I, L188R) or (S72G, A74I, T146F) or (S72G, A74I, T146F, L181Y) or (S72G, A74I, T146F, L181Y, L188F) or (S72G, A74I, T146F, L181Y, L188K) or (S72G, A74I, T146F, L181Y, L188R) or (S72G, A74I, T146F, L188F) or (S72G, A74I, T146F, L188K) or (S72G, A74I, T146F, V178P) or (S72G, A74I, T146F, V178P, L181Y) or (S72G, A74I, T146F, V178P, L181Y, L188F) or (S72G, A74I, T146F, V178P, L181Y, L188K) or (S72G, A74I, T146F, V178P, L181Y, L188R) or (S72G, A74I, T146F, V178P, L188F) or (S72G, A74I, T146F, V178P, L188K) or (S72G, A74I, T146F, V178P, L188R) or (S72G, A74I, T146F, V178W) or (S72G, A74I, T146F, V178W, L181Y) or (S72G, A74I, T146F, V178W, L181Y, L188F) or (S72G, A74I, T146F, V178W, L181Y, L188K) or (S72G, A74I, T146F, V178W, L181Y, L188R) or (S72G, A74I, T146F, V178W, L188F) or (S72G, A74I, T146F, V178W, L188K) or (S72G, A74I, T146F, V178W, L188R) or (S72G, A74I, V178P) or (S72G, A74I, V178P, L181Y) or (S72G, A74I, V178P, L181Y, L188R) or (S72G, A74I, V178P, L188F) or (S72G, A74I, V178P, L188K) or (S72G, A74I, V178P, L188R) or (S72G, A74I, V178W) or (S72G, A74I, V178W, L181Y, L188F) or (S72G, A74I, V178W, L181Y, L188R) or (S72G, A74I, V178W, L188F) or (S72G, A74I, V178W, L188R) or (S72G, A74L) or (S72G, A74M, A184Y, L188K) or (S72G, A74S) or (S72G, A74T) or (S72G, A74T, L181Y, A184D, L188K) or (S72G, A74T, L181Y, L188F) or (S72G, A74T, L181Y, L188K) or (S72G, A74T, L181Y, L188R) or (S72G, A74T, L188F) or (S72G, A74T, L188K) or (S72G, A74T, L188R) or (S72G, A74T, T146F) or (S72G, A74T, T146F, L181Y) or (S72G, A74T, T146F, L181Y, L188K) or (S72G, A74T, T146F, L181Y, L188R) or (S72G, A74T, T146F, L188F) or (S72G, A74T, T146F, L188K) or (S72G, A74T, T146F, L188R) or (S72G, A74T, T146F, V178P) or (S72G, A74T, T146F, V178P, L181Y) or (S72G, A74T, T146F, V178P, L188F) or (S72G, A74T, T146F, V178P, L188K) or (S72G, A74T, T146F, V178P, L188R) or (S72G, A74T, T146F, V178W) or (S72G, A74T, T146F, V178W, L181Y) or (S72G, A74T, T146F, V178W, L181Y, L188F) or (S72G, A74T, T146F, V178W, L181Y, L188K) or (S72G, A74T, T146F, V178W, L181Y, L188R) or (S72G, A74T, T146F, V178W, L188F) or (S72G, A74T, T146F, V178W, L188K) or (S72G, A74T, T146F, V178W, L188R) or (S72G, A74T, V178P) or (S72G, A74T, V178P, L181Y) or (S72G, A74T, V178P, L181Y, L188F) or (S72G, A74T, V178P, L181Y, L188K) or (S72G, A74T, V178P, L188F) or (S72G, A74T, V178P, L188K) or (S72G, A74T, V178P, L188R) or (S72G, A74T, V178W) or (S72G, A74T, V178W, L181Y, L188F) or (S72G, A74T, V178W, L181Y, L188K) or (S72G, A74T, V178W, L188F) or (S72G, A74T, V178W, L188K) or (S72G, A74T, V178W, L188R) or (S72G, A74V) or (S72G, A74V, A184W) or (S72G, A74V, A221V) or (S72G, A74V, F77A) or (S72G, A74V, F77A, M177Y) or (S72G, A74V, F77L, M177Y) or (S72G, A74V, F77S) or (S72G, A74V, L181Y) or (S72G, A74V, L181Y, L188F) or (S72G, A74V, L181Y, L188K) or (S72G, A74V, L188F) or (S72G, A74V, L188K) or (S72G, A74V, L188R) or (S72G, A74V, T146F) or (S72G, A74V, T146F, L181Y) or (S72G, A74V, T146F, L181Y, L188F) or (S72G, A74V, T146F, L181Y, L188K) or (S72G, A74V, T146F, L181Y, L188R) or (S72G, A74V, T146F, L188F) or (S72G, A74V, T146F, L188K) or (S72G, A74V, T146F, L188R) or (S72G, A74V, T146F, V178P) or (S72G, A74V, T146F, V178P, L181Y) or (S72G, A74V, T146F, V178P, L181Y, L188F) or (S72G, A74V, T146F, V178P, L181Y, L188K) or (S72G, A74V, T146F, V178P, L181Y, L188R) or (S72G, A74V, T146F, V178P, L188F) or (S72G, A74V, T146F, V178P, L188K) or (S72G, A74V, T146F, V178P, L188R) or (S72G, A74V, T146F, V178W) or (S72G, A74V, T146F, V178W, L181Y) or (S72G, A74V, T146F, V178W, L181Y, L188K) or (S72G, A74V, T146F, V178W, L181Y, L188R) or (S72G, A74V, T146F, V178W, L188F) or (S72G, A74V, T146F, V178W, L188K) or (S72G, A74V, T146F, V178W, L188R) or (S72G, A74V, V178P) or (S72G, A74V, V178P, L181Y) or (S72G, A74V, V178P, L181Y, L188F) or (S72G, A74V, V178P, L181Y, L188K) or (S72G, A74V, V178P, L181Y, L188R) or (S72G, A74V, V178P, L188F) or (S72G, A74V, V178P, L188K) or (S72G, A74V, V178P, L188R) or (S72G, A74V, V178W) or (S72G, A74V, V178W, L181Y) or (S72G, A74V, V178W, L181Y, L188F) or (S72G, A74V, V178W, L181Y, L188K) or (S72G, A74V, V178W, L181Y, L188R) or (S72G, A74V, V178W, L188F) or (S72G, A74V, V178W, L188K) or (S72G, A74V, V178W, L188R) or (S72G, A74Y) or (S72G, L181Y) or (S72G, L181Y, L188F) or (S72G, L181Y, L188K) or (S72G, L181Y, L188R) or (S72G, L188F) or (S72G, L188K) or (S72G, L188R) or (S72G, M177Y, V178W) or (S72G, M177Y, V178W, L188K) or (S72G, T146F) or (S72G, T146F, L181Y) or (S72G, T146F, L181Y, L188F) or (S72G, T146F, L181Y, L188K) or (S72G, T146F, L181Y, L188R) or (S72G, T146F, L188F) or (S72G, T146F, L188K) or (S72G, T146F, L188R) or (S72G, T146F, V178P) or (S72G, T146F, V178P, L181Y) or (S72G, T146F, V178P, L181Y, L188F) or (S72G, T146F, V178P, L181Y, L188K) or (S72G, T146F, V178P, L181Y, L188R) or (S72G, T146F, V178P, L188F) or (S72G, T146F, V178P, L188K) or (S72G, T146F, V178P, L188R) or (S72G, T146F, V178W) or (S72G, T146F, V178W, L181Y) or (S72G, T146F, V178W, L181Y, L188F) or (S72G, T146F, V178W, L181Y, L188K) or (S72G, T146F, V178W, L188F) or (S72G, T146F, V178W, L188K) or (S72G, T146F, V178W, L188R) or (S72G, V178P) or (S72G, V178P, L181Y) or (S72G, V178P, L181Y, L188F) or (S72G, V178P, L181Y, L188K) or (S72G, V178P, L181Y, L188R) or (S72G, V178P, L188F) or (S72G, V178P, L188K) or (S72G, V178P, L188R) or (S72G, V178W) or (S72G, V178W, L181Y) or (S72G, V178W, L181Y, L188F) or (S72G, V178W, L181Y, L188K) or (S72G, V178W, L181Y, L188R) or (S72G, V178W, L188F) or (S72G, V178W, L188K) or (S72G, V178W, L188R) or (S72H, A74C) or (S72N, A74C) or (S72N, A74I) or (S72N, A74V) or (S72W) or (S72W, A74V) or (S89C) or (S89E) or (S89G) or (S89I) or (S89L) or (S89N) or (S89Q) or (S89R) or (S89T) or (S89V) or (T146A) or (T146A, D222Y, I263M) or (T146A, F173D) or (T146A, F173V) or (T146A, F205G) or (T146A, F261C) or (T146A, F261S) or (T146A, F261V) or (T146A, G271S) or (T146A, G271T) or (T146A, H266V) or (T146A, I258T, I259S) or (T146A, I259H) or (T146A, I263F) or (T146A, I263L) or (T146A, I263N) or (T146A, I263V) or (T146A, L150A) or (T146A, L150G) or (T146A, L150K) or (T146A, L150R, F173I) or (T146A, L272H) or (T146A, L272S) or (T146A, L272W) or (T146A, M212A) or (T146C) or (T146D) or (T146E) or (T146F) or (T146F, L181Y) or (T146F, L181Y, L188F) or (T146F, L181Y, L188K) or (T146F, L188K) or (T146F, L188R) or (T146F, V178P) or (T146F, V178P, L181Y, L188R) or (T146F, V178P, L188K) or (T146F, V178P, L188R) or (T146F, V178W) or (T146F, V178W, L181Y, L188R) or (T146F, V178W, L188F) or (T146F, V178W, L188K) or (T146F, V178W, L188R) or (T146G) or (T146H) or (T146L) or (T146M) or (T146N) or (T146P) or (T146R) or (T146S) or (T146V) or (T146V, A191T) or (T146Y) or (T149P) or (T268A) or (T268S) or (T269K) or (T327A) or (T327A, A330W) or (T327C) or (T327D) or (T327E) or (T327I) or (T327L) or (T327M) or (T327N) or (T327P) or (T327Q) or (T327S) or (T327V) or (T365C) or (T365D) or (T365F) or (T365G) or (T365H) or (T365I) or (T365K) or (T365L) or (T365N) or (T365P) or (T365Q) or (T365V) or (T365W) or (T365Y) or (T436A) or (T436C) or (T436D) or (T436E) or (T436F) or (T436G) or (T436H) or (T436I) or (T436K) or (T436L) or (T436M) or (T436N) or (T436P) or (T436Q) or (T436R) or (T436S) or (T436V) or (T436W) or (T436Y) or (T438A) or (T438C) or (T438I) or (T438S) or (T438V) or (T49A) or (T49C) or (T49D) or (T49E) or (T49F) or (T49G) or (T49H) or (T49I) or (T49K) or (T49L) or (T49L, S54N) or (T49M) or (T49N) or (T49P) or (T49Q) or (T49R) or (T49R, R50C) or (T49R, R50S, M177Y, V178W, A184Y) or (T49S) or (T49V) or (T49W) or (T49Y) or (T88C) or (T88S) or (T88V) or (V178D) or (V178H) or (V178I) or (V178K) or (V178L) or (V178M) or (V178N) or (V178P) or (V178P, L181Y) or (V178P, L181Y, L188R) or (V178P, L188F) or (V178P, L188K) or (V178P, L188R) or (V178Q) or (V178R) or (V178S) or (V178T) or (V178W) or (V178W, A184Y) or (V178W, L181Y, L188K) or (V178W, L181Y, L188R) or (V178W, L188R) or (V178W, R50S) or (V178Y) or (V26A) or (V26C) or (V26F) or (V26G) or (V26H) or (V26I) or (V26K) or (V26L) or (V26M) or (V26N) or (V26N, R50S, M177Y, V178W, A184Y) or (V26Q) or (V26S) or (V26T) or (V26W) or (V26Y) or (V314A) or (V314E) or (V314F) or (V314H) or (V314P) or (V314Q) or (V314S) or (V314Y) or (V48A) or (V48D) or (V48E) or (V48F) or (V48G) or (V48H) or (V48I) or (V48L) or (V48M) or (V48P) or (V48Q) or (V48R) or (V48S) or (V48T) or (V48W) or (V48Y) or (W325A) or (W325C) or (W325D) or (W325E) or (W325F) or (W325G) or (W325I) or (W325K) or (W325L) or (W325M) or (W325Q) or (W325S) or (W325T) or (W325V) or (W325Y) or (W90Y) or (Y51A) or (Y51C) or (Y51F) or (Y51H) or (Y51I) or (Y51L) or (Y51M) or (Y51P) or (Y51S, F77V) or (Y51T) or (Y51V) or (Y51W).

According to **one embodiment** there is provided a BM3 variant wherein said variant has an improved selectivity and/or product yield for C19 hydroxylation compared with at least one BM3 variants according to SEQ ID No. 2 to 28, preferably with at least one BM3 variant selected from BM3-254, BM3-261, BM3-263, or BM3-268. This embodiment can be and is suggested to be combined with all previous embodiments, in particular with the first, second, third, fourth, fifth and/or sixth embodiment or the described subsets.

According to a **second aspect** of the current invention, there is provided a nucleic acid encoding for a Cytochrome P450 BM3 monooxygenase variant as described herein.

A defined protein or amino acid sequence may be encoded by various nucleic acid sequences. Where an amino acid sequence has been defined herein, each of the nucleic acids encoding this amino acid sequence shall also be deemed disclosed herein. It is therefore well understood that the nucleic acid sequence as provided e.g. in SEQ ID No. 59 for the wildtype sequence has to be read in an exemplary fashion.

Preferably, the nucleic acid encodes for a variant according to the first aspect. Even more preferably, the nucleic acid encodes for a variant according to the first aspect, first embodiment or the nucleic acid encodes for a variant according to the first aspect, second embodiment, e.g. variants of SEQ ID No. 2, 3, 4, 5, 6, 7, 8, 9, 10, 11, 12, 13, 14, 15, 16, 17, 18, 19, 20, 21, 22, 23, 24, 25, 26, 27 or 28, or a variant derived thereof. Furthermore, the nucleic acid can be a nucleic acid encoding for a variant according to the third, fourth, fifth, or sixth embodiment of the first aspect.

Nucleic acids according to the second aspect can be generated *de novo* as known in the art or can be generated by mutating available BM3 nucleic acid sequences. The nucleic acid encoding the P450-BM3 variant according to the current invention can then be introduced into a suitable expression system for expression of the BM3 variant. Sequences of the nucleic acids can be verified as known in the art. Exemplary methods for sequence verification are disclosed for example in (Green and Sambrook 2012).

According to a **third aspect** of the current invention, there is provided a host cell for the production of a Cytochrome P450 BM3 monooxygenase variant. In a preferred embodiment, the host cell comprises a nucleic acid encoding for a BM3 variant according to any of the aspects dercribed herein.

Suitable expression systems are well known in the art and example systems comprise *E. coli* strains such as DH5alpha, BL21 (DE3), or Rosetta (DE3), but also various other bacterial and non bacterial systems. In some preferred embodiments, the host cell is a procaryotic cell (e.g. an *E. coli* cell) or a eukaryotic cell (e.g. a CHO cell). In a preferred embodiment, the host cell is an *E. coli* cell, e.g. DH5alpha, BL21 (DE3), or Rosetta (DE3). In another preferred embodiment, the host cell is from *E. coli* strain W3110 (NC_007779.1), JM101 or MG1655 (NC_000913.3). Introduction of nucleic acids encoding for a BM3 variant, e.g. according to the second aspect, into the host cell may occur as known in the art, i.e. using suitable vectors or transfer systems. For example, where bacterial expression systems such as DH5alpha, BL21 (DE3), or Rosetta (DE3) are used, suitable vectors are known in the art, such as pSE420 (Invitrogen), pET21a (EMD Biosciences), pET22b (EMD Millipore), pLys (EMD Biosciences) or pETM11 (EMBL Vector Collection, Germany). For a detailed decription see e.g. section 4.2 *"Cloning of cyp102A1 and pETM11-BM3 construction"* of Supporting Information of (Acevedo-Rocha 2018) and the examples provided herein.

According to a **fourth aspect** of the current invention, there is provided the use of a Cytochrome P450 BM3 monooxygenase (BM3) variant for the production of a compound according to formula I, wherein R¹ and R² form a six-membered ring as part of a steroid.

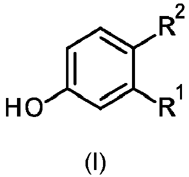

In a preferred embodiment according to the fourth aspect, formula I is formula (II), wherein R³ is a beta-hydroxy group (-OH) or an oxo group (=O).

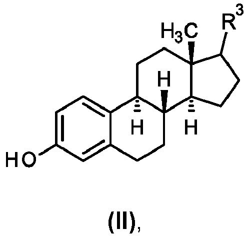

As described also elsewhere herein, it was surprisingly found, that BM3 variants can be used for the production of a compound according to formula I and II in a commercially relevant setup, i.e. for an industrial application. To this end, a BM3 variant suitable for catalyzing C19 hydroxylation is incubated with a suitable substrate, e.g. as described in examples 2, 8, 10, 13, 17, 20, 21, 24, 25.

In some highly preferred embodiments according to the fourth aspect, the substrate for the BM3 variant is a steroid or steroid derivative.

According to a **fifth aspect** of the current invention, there is provided the use of a BM3 variant for the C19-hydroxylation of a steroid or steroid derivative.

As described also elsewhere herein, it was surprisingly found, that BM3 variants can be used for the C19-hydroxylation of a steroid or steroid derivative in a commercially relevant setup, i.e. for an industrial application. To this end, a BM3 variant suitable for catalyzing C19 hydroxylation is incubated with a suitable substrate, e.g. as described in examples 2, 8, 10, 13, 17, 20, 21, 24, 25.

According to some highly preferred embodiments of the **fourth or fifth aspect,** the BM3 variant is a variant as described herein. Preferably, the BM3 variant can be a variant according to the first aspect. Even more preferably, the BM3 variant can be a variant according to the first aspect, first embodiment or the BM3 variant can be a variant according to the first aspect, second embodiment, e.g. variant according to SEQ ID No. 2, 3, 4, 5, 6, 7, 8, 9, 10, 11, 12, 13, 14, 15, 16, 17, 18, 19, 20, 21, 22, 23, 24, 25, 26, 27 or 28, or a variant derived thereof. Furthermore, the BM3 variant can be a variant according to the third, fourth, fifth, or sixth embodiment of the first aspect.

In some embodiments according to the **fourth or fifth aspect,** the BM3 variant is purified prior to incubation with the substrate using a purification technique known in the art, such as but not restricted to immobilized metal affinity chromatography, ion exchange chromatography, size exclusion chromatography. In some other embodiments according to the fourth aspect, the BM3 variant remains in a cell suspension when incubated with the substrate, e.g. no separation of BM3 variant and expression system occurs prior to the biotransformation, e.g. whole-cell biotransformation.

According to some preferred embodiments of the **fourth or fifth aspect** said steroid or steroid derivatives comprise a 1,4-dien-3-one-A-ring or a 4-en-3-one-A-ring. According to some even more preferred embodiments according to the **fourth or fifth aspect** said steroid or steroid derivative is androsta-1,4-dien-3,17-dione (ADD) or (17beta)-17-hydroxyandrosta-1,4-dien-3-one (delta1 testosterone).

In some embodiments of the **fourth or fifth aspect** the substrate steroid or steroid derivative is added to the biotransformation reaction as a purified compound. In some embodiments the substrate steroid or steroid derivative is dissolved in a suitable solvent and can be added in solution to the biotransformation mixture. In other embodiments the substrate steroid or steroid derivative is formed in the course of a reaction preceding the C19 hydroxylation. The reaction preceding the C19 hydroxylation reaction may be another biotransformation reaction or a chemical reaction, e.g. a reduction of delta1 testosterone to testosterone or an oxidation of delta1 testosterone to ADD.

In some embodiments of the fourth or fifth aspect the formed C19 hydroxylated steroid or steroid derivative is extracted from the biotransformation reaction mixture using a method known in the art, e.g. with 4-methyl-2-pentanone (see example 10). In some other embodiments according to the fourth or fifth aspect the formed C19 hydroxylated steroid or steroid derivative is converted into a secondary product. This conversion may occur e.g. by means of biotransformation or chemical reaction. A secondary product may be for example estrone or a derivative thereof.

Extraction of the C19 hydroxylated steroid or steroid derivative or a secondary product thereof may occur as known in the art, e.g. by stirring the reaction mixture with 4-methyl-2-pentanone, separation of the organic phase and concentration by evaporation of the organic solvents. Further purification of the C19 hydroxylated steroid or steroid derivative or a secondary product thereof may be performed using chromatographic techniques, e.g. flash chromatography using silica gel or preparative HPLC, or crystallization.

Analysis of the production of the C19 hydroxylated steroid, steroid derivative or secondary product can occur as known in the art, e.g. by using MS- or NMR-methods.

Suitable biotransformation conditions are described in the examples provided herein. However, it is routine for the skilled person to optimize the reaction conditions for a BM3 variant to obtain optimal yields for the reaction product. For example, incubation may occur in a KH2PO4/K2HPO4 buffer comprising an aqueous EDTA solution in a pH range of 6.6 to 7.4. Suitable incubation times may be for example between 12 and 48 hours.

According to a **sixth aspect** of the current invention, there is provided a process for C19-hydroxylation of a steroid or derivative thereof comprising
(i)
   (a) culturing a recombinant Cytochrome P450 BM3 monooxygenase (BM3) variant producing microorganism in a culture medium, in the presence of an exogenous or intermediately formed substrate; or
   (b) incubating a substrate-containing reaction medium with a Cytochrome P450 BM3 monooxygenase; and
(ii) isolating the oxidation product formed or a secondary product thereof from the medium;
said process being further characterized in that said BM3 variant is a BM3 variant as described herein.

The recombinant BM3 variant producing microorganism is a microorganism comprising a nucleic acid encoding a BM3 variant. The recombinant BM3 variant producing microorganism can be derived from any suitable expression system. Preferably, the recombinant BM3 variant producing microorganism is a microorganism according to the third aspect. For example, the BM3 variant may be a variant according to the first aspect.

In some embodiments of the sixth aspect, the process comprises culturing the recombinant BM3 variant producing microorganism in a culture medium. This can occur as known in the art, e.g. as described in the examples. However, the skilled person is aware of a variety of setups for the cultivation of microorganisms. Suitable culture media are described in the art for the respective expression system and can be easily adapted.

In some embodiments of the sixth aspect, the BM3 variant is purified prior to incubation with the substrate using a purification technique known in the art, such as immobilized metal affinity chromatography, ion exchange chromatography, size exclusion chromatography. In some other embodiments of the sixth aspect, the BM3 variant remains in a cell suspension when incubated with the substrate, e.g. no separation of BM3 variant and expression system occurs prior to the biotransformation, e.g. whole-cell biotransformation.

In some embodiments, the process comprises incubating a substrate-containing reaction medium with a BM3 variant (e.g. purified or crude mixture with cells of the expression system). Suitable media and conditions are described in the examples provided herein. However, it is routine for the skilled person to optimize the reaction conditions for a BM3 variant to optimize yields for the reaction product.

For example, the substrate can be an exogenous substrate. Such an exogenous substrate is added to the biotransformation reaction, e.g. as a purified compound, solution or mixture. The substrate can also be an intermediately formed substrate, i.e. the substrate can be formed in the course of a reaction preceding the C19 hydroxylation. The reaction preceding the C19 hydroxylation reaction may be another biotransformation reaction or a chemical reaction.

Suitable substrates are steroids or steroid derivatives, in particular those comprising a 1,4-dien-3-one-A-ring or a 4-en-3-one-A-ring. For example ADD or d1-testosterone are suitable substrates for the described BM3 variants. In some preferred embodiments according to the sixth aspect, said steroid or derivative thereof comprises a 1,4-dien-3-one-A-ring and a 19-methyl group. In some preferred embodiments according to the sixth aspect, said steroid or derivative thereof comprises a 4-en-3-one-A-ring and a 19-methyl group.

Following the biotransformation, the C19 hydroxylation product can be isolated from the reaction medium as described in the art. In the alternative, a secondary product may be isolated from the reaction medium. The isolation may be performed for example by chromatogrpahic techniques such as preparative or analytical HPLC, or by extraction. Extraction of the C19 hydroxylated steroid or steroid derivative or a secondary product thereof may occur as known in the art, e.g. with 4-methyl-2-pentanone (see example 10). Further purification of the C19 hydroxylated steroid or steroid derivative or a secondary product thereof may be performed using chromatographic techniques, e.g. flash chromatography using silica gel or preparative HPLC, or crystallization.

Analysis of the production of the C19 hydroxylated steroid, steroid derivative or secondary product can occur as known in the art, e.g. by MS- or NMR-methods.

According to a seventh aspect there is provided a method for obtaining optimized BM3 variants for the C19 hydroxylation of steroids, said method comprising
(i)
   (a) culturing a recombinant microorganism expressing a BM3 variant (test variant) in a culture medium, in the presence of an exogenous or intermediately formed steroid or steroid derivative; or
   (b) incubating a steroid or steroid derivative-containing reaction medium with a BM3 variant (test variant); and
(ii) Comparing the obtained product yield or selectiviy factor for the C19 hydroxylation product formed by the test variant or a secondary product thereof with the respective value obtained for a parent variant of the test variant capable of catalyzing the C19 hydroxylation of a steroid, and
(iii) Selecting the test variant as optimized for the C19 hydroxylation of steroids, if the test variant has an improved product yield/and or selectivity factor compared to the parent variant.

The description provided for the process according to the fifth and sixth aspect applies *mutatis mutandis,* in particular with regard to step (i) a and b.

In a preferred embodiment, the steroid or steroid derivative comprises an 1,4-dien-3-one-A-ring or a 4-en-3-one-A-ring. In a highly preferred embodiment the steroid or steroid derivative is ADD or d1-testosterone. In some preferred embodiments of the seventh aspect, the steroid or derivative thereof comprises a 1,4-dien-3-one-A-ring and a 19-methyl group. In some preferred embodiments of the seventh aspect, said steroid or derivative thereof comprises a 4-en-3-one-A-ring and a 19-methyl group.

Product yield and selectivity factor are calculated as disclosed elsewhere herein. The product yield can be the product yield for the C19 hydroxylated steroid or steroid derivative, or a product derived thereof. Preferably the product yield is the product yield for estradiol or estrone. The selectivity factor can be the selectivity factor for the C19 hydroxylated steroid or steroid derivative.

Preferably, the daughter variant has the sequence of its parent variant and at least one further mutation. Preferably, the parent variant is a variant according to the first aspect.

### EXAMPLES

If not stated otherwise, culture media were prepared in demineralized water and sterilized at 121 °C for 20 minutes or sterile filtered. All solutions were prepared with MilliQ water and sterile filtered, unless otherwise described. All preparations were performed under sterile conditions.

Oxford trace element solution contained FeCl3 x 6 H2O (27 g/L), ZnCl2 (1.31 g/L), CoCl2 x 6 H2O (2.87 g/L), CuCl2 x 2 H2O (1.27 g/L), Boric acid (0.5 g/L), CaCl2 x 2 H2O (1.32 g/L), Na2MoO4 x 2 H2O (2.35 g/L), Hydrochloric acid (37%) (100 mL/L).

List of abbreviations: Δ...deletion from amino acid; DMSO...Dimethyl sulfoxide; DMF...Dimethylformamide; EDTA...Ethylendiamintetraacetate; × g...times gravity; IPTG...isopropyl β-D-thiogalactopyranoside; LB...Luria-Bertani broth; OD550... optical density at 550 nm; rpm... revolutions per minute;

SOC Outgrowth Medium...#B9020S-from BioLabs: 2 % Vegetable Peptone, 0.5% Yeast Extract, 10 mM NaCl, 2.5 mM KCI, 10 mM MgCl2, 10 mM MgSO4, 20 mM Glucose.

Pluronic® PE 8100... Pluronic PE types are low-foaming, nonionic surfactants. They are block copolymers in which the central polypropylene glycol group is flanked by two polyethylene glycol groups. PE 8100 conforms to the following structural formula:

HO(CH₂CH₂O)ₓ(CH₂C(CH₃)HO)_{y}(CH₂CH₂O)_{z}H.

PE 8100 is a polypropylene glycol block copolymer with a molar mass of 2300 g/mol and 10 % polypropylene glycol in the molecule.

### Example 1

### Cloning of P450 BM3 (BM3) variants

Nucleotide sequences encoding P450-BM3 variants as described herein can be synthesized as known in the art, e.g. as offered by respective service providers such as Eurofins Genomics GmbH (Eurofins Genomics GmbH, Anzinger Str. 7a, 85560 Ebersberg, Germany). In brief, nucleic acid sequences encoding P450-BM3 WT (e.g. SEQ ID No. 59) or other P450-BM3 variants as described herein were cloned into an expression vector based on the vector pETM-11 (EMBL Vector Collection, Germany) or another suitable vector. For a detailed description see section 4.2 *"Cloning of cyp102A1 and pETM11-BM3 construction"* of Supporting Information of (Acevedo-Rocha 2018). Genetic elements were introduced into the modified pETM-11 vector by means of commonly known methods. For expression of BM3 variants, the expression vector was introduced into *Escherichia coli* strain BL21 Star™ (DE3) (Invitrogen™, Lifetechnologies) cells, *E. coli* BL 21 (DE3) cells or *E. coli* BL 21 Gold (DE3) ΔdkgA :: FRT T7 gdh (the construction is described in (Agudo R 2012)) cells.

### Generation of enzyme variants

Nucleotide substitutions (replacements) were introduced into the nucleic acid parent sequences, e.g. to obtain a T88 exchange into other amino acids on SEQ ID No. 5 as parent. Several Molecular Biological methods can be used to achieve these replacements. A useful method for preparing a mutated nucleic acid according to the invention and the corresponding protein comprises carrying out site-directed mutagenesis on codons encoding one or more amino acids, which are selected in advance. The methods for obtaining these site-directed mutations are well known to the skilled person and widely described in the literature (in particular: Directed Mutagenesis: A Practical Approach, 1991, Edited by M.J. McPHERSON, IRL PRESS), or are methods for which it is possible to employ commercial kits (for example the QUIKCHANGE™ lightening mutagenesis kit from Qiagen or Stratagene). After site-directed mutagenesis, nucleic acids were transformed into the *Escherichia coli* strain BL21 Star™ (DE3) (Invitrogen™, Lifetechnologies). Transformed cells were tested in appropriate biotransformation reactions, in order to determine product yield and selectivity. Appropriate biotransformation reactions are described below, see e.g. Example 2. Sequence verification was performed as known in the art.

Glycerol stocks of the *E*. *coli* cultures transformed with the respective expression plasmids were prepared by adding one volume of 40% glycerol solution to one volume of *E*. *coli* culture.

For isolation of single bacterial colonies, appropriate dilutions of *E.coli* cultures were plated onto LB-Agar plates containing suitable concentrations of kanamycin and incubated at 37°C until single colonied were obtained.

### Example 2

### Analysis of P450-BM3 variants for estradiol or estrone production

### Cultivation

For screening purposes *E*. *coli* strain BL21 Star™ (DE3) (Invitrogen™, Lifetechnologies) was used as host for expression plasmids (Example 1). For pre-cultures sterilized 2.5-mL 96-well deep-well plates (850301, HJ-Bioanalytik, Erkelenz, Germany) were filled with 490 µL LB-medium (LB Broth Miller, Fisher Bioreagents, BP1426-500) supplemented with kanamycin (Sigma-Aldrich, 50 µg/mL), inoculated with 10 µL of glycerol stocks of the respective variants (that were prepared in 96-well microtiter plates) or alternatively 500 µL LB-medium were inoculated with cell material from Agar plate colonies. Pre-cultures were incubated for 17 hours at 37°C and 250 rpm in a climo shaker ISF1-X (Kühner AG, Birsfelden, Switzerland).

Medium for expression cultures contained Tryptone (12 g/L), Yeast extract (24 g/L), Meat Peptone, tryptically digested (2 g/L), KH2PO4 (2.2 g/L), K2HPO4 (9.4 g/L), glycerol (0.4% [v/v]), Riboflavin (0.001 g/L), Thiamine hydrochloride (0.337 g/L) and Oxford trace element solution (250 µL/L). Tryptone, Yeast extract and Meat peptone were prepared as 50 g/L stock solutions, and K-PO4 was prepared as a 50x stock solution. These stock solutions were sterilized at 121 °C for 20 minutes. Riboflavin and Thiamine hydrochloride were freshly prepared as 0.06 g/L and 10 g/L stock solutions and sterile-filtered, respectively. The medium was prepared from the stock solutions and Kanamycin (Kanamycin solution, K0254, Sigma-Aldrich, St. Louis, MO, USA) was added for a final concentration of 50 mg/L.

For expression cultures sterilized 2.5-mL 96-well deep-well plates (850301, HJ-Bioanalytik, Erkelenz, Germany) were filled with 440 µL expression medium supplemented with kanamycin (50 mg/L); expression cultures were inoculated with 50 µL of pre-cultures. The expression culture plates were incubated at 37°C and 250 rpm in a climo shaker. After 4 h incubation 10 µL of 30 mM IPTG diluted in expression medium (supplemented with 50 mg/L kanamycin) was added per well to induce enzyme expression (resulting in a final concentration of 0.6 mM IPTG). The expression culture plates were subsequently incubated for 20 hours at 27°C in a shaking incubator.

Cells were harvested by centrifugation of the expression cultures for 15 minutes at 4°C and 2500 × g. The culture supernatant was discarded, and the remaining cell pellets were resuspended in 495 µL of P450 assay buffer (100 mM KPO4-buffer at pH 7.4, 50 g/L glucose, 35 g/L glycerol, 1 mM EDTA). The deep-well plates were stored at -80°C until use.

### Biotransformation

The deep-well plates containing the cell suspensions were thawed at room temperature. The biotransformation reaction was started by addition of 5 µL of Δ1-testosterone or androsta-1,4-diene-3,17-dione (ADD) stock solution (at 5 mg/mL or 10 mg/mL in *N,N*-Dimethylformamide) and incubated for 22 h at 27°C in a shaking incubator. Reactions were stopped by adding 500 µL of Stop solution (containing Acetonitrile, Methanol and Dimethylsulfoxide in a 1:1:1 ratio), and for extraction of steroides incubated on a shaker for 30 min at 20°C. Afterwards the plates were centrifuged for 15 min at 4°C and 2500x g. From each well 120 µL of the supernatant was transferred to a 96-well filter plate (Corning, 3504) that was subsequently centrifuged for 5 min at 4°C and 650x g. The filtered samples were stored at -20°C or directly submitted to HPLC analysis.

### Analytical HPLC method

Analytical HPLC was performed with the following setup:
Instrument: Agilent Technologies 1290 Infinity; Column: Ascentis Express C18, 50 x 2.1mm, 2.7 µm; Eluent A: Water (+0.025% Trifluoroacetic acid); Eluent B: Acetonitrile; Flow 1.5 mL/min; Temperature: 45 °C; injection volume: 4 µL, detection at 280 nm. Gradient: 0 - 0.18 min 5% solvent B, 0.18 - 0.4 min 5 - 10% solvent B, 0.4 - 3.6 min 10 - 27% solvent B, 3.6 - 6.0 min 27 - 95% solvent B, 6.0 - 6.25 min 5% solvent B.

Androsta-1,4-diene-3,17-dione (ADD), Δ1-testosterone, estradiol and estrone (dissolved in *N,N-*Dimethylformamide) were used as reference substances and as a standard for quantification. Appropriate dilutions were prepared covering the range up to the maximum concentration used in the biotransformation.

Observed retention times were 4.13 min for Δ1-testosterone, 4.38 min for ADD, 4.53 min for estradiol and 4.98 min for estrone. The concentration of the respective compounds in samples was calculated by comparing the obtained peak areas with those of the standards.

### Example 3

### Conversion of Δ1-testosterone to estradiol using BM3 variants based on parent type BM3-268 (Seq ID No. 5)

Variants of BM3-268 (Seq ID No. 5) with mutations in one or two positions were prepared as described in Example 1 and screened for Δ1-testosterone to estradiol conversion. Cultivation, biotransformation and HPLC analysis was performed as described in Example 2. Glycerol stocks were used as inoculum for cultivation, and the initial substrate (Δ1-testosterone) concentration was 50 mg/L.

Results for estradiol product yield and the selectivity factor for estradiol are shown in Figures 1 a, b and 2 a, b. For BM3-268 derived variants comprising mutations at position L78, E82 or A87 results for Δ1-testosterone to estradiol conversion are presented in Figure 1 c.

### Example 4

### Conversion of Δ1-testosterone to estradiol using BM3 variants based on BM3-268; M177Y, A184Y (SEQ ID No. 15)

Variants of the BM3 derivative BM3-268; M177Y, A184Y (Seq ID No. 15) with further mutations in up to six positions (Example 1) were screened for Δ1-testosterone to estradiol conversion. Cultivation, biotransformation and HPLC analysis was performed as described in Example 2. Glycerol stocks were used as inoculum for cultivation, and the initial Δ1-testosterone concentration used in the experiment was 100 mg/L.

Resulting estradiol product yields and estradiol selectivity factors are shown in Figure 3 a, b and Figure 4 a, b, c, d, e, f. For Seq ID No. 15 derived variants comprising mutations at position L78, E82 or A87 results for Δ1-testosterone to estradiol conversion are presented in Figure 3 c.

### Example 5

### Conversion of Δ1-testosterone to estradiol using BM3-268 (Seq ID No. 5) variants with up to seven modifications.

Variants of BM3-268 (Seq ID No. 5) with up to seven mutations in different positions (Example 1) were screened for Δ1-testosterone to estradiol conversion. Cultivation, biotransformation and HPLC analysis was performed as described in Example 2. Agar plate colonies were used as inoculum for cultivation, and the initial Δ1-testosterone concentration used in the experiment was 50 mg/L. Results for estradiol yield and the selectivity factor for estradiol are shown in Figure 5 a and b.

### Example 6

### Conversion of androsta-1,4-diene-3,17-dione (ADD) to estrone using BM3 variants based on BM3-268; M177Y, A184Y (SEQ ID No. 15)

Variants of the BM3-268; M177Y, A184Y (Seq ID No. 15) with further mutations in up to six positions (Example 1) were screened for androsta-1,4-diene-3,17-dione (ADD) to estrone conversion. Cultivation, biotransformation and HPLC analysis was performed as described in Example 2. Glycerol stocks were used as inoculum for cultivation, and the initial ADD concentration used in the experiment was 100 mg/L.

Results for estrone yield and selectivity factor for estrone are shown in Figure 6 a, b and Figure 7 a, b, c, d, e, f. For Seq ID No. 15 derived variants comprising mutations at position L78, E82 or A87 results for ADD to estrone conversion are presented in Figure 6 c.

### Example 7

### Conversion of androsta-1,4-diene-3,17-dione (ADD) to estrone using BM3-268 (Seq ID No. 5) variants with up to six modifications.

Variants of BM3-268 (Seq ID No. 5) with up to six mutations in different positions (Example 1) were screened for androsta-1,4-diene-3,17-dione (ADD) to estrone conversion. Cultivation, biotransformation and HPLC analysis was performed as described in Example 2. Agar plate colonies were used as inoculum for cultivation, and the initial ADD concentration used in the experiment was 100 mg/L. Results for estrone yield and the selectivity factor for estrone are shown in Figure 8.

### Example 8

### Method for screening P450-BM3 variants for the C19 hydroxylation of (17beta)-17-hydroxyandrosta-1,4-dien-3-one (delta-1-testosterone)

### Media

Tryptone (10 g/L), sodium chloride (10 g/L) and yeast extract (5 g/L) in demineralized water. The medium was sterilized at 121 °C for 20 minutes. Afterwards kanamycin (50 mg/L) was added.

### Preculture

Each well of a 96 deep well plate contained 0.25 ml of the medium which was inoculated with the *E. coli* strains (2 µL) containing the P450-BM3 variants from glycerol stocks. The plate was shaken at 37 °C and 800 rpm for 17 hours.

### Main culture

Each well of a 96 deep well plate contained 1.5 ml of the medium which was inoculated with 50 µL from the preculture. The cultures were shaken at 37 °C and 800 rpm for 3 hours. Then the temperature was decreased to 27 °C within 1 hour and IPTG (1 mM) in demineralized water was added to start the protein expression. After 4 hours of expression, the plates were harvested by centrifugation, the supernatant was discarded, the pellets were resuspended in 500 µL reaction buffer (KH2PO4 (4 g/L), K2HPO4 (12.3 g/L), glycerol (4% (v/v)), glucose (5% (w/v)), EDTA (0.5 mM)), frozen in liquid nitrogen and stored at -80 °C.

### Biotransformation

The plates were thawed and the reaction was started by addition of 5 µL of steroid stock solution of (17beta)-17-hydroxyandrosta-1,4-dien-3-one (12.5 µg dissolved in 5 µL of DMF) and plates were shaken for 22 h at 700 rpm and 27 °C while covered with lids. The plates were frozen in liquid nitrogen and stored at -80 °C. The thawed samples were prepared for HPLC screening by adding 500 µL mixture of acetonitrile / methanol / DMSO 1:1:1 (HPLC grade). The plates were shaken and centrifuged and 200 µl of the supernatant was transferred to an analytics plate. The plate was covered with a silicone sealing mat and submitted to HPLC analysis.

### HPLC Analysis

### Analytical chiral HPLC method:

Instrument: Waters Acquity UPLCMS SingleQuad; Column: Thermo Accucore C30 2.6 µm, 50x2.1 mm; Eluent A: water + 0.05 vol % formic acid, eluent B: acetonitrile + 0.05 vol % formic acid; Gradient: 0.0-2.0 min 5-30 % B, 2.0-5.0 min 85 % B; injection volume 4 µL; flow 0.9 mL/min; temperature: 45 °C; DAD scan: 248-280 nm.

**Table E1: HPLC retention times**

| **Substance** | **Retention Time** |
|---|---|
| Delta-1-testosterone | 2.40 |
| Estradiol (Example 10) | 2.55 |
| (15beta-OH)-delta-1-testosterone (Example 12) | 1.39 |
| (1alpha,2alpha-epoxy)-testosterone (Example 11) | 2.46 |
| (1alpha,2alpha-epoxy-15beta-OH)-testosterone (Example 15) | 1.35 |
| (6beta-OH)-delta-1-testosterone (Example 16) | 1.50 |
| (1alpha,2alpha-epoxy-19-OH)-testosterone (Example 14) | 1.56 |
| (15beta-OH)-estradiol (Example 13) | 1.69 |

### Results

After testing hundreds of P450-BM3 variants, only few mutants allowed for the C19-hydroxylation of delta-1-testosterone leading to estradiol as final product:

**Table E2: Estradiol product yield in % for some P450 BM3 variants.**

| | mutations | substrate | product | Screening yield (%) |
|---|---|---|---|---|
| BM3 254 | V78Y, A82E, F87A | delta-1-testosterone | estradiol | 1.9 |
| BM3 261 | V78M, A82E, F87A | delta-1-testosterone | estradiol | 6 |
| BM3 263 | V78I, A82E, F87A | delta-1-testosterone | estradiol | 4.8 |
| BM3 268 | V78L, A82E, F87A | delta-1-testosterone | estradiol | 6.6 |

In a subsequent screening experiment of four BM3 variants the following screening yields were found for the following (side) products:

**Table E3: Products formed with delta-1-testosterone as substrate for some P450 BM3 variants.**

| Variant | Substrate | Screening yield (%) | | | | | |
|---|---|---|---|---|---|---|---|
| | | Example 10 | Example 12 | Example 11 | Example 15 | Example 16 | Example 14 |
| BM3 254 | delta-1-testosterone | 2.5 | 10.3 | 8.6 | 3.4 | 2.2 | - |
| BM3 261 | delta-1-testosterone | 6.3 | 15 | 13.2 | 9.3 | 0.4 | 10.1 |
| BM3 263 | delta-1-testosterone | 3 | 21.6 | 1.6 | 11.8 | 1.5 | 6.5 |
| BM3 268 | delta-1-testosterone | 6.7 | 26.3 | 5.4 | 7.8 | 4.1 | 10.3 |

### Example 9

### Method for screening P450-BM3 variants for the C19 hydroxylation of (17beta)-17-hydroxyandrost-4-en-3-one (testosterone) as substrate

Media, preculture and main culture were prepared as decribed for delta-1-testosterone (Example 8).

### HPLC Analysis

### Analytical chiral HPLC method:

Instrument: Waters Acquity UPLCMS SingleQuad; Column: Ascentis Express C18 2.7 µm, 50x2.1 mm; Eluent A: water + 0.05 vol % formic acid, eluent B: acetonitrile + 0.05 vol % formic acid; Gradient: 0.0-3.0 min 5-30 % B, 3.0-5.0 95 % B, 5.0-5.5 5 % B; injection volume 4 µL; flow 0.9 mL/min; temperature: 45 °C; DAD scan: 244 nm.

**Table E4: HPLC retention times**

| **Substance** | **Retention Time** |
|---|---|
| Testosterone | 3.45 |
| (19-OH)-testosterone (Example 17) | 2.19 |
| (2beta,19-di-OH)-testosterone (Example 20) | 1.63 |
| (15beta,19-di-OH)-testosterone (Example 18) | 1.23 |
| (2beta,15beta-di-OH)-testosterone (Example 19) | 1.17 |
| (15beta-OH)-testosterone | 1.95 |
| (2beta-OH)-testosterone | 2.92 |

### Results

After testing hundreds of P450-BM3 variants, only few variants allowed for the C19-hydroxylation of testosterone:

**Table E5: Products formed with testosterone as substrate for some P450 BM3 variants.**

| Variant | Substrate | Screening yield (%) | | | | | |
|---|---|---|---|---|---|---|---|
| | | Example 17 | Example 20 | Example 18 | Example 19 | (2beta-OH)-testosterone | 15beta-OH)-testosterone |
| BM3 254 | testosterone | 8 | 4 | - | - | 29 | 5 |
| BM3 261 | testosterone | - | 23 | - | 13 | 31 | 17 |
| BM3 263 | testosterone | - | 11 | 4 | 5 | 35 | 16 |
| BM3 268 | testosterone | - | 39 | 5 | 7 | 9 | 20 |

### Example 10

### (17beta)-estra-1(10),2,4-triene-3,17-diol

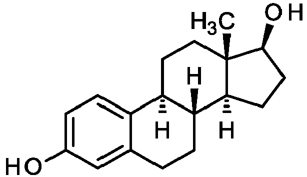

### Expression system

Expression system *E. coli* BL 21(DE3) pETM11 with BM3-268 was provided by M. T. Reetz and is described in (Kille Sabrina 2011) (DOI:10.1038/NCHEM.1113). The respective protein and gene sequence such as pETM11-BM3-268 is given in SEQ ID No. 5 and 63, respectively.

### Cultivation

Preculture medium comprised tryptone (10 g/L), sodium chloride (10 g/L) and yeast extract (5 g/L) in demineralized water. The medium was sterilized before kanamycin (50 mg/L) was added. One preculture (100 mL) was inoculated with the strain *E. coli* BL 21 (DE3) pETM11-BM3-268 (SEQ ID No.5) (50 µL) containing the desired plasmids and was shaken at 37 °C and 165 rpm for 16 hours. This preculture (100 mL) was used to inoculate one 5 L steel fermenter. The cultivation medium was prepared in the fermenter. Tryptone (20 g/L), sodium chloride (20 g/L), yeast extract (10 g/L) and Pluronic® PE 8100 (0.5 mL) were dissolved in demineralized water (4.0 L) and sterilized for 30 minutes at 121 °C in the fermenter. Afterwards kanamycin (0.25 g) in demineralized water (10 mL) was added. After the inoculation of the fermenter (16 hours old preculture), the inoculated culture was stirred at 500 rpm at 37 °C with an aeration rate of 2.5 L/min and an oxygen partial pressure of 30 % regulated by the stirring rate of up to 1500 rpm. After 3 hours an aqueous glucose solution (25 %, 40 g/h) was added. After 4 hours and 25 minutes an OD550 of 16.16 was reached, the temperature was decreased to 28 °C within 10 minutes and IPTG (1.19 g) in demineralized water (50 mL) was added to start the protein expression. After additional 8 hours and 35 minutes the cells (124.6 g) were harvested by centrifugation, suspended in buffer (125 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 4 x 45 mL, 1 x 35 mL und 10 x 1 mL.

### Biotransformation

To a 5 L steel fermenter which contained KH2PO4 (15 g), K2HPO4 (46.15 g), Pluronic® PE 8100 (0.5 mL), demineralized water (3.8 L), aqueous glucose solution (50 %, 375 mL) and aqueous EDTA solution (0.5 M, 3.75 mL) (17beta)-17-hydroxyandrosta-1,4-dien-3-one (500 mg, 1.75 mmol) dissolved in DMF (10 mL) was added and the mixture was stirred at 140 rpm at 27 °C with an aeration rate of 2.0 L/min. Cells in buffer (215 mL) were added and the biotransformation was stirred at an oxygen partial pressure of 50 % regulated by the stirring rate of up to 800 rpm. The pH value was maintained at pH 7.4 by addition of an aqueous solution of sodium hydroxide (16 %). After 26 hours and 5 minutes the biotransformation was extracted with 4-methyl-2-pentanone. The organic layer was concentrated to give a crude oil (4.98 g) which was slurried with methanol, filtered and concentrated to give an oil (4 g). The crude product was further purified by flash chromatography using silica gel (dichloromethane/acetone gradient) and by preparative HPLC to give the title compound (4.3 mg, 0.8 % yield).

### Preparative chiral HPLC method

Instrument: Waters Autopurificationsystem; Column: Waters XBrigde C18 5 µ 100x30 mm; Eluent A: water + 0.2 vol-% aqueous ammonia (32 %), Eluent B: acetonitrile; gradient: 0.00-0.50 min 17 % B (40-70 mL/min), 0.51-5.50 min 34-44 % B (70 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1 mm; eluent A: water + 0.2 vol % aqueous ammonia (32 %), eluent B: acetonitrile; gradient: 0-1.6 min 1-99 % B, 1.6-2.0 min 99 % B; flow 0.8 mL/min; temperature: 60 °C; DAD scan: 210-400 nm.
LC-MS: Rt = 1.01 min; MS (ESIneg): m/z = 271 [M-H]⁻
¹H-NMR (400 MHz, CHLOROFORM-d) δ [ppm]: 0.000 (1.83), 0.710 (4.29), 1.102 (0.18), 1.113 (0.30), 1.132 (0.31), 1.141 (0.29), 1.160 (0.32), 1.185 (0.85), 1.213 (0.54), 1.224 (0.54), 1.243 (0.60), 1.255 (0.59), 1.273 (0.57), 1.286 (0.46), 1.303 (0.52), 1.317 (0.44), 1.329 (0.32), 1.348 (0.47), 1.355 (0.45), 1.375 (0.54), 1.398 (0.55), 1.407 (0.56), 1.429 (0.57), 1.438 (0.51), 1.460 (0.41), 1.471 (0.41), 1.509 (0.63), 1.590 (0.24), 1.599 (0.21), 1.616 (0.31), 1.621 (0.29), 1.633 (0.23), 1.640 (0.26), 1.775 (0.23), 1.783 (0.23), 1.790 (0.24), 1.807 (0.22), 1.814 (0.24), 1.821 (0.20), 1.851 (0.23), 1.859 (0.34), 1.867 (0.24), 1.882 (0.22), 1.890 (0.31), 1.898 (0.20), 2.030 (0.22), 2.043 (0.24), 2.053 (0.23), 2.063 (0.26), 2.077 (0.30), 2.100 (0.32), 2.211 (0.25), 2.218 (0.25), 2.244 (0.24), 2.252 (0.22), 2.554 (16.00), 2.736 (0.53), 2.749 (0.54), 2.776 (0.22), 3.642 (0.29), 3.664 (0.50), 3.685 (0.27), 6.498 (0.70), 6.546 (0.36), 6.552 (0.31), 6.567 (0.39), 6.574 (0.34), 7.076 (0.58), 7.097 (0.53).

### Example 11

### (1alpha,2alpha,17beta)-17-hydroxy-1,2-epoxyandrost-4-en-3-one

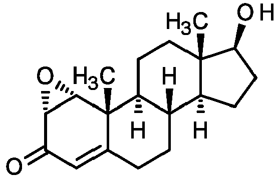

For the preparation of the title compound see preparation of Example 10. The crude product was further purified by flash chromatography using silica gel (dichloromethane/acetone gradient) and by preparative HPLC to give the title compound (13 mg).

### Preparative chiral HPLC method

Instrument: Waters Autopurification system; Column: Waters XBrigde C18 5 µ 100x30 mm; Eluent A: Water + 0.1 Vol-% Formic acid (99 %), Eluent B: Acetonitrile; Gradient: 0.00-0.50 min 16 % B (25-70 mL/min), 0.51-5.50 min 33-48 % B (70 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1 mm; Eluent A: Water + 0.1 Vol-% Formic acid (99 %), Eluent B: Acetonitrile; Gradient: 0-1.6 min 1-99 % B, 1.6-2.0 min 99 % B; Flow: 0.8 mL/min; Temperature: 60 °C; DAD scan: 210-400 nm.
Analytical Chiral HPLC: Rt = 2.04 min.
¹H-NMR (600MHz, Pyridine-d5): δ [ppm]: 0.77 - 0.91 (m, 2H), 0.98 - 1.05 (m, 1H), 0.99 (s, 3H), 1.11 (td, 1H), 1.23 - 1.32 (m, 1H), 1.34 (s, 3H), 1.43 - 1.53 (m, 2H), 1.55 - 1.67 (m, 2H), 1.73 - 1.81 (m, 2H), 2.01 - 2.14 (m, 3H), 2.20 (td, 1H), 3.63 (dd, 1H), 3.74 (d, 1H), 3.89 (td, 1H), 5.91 - 5.93 (m, 1H), 6.18 (d, 1H).

### Example 12

### (15beta,17beta)-15,17-dihydroxyandrosta-1,4-dien-3-one

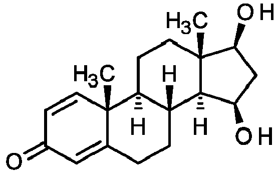

For the preparation of the title compound see preparation of Example 10. The crude product was further purified by flash chromatography using silica gel (dichloromethane/acetone gradient) and by preparative HPLC to give the title compound (78 mg).
Analytical HPLC: Rₜ = 0.74 min; MS (ESIpos): m/z = 303 [M+H]⁺
¹H-NMR (600 MHz, CDCl3) δ [ppm]: 0.82 (dd, 1H), 1.05 - 1.16 (m, 3H), 1.11 (s, 3H), 1.28 (s, 3H), 1.58 - 1.65 (m, 1H), 1.71 (qd, 1H), 1.75 - 1.80 (m, 1H), 1.86 (dt, 1H), 2.09 (qd, 1H), 2.20 - 2.29 (m, 1H), 2.37 - 2.42 (m, 1H), 2.55 (tdd, 1H), 2.63 (ddd, 1H), 3.58 (t, 1H), 4.21 (ddd, 1H), 6.09 (s, 1H), 6.24 (dd, 1H), 7.08 (d, 1H).

### Example 13

### (15beta, 17beta)-estra-1(10),2,4-triene-3,15,17-triol

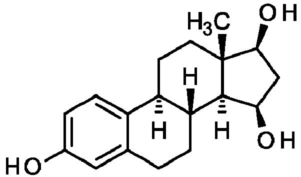

### Cultivation

Medium of the preculture with tryptone (16 g/L), sodium chloride (10 g/L) and yeast extract (10 g/L) in demineralized water was adjusted to pH 7.3 with sodium hydroxide solution (16 % in water), and sterilized at 121 °C for 20 minutes. Afterwards kanamycin (50 mg/L) was added.

One preculture (100 mL each) was inoculated with the *Escherichia coli* strain BL 21 Gold (DE3) ΔdkgA :: FRT T7 gdh (50 µL) expressing BM3-268; F77L and was shaken at 37 °C and 165 rpm for 16 hours. These precultures (100 mL) were used to inoculate one 10 L steel fermenter. The cultivation media was prepared in the fermenters. Tryptone (12 g/L), yeast extract (24 g/L), predigested beef extract (2 g/L), KH2PO4 (2.2 g/L), K2HPO4 (9.4 g/L) and glycerol (87 %, 4.6 g/L) were dissolved in demineralized water (9.2 L) and sterilized for 20 minutes at 121 °C in the fermenter. Afterwards kanamycin (0.5 g) in water (20 mL), riboflavin (10 mg) in water (20 mL), thiamine hydrochloride (3.37 g) in water (10 mL) and Oxford trace metal solution (2.5 mL) were added. After the inoculation with the 17 hours old preculture, the inoculated culture was stirred at 315 rpm at 37 °C with an aeration rate of 3.3 L/min partial pressure at pH 6.6 which was regulated by addition of aqueous sodium hydroxide solution (16 %) or aqueous phosphoric acid solution (16 %). After 2 hours an OD550 of 0.92 was reached, the temperature was decreased to 27 °C within 15 minutes and IPTG (2.38 g) in demineralized water (40 mL) and aminolevulinic acid (838 mg) in demineralized water (40 mL) were added to start the protein expression. After 12 hours the aqueous phosphoric acid solution (16 %) was substituted for an aqueous glucose solution (50 %) for pH regulation. After additional 24 hours the cells were harvested by centrifugation (96.86 g), suspended in buffer (97 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 2 x 40 mL, 1 x 80 mL und 5 x 1 mL.

### Biotransformation 1

To one 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrosta-1,4-dien-3-one (50 mg, 174.5 µmol) dissolved in DMF (4 mL) was added and the mixture was stirred at 300 rpm at 27 °C with an aeration rate of 0.5 L/min. The pH value was maintained at pH 7.4. Cells in buffer (20 mL) were added and the biotransformation was maintained at an oxygen partial pressure of 50 % by varying the stirring rate to up to 1200 rpm. After 24 hours and 40 minutes the biotransformation was frozen and stored at -20 °C.

### Biotransformation 2

To one 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrosta-1,4-dien-3-one (50 mg, 174,5 µmol) dissolved in DMF (4 mL) was added and the mixture was stirred at 300 rpm at 27 °C with an aeration rate of 0.5 L/min. The pH value was maintained at pH 7.4. Cells in buffer (40 mL) were added and the biotransformation was maintained at an oxygen partial pressure of 50 % by varying the stirring rate to up to 1200 rpm. After 24 hours and 40 minutes the biotransformation was harvested, frozen and stored at -20 °C.

### Biotransformation 3 + 4

To two 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrosta-1,4-dien-3-one (50 mg, 174,5 µmol) dissolved in DMF (4 mL) was added and the mixture was stirred at 300 rpm at 27 °C with an aeration rate of 0.5 L/min. The pH value was maintained at pH 7.4. Cells in buffer (40 mL) were added and the biotransformation was maintained at an oxygen partial pressure of 50 % by varying the stirring rate to up to 1200 rpm. After 5 hours and 20 minutes the culture was harvested, frozen and stored at -20 °C.

The harvested four biotransformations were thawed, combined and extracted with 4-methyl-2-pentanone.

The organic phases were concentrated to give a crude oil (1.69 g) which was slurried with methanol and was filtered and concentrated to give an oil (1.04 g). The crude product was further purified by flash chromatography using silica gel (dichloromethane/ethanol gradient) and by preparative HPLC to give the title compound (13.0 mg).

### Preparative chiral HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 5 % B (50-100 mL/min), 0.51-8.00 min 5 - 35 % B (100 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.
Analytical Chiral HPLC: Rₜ = 0.78 min.
¹H-NMR (600MHz, Pyridine-d5): δ [ppm]: 1.08 (dd, 1H), 1.37 (td, 1H), 1.49 (qd, 1H), 1.61 (s, 3H), 1.65 - 1.74 (m, 1H), 2.17 - 2.24 (m, 2H), 2.28 (ddd, 1H), 2.32 - 2.39 (m, 2H), 2.49 - 2.56 (m, 1H), 2.81 - 2.89 (m, 2H), 2.94 - 3.01 (m, 1H), 3.96 (td, 1H), 4.55 (s, 1H), 5.85 (d, 1H), 6.25 (d, 1H), 7.02 (d, 1H), 7.12 (dd, 1H), 7.38 (d, 1H), 11.16 (s, 1H).

### Example 14

### (1alpha,2alpha,17beta)-17,19-dihydroxy-1,2-epoxyandrost-4-en-3-one

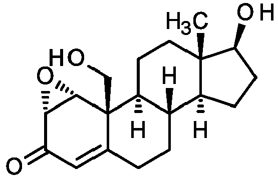

For the preparation of the title compound see Example 13. The crude product (1.04 g) was further purified by flash chromatography using silica gel (dichloromethane/ethanol gradient) and by preparative HPLC to give the title compound (7.4 mg).

### Preparative chiral HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 5 % B (50-100 mL/min), 0.51-8.00 min 5 - 35 % B (100 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.
Analytical Chiral HPLC: Rt = 0.80 min.
¹H-NMR (600 MHz, CDCl3) δ [ppm]: -0.006 (0.17), 0.000 (4.06), 0.005 (0.19), 0.772 (0.44), 0.816 (16.00), 0.880 (0.19), 0.969 (0.54), 0.981 (0.62), 0.987 (0.73), 0.989 (0.80), 0.999 (0.79), 1.001 (0.80), 1.007 (0.70), 1.015 (0.41), 1.020 (0.77), 1.036 (0.77), 1.044 (0.88), 1.057 (0.93), 1.064 (0.94), 1.069 (1.08), 1.076 (1.00), 1.086 (0.47), 1.090 (0.59), 1.096 (0.51), 1.253 (0.81), 1.260 (0.82), 1.266 (0.73), 1.278 (0.76), 1.282 (1.02), 1.287 (0.83), 1.299 (0.81), 1.305 (0.86), 1.313 (0.39), 1.324 (0.90), 1.334 (0.94), 1.344 (1.03), 1.354 (1.03), 1.365 (0.45), 1.374 (0.43), 1.432 (0.59), 1.437 (0.64), 1.445 (0.72), 1.451 (0.96), 1.454 (0.88), 1.457 (0.79), 1.460 (0.87), 1.465 (0.68), 1.468 (0.78), 1.471 (0.71), 1.474 (0.96), 1.480 (0.52), 1.488 (0.46), 1.494 (0.47), 1.506 (0.43), 1.512 (0.49), 1.528 (0.85), 1.534 (1.04), 1.550 (0.85), 1.556 (0.84), 1.572 (0.55), 1.574 (0.61), 1.579 (0.75), 1.586 (0.60), 1.590 (0.81), 1.595 (0.92), 1.602 (0.83), 1.607 (1.01), 1.611 (0.91), 1.616 (0.83), 1.623 (1.01), 1.628 (1.22), 1.640 (1.51), 1.761 (0.34), 1.767 (0.37), 1.779 (0.68), 1.785 (0.77), 1.799 (0.70), 1.806 (0.68), 1.816 (0.29), 1.823 (0.30), 1.862 (0.36), 1.868 (0.80), 1.873 (1.01), 1.882 (1.30), 1.884 (1.47), 1.889 (1.08), 1.898 (1.30), 1.905 (1.12), 1.910 (0.58), 1.992 (0.36), 2.000 (0.71), 2.004 (0.72), 2.014 (0.49), 2.022 (0.68), 2.026 (0.67), 2.033 (0.35), 2.052 (0.38), 2.062 (0.43), 2.067 (0.72), 2.074 (0.56), 2.077 (0.75), 2.083 (0.62), 2.090 (0.70), 2.092 (0.54), 2.100 (0.62), 2.105 (0.40), 2.115 (0.33), 2.312 (0.50), 2.319 (0.68), 2.324 (0.56), 2.334 (0.85), 2.339 (1.05), 2.346 (0.78), 2.372 (0.43), 2.396 (0.84), 2.403 (0.73), 2.405 (0.74), 2.418 (0.98), 2.426 (0.94), 2.439 (0.42), 2.441 (0.42), 2.447 (0.39), 3.358 (2.18), 3.361 (2.22), 3.364 (2.35), 3.367 (2.14), 3.633 (0.88), 3.647 (1.53), 3.662 (0.85), 3.944 (2.87), 3.950 (2.82), 4.173 (0.59), 4.263 (2.71), 4.281 (1.88), 5.900 (2.52), 7.267 (12.12).

### Example 15

### (1alpha,2alpha,15beta,17beta)-15,17-dihydroxy-1,2-epoxyandrost-4-en-3-one

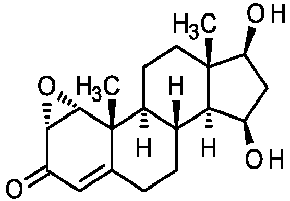

For the preparation of the title compound see Example 13. The crude product (1.04 g) was further purified by flash chromatography using silica gel (dichloromethane/ethanol gradient) and by preparative HPLC to give the title compound (2.8 mg).

### Preparative chiral HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 5 % B (50-100 mL/min), 0.51-8.00 min 5 - 35 % B (100 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.
Analytical Chiral HPLC: Rt = 0.73 min.
¹H-NMR (600 MHz, CHLOROFORM-d) δ [ppm]: -0.006 (0.48), 0.000 (11.79), 0.005 (0.44), 0.825 (1.12), 0.835 (1.15), 0.844 (1.18), 0.854 (1.23), 0.857 (1.22), 0.881 (0.35), 1.070 (0.62), 1.076 (0.66), 1.091 (1.49), 1.104 (13.48), 1.112 (1.31), 1.119 (1.11), 1.134 (0.39), 1.141 (0.37), 1.227 (0.58), 1.234 (0.66), 1.254 (2.09), 1.266 (0.93), 1.272 (0.78), 1.398 (0.70), 1.440 (15.22), 1.457 (1.29), 1.513 (0.45), 1.546 (0.44), 1.571 (2.00), 1.576 (2.63), 1.590 (16.00), 1.610 (1.91), 1.614 (2.15), 1.619 (1.27), 1.635 (0.91), 1.641 (0.91), 1.657 (0.40), 1.663 (0.37), 1.786 (0.38), 1.792 (0.80), 1.797 (0.85), 1.804 (0.43), 1.808 (0.34), 1.815 (0.61), 1.820 (0.62), 1.872 (0.61), 1.877 (0.96), 1.883 (0.58), 1.893 (0.64), 1.898 (0.85), 1.905 (0.51), 2.108 (0.34), 2.114 (0.32), 2.126 (0.65), 2.133 (0.70), 2.145 (0.62), 2.152 (0.65), 2.215 (0.47), 2.219 (0.62), 2.226 (0.54), 2.236 (0.60), 2.240 (0.78), 2.247 (0.68), 2.262 (0.38), 2.269 (0.60), 2.274 (0.52), 2.283 (0.45), 2.291 (0.57), 2.294 (0.51), 2.405 (0.51), 2.414 (0.47), 2.428 (0.86), 2.435 (0.79), 2.449 (0.41), 2.457 (0.36), 2.603 (0.67), 2.616 (0.88), 2.617 (0.84), 2.627 (0.79), 2.630 (0.83), 2.640 (0.83), 2.642 (0.82), 2.654 (0.65), 3.385 (1.56), 3.388 (1.69), 3.391 (1.66), 3.395 (1.55), 3.571 (0.58), 3.585 (1.12), 3.600 (0.56), 3.678 (2.43), 3.684 (2.34), 4.182 (0.51), 4.191 (0.87), 4.199 (0.51), 5.746 (2.23).

### Example 16

### (6beta,17beta)-6,17-dihydroxyandrosta-1,4-dien-3-one

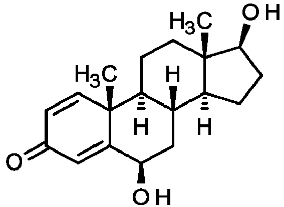

For the preparation of the title compound see Example 13. The crude product (1.04 g) was further purified by flash chromatography using silica gel (dichloromethane/ethanol gradient) and by preparative HPLC to give the title compound (2.4 mg).

### Preparative chiral HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 5 % B (50-100 mL/min), 0.51-8.00 min 5 - 35 % B (100 mL/min), DAD scan: 210-400 nm.

### Analytical chiral HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.
Analytical Chiral HPLC: Rt = 0.75 min.
¹H-NMR (600 MHz, CDCl3) δ [ppm]: 0.000 (7.05), 0.850 (0.34), 0.857 (10.48), 0.868 (0.34), 0.960 (0.40), 0.972 (0.39), 0.979 (0.55), 0.990 (0.55), 0.998 (0.48), 1.010 (0.46), 1.056 (0.37), 1.065 (0.43), 1.075 (0.61), 1.082 (0.65), 1.089 (0.52), 1.094 (0.57), 1.096 (0.56), 1.103 (1.02), 1.110 (0.72), 1.118 (0.75), 1.131 (0.41), 1.139 (0.42), 1.215 (0.47), 1.220 (0.44), 1.235 (0.62), 1.238 (0.70), 1.241 (0.73), 1.243 (0.71), 1.253 (1.01), 1.258 (1.09), 1.264 (0.79), 1.377 (0.64), 1.387 (0.69), 1.398 (0.76), 1.407 (0.80), 1.418 (0.40), 1.427 (0.45), 1.440 (1.03), 1.456 (12.77), 1.464 (0.72), 1.470 (0.66), 1.473 (0.63), 1.476 (0.51), 1.478 (0.58), 1.487 (0.52), 1.490 (0.47), 1.492 (0.64), 1.499 (0.39), 1.506 (0.37), 1.512 (0.39), 1.562 (0.37), 1.571 (0.42), 1.602 (1.00), 1.608 (1.08), 1.614 (1.25), 1.619 (1.41), 1.623 (1.48), 1.628 (1.35), 1.631 (1.37), 1.635 (1.48), 1.638 (1.35), 1.644 (1.17), 1.651 (1.05), 1.656 (0.96), 1.705 (0.40), 1.712 (0.39), 1.727 (0.56), 1.734 (0.56), 1.748 (0.66), 1.754 (0.70), 1.768 (0.66), 1.773 (0.86), 1.777 (0.87), 1.780 (0.57), 1.785 (0.56), 1.792 (0.36), 1.795 (0.39), 1.799 (0.37), 1.883 (0.57), 1.889 (0.73), 1.895 (0.53), 1.905 (0.56), 1.910 (0.66), 1.916 (0.46), 2.052 (0.49), 2.057 (0.81), 2.062 (0.63), 2.069 (0.34), 2.074 (0.85), 2.081 (1.05), 2.085 (1.41), 2.090 (0.63), 2.093 (0.33), 2.097 (0.54), 2.102 (0.65), 2.107 (0.74), 2.112 (0.39), 2.122 (0.72), 2.127 (0.45), 3.646 (0.92), 3.660 (1.30), 3.674 (0.85), 4.543 (0.81), 4.548 (1.50), 4.553 (0.85), 6.162 (2.12), 6.165 (2.42), 6.210 (1.34), 6.213 (1.21), 6.226 (1.36), 6.229 (1.27), 7.052 (1.95), 7.069 (1.96), 7.264 (16.00).

### Example 17

### (17beta)-17,19-dihydroxyandrost-4-en-3-one

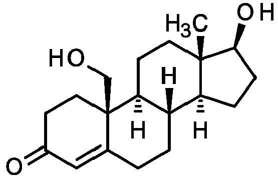

### Preparation 1

### Expression system

The strain *E. coli* BL 21 (DE3) pETM11-BM3-254 was provided by M. T. Reetz and is described in (Kille Sabrina 2011) (DOI:10.1038/NCHEM.1113). The protein and gene sequence are listed under SEQ ID No. 2 and 60, respectively.

### Cultivation

Medium of the preculture: tryptone (10 g/L), sodium chloride (10 g/L) and yeast extract (5 g/L) in demineralized water. The media was sterilized at 121 °C for 20 minutes. Afterwards kanamycin (50 mg/L) was added.

One preculture (100 mL) was inoculated with the strain *E. coli* BL 21 (DE3) pETM11-BM3-254 (50 µL) containing the desired plasmids and was shaken at 37 °C and 165 rpm for 16 hours. This preculture (10 mL) was used to inoculate one 1 L Biostat Q fermenter. The cultivation medium was prepared in the fermenter. Tryptone (20 g/L), sodium chloride (20 g/L), yeast extract (10 g/L) and Pluronic® PE 8100 (0.1 mL) were dissolved in demineralized water (1.0 L) and sterilized for 30 minutes at 121 °C in the fermenter. Afterwards kanamycin (50 mg) in demineralized water (5 mL) was added. After the inoculation of the fermenter the inoculated culture was stirred at 500 rpm at 37 °C with an aeration rate of 0.4 L/min and an oxygen partial pressure of 30 % was maintained by the stirring at a rate of up to 1500 rpm. After 3 hours an aqueous glucose solution (50%, 4 g/h) was added. After 4 hours and 50 minutes an OD550 of 16.1 was reached, the temperature was decreased to 28 °C within 5 minutes and IPTG (238 mg) in demineralized water (10 mL) was added to start the protein expression. After additional 9 hours the cells (39.1 g) were harvested by centrifugation, suspended in buffer (39.1 mL; KH2PO4 (4 g/L), K2HPO4 (12.3 g/L), glycerol (4 % (v/v)), glucose (5 % (w/v)), EDTA (0.5 mM)), frozen in liquid nitrogen and stored at -80 °C until further use in aliquots of 1 x 75 mL, 3 x 1 mL.

### Biotransformation

To two 1 L Biostat Q fermenter each containing KH2PO4 (3 g), K2HPO4 (9.23 g), Pluronic® PE 8100 (0.1 mL), demineralized water (0.75 L), aqueous glucose solution (50 %, 15 mL) and aqueous EDTA solution (0.5 M, 0.75 mL) (17beta)-17-hydroxyandrost-4-en-3-one (75.0 mg, 260 µmol) dissolved in DMF (5 mL) was added and the mixture was stirred at 300 rpm at 27 °C with an aeration rate of 0.5 L/min. Cells in buffer (each 37 mL) were added and the biotransformation was maintained at an oxygen partial pressure of 50 % by varying the stirring rate to up to 800 rpm. The pH value was maintained at pH7.4. After 26 hours aqueous glucose solution was added (50 %, 60 mL). After 102.5 hours the biotransformation was extracted with 4-methyl-2-pentanone. The organic layer was concentrated to give an oil (1.09 g) which was dissolved in methanol and was concentrated to give an oil (0.91 g). The crude product was further purified by flash chromatography using silica gel (dichloromethane/ methanol gradient) and by preparative HPLC to give the title compound (4.0 mg, 2,52 % yield).

### Preparative chiral HPLC method:

Instrument: Labomatic HD5000, Labocord-5000; Gilson GX-241, Labcol Vario 4000, Column: Chiralpak IA 5 µ 250x30 mm; Eluent A: Acetonitrile + 0.1 Vol-% Diethylamine (99 %); Eluent B: Ethanol; Isocratic: 90 % A + 10 % B; Flow 50.0 mL/min; UV 254 nm.

### Analytical chiral HPLC method:

Instrument: Agilent HPLC 1260; Column: Chiralpak IA 3 µ 100x4.6 mm; Eluent A: Acetonitrile + 0.1 Vol-% Diethylamine (99 %); Eluent B: Ethanol; Isocratic: 90 % A + 10 % B; Flow 1.4 mL/min; Temperature: 25 °C; DAD 254 nm.
¹H-NMR (600 MHz, CHLOROFORM-d) δ [ppm]: 0.79 (s, 3H), 0.92 - 0.99 (m, 1H), 1.02 - 1.09 (m, 3H), 1.31 (qd, 2H), 2.05 - 2.13 (m, 1H), 2.32 - 2.44 (m, 4H), 2.75 (ddd, 1H), 3.65 (t, 1H), 3.92 (d, 1H), 4.08 (dd, 1H), 5.96 (s, 1H).

### Preparation 2

### Expression system

In a second preparation the title compound was synthesized with the strain *E. coli* BL 21 Gold (DE3) ΔdkgA :: FRT T7 gdh and plasmid pETM11-BM3-254. The construction of the *E. coli* BL 21 Gold (DE3) ΔdkgA :: FRT T7 gdh strain is described in (Agudo R 2012). The strain was rendered competent and transformed with pETM11-BM3-254.

### Preparation of competent cells & transformation

Required solutions: TFB1 buffer containing KCH3COO (2.95 g/L), KCI (7.46 g/L), CaCl2 (1.11 g/L), glycerol (130 mL). TFB2 buffer containing KCI (0.75 g/L), CaCl2(11.1 g/L), MOPS buffer (2.1 g/L), glycerol (130 mL). The buffers were autoclaved. A solution of MgCl2x6H2O (95.2 g/L) was prepared including sterile filtration.

A culture (5 mL) of Luria-Bertani broth medium (10 g/L bacto tryptone, 5 g/L bacto yeast extract, 10 g/L sodium chloride) was inoculated with a glycerol culture (2 µl) of the strain *E. coli* BL 21 Gold (DE3) ΔdkgA :: FRT T7 gdh. This culture was shaken at 37 °C and 165 rpm overnight. The overnight culture (1 mL) was used to inoculate a culture (100 mL) of Luria-Bertani broth medium. The culture was incubated at 37 °C and 165 rpm until an OD600 of 0.4-0.5 was reached, whereupon the culture was subjected to centrifugation at 4 °C and 4000 rpm for 15 minutes. All following substances were pre-cooled in ice. The supernatant of the culture was discarded and the cell pellet was resuspended in TFB1 buffer (30 mL) and 3.2 ml MgCl2 solution (3.2 mL) and incubated on ice for 15 min. The mixture was subjected to centrifugation at 4 °C and 4000 rpm for 10 minutes. The supernatant was discarded and the cell pellet was resuspended in TFB2 buffer (4 mL) and was incubated for 15 min on ice. The cells were aliquoted a 50 µL, were snap frozen in liquid nitrogen and stored at -80 ° C.

For transformation 50 µL of the competent cells were thawed on ice for 15 min, 1-2 µL of plasmid (pETM11-BM3-254) were added, and the mixture was incubated on ice for 30 min. A heat shock was carried out for 45 sec at 42 °C, followed by incubation on ice for 5 min. After the addition of SOC Outgrowth Medium (350 µL), the mixture was incubated for 1 h at 37 °C and 1000 rpm shaking. The cells (100 µL) were placed on Luria-Bertani broth agar plates with kanamycin (50 mg/L) and incubated overnight at 37 °C. Single colonies were picked and sequenced.

### Cultivation of E. coli BL 21 Gold (DE3) ΔdkgA :: FRT T7 gdh pETM11-BM3-254 and protein expression:

Media of the preculture: tryptone (10 g/L), sodium chloride (10 g/L) and yeast extract (5 g/L) in demineralized water. The media was sterilized at 121 °C for 20 minutes. Afterwards kanamycin (50 mg/L) was added.

### Cell cultivation 1

One preculture (100 mL) was inoculated with the strain *E. coli* BL 21 Gold (DE3) ΔdkgA :: FRT T7 gdh pETM11-BM3-254 (50 µL) containing the desired plasmid and was shaken at 37 °C and 165 rpm for 16 hours. This preculture (10 mL) was used to inoculate one 1 L Biostat Q fermenter. The cultivation media was prepared in the fermenters. Tryptone (20 g/L), sodium chloride (20 g/L), yeast extract (10 g/L) and Pluronic® PE 8100 (0.1 mL) were dissolved in demineralized water (1.0 L) and sterilized for 30 minutes at 121 °C in the fermenter. Afterwards kanamycin (50 mg) in demineralized water (5 mL) and Oxford trace metal solution (0.25 mL) was added. After the inoculation of the fermenter, the inoculated culture was stirred at 500 rpm at 37 °C with an aeration rate of 0.4 L/min and an oxygen partial pressure of 30 % maintained by the stirring up to 1500 rpm. After 3 hours an aqueous glucose solution was added (25 %, 8 g/h). After 5 hours and 30 minutes OD550 of 15.72 was reached, the temperature was decreased to 28 °C within 10 minutes and IPTG (238 mg) in demineralized water (10 mL) was added to start the protein expression. After additional 9 hours and 50 minutes the cells (34.88 g) were harvested by centrifugation, suspended in buffer (34.88 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 2 x 25 mL, 3 x 1 mL and 1 x 13 ml.

### Cell cultivation 2

One preculture (100 mL) was inoculated with the strain *E. coli* BL 21 Gold (DE3) ΔdkgA :: FRT T7 gdh pETM11-BM3-254 (50 µL) containing the desired plasmid and was shaken at 37 °C and 165 rpm for 16 hours. This preculture (10 mL) was used to inoculate one 1 L Biostat Q fermenter. The cultivation media was prepared in the fermenters. Tryptone (20 g/L), sodium chloride (20 g/L), yeast extract (10 g/L) and Pluronic® PE 8100 (0.1 mL) was dissolved in demineralized water (4.0 L) and sterilized for 30 minutes at 121 °C in the fermenter. Afterwards kanamycin (50 mg) in demineralized water (5 mL) and Oxford trace metal solution (0.25 mL) was added. After inoculation of the fermenter, the inoculated culture was stirred at 500 rpm at 37 °C with an aeration rate of 0.4 L/min, and an oxygen partial pressure of 30 % was maintained by the stirring up to 1500 rpm. After 3 hours an aqueous glucose solution (25%, 8 g/h) was added. After 5 hours and 5 minutes an OD550 of 16.72 was reached, the temperature was decreased to 28 °C within 5 minutes and IPTG (238 mg) in demineralized water (10 mL) and aminolevulinic acid (42 mg) in demineralized water (5 mL) were added to start the protein expression. After additional 9 hours and 10 minutes the cells (38 g) were harvested by centrifugation, suspended in buffer (38 g; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 2 x 25 mL, 3 x 1 mL and 1 x 19 ml.

### Cell cultivation 3

One preculture (100 mL) was inoculated with the strain *E. coli* BL 21 Gold (DE3) ΔdkgA :: FRT T7 gdh pETM11-BM3-254 (50 µL) containing the desired plasmid and was shaken at 37 °C and 165 rpm for 16 hours. This preculture (100 mL) was used to inoculate one 10 L steel fermenter. The cultivation media was prepared in the fermenter. Tryptone (20 g/L), sodium chloride (20 g/L), yeast extract (10 g/L) and Pluronic® PE 8100 (0.1 mL) were dissolved in demineralized water (8.8 L) and sterilized for 30 minutes at 121 °C in the fermenter. Afterwards kanamycin (500 mg) in demineralized water (10 mL) and Oxford trace metal solution (2.5 mL) was added. After inoculation of the fermenter (16 hours old preculture), the inoculated culture was stirred at 400 rpm at 37 °C with an aeration rate of 4.0 L/min and an oxygen partial pressure of 30 % maintained by stirring up to 1200 rpm. After 3 hours an aqueous glucose solution (25%, 80 g/h) was added. After 5 hours and 20 minutes an OD550 of 16.69 was reached, the temperature was decreased to 28 °C within 5 minutes and IPTG (2.38 g) in demineralized water (20 mL) and aminolevulinic acid (420 mg) in demineralized water (20 mL) was added to start the protein expression. After additional 9 hours and 25 minutes the cells (298.42g) were harvested by centrifugation, suspended in buffer (298.42 g; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 7 x 25 mL, 3 x 1 mL and 7 x 50 ml.

### Biotransformation 1

To one 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrost-4-en-3-one (100 mg, 347 µmol) dissolved in DMF (5 mL) was added and the mixture was stirred at 300 rpm at 27 °C with an aeration rate of 0.5 L/min. The pH value was maintained at pH 7.4. Cells in buffer (50 mL of cell cultivation 1) were added, and the biotransformation was maintained at an oxygen partial pressure of 50 % by varying the stirring rate to up to 800 rpm. After 26.5 hours the biotransformation was extracted with 4-methyl-2-pentanone.

### Biotransformation 2

To one 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrost-4-en-3-one (100 mg, 347 µmol) dissolved in DMF (5 mL) was added and the mixture was stirred at 300 rpm at 27 °C with an aeration rate of 0.5 L/min. The pH value was maintained at pH 7.4. Cells in buffer (50 mL of cell cultivation 2) were added, and the biotransformation was maintained at an oxygen partial pressure of 50 % by varying the stirring rate to up to 800 rpm. After 26.5 hours the biotransformation was extracted with 4-methyl-2-pentanone.

### Biotransformation 3

To one 1 L Biostat Q fermenter which contained KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrost-4-en-3-one (100 mg, 347 µmol) dissolved in DMF (5 mL) was added and the mixture was stirred at 300 rpm at 27 °C with an aeration rate of 0.5 L/min. The pH value was maintained at pH 7.4. Cells in buffer (50 mL of cell cultivation 3) were added, and the biotransformation was maintained at an oxygen partial pressure of 50 % by varying the stirring rate to up to 1200 rpm. After 4 hours additional cells in buffer (25 mL of cell cultivation 3) were added. After 26 hours and 25 minutes the biotransformation was extracted with 4-methyl-2-pentanone.

### Biotransformation 4 and 5

To two 1 L Biostat Q fermenter each containing KH2PO4 (4 g), K2HPO4 (12.3 g), Pluronic® PE 8100 (0.1 mL), demineralized water (930 mL), aqueous glucose solution (50 %, 20 mL) and aqueous EDTA solution (0.5 M, 1 mL) (17beta)-17-hydroxyandrost-4-en-3-one (25 mg, 87 µmol) dissolved in DMF (2.5 mL) was added and the mixture was stirred at 300 rpm at 27 °C with an aeration rate of 0.5 L/min. The pH value was maintained at pH 7.4. Cells in buffer (50 mL of cell cultivation 3) were added, and the biotransformation was maintained at an oxygen partial pressure of 50 % by varying the stirring rate to up to 800 rpm. After 26 hours and 25 minutes the biotransformation was extracted with 4-methyl-2-pentanone.

The organic phases of the five biotransformations were combined and concentrated to give an oil (4.45 g) which was slurried with methanol and was filtered and concentrated to give an oil (1.23 g). The crude product was further purified by flash chromatography using silica gel (dichloromethane/ethanol gradient) and by preparative HPLC to give the title compound (13.0 mg).

### Analytical HPLC method:

Instrument: Waters Acquity UPLCMS SingleQuad; Column: Acquity UPLC BEH C18 1.7 µm, 50x2.1 mm; Eluent A: Water + 0.1 Vol-% formic acid (99 %), Eluent B: Acetonitrile; Gradient: 0-1.6 min 1-99 % B, 1.6-2.0 min 99 % B; Flow 0.8 mL/min; Temperature: 60 °C; DAD scan: 210-400 nm.

### Preparative HPLC method:

Instrument: Waters Autopurificationsystem; Column: Waters XBrigde C18 5 µ 150x50 mm; Eluent A: Water + 0.1 Vol-% formic acid (99 %), Eluent B: Acetonitril; Gradient: 0.00-0.50 min 9 % B (40-100 mL/min), 0.51-8.50 min 19-41 % B (100 mL/min), DAD scan: 210-400 nm.

### Example 18

### (15beta, 17beta)-15, 17, 19-trihydroxyandrost-4-en-3-one

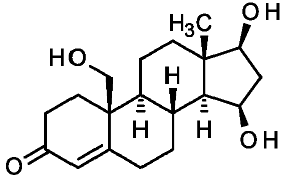

For the preparation of the title compound see second preparation of Example 17. The crude product (1.23 g) was further purified by flash chromatography using silica gel (dichloromethane/ethanol gradient) and by preparative HPLC to give the title compound (2.2 mg).

### Preparative chiral HPLC method:

Instrument: Waters Autopurificationsystem; Column: Waters XBrigde C18 5 µ 150x50 mm; Eluent A: Water + 0.1 Vol-% Formic acid (99 %), Eluent B: Acetonitrile; Gradient: 0.00-0.50 min 5 % B (40-100 mL/min), 0.51-8.50 min 5-30 % B (100 mL/min), DAD scan: 210-400 nm.

### Analytical chiral HPLC method:

Instrument: Waters Acquity UPLCMS SingleQuad; Column: Acquity UPLC BEH C18 1.7 µm, 50x2.1 mm; Eluent A: Water + 0.1 Vol-% Formic acid (99 %), Eluent B: Acetonitrile; Gradient: 0-1.6 min 1-99 % B, 1.6-2.0 min 99 % B; Flow: 0.8 mL/min; Temperature: 60 °C; DAD scan: 210-400 nm.
Analytical Chiral HPLC: Rₜ = 0.63 min.
¹H-NMR (600MHz, DMSO-d6) δ [ppm]: 0.61 (dd, 1H), 0.82 - 0.97 (m, 6H), 1.37 - 1.47 (m, 2H), 1.51 - 1.57 (m, 1H), 1.61 (td, 1H), 1.64 - 1.68 (m, 1H), 1.89 - 1.97 (m, 1H), 2.07 - 2.17 (m, 2H), 2.22 - 2.32 (m, 3H), 2.37 - 2.45 (m, 2H), 2.58 - 2.67 (m, 2H), 3.26 - 3.32 (m, 1H), 3.73 (dd, 1H), 3.85 (dd, 1H), 3.91 - 3.98 (m, 1H), 4.35 (d, 1H), 4.46 (d, 1H), 4.72 (t, 1H), 5.74 (s, 1H).
¹H-NMR (600 MHz, DMSO-d6) δ [ppm]: -0.006 (0.85), 0.005 (0.77), 0.593 (1.06), 0.603 (1.08), 0.612 (1.10), 0.621 (1.08), 0.832 (0.48), 0.847 (0.83), 0.853 (0.89), 0.869 (0.54), 0.875 (0.56), 0.889 (16.00), 0.900 (0.68), 0.907 (0.64), 0.924 (1.06), 0.943 (1.10), 0.957 (0.56), 0.964 (0.54), 1.383 (0.68), 1.387 (0.70), 1.398 (0.85), 1.402 (0.95), 1.406 (0.83), 1.410 (0.77), 1.421 (1.12), 1.425 (1.31), 1.440 (0.68), 1.446 (0.66), 1.529 (0.77), 1.535 (0.83), 1.552 (0.58), 1.557 (0.54), 1.588 (0.46), 1.602 (0.83), 1.611 (0.85), 1.625 (0.48), 1.634 (0.46), 1.651 (1.02), 1.656 (0.60), 1.666 (0.64), 1.671 (0.95), 1.918 (0.73), 1.924 (0.79), 1.937 (0.73), 1.942 (0.75), 2.076 (0.44), 2.087 (0.60), 2.092 (0.79), 2.099 (0.56), 2.113 (0.73), 2.120 (1.37), 2.126 (1.14), 2.134 (0.54), 2.141 (0.58), 2.145 (0.64), 2.230 (0.77), 2.234 (0.56), 2.248 (0.79), 2.255 (1.31), 2.270 (1.35), 2.275 (0.93), 2.280 (1.24), 2.283 (1.22), 2.294 (1.43), 2.306 (0.77), 2.382 (0.81), 2.385 (1.16), 2.388 (1.64), 2.391 (1.31), 2.405 (0.75), 2.411 (0.70), 2.519 (3.59), 2.522 (3.63), 2.525 (3.05), 2.600 (0.58), 2.610 (1.08), 2.613 (1.04), 2.616 (1.33), 2.619 (1.02), 2.624 (0.79), 2.628 (0.75), 2.633 (0.70), 2.637 (0.62), 2.652 (0.62), 2.661 (0.48), 3.265 (0.54), 3.273 (0.58), 3.279 (1.14), 3.287 (1.16), 3.293 (0.58), 3.302 (0.56), 3.713 (0.99), 3.723 (1.04), 3.732 (1.31), 3.741 (1.26), 3.837 (1.02), 3.846 (1.08), 3.855 (0.81), 3.863 (0.77), 3.943 (0.87), 3.949 (0.87), 4.348 (2.88), 4.355 (2.82), 4.454 (2.47), 4.462 (2.38), 4.711 (1.18), 4.719 (2.47), 4.728 (1.16), 5.740 (3.34), 6.552 (0.56), 8.318 (1.16).

### Example 19

### (2beta, 15beta, 17beta)-2, 15, 17-trihydroxyandrost-4-en-3-one

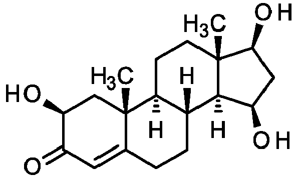

For the preparation of the title compound see second preparation of Example 17. The crude product (1.23 g) was further purified by flash chromatography using silica gel (dichloromethane/ethanol gradient) and by preparative HPLC to give the title compound (1 mg).

### Preparative chiral HPLC method:

Instrument: Waters Autopurificationsystem; Column: Waters XBrigde C18 5 µ 150x50 mm; Eluent A: Water + 0.1 Vol-% Formic acid (99 %), Eluent B: Acetonitrile; Gradient: 0.00-0.50 min 5 % B (40-100 mL/min), 0.51-8.50 min 5-30 % B (100 mL/min), DAD scan: 210-400 nm.

### Analytical chiral HPLC method:

Instrument: Waters Acquity UPLCMS SingleQuad; Column: Acquity UPLC BEH C18 1.7 µm, 50x2.1 mm; Eluent A: Water + 0.1 Vol-% Formic acid (99 %), Eluent B: Acetonitrile; Gradient: 0-1.6 min 1-99 % B, 1.6-2.0 min 99 % B; Flow: 0.8 mL/min; Temperature: 60 °C; DAD scan: 210-400 nm.
Analytical Chiral HPLC: Rₜ = 0.64 min.
¹H-NMR (400 MHz, CHLOROFORM-d) δ [ppm]: 0.774 (1.91), 0.788 (1.77), 0.802 (1.30), 0.816 (1.29), 0.997 (0.71), 1.010 (14.33), 1.027 (0.71), 1.031 (0.70), 1.040 (0.91), 1.051 (1.46), 1.061 (0.86), 1.072 (0.57), 1.082 (0.82), 1.092 (0.42), 1.129 (0.40), 1.136 (0.42), 1.148 (0.61), 1.160 (16.00), 1.170 (0.89), 1.174 (0.86), 1.184 (3.29), 1.191 (1.47), 1.210 (0.65), 1.349 (0.55), 1.358 (0.57), 1.381 (0.95), 1.389 (0.89), 1.407 (0.92), 1.415 (0.98), 1.434 (0.69), 1.439 (0.56), 1.443 (0.76), 1.456 (0.60), 1.464 (2.08), 1.474 (1.47), 1.498 (3.80), 1.507 (1.42), 1.517 (1.52), 1.523 (1.55), 1.533 (2.86), 1.538 (1.94), 1.554 (1.66), 1.559 (1.68), 1.640 (0.47), 1.708 (0.49), 1.716 (0.89), 1.724 (0.89), 1.733 (0.51), 1.740 (0.42), 1.748 (0.63), 1.757 (0.62), 1.779 (0.74), 1.788 (1.01), 1.796 (0.61), 1.811 (0.61), 1.819 (0.93), 1.828 (0.54), 1.943 (0.50), 1.979 (0.98), 2.026 (1.06), 2.036 (0.61), 2.057 (0.70), 2.063 (0.57), 2.106 (0.64), 2.191 (0.71), 2.198 (1.06), 2.207 (1.84), 2.215 (0.62), 2.227 (1.79), 2.234 (1.28), 2.240 (0.81), 2.400 (1.05), 2.414 (1.07), 2.434 (0.99), 2.448 (0.98), 2.512 (0.43), 2.516 (0.45), 2.521 (0.76), 2.532 (0.66), 2.535 (0.71), 2.540 (1.23), 2.543 (1.28), 2.558 (0.89), 2.562 (1.02), 2.568 (0.44), 2.577 (1.01), 2.580 (1.05), 2.598 (0.68), 3.504 (1.26), 3.526 (2.58), 3.547 (1.18), 4.093 (1.59), 4.101 (0.86), 4.107 (1.71), 4.114 (1.31), 4.119 (0.79), 4.127 (1.90), 4.133 (0.80), 4.142 (1.13), 5.234 (4.88), 5.755 (3.02), 5.757 (2.97).

### Example 20

### (2beta, 17beta)-2, 17, 19-trihydroxyandrost-4-en-3-one

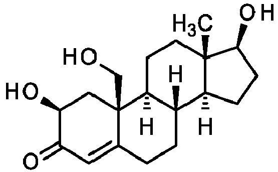

Media of the preculture: tryptone (10 g/L), sodium chloride (10 g/L) and yeast extract (5 g/L) in demineralized water. The media was sterilized at 121 °C for 20 minutes. Afterwards kanamycin (50 mg/L) was added.

### Cell cultivation 1

One preculture (100 mL) was inoculated with the strain *E. coli* BL 21 Gold (DE3) ΔdkgA :: FRT T7 gdh pETM11-BM3-254 (50 µL) containing the desired plasmids and was shaken at 37 °C and 165 rpm for 16 hours. This preculture (10 mL) was used to inoculate one 1 L Biostat Q fermenter. The cultivation media was prepared in the fermenter. Tryptone (20 g/L), sodium chloride (20 g/L), yeast extract (10 g/L) and Pluronic® PE 8100 (0.5 mL) were dissolved in demineralized water (1.0 L) and sterilized for 30 minutes at 121 °C in the fermenter. Afterwards kanamycin (50 mg) in demineralized water (5 mL) was added. After the inoculation of the fermenter, the inoculated culture was stirred at 500 rpm at 37 °C with an aeration rate of 0.4 L/min and an oxygen partial pressure of 30 % was maintained by stirring of up to 1500 rpm. After 3 hours an aqueous glucose solution (25 %, 8 g/h) was added. After 5 hours and 30 minutes an OD550 of 16.2 was reached, the temperature was decreased to 28 °C within 15 minutes and IPTG (0.24 g) in demineralized water (10 mL) was added to start the protein expression. After additional 10 hours and 5 minutes the cells were harvested by centrifugation (38.8 g), suspended in buffer (38.8 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 3 x 1 mL and 7 x 10 mL.

### Cell cultivation 2

One preculture (100 mL) was inoculated with the strain *E. coli* BL 21 Gold (DE3) ΔdkgA :: FRT T7 gdh pETM11-BM3-254 (50 µL) containing the desired plasmids and was shaken at 37 °C and 165 rpm for 16 hours. This preculture (10 mL) was used to inoculate one 1 L Biostat Q fermenter. The cultivation media was prepared in the fermenter. Tryptone (20 g/L), sodium chloride (20 g/L), yeast extract (10 g/L) and Pluronic® PE 8100 (0.5 mL) were dissolved in demineralized water (1.0 L) and sterilized for 30 minutes at 121 °C in the fermenter. Afterwards kanamycin (50 mg) in demineralized water (5 mL) was added. After the inoculation of the fermenter, the inoculated culture was stirred at 500 rpm at 37 °C with an aeration rate of 0.4 L/min and an oxygen partial pressure of 30 % was maintained by stirring of up to 1500 rpm. After 3 hours an aqueous glucose solution (25 %, 8 g/h) was added. After 5 hours an OD550 of 16.6 was reached, the temperature was decreased to 28 °C within 15 minutes and IPTG (0.24 g) in demineralized water (10 mL) was added to start the protein expression. After additional 9 hours and 15 minutes the cells were harvested by centrifugation (42 g), suspended in buffer (42 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 3 x 1 mL and 7 x 10 mL.

### Cell cultivation 3

One preculture (100 mL) was inoculated with the strain *E. coli* BL 21 Gold (DE3) ΔdkgA :: FRT T7 gdh pETM11-BM3-254 (50 µL) containing the desired plasmids and was shaken at 37 °C and 165 rpm for 16 hours. This preculture (10 mL) was used to inoculate one 1 L Biostat Q fermenter. The cultivation media was prepared in the fermenter. Tryptone (20 g/L), sodium chloride (20 g/L), yeast extract (10 g/L) and Pluronic® PE 8100 (0.5 mL) were dissolved in demineralized water (1.0 L) and sterilized for 30 minutes at 121 °C in the fermenter. Afterwards kanamycin (50 mg) in demineralized water (5 mL) was added. After the inoculation of the fermenter, the inoculated culture was stirred at 500 rpm at 37 °C with an aeration rate of 0.4 L/min and an oxygen partial pressure of 30 % was maintained by stirring of up to 1500 rpm. After 3 hours an aqueous glucose solution (25 %, 8 g/h) was added. After 5 hours and 5 minutes an OD550 of 16.72 was reached, the temperature was decreased to 28 °C within 15 minutes and IPTG (0.238 g) in demineralized water (10 mL) and 5-aminolevulinic acid hydrochloride (0.042 g) in demineralized water (5 mL) was added to start the protein expression. After additional 9 hours and 40 minutes the cells were harvested by centrifugation (43 g), suspended in buffer (43 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 3 x 1 mL and 5 x 15 mL.

### Biotransformation 1

To a 1 L Biostat Q fermenter which contained KH2PO4 (4 g), K2HPO4 (12.3 g), Pluronic® PE 8100 (0.1 mL), demineralized water (0.93 L), aqueous glucose solution (50 %, 20 mL) and aqueous EDTA solution (0.5 M, 1 mL) (17beta)-17-hydroxyandrosta-4-en-3-one (100 mg, 0.35 mmol) dissolved in DMF (5 mL) was added, and the mixture was stirred at 300 rpm at 27 °C with an aeration rate of 0.5 L/min. Cells in buffer (50 mL of preculture 2) were added, and the biotransformation was maintained at an oxygen partial pressure of 50 % by varying the stirring rate to up to 800 rpm. The pH value was maintained at pH 7.4 by addition of an aqueous solution of sodium hydroxide (16 %). After 4 hours, cells (20 mL) in buffer were added again. After 28 hours and 10 minutes the biotransformation was stopped.

### Biotransformation 2

To a 1 L Biostat Q fermenter which contained KH2PO4 (4 g), K2HPO4 (12.3 g), Pluronic® PE 8100 (0.1 mL), demineralized water (0.93 L), aqueous glucose solution (50 %, 20 mL) and aqueous EDTA solution (0.5 M, 1 mL) (17beta)-17-hydroxyandrosta-4-en-3-one (100 mg, 0.35 mmol) dissolved in DMF (5 mL) was added and the mixture was stirred at 300 rpm at 27 °C with an aeration rate of 0.5 L/min. Cells in buffer (50 mL of cell cultivation 3) were added, and the biotransformation was maintained at an oxygen partial pressure of 50 % by varying the stirring rate to up to 800 rpm. The pH value was maintained at pH 7.4 by addition of an aqueous solution of sodium hydroxide (16 %). After 27 hours and 45 minutes the biotransformation was stopped.

### Biotransformation 3

To a 1 L Biostat Q fermenter which contained KH2PO4 (4 g), K2HPO4 (12.3 g), Pluronic® PE 8100 (0.1 mL), demineralized water (0.93 L), aqueous glucose solution (50 %, 20 mL) and aqueous EDTA solution (0.5 M, 1 mL). (17beta)-17-hydroxyandrosta-4-en-3-one (4 x 25 mg, 0.35 mmol) dissolved in DMF (4 x 2.5 mL) was added in intervals after 0 h, 2 h, 4 h and 6 h and the mixture was stirred at 300 rpm at 27 °C with an aeration rate of 0.5 L/min. Cells in buffer (50 mL of cell cultivation 1) were added at 0 h, and the biotransformation was maintained at an oxygen partial pressure of 50 % by varying the stirring rate between 800 and 1200 rpm. The pH value was maintained at pH 7.4 by addition of an aqueous solution of sodium hydroxide (16 %). After 28 hours the biotransformation.

The three biotransformations were combined and extracted with 4-methyl-2-pentanone. The organic layer was concentrated to give an oil (2.2 g) which was slurried with methanol and was filtered and concentrated to give an oil (1.06 g). The crude product was further purified by flash chromatography using silica gel (dichloromethane/methanol gradient) and by preparative HPLC to give the title compound (7.8 mg, 2 % yield).

### Preparative chiral HPLC method:

Instrument: Waters Autopurificationsystem; Column: Waters XBrigde C18 5 µ 100x30 mm; Eluent A: water + 0.1 vol-% aqueous ammonia (99 %), Eluent B: acetonitrile; gradient: 0.00-0.50 min 10 % B (40-70 mL/min), 0.51-5.50 min 10-40 % B (70 mL/min), DAD scan: 210-400 nm.

### Analytical chiral HPLC method:

Instrument: Waters Acquity UPLCMS SingleQuad; Column: Acquity UPLC BEH C18 1.7 µm, 50x2.1 mm; eluent A: water + 0.1 vol % aqueous ammonia (99 %), eluent B: acetonitrile; gradient: 0-1.6 min 1-99 % B, 1.6-2.0 min 99 % B; flow 0.8 mL/min; temperature: 60 °C; DAD scan: 210-400 nm.
Analytical Chiral HPLC: Rt = 0.73 min.
¹H-NMR (600 MHz, CDCl3) δ [ppm]: 0.79 (s, 3H), 0.95 - 1.11 (m, 3H), 1.24 - 1.36 (m, 3H), 1.38 - 1.50 (m, 3H), 1.57 - 1.63 (m, 1H), 1.69 - 1.77 (m, 1H), 1.81 (dq, 1H), 1.87 (dt, 1H), 1.95 - 2.02 (m, 1H), 2.03 - 2.14 (m, 1H), 2.20 - 2.27 (m, 2H), 2.30 (s, 1H), 2.35 - 2.41 (m, 1H), 2.44 - 2.54 (m, 1H), 3.60 - 3.73 (m, 3H), 4.09 - 4.17 (m, 2H).

### Example 21

### 3-hydroxyestra-1(10),2,4-trien-17-one

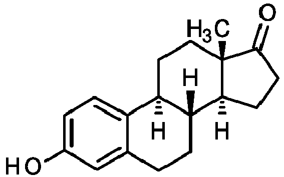

### Expression system

### E. coli BL21 Star™ (DE3) pETM11-BM3-268; S72G, A74V, F77A

### Cultivation

Media of the preculture: tryptone (16 g/L), sodium chloride (10 g/L) and yeast extract (10 g/L) in demineralized water. The pH value was maintained at pH 7.3. The medium was sterilized at 121 °C for 20 minutes. Afterwards kanamycin (50 mg/L) was added.

One preculture (100 mL) was inoculated with the strain *E. coli* BL21 Star™ (DE3) pETM11-BM3-268; S72G, A74V, F77A (50 µL) containing the desired plasmids and was shaken at 37 °C and 165 rpm for 17 hours. This preculture (100 mL) was used to inoculate one 10 L steel fermenter. The cultivation media were prepared in the fermenter. Tryptone (12 g/L), yeast extract (24 g/L), predigested beef extract (2 g/L), KH2PO4 (2.2 g/L), K2HPO4 (9.4 g/L) and glycerol (87%, 4.6 g/L) were dissolved in demineralized water (9.2 L) and sterilized for 20 minutes at 121°C in the fermenter. Afterwards kanamycin (0.5 g) in water (20 mL), riboflavin (10 mg) in water (20 mL), thiamine hydrochloride (3.37 g) in water (10 mL) and Oxford trace metal solution (2.5 mL) were added. After the inoculation of the fermenter, the culture was stirred at 315 rpm at 37 °C with an aeration rate of 3.3 L/min at pH 6.6 which was regulated by addition of aqueous sodium hydroxide solution (16%) or aqueous phosphoric acid solution (16%). After 2.55 hours, an OD550 of 0.94 was reached, the temperature was decreased to 27 °C within 10 minutes and IPTG (1.43 g) in water (40 mL) were added to start the protein expression. After 9.15 hours the aqueous phosphoric acid solution (16%) was substituted for an aqueous glucose solution (50%) for pH regulation. After additional 23.5 hours an OD550 of 23.92 was reached, the cells were harvested by centrifugation (215.6 g), suspended in buffer (215.6 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 1 x 150 mL, 1 x 130 mL, 2 x 40 mL und 5 x 1 mL.

### Biotransformation

To a 10 L steel fermenter which contained KH2PO4 (40 g), K2HPO4 (123 g), Pluronic® PE 8100 (1 mL), demineralized water (9.0 L), aqueous glucose solution (50 %, 200 mL) and aqueous EDTA solution (0.5 M, 10 mL) androsta-1,4-diene-3,17-dione (500 mg, 1.75 mmol) dissolved in DMF (10 mL) was added and the mixture was stirred at 315 rpm at 27 °C with an aeration rate of 3.3 L/min. Cells in buffer (400 mL) were added, and the biotransformation was maintained at an oxygen partial pressure of 50 % by varying the stirring rate to up to 1200 rpm. The pH value was maintained at pH 7.4 by addition of an aqueous solution of sodium hydroxide (16 %). After 26 hours the biotransformation was extracted with 4-methyl-2-pentanone. The organic layer was concentrated to give an oil (2.37 g) which was slurried with methanol and was filtered and concentrated to give an oil (2.05 g). The crude product was further purified by flash chromatography using silica gel (dichloromethane/methanol gradient) and twice by preparative HPLC to give the title compound (14 mg).

### 1. Preparative HPLC method

Instrument: Waters Autopurificationsystem; Column: Waters XBrigde C18 5µ 50x50 mm; Eluent A: Water + 0.2 Vol-% Formic acid (99 %), Eluent B: Acetonitrile; Gradient: 0.00-0.50 min 23 % B (50-100 mL/min), 0.51-13.50 min 23 - 43 % B (100 mL/min), DAD scan: 210-400 nm.

### 1. Analytical HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-2.6 min 1-99 % B, 2.6-3.0 min 99 % B; Flow 0.8 mL/min; Temperature: 60 °C; DAD scan: 210-400 nm.
Analytical HPLC: Rt = 1.10 min.

### 2. Preparative HPLC method

Instrument: Sepiatec: Prep SFC100; Column: Chiralpak IG 5µ 250x30 mm; eluent A: CO2; eluent B: methanol; isocratic: 20 % B; flow: 100 mL/min; temperature: 40 °C; BPR: 150 bar; UV: 280 nm.

### 2. Analytical HPLC method

Instrument: Agilent: 1260, Aurora SFC-Modul; Column: Chiralpak IG 5µ 100x4.6 mm; eluent A: CO2; eluent B: methanol; isocratic: 20 % B; flow: 4 mL/min; temperature: 37.5 °C; BPR: 100 bar; UV: 280 nm.
Analytical HPLC: Rₜ = 3.66 min.
¹H-NMR (400 MHz, DMSO-d6) δ [ppm]: 0.820 (16.00), 1.293 (0.48), 1.322 (0.95), 1.329 (0.75), 1.348 (2.38), 1.371 (1.82), 1.401 (0.24), 1.450 (0.87), 1.461 (0.83), 1.476 (1.50), 1.492 (0.75), 1.523 (0.55), 1.529 (0.44), 1.545 (0.36), 1.552 (0.59), 1.575 (0.36), 1.583 (0.32), 1.726 (0.99), 1.733 (0.91), 1.748 (0.79), 1.754 (0.67), 1.882 (0.51), 1.890 (0.55), 1.896 (0.59), 1.915 (0.75), 1.921 (0.83), 1.935 (0.63), 1.943 (0.67), 1.964 (0.48), 1.976 (0.44), 2.011 (0.59), 2.033 (0.91), 2.058 (0.83), 2.071 (0.20), 2.080 (1.19), 2.102 (0.51), 2.136 (0.55), 2.284 (0.40), 2.301 (0.71), 2.314 (0.71), 2.326 (0.51), 2.331 (0.24), 2.394 (0.95), 2.414 (1.07), 2.440 (0.75), 2.461 (0.95), 2.518 (0.71), 2.522 (0.48), 2.539 (0.44), 2.664 (0.20), 2.669 (0.24), 2.673 (0.20), 2.710 (0.20), 2.735 (1.35), 2.747 (1.43), 2.775 (0.51), 3.298 (0.20), 6.444 (2.06), 6.450 (2.42), 6.496 (1.35), 6.502 (1.07), 6.517 (1.39), 6.523 (1.15), 7.036 (1.82), 7.056 (1.66), 9.033 (4.63).
¹H-NMR (400 MHz, DMSO-d6) δ [ppm]: 0.82 (s, 3H), 1.22 - 1.41 (m, 3H), 1.42 - 1.61 (m, 3H), 1.69 - 1.79 (m, 1H), 1.86 - 1.99 (m, 2H), 2.00 - 2.19 (m, 2H), 2.25 - 2.35 (m, 1H), 2.43 (dd, 1H), 2.68 - 2.83 (m, 2H), 6.45 (d, 1H), 6.51 (dd, 1H), 7.05 (d, 1H), 9.03 (s, 1H).

For another preparation of the title compound see Example 25. The crude product was further purified by flash chromatography using silica gel (dichloromethane/methanol gradient) and by preparative HPLC to give the title compound (91 mg).

### Example 22

### 15alpha-hydroxyandrosta-1,4-diene-3,17-dione

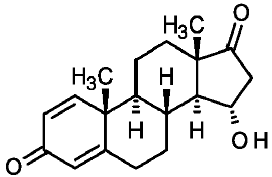

For the preparation of the title compound see Example 21. The crude product was further purified by flash chromatography using silica gel (dichloromethane/methanol gradient) and twice by preparative HPLC to give the title compound (14 mg).

### 1. Preparative HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 17 % B (50-100 mL/min), 0.51-8.50 min 17 - 37 % B (100 mL/min), DAD scan: 210-400 nm.

### 1. Analytical HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-2.6 min 1-99 % B, 2.6-3.0 min 99 % B; Flow 0.8 mL/min; Temperature: 60 °C; DAD scan: 210-400 nm.
Analytical HPLC: Rt = 0.82 min.

### 2. Preparative HPLC method

Instrument: PrepCon Labomatic HPLC; Column: YMC Cellulose SC 5µ, 250x30 mm; eluent A: hexane + 0.1 vol % diethylamine; eluent B: 2-propanol; isocratic: 70 % A + 30 % B; flow: 50 mL/min; temperature: 25 °C; UV: 254 nm.

### 2. Analytical HPLC method

Instrument: Waters Alliance 2695; Column: YMC Cellulose SC 3µ, 100x4.6 mm; eluent A: hexane + 0.1 vol % diethylamine; eluent B: 2-propanol; isocratic: 70 % A + 30 % B; flow: 1.4 mL/min; temperature: 25 °C; UV: 254 nm.
Analytical HPLC: Rt = 9.28 min.
¹H-NMR (600 MHz, CDCl3) δ [ppm]: 0.000 (2.45), 0.736 (0.49), 0.839 (0.64), 0.848 (0.52), 0.856 (0.53), 0.862 (0.46), 0.869 (0.88), 0.881 (1.19), 0.892 (0.62), 1.071 (0.16), 1.139 (0.57), 1.146 (0.65), 1.157 (0.67), 1.159 (0.78), 1.163 (0.80), 1.166 (0.69), 1.177 (0.65), 1.184 (0.62), 1.189 (0.46), 1.197 (0.55), 1.210 (0.78), 1.217 (0.88), 1.233 (1.06), 1.239 (1.17), 1.254 (5.41), 1.262 (3.03), 1.270 (14.17), 1.281 (2.20), 1.288 (1.62), 1.297 (1.04), 1.306 (16.00), 1.333 (0.30), 1.348 (0.42), 1.356 (0.21), 1.376 (0.22), 1.399 (0.16), 1.412 (0.17), 1.601 (0.17), 1.642 (0.51), 1.700 (0.19), 1.724 (0.23), 1.731 (0.33), 1.747 (0.53), 1.753 (0.50), 1.768 (0.70), 1.774 (0.62), 1.789 (0.32), 1.796 (0.33), 1.840 (0.70), 1.844 (1.02), 1.851 (1.63), 1.858 (0.95), 1.861 (0.97), 1.866 (1.73), 1.868 (1.14), 1.873 (1.06), 1.877 (0.57), 1.884 (0.28), 2.163 (0.24), 2.169 (0.28), 2.181 (0.55), 2.187 (0.64), 2.201 (0.54), 2.206 (0.60), 2.219 (0.21), 2.225 (0.26), 2.267 (0.30), 2.271 (0.41), 2.276 (0.50), 2.280 (0.46), 2.288 (0.41), 2.292 (0.42), 2.297 (0.46), 2.301 (0.44), 2.307 (0.28), 2.434 (0.41), 2.438 (0.48), 2.441 (0.51), 2.445 (0.42), 2.456 (0.59), 2.461 (0.67), 2.463 (0.68), 2.468 (0.52), 2.503 (0.69), 2.513 (0.64), 2.535 (1.79), 2.545 (1.79), 2.567 (0.41), 2.569 (0.53), 2.575 (2.58), 2.590 (0.64), 2.592 (0.66), 2.598 (0.63), 2.600 (0.64), 2.607 (0.88), 2.612 (0.34), 2.614 (0.31), 2.621 (0.27), 2.623 (0.37), 4.576 (0.66), 4.585 (1.06), 4.593 (0.65), 6.107 (1.41), 6.109 (2.45), 6.112 (1.50), 6.245 (1.70), 6.248 (1.64), 6.262 (1.64), 6.266 (1.59), 7.060 (2.35), 7.078 (2.17), 7.267 (8.94).

### Example 23

### 6beta-hydroxyandrosta-1,4-diene-3,17-dione

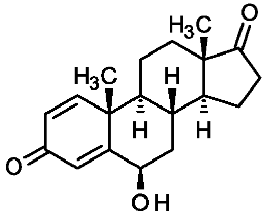

For the preparation of the title compound see Example 21. The crude product was further purified by flash chromatography using silica gel (dichloromethane/methanol gradient) and twice by preparative HPLC to give the title compound (2 mg).

### 1. Preparative HPLC method

Instrument: Waters Autopurificationsystem; Column: Waters XBrigde C18 5µ 50x50 mm; Eluent A: Water + 0.1 Vol-% Formic acid (99 %), Eluent B: Acetonitrile; Gradient: 0.00-0.50 min 17 % B (50-100 mL/min), 0.51-8.50 min 17 - 37 % B (100 mL/min), DAD scan: 210-400 nm.

### 1. Analytical HPLC method

Instrument: Waters Acquity UPLCMS SingleQuad; Column: Acquity UPLC BEH C18 1.7 µm, 50x2.1mm; Eluent A: Water + 0.1 Vol-% Formic acid (99 %), Eluent B: Acetonitrile; Gradient: 0-2.6 min 1-99 % B, 2.6-3.0 min 99 % B; Flow 0.8 mL/min; Temperature: 60 °C; DAD scan: 210-400 nm.
Analytical HPLC: Rₜ = 0.82 min.

### 2. Preparative HPLC method

Instrument: PrepCon Labomatic HPLC; Column: YMC Cellulose SC 5µ, 250x30 mm; eluent A: hexane + 0.1 vol % diethylamine; eluent B: 2-propanol; isocratic: 70 % A + 30 % B; flow: 50 mL/min; temperature: 25 °C; UV: 254 nm.

### 2. Analytical HPLC method

Instrument: Waters Alliance 2695; Column: YMC Cellulose SC 3µ, 100x4.6 mm; eluent A: hexane + 0.1 vol % diethylamine; eluent B: 2-propanol; isocratic: 70 % A + 30 % B; flow: 1.4 mL/min; temperature: 25 °C; UV: 254 nm.
Analytical HPLC: Rt = 6.58 min.
¹H-NMR (400 MHz, DMSO-d6) δ [ppm]: 0.697 (1.62), 0.717 (0.16), 0.833 (0.65), 0.851 (1.06), 0.879 (16.00), 0.986 (0.57), 0.996 (0.57), 1.014 (0.89), 1.023 (0.97), 1.042 (0.73), 1.052 (0.65), 1.083 (0.32), 1.147 (0.65), 1.164 (1.06), 1.172 (0.89), 1.184 (1.38), 1.195 (1.87), 1.232 (5.28), 1.256 (1.38), 1.272 (1.06), 1.289 (1.38), 1.293 (1.46), 1.300 (1.22), 1.317 (0.97), 1.330 (0.97), 1.347 (0.65), 1.351 (0.65), 1.373 (16.00), 1.472 (0.41), 1.495 (0.81), 1.502 (0.65), 1.517 (0.57), 1.526 (1.14), 1.534 (0.57), 1.548 (0.73), 1.557 (0.65), 1.579 (0.32), 1.598 (0.32), 1.609 (0.41), 1.631 (0.65), 1.641 (0.81), 1.668 (2.60), 1.692 (0.89), 1.698 (1.22), 1.708 (0.65), 1.784 (0.73), 1.795 (0.81), 1.805 (0.57), 1.822 (0.65), 1.833 (0.81), 1.847 (0.65), 1.853 (0.73), 1.867 (0.73), 1.883 (0.57), 1.898 (0.49), 1.959 (0.81), 1.967 (0.81), 1.975 (1.22), 1.981 (1.71), 2.006 (1.46), 2.017 (0.73), 2.028 (1.38), 2.051 (0.49), 2.080 (0.32), 2.089 (0.32), 2.109 (0.73), 2.116 (0.65), 2.138 (0.73), 2.165 (0.32), 2.173 (0.24), 2.331 (0.97), 2.336 (0.49), 2.380 (1.14), 2.401 (1.14), 2.428 (1.30), 2.450 (1.79), 2.518 (3.65), 2.522 (2.44), 2.539 (0.49), 2.673 (0.89), 2.678 (0.41), 4.398 (1.71), 4.405 (1.71), 5.331 (2.27), 5.336 (2.19), 6.054 (3.49), 6.058 (4.30), 6.084 (2.60), 6.089 (1.79), 6.109 (2.36), 6.114 (1.95), 7.168 (3.17), 7.193 (3.09).

### Example 24

### 16beta-hydroxyandrosta-1,4-diene-3,17-dione

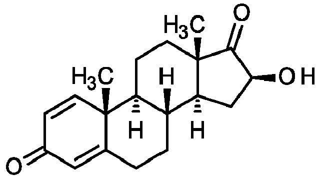

### Expression system

### E. coli BL21 Star™ (DE3) pETM11-BM3-268; F77A, M177Y

### Cultivation

Media of the preculture: tryptone (10 g/L), sodium chloride (10 g/L) and yeast extract (5 g/L) in demineralized water. The media was sterilized at 121 °C for 20 minutes. Afterwards kanamycin (50 mg/L) was added.

One preculture (100 mL) was inoculated with the strain *E. coli* BL21 Star™ (DE3) pETM11-BM3-268; F77A, M177Y (50 µL) containing the desired plasmids and was shaken at 37 °C and 165 rpm for 17 hours. This preculture (100 mL) was used to inoculate one 10 L steel fermenter. The cultivation media were prepared in the fermenter. Tryptone (12 g/L), yeast extract (24 g/L), predigested beef extract (2 g/L), KH2PO4 (2.2 g/L), K2HPO4 (9.4 g/L) and glycerol (87%, 4.6 g/L) were dissolved in demineralized water (9.2 L) and sterilized for 20 minutes at 121°C in the fermenter. Afterwards kanamycin (0.5 g) in water (20 mL), riboflavin (10 mg) in water (20 mL), thiamine hydrochloride (3.37 g) in water (10 mL) and Oxford trace metal solution (2.5 mL) were added. After the inoculation of the fermenter, the culture was stirred at 315 rpm at 37 °C with an aeration rate of 3.3 L/min at pH 6.6 which was regulated by addition of aqueous sodium hydroxide solution (16%) or aqueous phosphoric acid solution (16%). After 3.15 hours, an OD550 of 1.0 was reached, the temperature was decreased to 27 °C within 10 minutes and IPTG (1.43 g) in water (40 mL) were added to start the protein expression. After 8 hours the aqueous phosphoric acid solution (16%) was substituted for an aqueous glucose solution (50%) for pH regulation. After additional 24 hours an OD550 of 25.2 was reached, the cells were harvested by centrifugation (284.2 g), suspended in buffer (284.2 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 2 x 150 mL, 1 x 140 mL, 2 x 40 mL und 5 x 1 mL.

### Biotransformation

To a 10 L steel fermenter which contained KH2PO4 (40 g), K2HPO4 (123 g), Pluronic® PE 8100 (1 mL), demineralized water (9.0 L), aqueous glucose solution (50 %, 200 mL) and aqueous EDTA solution (0.5 M, 10 mL) androsta-1,4-diene-3,17-dione (500 mg, 1.75 mmol) dissolved in DMF (10 mL) was added and the mixture was stirred at 315 rpm at 27 °C with an aeration rate of 3.3 L/min. Cells in buffer (400 mL) were added, and the biotransformation was maintained at an oxygen partial pressure of 50 % by varying the stirring rate to up to 1200 rpm. The pH value was maintained at pH7.4 by addition of an aqueous solution of sodium hydroxide (16 %). After 26 hours the biotransformation was extracted with 4-methyl-2-pentanone. The organic layer was concentrated to give an oil (1.71 g) which was slurried with methanol and was filtered and concentrated to give an oil (1.55 g). The crude product was further purified by flash chromatography using silica gel (dichloromethane/methanol gradient) and by preparative HPLC to give the title compound (11 mg).

### Preparative HPLC method

Instrument: Sepiatec: Prep SFC100; Column: Reprosil Chiral NR 8µ 250x30 mm; eluent A: CO2; eluent B: 2-propanol; isocratic: 25 % B; flow: 100 mL/min; temperature: 40 °C; BPR: 150 bar; UV: 254 nm.

### Analytical HPLC method

Instrument: Agilent: 1260, Aurora SFC-Modul; Column: Reprosil Chiral NR 5µ 100x4.6 mm; eluent A: CO2; eluent B: 2-propanol; isocratic: 25 % B; flow: 4 mL/min; temperature: 37.5 °C; BPR: 100 bar; UV: 254 nm.
Analytical HPLC: Rt = 3.41 min.
¹H-NMR (600 MHz, CDCl3) δ [ppm]: 0.000 (2.08), 1.032 (13.48), 1.117 (0.25), 1.124 (0.30), 1.129 (0.47), 1.137 (0.99), 1.147 (0.90), 1.150 (0.65), 1.154 (0.70), 1.157 (0.93), 1.168 (0.97), 1.175 (0.53), 1.180 (0.33), 1.188 (0.30), 1.222 (0.50), 1.230 (0.53), 1.237 (0.38), 1.239 (0.59), 1.244 (0.71), 1.248 (0.70), 1.252 (0.83), 1.256 (0.66), 1.262 (0.78), 1.272 (16.00), 1.322 (0.38), 1.330 (0.43), 1.337 (0.26), 1.344 (0.81), 1.351 (0.86), 1.366 (0.46), 1.373 (0.45), 1.558 (0.71), 1.573 (0.73), 1.578 (0.78), 1.580 (0.67), 1.593 (0.82), 1.596 (0.70), 1.600 (0.71), 1.616 (0.71), 1.723 (0.35), 1.730 (0.38), 1.745 (0.66), 1.752 (0.68), 1.767 (0.71), 1.774 (0.75), 1.788 (0.35), 1.796 (0.36), 1.850 (0.36), 1.854 (0.71), 1.857 (0.73), 1.861 (0.91), 1.869 (0.48), 1.872 (0.82), 1.879 (0.99), 1.884 (0.55), 1.892 (0.77), 1.897 (0.59), 1.909 (0.25), 1.916 (0.27), 1.928 (0.59), 1.933 (0.66), 1.935 (0.65), 1.940 (0.51), 1.950 (0.57), 1.954 (0.62), 1.957 (0.60), 1.961 (0.46), 2.073 (0.30), 2.077 (0.40), 2.081 (0.47), 2.083 (0.44), 2.085 (0.44), 2.092 (0.39), 2.099 (0.40), 2.102 (0.44), 2.104 (0.43), 2.113 (0.27), 2.380 (0.61), 2.388 (0.62), 2.393 (0.63), 2.400 (0.80), 2.408 (0.60), 2.413 (0.59), 2.422 (0.84), 2.426 (0.51), 2.430 (0.43), 2.441 (0.67), 2.446 (0.73), 2.448 (0.72), 2.453 (0.59), 2.504 (0.41), 2.507 (0.45), 2.512 (0.45), 2.515 (0.44), 2.526 (0.65), 2.529 (0.66), 2.535 (0.65), 2.538 (0.61), 2.549 (0.29), 2.552 (0.31), 2.557 (0.27), 2.560 (0.25), 2.623 (5.61), 3.948 (1.17), 3.962 (1.59), 3.977 (1.10), 6.101 (1.45), 6.103 (2.45), 6.107 (1.46), 6.241 (1.54), 6.244 (1.48), 6.258 (1.70), 6.262 (1.62), 7.031 (2.46), 7.048 (2.39), 7.266 (8.11).
¹H-NMR (600 MHz, CDCl3) δ [ppm]: 1.03 (s, 3H), 1.11 - 1.20 (m, 2H), 1.21 - 1.28 (m, 1H), 1.27 (s, 3H), 1.31 - 1.38 (m, 1H), 1.59 (ddd, 1H), 1.76 (qd, 1H), 1.84 - 1.97 (m, 3H), 2.06 - 2.12 (m, 1H), 2.37 - 2.46 (m, 2H), 2.50 - 2.57 (m, 1H), 2.78 (s, 1H), 3.96 (t, 1H), 6.10 (t, 1H), 6.25 (dd, 1H), 7.04 (d, 1H).

### Example 25

### 3,16beta-dihydroxyestra-1(10),2,4-trien-17-one

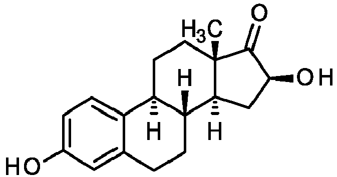

### Cultivation

Media of the preculture: tryptone (16 g/L), sodium chloride (10 g/L) and yeast extract (10 g/L) in demineralized water. The pH value was maintained at pH 7.3. The medium was sterilized at 121 °C for 20 minutes. Afterwards kanamycin (50 mg/L) was added.

Cell cultivation 1: One preculture (100 mL) was inoculated with the strain *E. coli* BL21 Star™ (DE3) pETM11-BM3-268; M177Y, A184Y, S72G, V178P, L181Y, L188F (50 µL) containing the desired plasmids and was shaken at 37 °C and 165 rpm for 17 hours. This preculture (100 mL) was used to inoculate one 10 L steel fermenter. The cultivation media was prepared in the fermenter. Tryptone (12 g/L), yeast extract (24 g/L), predigested beef extract (2 g/L), KH2PO4 (2.2 g/L), K2HPO4 (9.4 g/L) and glycerol (87%, 4.6 g/L) were dissolved in demineralized water (9.2 L) and sterilized for 20 minutes at 121 °C in the fermenter. Afterwards kanamycin (0.5 g) in water (20 mL), riboflavin (10 mg) in water (20 mL), thiamine hydrochloride (3.37 g) in water (10 mL) and Oxford trace metal solution (2.5 mL) were added. After the inoculation of the fermenter, the culture was stirred at 315 rpm at 37 °C with an aeration rate of 3.3 L/min at pH 6.6 which was regulated by addition of aqueous sodium hydroxide solution (16%) or aqueous phosphoric acid solution (16%). After 4 hours, an OD550 of 0.7 was reached, the temperature was decreased to 27 °C within 10 minutes and IPTG (1.43 g) in water (40 mL) were added to start the protein expression. After 9.50 hours the aqueous phosphoric acid solution (16%) was substituted for an aqueous glucose solution (50%) for pH regulation. After additional 24 hours an OD550 of 19.6 was reached, the cells were harvested by centrifugation (194.4 g), suspended in buffer (194.4 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 1 x 150 mL, 1 x 100 mL, 2 x 40 mL und 5 x 1 mL.

Cell cultivation 2: One preculture (100 mL) was inoculated with the strain *E. coli* BL21 Star™ (DE3) pETM11-BM3-268; M177Y, A184Y, S72G, V178P, L181Y, L188F (50 µL) containing the desired plasmids and was shaken at 37 °C and 165 rpm for 17 hours. This preculture (100 mL) was used to inoculate one 10 L steel fermenter. The cultivation media was prepared in the fermenter. Tryptone (12 g/L), yeast extract (24 g/L), predigested beef extract (2 g/L), KH2PO4 (2.2 g/L), K2HPO4 (9.4 g/L) and glycerol (87%, 4.6 g/L) were dissolved in demineralized water (9.2 L) and sterilized for 20 minutes at 121 °C in the fermenter. Afterwards kanamycin (0.5 g) in water (20 mL), riboflavin (10 mg) in water (20 mL), thiamine hydrochloride (3.37 g) in water (10 mL) and Oxford trace metal solution (2.5 mL) were added. After the inoculation of the fermenter, the culture was stirred at 315 rpm at 37 °C with an aeration rate of 3.3 L/min at pH 6.6 which was regulated by addition of aqueous sodium hydroxide solution (16%) or aqueous phosphoric acid solution (16%). After 3.55 hours, an OD550 of 0.69 was reached, the temperature was decreased to 27 °C within 10 minutes and IPTG (1.43 g) in water (40 mL) were added to start the protein expression. After 9 hours the aqueous phosphoric acid solution (16%) was substituted for an aqueous glucose solution (50%) for pH regulation. After additional 24 hours an OD550 of 27.75 was reached, the cells were harvested by centrifugation (231 g), suspended in buffer (231 mL; KH2PO4 (4 g/L), K2HPO4 (12.3 g/L), glycerol (4% (v/v)), glucose (5% (w/v)), EDTA (0.5 mM)), frozen in liquid nitrogen and stored at -80 °C until further use in aliquots of 2 x 150 mL, 1 x 50 mL, 2 x 40 mL und 5 x 1 mL.

### Biotransformation

To a 10 L steel fermenter which contained KH2PO4 (40 g), K2HPO4 (123 g), Pluronic® PE 8100 (1 mL), demineralized water (9.0 L), aqueous glucose solution (50 %, 200 mL) and aqueous EDTA solution (0.5 M, 10 mL) androsta-1,4-diene-3,17-dione (500 mg, 1.75 mmol) dissolved in DMF (10 mL) was added and the mixture was stirred at 315 rpm at 27 °C with an aeration rate of 3.3 L/min. Cells in buffer (330 mL of cell cultivation 1 and 150 ml of cell cultivation 2) were added, and the biotransformation was maintained at an oxygen partial pressure of 50 % by varying the stirring rate to up to 1200 rpm. The pH value was maintained at pH 7.4 by addition of an aqueous solution of sodium hydroxide (16 %). After 4 hours the biotransformation was extracted with 4-methyl-2-pentanone. The organic layer was concentrated to give an oil (5.65 g) which was dissolved in methanol, and was concentrated to give an oil (3.73 g).

The crude product was further purified by flash chromatography using silica gel (dichloromethane/ethanol gradient) and by preparative HPLC to give the title compound (54 mg).

### Preparative HPLC method

Instrument: Waters Autopurificationsystem; Column: XBrigde C18 5 µ, 100x30 mm; eluent A: water + 0.1 vol % formic acid; eluent B: acetonitrile; gradient: 0.0-0.5 min 23 % B (35-70 mL/min), 0.5-5.5 min 23-58 % B; flow: 70 mL/min; temperature: 25 °C; DAD scan: 210-400 nm.

### Analytical HPLC method

Instrument: Waters Acquity UPLCMS SingleQuad; Column: Acquity UPLC BEH C18 1.7 µ, 50x2.1 mm; eluent A: water + 0.1 vol % formic acid; eluent B: acetonitrile; gradient: 0-2.6 min 1-99 % B, 2.6-3.0 min 99 % B; flow: 0.8 mL/min; temperature: 60 °C; DAD scan: 210-400 nm.
Analytical HPLC: Rt = 0.84 min.
¹H-NMR (600 MHz, Pyridin-d5) δ [ppm]: 1.04 (s, 3H), 1.28 - 1.41 (m, 2H), 1.45 - 1.62 (m, 3H), 1.75 (ddd, 1H), 1.86 - 1.93 (m, 1H), 1.98 - 2.08 (m, 1H), 2.21 - 2.27 (m, 1H), 2.28 - 2.36 (m, 1H), 2.45 - 2.53 (m, 1H), 2.80 - 2.92 (m, 2H), 4.30 (t, 1H), 7.03 (d, 1H), 7.11 (dd, 1H), 7.28 (d, 1H), 7.91 (s, 1H), 11.24 (s, 1H).
¹H-NMR (600 MHz, Pyridin-d5) δ [ppm]: -0.002 (1.01), 0.000 (2.61), 0.965 (0.35), 1.038 (16.00), 1.292 (0.33), 1.303 (0.42), 1.313 (0.94), 1.323 (1.00), 1.333 (1.12), 1.344 (1.18), 1.353 (0.90), 1.355 (0.90), 1.365 (1.11), 1.370 (1.07), 1.374 (1.06), 1.378 (0.87), 1.388 (0.84), 1.396 (0.76), 1.461 (0.25), 1.467 (0.28), 1.483 (0.83), 1.489 (0.69), 1.504 (1.07), 1.508 (1.19), 1.524 (2.72), 1.539 (1.21), 1.545 (1.23), 1.553 (0.73), 1.557 (0.76), 1.561 (0.70), 1.571 (1.25), 1.574 (1.22), 1.590 (1.08), 1.593 (1.04), 1.608 (0.40), 1.612 (0.36), 1.722 (0.73), 1.737 (0.96), 1.741 (1.08), 1.744 (0.91), 1.757 (1.08), 1.759 (0.97), 1.764 (0.84), 1.779 (0.67), 1.875 (0.85), 1.881 (0.87), 1.886 (0.98), 1.890 (0.83), 1.896 (0.93), 1.901 (0.84), 1.907 (0.81), 2.007 (0.29), 2.026 (1.65), 2.031 (1.03), 2.042 (1.15), 2.046 (1.18), 2.051 (0.70), 2.224 (0.72), 2.241 (1.12), 2.253 (0.50), 2.260 (0.54), 2.302 (1.04), 2.313 (0.84), 2.317 (0.78), 2.324 (1.11), 2.329 (1.01), 2.474 (0.74), 2.482 (0.89), 2.487 (0.99), 2.494 (1.19), 2.502 (0.94), 2.506 (0.82), 2.515 (0.68), 2.813 (0.44), 2.823 (0.52), 2.841 (1.32), 2.849 (1.37), 2.858 (0.95), 2.869 (0.89), 2.879 (0.83), 2.888 (0.90), 2.907 (0.31), 2.917 (0.27), 3.615 (0.54), 4.287 (0.83), 4.301 (1.48), 4.314 (0.78), 5.038 (1.80), 7.025 (2.83), 7.029 (2.71), 7.099 (1.47), 7.103 (1.36), 7.113 (1.74), 7.117 (1.46), 7.212 (11.96), 7.270 (2.43), 7.284 (2.07), 7.580 (5.57), 7.908 (0.88), 8.729 (10.46), 11.240 (0.77).

### Example 26

### 1alpha,2alpha-1,2-epoxyandrost-4-ene-3,17-dione

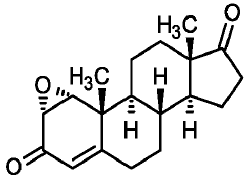

For the preparation of the title compound see Example 25. The crude product was further purified by flash chromatography using silica gel (dichloromethane/ethanol gradient) and by preparative HPLC to give the title compound (5.8 mg).

### Preparative HPLC method

Instrument: Waters Autopurificationsystem; Column: XBrigde C18 5 µ, 100x30 mm; eluent A: water+ 0.1 vol % formic acid; eluent B: acetonitrile; gradient: 0.0-0.5 min 23 % B (35-70 mL/min), 0.5-5.5 min 23-58 % B; flow: 70 mL/min; temperature: 25 °C; DAD scan: 210-400 nm.

### Analytical HPLC method

Instrument: Waters Acquity UPLCMS SingleQuad; Column: Acquity UPLC BEH C18 1.7 µ, 50x2.1 mm; eluent A: water + 0.1 vol % formic acid; eluent B: acetonitrile; gradient: 0-2.6 min 1-99 % B, 2.6-3.0 min 99 % B; flow: 0.8 mL/min; temperature: 60 °C; DAD scan: 210-400 nm.
Analytical HPLC: Rt = 1.06 min.
¹H-NMR (600 MHz, CHLOROFORM-d) δ [ppm]: 0.95 (s, 3H), 1.13 (qd, 1H), 1.23 - 1.36 (m, 4H), 1.42 (s, 3H), 1.54 - 1.68 (m, 2H), 1.84 - 1.94 (m, 2H), 1.88 - 1.98 (m, 1H), 2.05 - 2.15 (m, 2H), 2.22 - 2.31 (m, 1H), 2.36 - 2.43 (m, 1H), 2.48 (dd, 1H), 3.40 (dd, 1H), 3.67 (d, 1H), 5.76 (s, 1H).
¹H-NMR (600 MHz, CHLOROFORM-d) δ [ppm]: 0.000 (1.78), 0.880 (0.44), 0.935 (0.39), 0.949 (14.78), 0.988 (1.17), 1.093 (0.36), 1.100 (0.42), 1.114 (0.85), 1.121 (0.85), 1.136 (0.89), 1.143 (0.90), 1.157 (0.45), 1.164 (0.41), 1.238 (0.73), 1.244 (0.81), 1.255 (2.21), 1.279 (1.39), 1.288 (1.42), 1.303 (1.60), 1.309 (1.87), 1.318 (0.94), 1.327 (1.16), 1.419 (16.00), 1.545 (0.66), 1.560 (1.16), 1.565 (1.06), 1.576 (0.92), 1.581 (1.72), 1.587 (1.04), 1.596 (1.54), 1.602 (1.28), 1.610 (1.26), 1.617 (1.71), 1.633 (1.26), 1.638 (1.20), 1.654 (0.67), 1.662 (0.58), 1.849 (0.39), 1.856 (0.43), 1.868 (1.11), 1.875 (1.61), 1.880 (1.14), 1.887 (1.23), 1.895 (1.53), 1.900 (2.16), 1.918 (0.88), 1.925 (1.35), 1.936 (0.62), 1.940 (0.70), 1.950 (0.73), 1.956 (0.56), 1.961 (0.61), 1.971 (0.54), 2.013 (0.39), 2.072 (0.90), 2.086 (1.84), 2.093 (0.67), 2.103 (1.67), 2.119 (1.48), 2.134 (0.65), 2.245 (0.61), 2.252 (0.74), 2.257 (0.60), 2.267 (0.85), 2.271 (0.99), 2.278 (0.76), 2.291 (0.41), 2.371 (0.67), 2.380 (0.75), 2.393 (1.15), 2.402 (1.07), 2.414 (0.57), 2.423 (0.47), 2.454 (1.10), 2.468 (1.06), 2.486 (0.96), 2.501 (0.92), 2.625 (0.45), 3.393 (1.58), 3.396 (1.81), 3.398 (1.89), 3.402 (1.65), 3.662 (2.62), 3.668 (2.55), 5.756 (2.73), 7.264 (14.93).

### Example 27

### 19-hydroxy-1alpha,2alpha-1,2-epoxyandrost-4-ene-3,17-dione

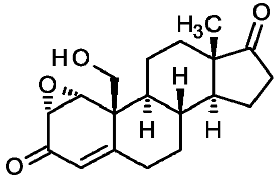

For the preparation of the title compound see Example 25. The crude product was further purified by flash chromatography using silica gel (dichloromethane/ethanol gradient) and twice by preparative HPLC to give the title compound (3.7 mg).

### 1. Preparative HPLC method

Instrument: Waters Autopurificationsystem; Column: XBrigde C18 5 µ, 100x30 mm; eluent A: water + 0.1 vol % formic acid; eluent B: acetonitrile; gradient: 0.0-0.5 min 23 % B (35-70 mL/min), 0.5-5.5 min 23-58 % B; flow: 70 mL/min; temperature: 25 °C; DAD scan: 210-400 nm.

### 1. Analytical HPLC method

Instrument: Waters Acquity UPLCMS SingleQuad; Column: Acquity UPLC BEH C18 1.7 µ, 50x2.1 mm; eluent A: water + 0.1 vol % formic acid; eluent B: acetonitrile; gradient: 0-2.6 min 1-99 % B, 2.6-3.0 min 99 % B; flow: 0.8 mL/min; temperature: 60 °C; DAD scan: 210-400 nm.

### 2. Preparative HPLC method

Instrument: Waters Autopurificationsystem; Column: XBrigde C18 5 µ, 100x30 mm; eluent A: water + 0.1 vol % formic acid; eluent B: acetonitrile; gradient: 0.0-0.5 min 20 % B (35-70 mL/min), 0.5-5.5 min 20-30 % B; flow: 70 mL/min; temperature: 25 °C; DAD scan: 210-400 nm.

### 2. Analytical HPLC method

Instrument: Waters Acquity UPLCMS SingleQuad; Column: Acquity UPLC BEH C18 1.7 µ, 50x2.1 mm; eluent A: water + 0.1 vol % formic acid; eluent B: acetonitrile; gradient: 0-2.6 min 1-99 % B, 2.6-3.0 min 99 % B; flow: 0.8 mL/min; temperature: 60 °C; DAD scan: 210-400 nm.
Analytical HPLC: Rt = 0.82 min.
¹H-NMR (600 MHz, CHLOROFORM-d) δ [ppm]: 0.95 (s, 3H), 1.12 - 1.21 (m, 1H), 1.23 - 1.37 (m, 3H), 1.54 - 1.65 (m, 2H), 1.84 - 2.01 (m, 4H), 2.06 - 2.18 (m, 2H), 2.33 - 2.41 (m, 2H), 2.43 - 2.52 (m, 2H), 3.38 (dd, 1H), 3.95 (d, 1H), 4.16 (t, 1H), 4.29 (d, 1H), 5.92 (s, 1H).
¹H-NMR (600 MHz, CDCl3) δ [ppm]: 0.881 (0.18), 0.904 (0.57), 0.927 (0.28), 0.948 (16.00), 1.136 (0.33), 1.143 (0.36), 1.157 (0.87), 1.164 (0.89), 1.179 (0.96), 1.186 (0.96), 1.200 (0.48), 1.208 (0.57), 1.240 (0.68), 1.247 (0.73), 1.263 (1.38), 1.269 (1.30), 1.287 (1.12), 1.291 (0.93), 1.297 (0.82), 1.308 (0.99), 1.315 (0.97), 1.318 (0.96), 1.326 (1.21), 1.331 (0.88), 1.336 (0.89), 1.347 (1.18), 1.364 (0.77), 1.369 (0.76), 1.414 (0.40), 1.545 (0.55), 1.552 (0.60), 1.568 (1.36), 1.574 (1.53), 1.590 (2.54), 1.597 (2.84), 1.606 (4.35), 1.621 (2.17), 1.627 (1.63), 1.642 (0.73), 1.881 (0.80), 1.887 (1.28), 1.893 (0.88), 1.904 (0.80), 1.909 (1.20), 1.915 (0.87), 1.926 (0.50), 1.937 (1.32), 1.945 (1.51), 1.952 (1.50), 1.958 (2.52), 1.963 (2.30), 1.970 (1.32), 1.981 (1.80), 2.076 (0.82), 2.090 (1.33), 2.108 (1.19), 2.115 (0.59), 2.123 (2.28), 2.138 (1.12), 2.143 (0.92), 2.148 (0.85), 2.367 (1.13), 2.373 (1.49), 2.388 (1.21), 2.394 (1.41), 2.399 (0.97), 2.435 (0.78), 2.443 (0.82), 2.458 (1.29), 2.464 (2.12), 2.478 (1.61), 2.487 (0.59), 2.495 (1.14), 2.510 (1.01), 2.623 (0.54), 3.377 (1.81), 3.380 (2.08), 3.383 (2.14), 3.387 (1.91), 3.942 (2.83), 3.949 (2.78), 4.146 (0.55), 4.163 (1.05), 4.179 (0.71), 4.278 (2.45), 4.297 (1.82), 5.920 (3.16), 7.266 (11.03).

### Example 28

### 3,15beta-dihydroxyestra-1(10),2,4-trien-17-one

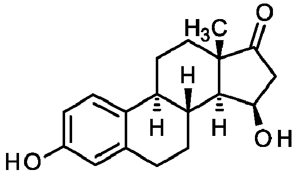

For the preparation of the title compound see Example 25. The crude product was further purified by flash chromatography using silica gel (dichloromethane/ethanol gradient) and by preparative HPLC to give the title compound (0.8 mg).

### Preparative HPLC method

Instrument: Waters Autopurificationsystem; Column: XBrigde C18 5 µ, 100x30 mm; eluent A: water + 0.1 vol % formic acid; eluent B: acetonitrile; gradient: 0.0-0.5 min 25 % B (35-70 mL/min), 0.5-5.5 min 25-35 % B; flow: 70 mL/min; temperature: 25 °C; DAD scan: 210-400 nm.

### Analytical HPLC method

Instrument: Waters Acquity UPLCMS SingleQuad; Column: Acquity UPLC BEH C18 1.7 µ, 50x2.1 mm; eluent A: water + 0.1 vol % formic acid; eluent B: acetonitrile; gradient: 0-2.6 min 1-99 % B, 2.6-3.0 min 99 % B; flow: 0.8 mL/min; temperature: 60 °C; DAD scan: 210-400 nm.
Analytical HPLC: Rt = 0.85 min.
¹H-NMR (600 MHz, Pyr) δ [ppm]: 0.000 (1.30), 1.267 (0.19), 1.452 (0.59), 1.458 (0.76), 1.469 (0.83), 1.477 (0.91), 1.492 (4.48), 1.530 (0.23), 1.551 (0.43), 1.574 (0.61), 1.596 (0.42), 1.618 (0.31), 2.018 (0.47), 2.037 (0.49), 2.137 (0.40), 2.155 (0.41), 2.172 (0.16), 2.301 (0.26), 2.318 (0.42), 2.336 (0.26), 2.352 (0.45), 2.379 (0.51), 2.496 (0.25), 2.666 (0.30), 2.676 (0.32), 2.697 (0.48), 2.707 (0.47), 2.816 (0.89), 2.833 (0.26), 2.848 (0.98), 2.859 (0.55), 2.879 (0.32), 2.887 (0.31), 2.898 (0.31), 4.782 (0.56), 4.989 (1.05), 6.502 (0.65), 7.002 (1.01), 7.106 (0.53), 7.118 (0.60), 7.211 (16.00), 7.313 (0.74), 7.327 (0.66), 7.578 (7.55), 8.731 (12.51), 11.201 (0.27).

### References

Patent WO200107630 A1. n.d.
Patent EP1196603 A1 . n.d.
Patent WO2016007623 A1. n.d.
Patent WO200107630 A1 . n.d.
Patent EP1131440 A2. n.d.
Acevedo-Rocha, CG, Gamble, C, Lonsdale, R, Li, A, Nett, N, Hoebenreich, S, Lingnau, JB, Wirtz, C, Fares, C, Hinrichs, H, Deege, A, Mulholland, AJ, Nov, Y, Leys, D, Mclean, K, Munro, A & Reetz, MT. "P450-Catalyzed Regio- and Diastereoselective Steroid Hydroxylation: Efficient Directed Evolution Enabled by Mutability Landscaping." ACS Catalysis 8, no. 4 (2018): p. 3395-3410.
Agudo R, Roiban GD, Reetz MT. "Achieving regio- and enantioselectivity of P450-catalyzed oxidative CH activation of small functionalized molecules by structure-guided directed evolution." Chembiochem, 2012: 1465 -- 73.
Capdevila, Jorge H. and Wei, Shozou and Helvig, Christian and Falck, John R. and Belosludtsev, Yuri and Truan, Gilles and Graham-Lorence, Sandra E. and Peterson, Julian A. "The Highly Stereoselective Oxidation of Polyunsaturated Fatty Acids by Cytochrome P450BM-3." Journal of Biological Chemistry 271, no. 37 (1996): p. 22663-22671.
F. Templeton, John and Lin, Weiyang and Ling, Yangzhi and Majgier-Baranowska, Helena and Marat, Kirk. "Synthesis of 19-hydroxy-1β{,}19-cyclosteroids." J. Chem. Soc., Perkin Trans. 1, 1997: 2037-2044.
Green, Michael R, and Joseph Sambrook. Molecular cloning: a laboratory manual. 4th. Cold Spring Harbor, N.Y.: Cold Spring Harbor Laboratory Press, 2012.
Hazel M Girvan, Andrew W Munro. "Applications of microbial cytochrome P450 enzymes in biotechnology and synthetic biology." Current Opinion in Chemical Biology 31 (2016): 136-145.
Hershberg, E B,Rubin, Martin,Schwenk, Erwin. "SYNTHESIS OF ESTRONE FROM ANDROSTADIENEDIONE." The Journal of Organic Chemistry 15, no. 2 (1950): p. 292 - 300.
Inhoffen, H H. "Der Weg vom Cholesterin zum Follikelhormon Oestradiol." Angewandte Chemie 59, no. 7-8 (1947): 207 - 212.
IUPAC. Gold Book - Compendium of Chemical Terminology. n.d.
Kawahara R, Fujita K, Yamaguchi R. "Cooperative Catalysis by Iridium Complexes with a Bipyridonate Ligand: Versatile Dehydrogenative Oxidation of Alcohols and Reversible Dehydrogenation-Hydrogenation between 2-Propanol and Acetone." 2012: 12790-12794.
Kille Sabrina, Zilly Felipe E., Acevedo Juan P., Reetz Manfred T. "Regio- and stereoselectivity of P450-catalysed hydroxylation of steroids controlled by laboratory evolution." Nat Chem. 3, no. 9 (2011): p. 738-743.
Kille, Sabrina. Flavoproteins in directed evolution : iterative CASTing to evolve YqjM and P450BM3. Bochum, 2010.
McPherson, M. J. Directed Mutagenesis: A Practical Approach. Vol. 73. IRL Press, 1991.
Mitsuteru Numazawa, Kouwa Yamashita, Nao Kimura and Madoka Takahashi. "Chemical aromatization of 19-hydroxyandrosta-1,4-diene-3,17-dione with acid or alkaline: Elimination of the 19-hydroxymethyl group as formaldehyde." Steroids 74, no. 2 (2009): p. 208 - 211.
Munro, Andrew W,Daff, Simon,Coggins, John R,Lindsay, J Gordon,Chapman, Stephen K. "Probing Electron Transfer in Flavocytochrome P-450 BM3 and Its Component Domains." European Journal of Biochemistry 239, no. 2 (1996): p. 403-409.
Narhi, L O and Fulco, A J. "Characterization of a catalytically self-sufficient 119,000-dalton cytochrome P-450 monooxygenase induced by barbiturates in Bacillus megaterium." Journal of Biological Chemistry 261, no. 16 (1986): p. 7160-7169.
Noble MA, Miles CS, Chapman SK, Lysek DA, MacKay AC, Reid GA, Hanzlik RP, Munro AW. "Roles of key active-site residues in flavocytochrome P450 BM3." The Biochemical journal 339, no. Pt 2 (1999): p. 371-379.

## Claims

1. A Cytochrome P450 BM3 monooxygenase (BM3) variant for catalyzing the C19 hydroxylation of a steroid or steroid derivative, wherein the BM3 variant comprises the mutation F87A and at least one and preferably two further mutations selected from
(i) a mutation at position V78, preferably V78F, V78Y, V78M, V78I or V78L,
(ii) a mutation at position A82, preferably A82E, A82Q or A82P,
and wherein the BM3 variant optionally comprises further mutations.

2. The BM3 variant according to claim 1, **characterized in** comprising
(i) at least the mutations
a) V78Y, A82E and F87A (BM3-254, SEQ ID No. 2),
b) V78M, A82E and F87A (BM3-261, SEQ ID No. 3),
c) V78I, A82E and F87A (BM3-263, SEQ ID No. 4),
d) V78L, A82E and F87A (BM3-268, SEQ ID No. 5).
e) V78Y, A82P and F87A (SEQ ID No. 6),
f) V78M, A82P and F87A (SEQ ID No. 7),
g) V78I, A82P and F87A (SEQ ID No. 8),
h) V78L, A82P and F87A (SEQ ID No. 9),
i) V78Y, A82Q and F87A (SEQ ID No. 10),
j) V78M, A82Q and F87A (SEQ ID No. 11),
k) V78I, A82Q and F87A (SEQ ID No. 12), or
l) V78L, A82Q and F87A (SEQ ID No. 13),
and at least one, two, three, four, five, six, seven, eight, nine or ten further mutation(s),
or
(ii) at least the mutations
m) V78F, A82E, F87A, M177Y and A184Y (SEQ ID No. 14),
n) V78L, A82E, F87A, M177Y and A184Y (SEQ ID No. 15),
o) V78I, A82E, F87A, M177Y and A184Y (SEQ ID No. 16),
p) V78M, A82E, F87A, M177Y and A184Y (SEQ ID No. 17),
q) V78Y, A82E, F87A, M177Y and A184Y (SEQ ID No. 18),
r) V78F, A82P, F87A, M177Y and A184Y (SEQ ID No. 19),
s) V78L, A82P, F87A, M177Y and A184Y (SEQ ID No. 20),
t) V78I, A82P, F87A, M177Y and A184Y (SEQ ID No. 21),
u) V78M, A82P, F87A, M177Y and A184Y (SEQ ID No. 22),
v) V78Y, A82P, F87A, M177Y and A184Y (SEQ ID No. 23),
w) V78F, A82Q, F87A, M177Y and A184Y (SEQ ID No. 24),
x) V78L, A82Q, F87A, M177Y and A184Y (SEQ ID No. 25),
y) V78I, A82Q, F87A, M177Y and A184Y (SEQ ID No. 26),
z) V78M, A82Q, F87A, M177Y and A184Y (SEQ ID No. 27), or
aa) V78Y, A82Q, F87A, M177Y and A184Y (SEQ ID No. 28),
and optionally further mutation(s).

3. A BM3 variant according to claim 1 or 2, which is **characterized by** a product yield for at least one C19 hydroxylated steroid or derivative thereof or a secondary product thereof which is > 5 %, preferably > 8 %, >10 %, or > 12 %, even more preferably > 15%, >20 %, >25 %, > 30 %, >35 %, > 40 %, > 45 %, or > 50 %, most preferably > 55 %, > 60 %, > 65 %, > 70 %, > 75 %.

4. A BM3 variant according to any of claims 1 to 3 which is **characterized by** a selectivity factor for the C19 hydroxylation of at least one steroid or steroid derivative which is > 0.1, preferably > 0.2 or > 0.3, even more preferably > 0.4 or > 0.5, most preferably > 0.5, 0.6, 0.7, 0.8 or 0.9.

5. The BM3 variant according to any of claims 1 to 4, said further mutation(s) comprising mutations at at least one, two, three, four, five, six, seven, eight, nine, ten or more position(s) selected from (A184) or (A191) or (A221) or (A264) or (A321) or (A328) or (A33) or (A330) or (A399) or (A74) or (D168) or (D208) or (D222) or (D363) or (E13) or (E143) or (E267) or (E352) or (E64) or (E82) or (E93) or (F173) or (F205) or (F261) or (F331) or (F393) or (F77) or (F81) or (G240) or (G271) or (G402) or (G415) or (G570) or (G677) or (G85) or (H266) or (H659) or (I153) or (I174) or (I258) or (I259) or (I263) or (I401) or (K210) or (K224) or (L150) or (L181) or (L188) or (L20) or (L262) or (L272) or (L29) or (L324) or (L333) or (L356) or (L437) or (L75) or (L78) or (L86) or (M118) or (M177) or (M185) or (M212) or (M354) or (N70) or (P243) or (P326) or (P329) or (P392) or (P9) or (R147) or (R161) or (R190) or (R203) or (R255) or (R323) or (R47) or (R50) or (S164) or (S176) or (S54) or (S72) or (S89) or (T146) or (T149) or (T260) or (T268) or (T269) or (T327) or (T365) or (T436) or (T438) or (T49) or (T88) or (V178) or (V26) or (V299) or (V314) or (V371) or (V48) or (W325) or (W367) or (W90) or (Y51).

6. A BM3 variant according to any of claims 1 to 5, wherein said variant has an improved selectivity or product yield for C19 hydroxylation compared with at least one BM3 variant selected from the variants according to SEQ ID No. 2 to 5.

7. A BM3 variant according to any of claims 1 to 6, wherein said monooxygenase comprises the mutations M177Y and/or A184Y.

8. A BM3 variant according to any of claims 1 to 7, wherein said variant comprises at least the mutation(s) (A184D) or (A184D, L188K) or (A184E) or (A184F) or (A184G) or (A184H) or (A184I) or (A184K) or (A184L) or (A184N) or (A184Q) or (A184R) or (A184W) or (A184W, L188F) or (A184Y) or (A184Y, L188K) or (A184Y, R50S) or (A221S) or (A264V) or (A321C) or (A321D) or (A321E) or (A321G) or (A321I) or (A321N) or (A321T) or (A321V) or (A328P) or (A330C) or (A330D) or (A330E) or (A330F) or (A330G) or (A330H) or (A330I) or (A330K) or (A330L) or (A330M) or (A330N) or (A330Q) or (A330R) or (A330S) or (A330T) or (A330T, E352A) or (A330T, F331V) or (A330V) or (A330W) or (A330Y) or (A330Y, W367C) or (A33V) or (A399C) or (A399E) or (A399G) or (A399I) or (A399L) or (A399M) or (A399N) or (A399Q) or (A399R) or (A399S) or (A399T) or (A399V) or (A74C) or (A74C, L75V) or (A74D) or (A74D, F81V) or (A74E) or (A74F) or (A74G) or (A74G, L75C) or (A74H) or (A74I) or (A74I, A184W, L188F) or (A74I, F77A, L181Y, A184W) or (A74I, L181Y, L188K) or (A74I, L181Y, L188R) or (A74I, L188F) or (A74I, L188K) or (A74I, L188R) or (A74I, M177Y, A184R, L188F) or (A74I, M177Y, V178P, A184G) or (A74I, T146F) or (A74I, T146F, L181Y) or (A74I, T146F, L181Y, L188K) or (A74I, T146F, L181Y, L188R) or (A74I, T146F, L188F) or (A74I, T146F, L188K) or (A74I, T146F, V178P) or (A74I, T146F, V178P, L181Y, L188F) or (A74I, T146F, V178P, L181Y, L188K) or (A74I, T146F, V178P, L188K) or (A74I, T146F, V178P, L188R) or (A74I, T146F, V178W) or (A74I, T146F, V178W, L181Y, L188F) or (A74I, T146F, V178W, L181Y, L188R) or (A74I, T146F, V178W, L188F) or (A74I, T146F, V178W, L188K) or (A74I, T146F, V178W, L188R) or (A74I, V178P) or (A74I, V178P, L181Y) or (A74I, V178P, L181Y, L188R) or (A74I, V178P, L188F) or (A74I, V178P, L188K) or (A74I, V178P, L188R) or (A74I, V178W, L188F) or (A74I, V178W, L188K) or (A74I, V178W, L188R) or (A74K) or (A74L) or (A74M) or (A74N) or (A74P) or (A74Q) or (A74R) or (A74R, L75V) or (A74S) or (A74T) or (A74T, A184D) or (A74T, F77A, L181Y, A184D, L188K) or (A74T, F77A, V178W, A184Y) or (A74T, F77L, L181Y, A184D, L188K) or (A74T, L181Y) or (A74T, L181Y, A184D, L188K) or (A74T, L181Y, A184Y, L188K) or (A74T, L181Y, L188K) or (A74T, L181Y, L188R) or (A74T, L188K) or (A74T, M177Y, L181Y, A184D, L188K) or (A74T, T146F, L181Y, A184D, L188K) or (A74T, T146F, L181Y, L188K) or (A74T, T146F, L181Y, L188R) or (A74T, T146F, L188F) or (A74T, T146F, L188K) or (A74T, T146F, L188R) or (A74T, T146F, V178P) or (A74T, T146F, V178P, L181Y) or (A74T, T146F, V178P, L188K) or (A74T, T146F, V178P, L188R) or (A74T, T146F, V178W) or (A74T, T146F, V178W, L181Y) or (A74T, T146F, V178W, L181Y, L188K) or (A74T, T146F, V178W, L188R) or (A74T, V178P) or (A74T, V178P, L188F) or (A74T, V178P, L188K) or (A74T, V178P, L188R) or (A74T, V178W, L181Y, L188K) or (A74T, V178W, L188K) or (A74T, V178W, L188R) or (A74V) or (A74V, F77A, A184N, L188F) or (A74V, L181Y) or (A74V, L181Y, A184D, L188K) or (A74V, L181Y, A184W, L188F) or (A74V, L181Y, L188F) or (A74V, L188F) or (A74V, L188K) or (A74V, L75V) or (A74V, T146F, L181Y, L188K) or (A74V, T146F, L181Y, L188R) or (A74V, T146F, L188K) or (A74V, T146F, L188R) or (A74V, T146F, V178W) or (A74V, T146F, V178W, L181Y, L188F) or (A74V, V178P, A184W, L188K) or (A74V, V178P, L181Y, L188F) or (A74V, V178P, L181Y, L188K) or (A74V, V178P, L181Y, L188R) or (A74V, V178P, L188F) or (A74V, V178W, L188K) or (A74W) or (A74Y) or (D168Y, F173D) or (D363G, T438C) or (E13D, R47L) or (E143A) or (E143C) or (E143D) or (E143F) or (E143G) or (E143I) or (E143K) or (E143L) or (E143M) or (E143N) or (E143P) or (E143Q) or (E143R) or (E143S) or (E143V) or (E143W) or (E143Y) or (E267A) or (E267C) or (E267D) or (E267G) or (E267K) or (E267P) or (E267R) or (E267S) or (E267T) or (E267Y) or (E352A) or (E352D) or (E352F) or (E352G) or (E352I) or (E352L) or (E352M) or (E352N) or (E352P) or (E352R) or (E352S) or (E352T) or (E352V) or (E352W) or (E352Y) or (E64A) or (E64G) or (E64H) or (E64I) or (E64K) or (E64L) or (E64M) or (E64N) or (E64Q) or (E64V) or (E64W) or (E64Y) or (E82P) or (E93G) or (F173C) or (F173C, F205G) or (F173D) or (F173I) or (F173K) or (F173L) or (F173M) or (F173N) or (F173P) or (F173Q) or (F173R) or (F173S) or (F173S, F205P) or (F173V) or (F173W) or (F173Y) or (F173Y, I174F) or (F205A) or (F205C) or (F205D) or (F205D, D208N) or (F205E) or (F205G) or (F205H) or (F205I) or (F205K) or (F205L) or (F205M) or (F205N) or (F205P) or (F205R) or (F205S) or (F205T) or (F205V) or (F205W) or (F205Y) or (F261A) or (F261C) or (F261D) or (F261G) or (F261I) or (F261L) or (F261M) or (F261N) or (F261Q) or (F261S) or (F261T) or (F261V) or (F261W) or (F261Y) or (F331C) or (F331H) or (F331I) or (F331L) or (F331M) or (F331N) or (F331P) or (F331T) or (F331V) or (F331W) or (F331Y) or (F393M) or (F393W) or (F77A) or (F77A, A184W) or (F77A, L181P, A184Y, L188K) or (F77A, M177Y) or (F77C) or (F77D) or (F77E) or (F77G) or (F77H) or (F77I) or (F77K) or (F77L) or (F77M) or (F77N) or (F77P) or (F77R) or (F77S) or (F77T) or (F77V) or (F77V, L86M) or (F77W) or (F77Y) or (F81A) or (F81C) or (F81D) or (F81I) or (F81L) or (F81P) or (F81R) or (F81S) or (F81T) or (F81V) or (F81W) or (F81Y) or (G271A) or (G271C) or (G271D) or (G271E) or (G271F) or (G271H) or (G271K) or (G271L) or (G271M) or (G271N) or (G271P) or (G271Q) or (G271R) or (G271S) or (G271T) or (G271V) or (G271W) or (G271Y) or (G402A) or (G415A) or (G415D) or (G415S) or (G415T) or (G415V) or (G85A) or (G85L) or (G85S) or (H266A) or (H266C) or (H266D) or (H266E) or (H266F) or (H266G) or (H266I) or (H266K) or (H266M) or (H266N) or (H266P) or (H266Q) or (H266R) or (H266S) or (H266T) or (H266V) or (H266W) or (H266Y) or (I153F, G271L) or (I153L) or (I153L, F173Y) or (I259A) or (I259C) or (I259D) or (I259F) or (I259G) or (I259H) or (I259K) or (I259L) or (I259M) or (I259N) or (I259Q) or (I259S) or (I259T) or (I259V) or (I259W) or (I259Y) or (I263A) or (I263C) or (I263E) or (I263F) or (I263G) or (I263H, A264G) or (I263K) or (I263L) or (I263M) or (I263N) or (I263Q) or (I263S) or (I263T) or (I263V) or (I263Y) or (I401A) or (I401L) or (I401M) or (I401T) or (I401V) or (K210E) or (K210T, G271V) or (K224C) or (K224E) or (K224F) or (K224H) or (K224I) or (K224L) or (K224M) or (K224P) or (K224Q) or (K224W) or (K224Y) or (L150A) or (L150C) or (L150D) or (L150E) or (L150F) or (L150G) or (L150H) or (L150I) or (L150K) or (L150M) or (L150N) or (L150Q) or (L150R) or (L150R, F205R) or (L150S) or (L150S, F173L) or (L150T) or (L150V) or (L150W) or (L150Y) or (L181H) or (L181I) or (L181M) or (L181P) or (L181V) or (L181Y) or (L181Y, A184D) or (L181Y, L188F) or (L181Y, L188K) or (L181Y, L188K, H659R) or (L188A) or (L188D) or (L188E) or (L188F) or (L188H) or (L188I) or (L188K) or (L188M) or (L188N) or (L188Q) or (L188R) or (L188S) or (L188W) or (L20C) or (L20D) or (L20E) or (L20F) or (L20G) or (L20G, R47L) or (L20I) or (L20M) or (L20N) or (L20P) or (L20R) or (L20S) or (L20T) or (L20V) or (L20W) or (L20Y) or (L262I) or (L262V) or (L262W) or (L262Y) or (L272A) or (L272C) or (L272E) or (L272F) or (L272G) or (L272I) or (L272K) or (L272M) or (L272N) or (L272Q) or (L272R) or (L272S) or (L272T) or (L272V) or (L272W) or (L272Y) or (L29A) or (L29C) or (L29D) or (L29F) or (L29H) or (L29I) or (L29M) or (L29M, R47G) or (L29P) or (L29Q) or (L29S) or (L29T) or (L29V) or (L29W) or (L29Y) or (L324F) or (L356C) or (L356F) or (L356H) or (L356I) or (L356M) or (L356N) or (L356Q) or (L356S) or (L356T) or (L356V) or (L356W) or (L437I) or (L437M) or (L75H, F81S) or (L75I) or (L75I, F81C) or (L75I, F81G) or (L75I, F81H) or (L75I, F81I) or (L75I, F81L) or (L75I, F81S) or (L75I, F81V) or (L75I, F81Y) or (L75V) or (L75V, F81C) or (L75V, F81H) or (L75V, F81I) or (L75V, F81L) or (L75V, F81V) or (L75V, F81Y) or (L78F) or (L78I) or (L78M) or (L78V) or (L78Y) or (L86A, S89T) or (L86I) or (L86I, S89T) or (L86M) or (L86M, S89T) or (L86N, S89T) or (L86V) or (L86V, S89T) or (M118A) or (M118E) or (M118F) or (M118G) or (M118H) or (M118I) or (M118K) or (M118L) or (M118N) or (M118P) or (M118Q) or (M118S) or (M118T) or (M118V) or (M118W) or (M118Y) or (M177A) or (M177C) or (M177C, V178Y) or (M177D) or (M177E) or (M177F) or (M177G) or (M177H) or (M177I) or (M177K) or (M177L) or (M177N) or (M177P) or (M177Q) or (M177R) or (M177T) or (M177V) or (M177Y) or (M177Y, A184W) or (M177Y, A184Y) or (M177Y, M185V) or (M177Y, R50S) or (M177Y, V178P) or (M177Y, V178P, A184Y, L188F) or (M177Y, V178W) or (M177Y, V178W, A184Y) or (M177Y, V178W, A184Y, L188F) or (M185C) or (M185D) or (M185E) or (M185G) or (M185H) or (M185K) or (M185L) or (M185N) or (M185Q) or (M185R) or (M185V) or (M185Y) or (M212A) or (M212C) or (M212C, I259L) or (M212D) or (M212E) or (M212F) or (M212G) or (M212H) or (M212K) or (M212L) or (M212L, I259F) or (M212P) or (M212Q) or (M212R) or (M212S) or (M212T) or (M212V) or (M212W) or (M212Y) or (M354A) or (M354C) or (M354D) or (M354E) or (M354G) or (M354I) or (M354K) or (M354K, D363Y) or (M354L) or (M354N) or (M354Q) or (M354R) or (M354S) or (M354T) or (M354V) or (M354W) or (M354Y) or (N70A) or (N70C) or (N70F) or (N70G) or (N70H) or (N70K) or (N70R) or (N70W) or (N70Y) or (P243T, H266L) or (P326C) or (P326D) or (P326G) or (P326N) or (P326S) or (P326T) or (P329A) or (P329C) or (P329G) or (P329K) or (P329S) or (P329T) or (P392C) or (P392G) or (P392Q) or (P392R) or (P392V) or (P9S) or (R147C, H266G) or (R161C, G271D) or (R190L, F261V) or (R203C) or (R255A) or (R255F) or (R255G) or (R255I) or (R255K) or (R255L) or (R255M) or (R255N) or (R255P) or (R255T) or (R255W) or (R323C) or (R47A) or (R47C) or (R47D) or (R47E) or (R47F) or (R47G) or (R47H) or (R47H, A74W) or (R47I) or (R47K) or (R47L) or (R47M) or (R47N) or (R47P) or (R47Q) or (R47S) or (R47T) or (R47V) or (R47W) or (R50A) or (R50C) or (R50D) or (R50E) or (R50F) or (R50G) or (R50I) or (R50L) or (R50M) or (R50N) or (R50P) or (R50Q) or (R50S) or (R50S, A184W, L188F) or (R50S, A74I, A184W, L188F) or (R50S, A74M, A184Y, L188K) or (R50S, A74T, L181Y, A184D, L188K, G240R) or (R50S, A74V) or (R50S, M177Y, V178P, A184Y) or (R50S, M177Y, V178W, A184W) or (R50S, M177Y, V178W, A184Y) or (R50S, M177Y, V178W, A184Y, A330R) or (R50S, M177Y, V178W, A184Y, A74V, G677D) or (R50S, M177Y, V178W, A184Y, H266S) or (R50S, M177Y, V178W, A184Y, L181P) or (R50S, M177Y, V178W, A184Y, L181Y) or (R50S, M177Y, V178W, A184Y, L188K) or (R50S, M177Y, V178W, A184Y, L188R) or (R50S, M177Y, V178W, A184Y, T146F) or (R50S, M177Y, V178W, A184Y, V26N) or (R50S, M177Y, V178W, L181Y, A184Y) or (R50S, S72G, A74V) or (R50S, S72G, A74V, M177Y, A184Y) or (R50S, S72G, A74V, M177Y, V178P, A184Y) or (R50S, S72G, A74V, M177Y, V178W, A184Y) or (R50S, S72G, A74V, M177Y, V178W, A184Y, L188R) or (R50S, T146F, M177Y, V178W, A184Y) or (R50V) or (R50Y) or (S164N, F261L) or (S176A, A184Y, L188K) or (S176C) or (S176D) or (S176E) or (S176F) or (S176G) or (S176H) or (S176K) or (S176L) or (S176M) or (S176N) or (S176P) or (S176Q) or (S176R) or (S72C, A74C) or (S72C, A74I) or (S72C, A74L) or (S72C, A74V) or (S72C, A74Y) or (S72D, A74C) or (S72D, A74F) or (S72G) or (S72G, A74C) or (S72G, A74F) or (S72G, A74H) or (S72G, A74I) or (S72G, A74I, A184W, L188F) or (S72G, A74I, F77A, M177Y) or (S72G, A74I, F77L) or (S72G, A74I, F77L, M177Y) or (S72G, A74I, L181Y) or (S72G, A74I, L181Y, L188F) or (S72G, A74I, L181Y, L188K) or (S72G, A74I, L181Y, L188R) or (S72G, A74I, L188F) or (S72G, A74I, L188K) or (S72G, A74I, L188R) or (S72G, A74I, T146F) or (S72G, A74I, T146F, L181Y) or (S72G, A74I, T146F, L181Y, L188F) or (S72G, A74I, T146F, L181Y, L188K) or (S72G, A74I, T146F, L181Y, L188R) or (S72G, A74I, T146F, L188F) or (S72G, A74I, T146F, L188K) or (S72G, A74I, T146F, L188R) or (S72G, A74I, T146F, V178P) or (S72G, A74I, T146F, V178P, L181Y) or (S72G, A74I, T146F, V178P, L181Y, L188F) or (S72G, A74I, T146F, V178P, L181Y, L188K) or (S72G, A74I, T146F, V178P, L181Y, L188R) or (S72G, A74I, T146F, V178P, L188F) or (S72G, A74I, T146F, V178P, L188K) or (S72G, A74I, T146F, V178P, L188R) or (S72G, A74I, T146F, V178W) or (S72G, A74I, T146F, V178W, L181Y) or (S72G, A74I, T146F, V178W, L181Y, L188F) or (S72G, A74I, T146F, V178W, L181Y, L188K) or (S72G, A74I, T146F, V178W, L181Y, L188R) or (S72G, A74I, T146F, V178W, L188F) or (S72G, A74I, T146F, V178W, L188K) or (S72G, A74I, T146F, V178W, L188R) or (S72G, A74I, V178P) or (S72G, A74I, V178P, L181Y) or (S72G, A74I, V178P, L181Y, L188K) or (S72G, A74I, V178P, L181Y, L188R) or (S72G, A74I, V178P, L188F) or (S72G, A74I, V178P, L188K) or (S72G, A74I, V178P, L188R) or (S72G, A74I, V178W) or (S72G, A74I, V178W, L181Y, L188F) or (S72G, A74I, V178W, L181Y, L188K) or (S72G, A74I, V178W, L181Y, L188R) or (S72G, A74I, V178W, L188F) or (S72G, A74I, V178W, L188K) or (S72G, A74I, V178W, L188R) or (S72G, A74L) or (S72G, A74M, A184Y, L188K) or (S72G, A74S) or (S72G, A74T) or (S72G, A74T, L181Y, A184D, L188K) or (S72G, A74T, L181Y, L188F) or (S72G, A74T, L181Y, L188K) or (S72G, A74T, L181Y, L188R) or (S72G, A74T, L188F) or (S72G, A74T, L188K) or (S72G, A74T, L188R) or (S72G, A74T, T146F) or (S72G, A74T, T146F, L181Y) or (S72G, A74T, T146F, L181Y, L188K) or (S72G, A74T, T146F, L181Y, L188R) or (S72G, A74T, T146F, L188F) or (S72G, A74T, T146F, L188K) or (S72G, A74T, T146F, L188R) or (S72G, A74T, T146F, V178P) or (S72G, A74T, T146F, V178P, L181Y) or (S72G, A74T, T146F, V178P, L188F) or (S72G, A74T, T146F, V178P, L188K) or (S72G, A74T, T146F, V178P, L188R) or (S72G, A74T, T146F, V178W) or (S72G, A74T, T146F, V178W, L181Y) or (S72G, A74T, T146F, V178W, L181Y, L188F) or (S72G, A74T, T146F, V178W, L181Y, L188K) or (S72G, A74T, T146F, V178W, L181Y, L188R) or (S72G, A74T, T146F, V178W, L188F) or (S72G, A74T, T146F, V178W, L188K) or (S72G, A74T, T146F, V178W, L188R) or (S72G, A74T, V178P) or (S72G, A74T, V178P, L181Y) or (S72G, A74T, V178P, L181Y, L188F) or (S72G, A74T, V178P, L181Y, L188K) or (S72G, A74T, V178P, L188F) or (S72G, A74T, V178P, L188K) or (S72G, A74T, V178P, L188R) or (S72G, A74T, V178W) or (S72G, A74T, V178W, L181Y, L188F) or (S72G, A74T, V178W, L181Y, L188K) or (S72G, A74T, V178W, L188F) or (S72G, A74T, V178W, L188K) or (S72G, A74T, V178W, L188R) or (S72G, A74V) or (S72G, A74V, A184W) or (S72G, A74V, A221V) or (S72G, A74V, F77A) or (S72G, A74V, F77A, M177Y) or (S72G, A74V, F77L, M177Y) or (S72G, A74V, F77S) or (S72G, A74V, L181Y) or (S72G, A74V, L181Y, L188F) or (S72G, A74V, L181Y, L188K) or (S72G, A74V, L188F) or (S72G, A74V, L188K) or (S72G, A74V, L188R) or (S72G, A74V, T146F) or (S72G, A74V, T146F, L181Y) or (S72G, A74V, T146F, L181Y, L188F) or (S72G, A74V, T146F, L181Y, L188K) or (S72G, A74V, T146F, L181Y, L188R) or (S72G, A74V, T146F, L188F) or (S72G, A74V, T146F, L188K) or (S72G, A74V, T146F, L188R) or (S72G, A74V, T146F, V178P) or (S72G, A74V, T146F, V178P, L181Y) or (S72G, A74V, T146F, V178P, L181Y, L188F) or (S72G, A74V, T146F, V178P, L181Y, L188K) or (S72G, A74V, T146F, V178P, L181Y, L188R) or (S72G, A74V, T146F, V178P, L188F) or (S72G, A74V, T146F, V178P, L188K) or (S72G, A74V, T146F, V178P, L188R) or (S72G, A74V, T146F, V178W) or (S72G, A74V, T146F, V178W, L181Y) or (S72G, A74V, T146F, V178W, L181Y, L188K) or (S72G, A74V, T146F, V178W, L181Y, L188R) or (S72G, A74V, T146F, V178W, L188F) or (S72G, A74V, T146F, V178W, L188K) or (S72G, A74V, T146F, V178W, L188R) or (S72G, A74V, V178P) or (S72G, A74V, V178P, L181Y) or (S72G, A74V, V178P, L181Y, L188F) or (S72G, A74V, V178P, L181Y, L188K) or (S72G, A74V, V178P, L181Y, L188R) or (S72G, A74V, V178P, L188F) or (S72G, A74V, V178P, L188K) or (S72G, A74V, V178P, L188R) or (S72G, A74V, V178W) or (S72G, A74V, V178W, L181Y) or (S72G, A74V, V178W, L181Y, L188F) or (S72G, A74V, V178W, L181Y, L188K) or (S72G, A74V, V178W, L181Y, L188R) or (S72G, A74V, V178W, L188F) or (S72G, A74V, V178W, L188K) or (S72G, A74V, V178W, L188R) or (S72G, A74Y) or (S72G, L181Y) or (S72G, L181Y, L188F) or (S72G, L181Y, L188K) or (S72G, L181Y, L188R) or (S72G, L188F) or (S72G, L188K) or (S72G, L188R) or (S72G, M177Y, V178W) or (S72G, M177Y, V178W, L188K) or (S72G, T146F) or (S72G, T146F, L181Y) or (S72G, T146F, L181Y, L188F) or (S72G, T146F, L181Y, L188K) or (S72G, T146F, L181Y, L188R) or (S72G, T146F, L188F) or (S72G, T146F, L188K) or (S72G, T146F, L188R) or (S72G, T146F, V178P) or (S72G, T146F, V178P, L181Y) or (S72G, T146F, V178P, L181Y, L188F) or (S72G, T146F, V178P, L181Y, L188K) or (S72G, T146F, V178P, L181Y, L188R) or (S72G, T146F, V178P, L188F) or (S72G, T146F, V178P, L188K) or (S72G, T146F, V178P, L188R) or (S72G, T146F, V178W) or (S72G, T146F, V178W, L181Y) or (S72G, T146F, V178W, L181Y, L188F) or (S72G, T146F, V178W, L181Y, L188K) or (S72G, T146F, V178W, L188F) or (S72G, T146F, V178W, L188K) or (S72G, T146F, V178W, L188R) or (S72G, V178P) or (S72G, V178P, L181Y) or (S72G, V178P, L181Y, L188F) or (S72G, V178P, L181Y, L188K) or (S72G, V178P, L181Y, L188R) or (S72G, V178P, L188F) or (S72G, V178P, L188K) or (S72G, V178P, L188R) or (S72G, V178W) or (S72G, V178W, L181Y) or (S72G, V178W, L181Y, L188F) or (S72G, V178W, L181Y, L188K) or (S72G, V178W, L181Y, L188R) or (S72G, V178W, L188F) or (S72G, V178W, L188K) or (S72G, V178W, L188R) or (S72H, A74C) or (S72H, A74G) or (S72H, A74S) or (S72H, A74Y) or (S72N, A74C) or (S72N, A74I) or (S72N, A74N) or (S72N, A74V) or (S72T) or (S72W) or (S72W, A74I, A184W, L188F) or (S72W, A74V) or (S72W, L333R) or (S72W, W90Y, V299G) or (S72Y, A74V) or (S89C) or (S89E) or (S89G) or (S89I) or (S89L) or (S89M) or (S89N) or (S89Q) or (S89R) or (S89T) or (S89V) or (T146A) or (T146A, D222Y, I263M) or (T146A, F173D) or (T146A, F173V) or (T146A, F205G) or (T146A, F261C) or (T146A, F261S) or (T146A, F261V) or (T146A, G271R) or (T146A, G271S) or (T146A, G271T) or (T146A, H266T) or (T146A, H266V) or (T146A, I258T, I259S) or (T146A, I259H) or (T146A, I263F) or (T146A, I263L) or (T146A, I263N) or (T146A, I263V) or (T146A, L150A) or (T146A, L150G) or (T146A, L150K) or (T146A, L150R, F173I) or (T146A, L272H) or (T146A, L272R) or (T146A, L272S) or (T146A, L272W) or (T146A, M212A) or (T146C) or (T146D) or (T146E) or (T146F) or (T146F, L181Y) or (T146F, L181Y, L188F) or (T146F, L181Y, L188K) or (T146F, L188K) or (T146F, L188R) or (T146F, V178P) or (T146F, V178P, L181Y, L188R) or (T146F, V178P, L188K) or (T146F, V178P, L188R) or (T146F, V178W) or (T146F, V178W, L181Y, L188R) or (T146F, V178W, L188F) or (T146F, V178W, L188K) or (T146F, V178W, L188R) or (T146G) or (T146H) or (T146L) or (T146M) or (T146N) or (T146P) or (T146R) or (T146S) or (T146V) or (T146V, A191T) or (T146Y) or (T149P) or (T260A) or (T260S) or (T260W) or (T268A) or (T268S) or (T269K) or (T327A) or (T327A, A330W) or (T327C) or (T327D) or (T327E) or (T327G) or (T327I) or (T327L) or (T327M) or (T327N) or (T327P) or (T327Q) or (T327S) or (T327V) or (T365A) or (T365C) or (T365D) or (T365F) or (T365G) or (T365H) or (T365I) or (T365K) or (T365L) or (T365N) or (T365P) or (T365Q) or (T365V) or (T365W) or (T365Y) or (T436A) or (T436C) or (T436D) or (T436E) or (T436F) or (T436G) or (T436H) or (T436I) or (T436K) or (T436L) or (T436M) or (T436N) or (T436P) or (T436Q) or (T436R) or (T436S) or (T436V) or (T436W) or (T436Y) or (T438A) or (T438C) or (T438G) or (T438I) or (T438L) or (T438S) or (T438V) or (T49A) or (T49C) or (T49D) or (T49E) or (T49F) or (T49G) or (T49H) or (T49I) or (T49K) or (T49L) or (T49L, S54N) or (T49M) or (T49N) or (T49P) or (T49Q) or (T49R) or (T49R, R50C) or (T49R, R50S, M177Y, V178W, A184Y) or (T49S) or (T49V) or (T49W) or (T49Y) or (T88A) or (T88C) or (T88S) or (T88V) or (V178A) or (V178D) or (V178F) or (V178H) or (V178I) or (V178K) or (V178L) or (V178M) or (V178N) or (V178P) or (V178P, L181Y) or (V178P, L181Y, L188R) or (V178P, L188F) or (V178P, L188K) or (V178P, L188R) or (V178Q) or (V178R) or (V178S) or (V178T) or (V178W) or (V178W, A184Y) or (V178W, L181Y, L188K) or (V178W, L181Y, L188R) or (V178W, L188R) or (V178W, R50S) or (V178Y) or (V26A) or (V26C) or (V26E) or (V26F) or (V26G) or (V26G, R47S) or (V26H) or (V26I) or (V26K) or (V26L) or (V26M) or (V26N) or (V26N, R50S, M177Y, V178W, A184Y) or (V26Q) or (V26R) or (V26S) or (V26T) or (V26W) or (V26Y) or (V314A) or (V314E) or (V314F) or (V314H) or (V314P) or (V314Q) or (V314S) or (V314Y) or (V371P) or (V48A) or (V48D) or (V48E) or (V48F) or (V48G) or (V48H) or (V48I) or (V48L) or (V48M) or (V48P) or (V48Q) or (V48R) or (V48S) or (V48T) or (V48W) or (V48Y) or (W325A) or (W325C) or (W325D) or (W325E) or (W325F) or (W325G) or (W325I) or (W325K) or (W325L) or (W325M) or (W325Q) or (W325R) or (W325S) or (W325T) or (W325V) or (W325Y) or (W90D) or (W90Y) or (W90Y, V299G, G570D) or (Y51A) or (Y51C) or (Y51E) or (Y51F) or (Y51G) or (Y51H) or (Y51I) or (Y51L) or (Y51M) or (Y51N) or (Y51P) or (Y51Q) or (Y51S) or (Y51S, F77V) or (Y51T) or (Y51V) or (Y51W).

9. A nucleic acid encoding for a Cytochrome P450 BM3 monooxygenase variant according to any of claims 1 to 8.

10. A host cell for the production of a Cytochrome P450 BM3 monooxygenase variant according to any of claims 1 to 8.

11. Use of a Cytochrome P450 BM3 monooxygenase (BM3) variant for the production of a compound according to formula I wherein R¹ and R² form a six-membered ring as part of a steroid or steroid derivative.

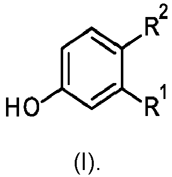

12. Use of a BM3 variant according to claim 11, wherein said compound according to formula I is estrone or estradiol.

13. Use of a BM3 variant for the C19-hydroxylation of a steroid or steroid derivative, said steroid or steroid derivative comprising a 1,4-dien-3-one-A-ring or 4-en-3-one-A-ring.

14. Use of a BM3 variant for the C19-hydroxylation of a steroid according to claim 13, wherein said steroid derivative is androsta-1,4-dien-3,17-dione (ADD) or (17beta)-17-hydroxyandrosta-1,4-dien-3-one (delta1 testosterone).

15. Use of a BM3 variant according to claim 11, 12, 13 or 14, wherein the BM3 variant is a variant according to any of claims 1 to 8.

16. A process for C19-hydroxylation of a steroid derivative comprising (i)
a. culturing a recombinant Cytochrome P450 BM3 monooxygenase (BM3) variant producing microorganism in a culture medium, in the presence of an exogenous or intermediately formed substrate; or
b. incubating a substrate-containing reaction medium with a BM3 variant;
and (ii) isolating from the medium the C19 hydroxylation product formed or a secondary product thereof; optionally further **characterized in that** said BM3 variant is a BM3 variant according to any of claims 1 to 8.

17. The process according to claim 16, wherein the steroid derivative comprises a 1,4-dien-3-one-A-ring or a 4-en-3-one-A-ring and a 19-methyl group.

18. A method for obtaining optimized BM3 variants for the C19 hydroxylation of steroids, said method comprising
(i)
(a) culturing a recombinant microorganism expressing a BM3 variant (test variant) in a culture medium, in the presence of an exogenous or intermediately formed steroid or steroid derivative; or
(b) incubating a steroid or steroid derivative-containing reaction medium with a BM3 variant (test variant); and
(ii) Comparing the obtained product yield and/or selectiviy factor for the C19 hydroxylation product formed by the test variant or a secondary product thereof with the respective value obtained for a parent variant of the test variant capable of catalyzing the C19 hydroxylation of a steroid, and
(iii) Selecting the test variant as optimized for the C19 hydroxylation of steroids, if the test variant has an improved product yield/and or selectivity factor compared to the parent variant.
